(19) Europäisches Patentamt
European Patent Office
Office européen des brevets

(11) Publication number: **0 556 396 B1**

(12) **EUROPEAN PATENT SPECIFICATION**

(45) Date of publication of patent specification: **20.09.95**

(51) Int. Cl.6: **C07D 401/06**, C07D 401/12, C07D 401/14, C07D 403/06, C07D 403/12, C07D 403/14, C07D 417/06, C07D 417/12, C07D 417/14, A01N 43/54, A01N 43/56, A01N 43/58, A01N 43/60, A01N 43/707, A01N 43/78

(21) Application number: **91919177.5**

(22) Date of filing: **08.11.91**

(86) International application number: **PCT/JP91/01538**

(87) International publication number: **WO 92/08715 (29.05.92 92/12)**

The file contains technical information submitted after the application was filed and not included in this specification

(54) **SUBSTITUTED PYRAZOLE DERIVATIVE AND AGROHORTICULTURAL BACTERICIDE.**

(30) Priority: **09.11.90 JP 305340/90**
**24.04.91 JP 94264/91**
**15.10.91 JP 266474/91**

(43) Date of publication of application: **25.08.93 Bulletin 93/34**

(45) Publication of the grant of the patent: **20.09.95 Bulletin 95/38**

(84) Designated Contracting States: **AT BE CH DE DK ES FR GB GR IT LI LU NL SE**

(56) References cited:
**JP-A- 1 125 379**

**PATENT ABSTRACTS OF JAPAN vol. 15, no. 357 (C-0866)10 September 1991**

(73) Proprietor: **NISSAN CHEMICAL INDUSTRIES, LIMITED**
**7-1, Kanda-Nishiki-cho 3-chome**
**Chiyoda-ku**
**Tokyo 101 (JP)**

(72) Inventor: **NAKAJIMA, Yasuyuki, Nissan Chem. Ind. Ltd.**
**Central Research Institute**
**722-1, Tsuboi-cho**
**Funabashi-shi**
**Chiba-ken 274 (JP)**
Inventor: **WATANABE, Junichi, Nissan Chem.Ind. Ltd.**
**Central Research Institute**
**722-1, Tsuboi-cho**
**Funabashi-shi**
**Chiba-ken 274 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**PATENT ABSTRACTS OF JAPAN vol. 9, no. 036 (C-266)15 February 1985**

**PATENT ABSTRACTS OF JAPAN vol. 012, no. 263 (C-514)22 July 1988**

Inventor: **HIROHARA, Yohji, Nissan Chem. Ind. Ltd.**
**Central Research Institute**
**722-1, Tsuboi-cho**
**Funabashi-shi**
**Chiba-ken 274 (JP)**
Inventor: **MITA, Takeshi, Nissan Chem. Ind. Ltd.**
**Central Research Institute**
**722-1, Tsuboi-cho**
**Funabashi-shi**
**Chiba-ken 274 (JP)**
Inventor: **SUZUKI, Hideo, Nissan Chem. Ind., Ltd.**
**Centr. Research Inst.**
**722-1, Tsuboi-cho,**
**Funabashi-shi**
**Chiba-ken 274 (JP)**
Inventor: **HANAUE, Masami, Nissan Chem. Ind., Ltd. Shiraoka**
**Res. St. of Biol. Sc.,**
**1470, Ohaza Siraoka**
**Minamisaitama-gun, Saitama-ken 349-02 (JP)**
Inventor: **OHYA, Hiroshi, Nissan Chem.Ind., Ltd. Shiraoka**
**Res. St. of Bio. Science,**
**1470, Ohaza Siraoka,**
**Minamisaitama-gun,**
**Saitama-ken 349-02 (JP)**
Inventor: **NAKAYAMA, Masato, Nissan Chem. Ind., Ltd.**
**Shiraoka Res. St. of Bio. Sc.**
**1470, Ohaza Siraoka,**
**Minamisaitama-gun, Saitama-ken 349-02 (JP)**
Inventor: **ITO, Tadashi, Nissan Chem. Ind., Ltd.**
**Shiraoka Res. St. of Bio. Sc.,**
**1470 Ohaza Siraoka**
**Minamisaitama-gun, Saitama-ken 349-02 (JP)**
Inventor: **TOYODA, Ryutaro, Nissan Chem. Ind., Ltd.**
**Shiraoka Res. St. of Bio. Sc.**
**1470, Ohaza Siraoka**
**Minamisaitama-gun, Saitama-ken 349-02 (JP)**

(74) Representative: **Hansen, Bernd, Dr.rer.nat. et al**
**Hoffmann, Eitle & Partner**
**Patentanwälte**
**Postfach 81 04 20**
**D-81904 München (DE)**

## Description

The present invention relates to novel pyrazole derivatives and fungicides for agricultural and horticultural use containing the derivative(s) as an active ingredient.

Various fungicides have heretofore been developed, but the potency of them could not be said to be always satisfactory due to appearance of resistant strains and other reasons.

Japanese Patent Application Laid-Open No. 1-125379 mentions that pyrazole derivatives of certain kinds have fungicidal activity.

The compounds as disclosed in the laid-open specification are not still satisfactory with respect to the potency, the residual effect and the phytotoxicity. Therefore, development of fungicides for agricultural and horticultural use, which are further more useful to plant diseases, is desired.

In view of the situation mentioned above, the present inventors repeatedly made various investigations so as to develop compounds having excellent fungicidal activity and, as a result, have found that substituted pyrazole derivatives of the following general formula (I) have excellent fungicidal activity. On the basis of the finding, they have achieved the present invention. Specifically, the present invention relates to substituted pyrazole derivatives of the general formula [1]:

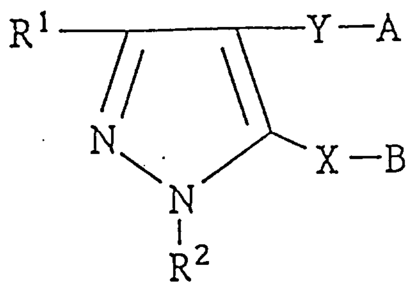

[1]

where $R^1$ represents a hydrogen atom, a halogen atom, an alkyl group, an alkoxy group, an alkylthio group or a haloalkyl group;

$R^2$ represents a hydrogen atom, an alkyl group, a haloalkyl group, a phenylalkyl group, $-COR^6$ or $-SO_2R^7$;

X represents -S-, -SO-, $-SO_2-$, $-N(R^3)-$, -CO- or - $C(R^4)(R^5)-$;

$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, a phenylalkyl group, $-COR^6$ or $-SO_2R^7$;

$R^4$ and $R^5$ independently represent a hydrogen atom, a halogen atom, an alkyl group, a haloalkyl group, an alkenyl group, an alkynyl group or $-OR^8$;

$R^8$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a phenylalkyl group, $-COR^6$ or $-SO_2R^7$;

$R^6$ represents a hydrogen atom, an alkyl group, a haloalkyl group, a phenyl group, a phenylalkyl group, an alkoxy group or

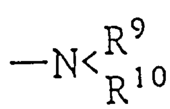

;

$R^7$ represents an alkyl group, a haloalkyl group, a phenyl group or

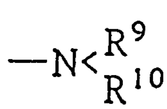

;

$R^9$ and $R^{10}$ independently represent a hydrogen atom, an alkyl group or a phenyl group;
Y represents an oxygen atom, -S-, -SO-, or $-SO_2-$;
A represents a phenyl group
B represents

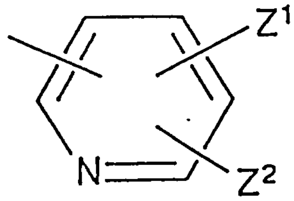
,
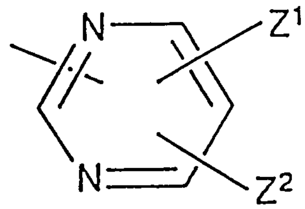
,
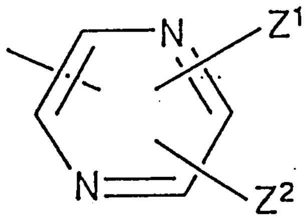
,
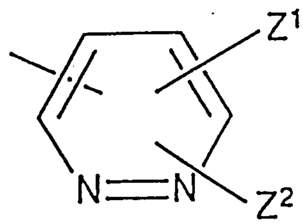
,
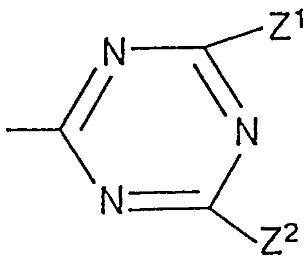
,
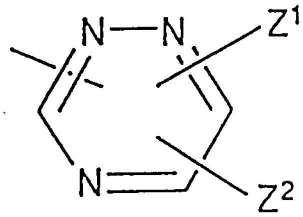

or

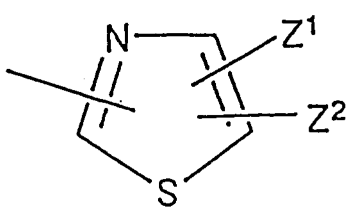
;

$Z^1$ and $Z^2$ independently represent a hydrogen atom, a halogen atom, an alkyl group, an alkoxy group or a haloalkyl group,
wherein the substituted pyrazole derivatives of the general formula [I] include compounds of formula [1a]

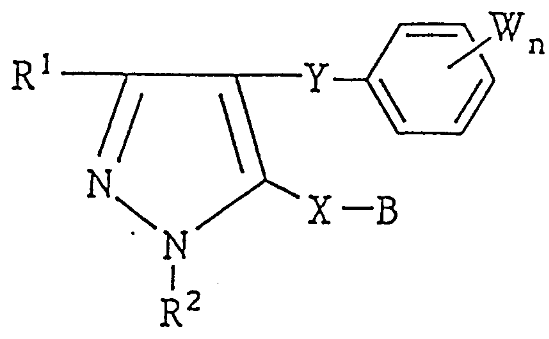

having the following meanings of substituents

Table 1

| Compound No. | $R^1$ | $R^2$ | X | Y | $W_n$ | B |
|---|---|---|---|---|---|---|
| 4 | H | $CH_3$ | S | S | 4-Cl | B1 |
| 5 | $CH_3$ | $CH_3$ | S | S | 4-Cl | B1 |
| 6 | $CF_3$ | $CH_3$ | S | S | 4-Cl | B1 |
| 7 | $CH_3$ | $CH_3$ | S | S | 4-$CH_3$ | B1 |
| 8 | $CF_3$ | $CH_3$ | S | S | 4-$CH_3$ | B1 |
| 9 | $CH_3$ | H | S | S | 4-Cl | B1 |
| 10 | $CH_3$ | H | S | S | 4-$CH_3$ | B1 |
| 11 | $CH_3$ | $CF_3$ | S | S | 4-Cl | B1 |

Table 1 (continued)

| Compound No. | $R^1$ | $R^2$ | X | Y | $W_n$ | B |
|---|---|---|---|---|---|---|
| 12 | $CH_3$ | $CF_3$ | S | S | 4-$CH_3$ | B1 |
| 13 | $CH_3$ | $CH_3$ | S | S | 4-$CF_3$ | B1 |
| 14 | $CH_3$ | $CH_3$ | S | S | 4-Br | B1 |
| 15 | $CH_3$ | $CH_3$ | S | S | 4-$NO_2$ | B1 |
| 16 | $CH_3$ | $CH_3$ | S | S | 4-$OCH_3$ | B1 |
| 17 | $CH_3$ | $CH_3$ | S | S | 4-$C_2H_5$ | B1 |
| 18 | $CH_3$ | $CH_3$ | S | S | 2-Cl | B1 |
| 19 | $CH_3$ | $CH_3$ | S | S | 2-Cl, 4-Cl | B1 |
| 20 | $CH_3$ | $CH_3$ | S | S | 2-Cl, 4-$CH_3$ | B1 |
| 21 | $CH_3$ | $CH_3$ | S | S | 4-Cl | B2 |
| 22 | $CF_3$ | $CH_3$ | S | S | 4-Cl | B2 |

Table 1 (continued)

| Compound No. | $R^1$ | $R^2$ | X | Y | $W_n$ | B |
|---|---|---|---|---|---|---|
| 23 | $CH_3$ | $CH_3$ | S | S | 4-$CH_3$ | B2 |
| 24 | $CF_3$ | $CH_3$ | S | S | 4-$CH_3$ | B2 |
| 25 | $CH_3$ | $CH_3$ | S | S | 2-Cl, 4-Cl | B2 |
| 26 | $CH_3$ | $CH_3$ | S | S | 2-Cl, 4-$CH_3$ | B2 |
| 27 | $CH_3$ | $CH_3$ | S | O | 4-Cl | B2 |
| 28 | $CH_3$ | $CH_3$ | S | O | 4-$CH_3$ | B2 |
| 29 | $CF_3$ | $CH_3$ | S | O | 4-$CH_3$ | B2 |
| 30 | $CH_3$ | $CH_3$ | S | O | 3-Cl, 4-$CH_3$ | B2 |
| 31 | $C_2H_5$ | $CH_3$ | S | S | H | B1 |
| 32 | $C_2H_5$ | $CH_3$ | S | S | 4-Cl | B1 |

Table 1 (continued)

| Compound No. | $R^1$ | $R^2$ | X | Y | $W_n$ | B |
|---|---|---|---|---|---|---|
| 33 | $C_2H_5$ | $CH_3$ | S | S | 4-$CH_3$ | B1 |
| 34 | $C_2H_5$ | $CH_3$ | S | S | 4-$OCH_3$ | B1 |
| 35 | $C_2H_5$ | $CH_3$ | S | S | 3-$CF_3$ | B1 |
| 36 | $C_2H_5$ | $CH_3$ | S | S | 2-Cl, 4-Cl | B1 |
| 37 | $C_2H_5$ | $CH_3$ | S | S | 3-Cl, 4-Cl | B1 |
| 38 | $C_2H_5$ | $CH_3$ | S | S | 2-Cl, 4-$CH_3$ | B1 |
| 39 | i-$C_3H_7$ | $CH_3$ | S | S | 4-Cl | B1 |
| 40 | i-$C_3H_7$ | $CH_3$ | S | S | 4-$CH_3$ | B1 |
| 41 | i-$C_3H_7$ | $CH_3$ | S | S | 4-$OCH_3$ | B1 |
| 42 | t-$C_4H_9$ | $CH_3$ | S | S | 4-Cl | B1 |

Table 1 (continued)

| Compound No. | $R^1$ | $R^2$ | X | Y | $W_n$ | B |
|---|---|---|---|---|---|---|
| 43 | t-$C_4H_9$ | $CH_3$ | S | S | 4-$CH_3$ | B1 |
| 44 | t-$C_4H_9$ | $CH_3$ | S | S | 4-$OCH_3$ | B1 |
| 45 | $CH_3$ | $C_2H_5$ | S | S | 4-Cl | B1 |
| 46 | $CH_3$ | $C_3H_7$ | S | S | 4-Cl | B1 |
| 47 | $CH_3$ | $CH_3$ | NH | S | H | B1 |
| 48 | $CF_3$ | $CH_3$ | NH | S | H | B1 |
| 49 | $CH_3$ | $CH_3$ | NH | S | 4-Cl | B1 |
| 50 | $CF_3$ | $CH_3$ | NH | S | 4-Cl | B1 |
| 51 | H | $CH_3$ | NH | S | 4-Cl | B1 |
| 52 | $CH_3$ | $CH_3$ | NH | S | 4-$CH_3$ | B1 |

Table 1 (continued)

| Compound No. | $R^1$ | $R^2$ | X | Y | $W_n$ | B |
|---|---|---|---|---|---|---|
| 53 | $CF_3$ | $CH_3$ | NH | S | 4-$CH_3$ | B1 |
| 54 | H | $CH_3$ | NH | S | 4-$CH_3$ | B1 |
| 55 | $CH_3$ | $CH_3$ | NH | S | 4-F | B1 |
| 56 | $CH_3$ | $CH_3$ | NH | S | 4-Br | B1 |
| 57 | $CH_3$ | $CH_3$ | NH | S | 4-I | B1 |
| 58 | $CH_3$ | $CH_3$ | NH | S | 2-F | B1 |
| 59 | $CH_3$ | $CH_3$ | NH | S | 2-Cl | B1 |
| 60 | $CH_3$ | $CH_3$ | NH | S | 2-Br | B1 |
| 61 | $CH_3$ | $CH_3$ | NH | S | 2-I | B1 |
| 62 | $CH_3$ | $CH_3$ | NH | S | 3-F | B1 |

Table 1 (continued)

| Compound No. | $R^1$ | $R^2$ | X | Y | $W_n$ | B |
|---|---|---|---|---|---|---|
| 63 | $CH_3$ | $CH_3$ | NH | S | 3-Cl | B1 |
| 64 | $CH_3$ | $CH_3$ | NH | S | 3-Br | B1 |
| 65 | $CH_3$ | $CH_3$ | NH | S | 3-I | B1 |
| 66 | $CH_3$ | $CH_3$ | NH | S | $2-OCH_3$ | B1 |
| 67 | $CH_3$ | $CH_3$ | NH | S | $3-OCH_3$ | B1 |
| 68 | $CH_3$ | $CH_3$ | NH | S | $4-OCH_3$ | B1 |
| 69 | $CH_3$ | $CH_3$ | NH | S | $2-CF_3$ | B1 |
| 70 | $CH_3$ | $CH_3$ | NH | S | $3-CF_3$ | B1 |
| 71 | $CH_3$ | $CH_3$ | NH | S | $4-CF_3$ | B1 |
| 72 | $CH_3$ | $CH_3$ | NH | S | $4-C_2H_5$ | B1 |

Table 1 (continued)

| Compound No. | $R^1$ | $R^2$ | X | Y | $W_n$ | B |
|---|---|---|---|---|---|---|
| 73 | $CH_3$ | $CH_3$ | NH | S | $4-C_3H_7$ | B1 |
| 74 | $CH_3$ | $CH_3$ | NH | S | $4-C_4H_9$ | B1 |
| 75 | $CH_3$ | $CH_3$ | NH | S | $4-i-C_3H_7$ | B1 |
| 76 | $CH_3$ | $CH_3$ | NH | S | $4-t-C_4H_9$ | B1 |
| 77 | $CH_3$ | $CH_3$ | NH | S | $2-OCF_3$ | B1 |
| 78 | $CH_3$ | $CH_3$ | NH | S | $3-OCF_3$ | B1 |
| 79 | $CH_3$ | $CH_3$ | NH | S | $4-OCF_3$ | B1 |
| 80 | $CH_3$ | $CH_3$ | NH | S | $3-NO_2$ | B1 |
| 81 | $CH_3$ | $CH_3$ | NH | S | $4-NO_2$ | B1 |
| 82 | $CH_3$ | $CH_3$ | NH | S | $3-NH_2$ | B1 |

Table 1 (continued)

| Compound No. | $R^1$ | $R^2$ | X | Y | $W_n$ | B |
|---|---|---|---|---|---|---|
| 83 | $CH_3$ | $CH_3$ | NH | S | 4-$NH_2$ | B1 |
| 84 | $CH_3$ | $CH_3$ | NH | S | 4-$NHCOCH_3$ | B1 |
| 85 | $CH_3$ | $CH_3$ | NH | S | 4-$NHSO_2CH_3$ | B1 |
| 86 | $CH_3$ | $CH_3$ | NH | S | 4-$NHCOCF_3$ | B1 |
| 87 | $CH_3$ | $CH_3$ | NH | S | 4-$NHSO_2CF_3$ | B1 |
| 88 | $CH_3$ | $CH_3$ | NH | S | 4-Ph | B1 |
| 89 | $CH_3$ | $CH_3$ | NH | S | 4-OPh | B1 |
| 90 | $CH_3$ | $CH_3$ | NH | S | 4-$OCF_2CF_2H$ | B1 |
| 91 | $CH_3$ | $CH_3$ | NH | S | 4-$COCH_3$ | B1 |
| 92 | $CH_3$ | $CH_3$ | NH | S | 4-NHCOPh | B1 |

Table 1 (continued)

| Compound No. | $R^1$ | $R^2$ | X | Y | $W_n$ | B |
|---|---|---|---|---|---|---|
| 93 | $CH_3$ | $CH_3$ | NH | S | 4-$NHCOOCH_3$ | B 1 |
| 94 | $CH_3$ | $CH_3$ | NH | S | 4-$NHCON(CH_3)_2$ | B 1 |
| 95 | $CH_3$ | $CH_3$ | NH | S | 2-Cl, 3-Cl | B 1 |
| 96 | $CH_3$ | $CH_3$ | NH | S | 2-Cl, 4-Cl | B 1 |
| 97 | $CH_3$ | $CH_3$ | NH | S | 2-Cl, 5-Cl | B 1 |
| 98 | $CH_3$ | $CH_3$ | NH | S | 2-Cl, 6-Cl | B 1 |
| 99 | $CH_3$ | $CH_3$ | NH | S | 3-Cl, 4-Cl | B 1 |
| 100 | $CH_3$ | $CH_3$ | NH | S | 3-Cl, 5-Cl | B 1 |
| 101 | $CH_3$ | $CH_3$ | NH | S | 2-$CH_3$, 3-$CH_3$ | B 1 |
| 102 | $CH_3$ | $CH_3$ | NH | S | 2-$CH_3$, 4-$CH_3$ | B 1 |

Table 1 (continued)

| Compound No. | $R^1$ | $R^2$ | X | Y | $W_n$ | B |
|---|---|---|---|---|---|---|
| 103 | $CH_3$ | $CH_3$ | NH | S | 2-$CH_3$, 5-$CH_3$ | B1 |
| 104 | $CH_3$ | $CH_3$ | NH | S | 2-$CH_3$, 6-$CH_3$ | B1 |
| 105 | $CH_3$ | $CH_3$ | NH | S | 3-$CH_3$, 4-$CH_3$ | B1 |
| 106 | $CH_3$ | $CH_3$ | NH | S | 3-$CH_3$, 5-$CH_3$ | B1 |
| 107 | $CH_3$ | $CH_3$ | NH | S | 2-Cl, 3-$CH_3$ | B1 |
| 108 | $CH_3$ | $CH_3$ | NH | S | 2-Cl, 4-$CH_3$ | B1 |
| 109 | $CH_3$ | $CH_3$ | NH | S | 2-Cl, 5-$CH_3$ | B1 |
| 110 | $CH_3$ | $CH_3$ | NH | S | 3-Cl, 4-$CH_3$ | B1 |
| 111 | $CH_3$ | $CH_3$ | NH | S | 3-Cl, 5-$CH_3$ | B1 |
| 112 | $CH_3$ | $CH_3$ | NH | S | 2-$CH_3$, 3-Cl | B1 |

Table 1 (continued)

| Compound No. | $R^1$ | $R^2$ | X | Y | $W_n$ | B |
|---|---|---|---|---|---|---|
| 113 | $CH_3$ | $CH_3$ | NH | S | 2-$CH_3$, 4-Cl | B1 |
| 114 | $CH_3$ | $CH_3$ | NH | S | 2-$CH_3$, 5-Cl | B1 |
| 115 | $CH_3$ | $CH_3$ | NH | S | 2-F, 3-F | B1 |
| 116 | $CH_3$ | $CH_3$ | NH | S | 2-F, 4-F | B1 |
| 117 | $CH_3$ | $CH_3$ | NH | S | 2-F, 5-F | B1 |
| 118 | $CH_3$ | $CH_3$ | NH | S | 2-F, 6-F | B1 |
| 119 | $CH_3$ | $CH_3$ | NH | S | 2-F, 3-Cl | B1 |
| 120 | $CH_3$ | $CH_3$ | NH | S | 2-F, 4-Cl | B1 |
| 121 | $CH_3$ | $CH_3$ | NH | S | 2-F, 5-Cl | B1 |
| 122 | $CH_3$ | $CH_3$ | NH | S | 2-F, 6-Cl | B1 |

Table 1 (continued)

| Compound No. | $R^1$ | $R^2$ | X | Y | $W_n$ | B |
|---|---|---|---|---|---|---|
| 123 | $CH_3$ | $CH_3$ | NH | S | 2-F, 3-$CH_3$ | B1 |
| 124 | $CH_3$ | $CH_3$ | NH | S | 2-F, 4-$CH_3$ | B1 |
| 125 | $CH_3$ | $CH_3$ | NH | S | 2-F, 3-Br | B1 |
| 126 | $CH_3$ | $CH_3$ | NH | S | 2-F, 4-Br | B1 |
| 127 | $CH_3$ | $CH_3$ | NH | S | 3-F, 4-Cl | B1 |
| 128 | $CH_3$ | $CH_3$ | NH | S | 3-F, 5-Cl | B1 |
| 129 | $CH_3$ | $CH_3$ | NH | S | 3-F, 4-$CH_3$ | B1 |
| 130 | $CH_3$ | $CH_3$ | NH | S | 3-F, 5-$CH_3$ | B1 |
| 131 | $CH_3$ | $CH_3$ | NH | S | 2-Br, 4-Cl | B1 |
| 132 | $CH_3$ | $CH_3$ | NH | S | 2-Br, 4-Br | B1 |

Table 1 (continued)

| Compound No. | $R^1$ | $R^2$ | X | Y | $W_n$ | B |
|---|---|---|---|---|---|---|
| 133 | $CH_3$ | $CH_3$ | NH | S | 2-Br, 4-$CH_3$ | B 1 |
| 134 | $CH_3$ | $CH_3$ | NH | S | 3-Br, 4-Cl | B 1 |
| 135 | $CH_3$ | $CH_3$ | NH | S | 3-Br, 4-Br | B 1 |
| 136 | $CH_3$ | $CH_3$ | NH | S | 3-Br, 4-$CH_3$ | B 1 |
| 137 | $CH_3$ | $CH_3$ | NH | S | 2-Cl, 4-Br | B 1 |
| 138 | $CH_3$ | $CH_3$ | NH | S | 3-Cl, 4-Br | B 1 |
| 139 | $CH_3$ | $CH_3$ | NH | S | 2-Cl, 4-I | B 1 |
| 140 | $CH_3$ | $CH_3$ | NH | S | 3-Cl, 4-I | B 1 |
| 141 | $CH_3$ | $CH_3$ | NH | S | 3-F, 4-Br | B 1 |
| 142 | $CH_3$ | $CH_3$ | NH | S | 3, 4-$OCH_2O$- | B 1 |

Table 1 (continued)

| Compound No. | $R^1$ | $R^2$ | X | Y | $W_n$ | B |
|---|---|---|---|---|---|---|
| 143 | $CH_3$ | $CH_3$ | NH | S | 2-$CF_3$, 3-Cl | B 1 |
| 144 | $CH_3$ | $CH_3$ | NH | S | 2-$CF_3$, 4-Cl | B 1 |
| 145 | $CH_3$ | $CH_3$ | NH | S | 2-$CF_3$, 3-Br | B 1 |
| 146 | $CH_3$ | $CH_3$ | NH | S | 2-$CF_3$, 4-Br | B 1 |
| 147 | $CH_3$ | $CH_3$ | NH | S | 2-F, 4-$NO_2$ | B 1 |
| 148 | $CH_3$ | $CH_3$ | NH | S | 3-F, 4-$NO_2$ | B 1 |
| 149 | $CH_3$ | $CH_3$ | NH | S | 2-Cl, 4-$NO_2$ | B 1 |
| 150 | $CH_3$ | $CH_3$ | NH | S | 3-Cl, 4-$NO_2$ | B 1 |
| 151 | $CH_3$ | $CH_3$ | NH | S | 2-F, 4-$OCH_3$ | B 1 |
| 152 | $CH_3$ | $CH_3$ | NH | S | 3-F, 4-$OCH_3$ | B 1 |

Table 1 (continued)

| Compound No. | $R^1$ | $R^2$ | X | Y | $W_n$ | B |
|---|---|---|---|---|---|---|
| 153 | $CH_3$ | $CH_3$ | NH | S | 2-Cl, 4-$OCH_3$ | B1 |
| 154 | $CH_3$ | $CH_3$ | NH | S | 3-Cl, 4-$OCH_3$ | B1 |
| 155 | $CH_3$ | $CH_3$ | NH | S | 2-$OCH_3$, 4-$OCH_3$ | B1 |
| 156 | $CH_3$ | $CH_3$ | NH | S | 3-$OCH_3$, 4-$OCH_3$ | B1 |
| 157 | $CH_3$ | $CH_3$ | NH | S | 2-Cl, 3-Cl, 4-Cl | B1 |
| 158 | $CH_3$ | $CH_3$ | NH | S | 2-Cl, 4-Cl, 5-Cl | B1 |
| 159 | $CH_3$ | $CH_3$ | NH | S | 2-Cl, 4-Cl, 6-Cl | B1 |
| 160 | $CH_3$ | $CH_3$ | NH | S | 3-Cl, 4-Cl, 5-Cl | B1 |
| 161 | $CH_3$ | $CH_3$ | NH | S | 2-Cl, 3-Cl, 4-$CH_3$ | B1 |
| 162 | $CH_3$ | $CH_3$ | NH | S | 2-Cl, 3-Cl, 4-Br | B1 |

Table 1 (continued)

| Compound No. | $R^1$ | $R^2$ | X | Y | $W_n$ | B |
|---|---|---|---|---|---|---|
| 163 | $CH_3$ | $CH_3$ | NH | S | 2-Cl, 3-Cl, 4-I | B1 |
| 164 | $CH_3$ | $CH_3$ | NH | S | 2-Cl, 4-Br, 6-Cl | B1 |
| 165 | $CH_3$ | $CH_3$ | NH | S | 2-Cl, 4-$CH_3$, 6-Cl | B1 |
| 166 | $CH_3$ | $CH_3$ | NH | S | 2-Br, 4-Cl, 6-Cl | B1 |
| 167 | $CH_3$ | $CH_3$ | NCHO | S | 4-Cl | B1 |
| 168 | $CH_3$ | $CH_3$ | $NCOCH_3$ | S | 4-Cl | B1 |
| 169 | $CH_3$ | $CH_3$ | $NCOOCH_3$ | S | 4-Cl | B1 |
| 170 | $CH_3$ | $CH_3$ | $NCON(CH_3)_2$ | S | 4-Cl | B1 |
| 171 | $CH_3$ | $CH_3$ | NCONHPh | S | 4-Cl | B1 |
| 172 | $CH_3$ | $CH_3$ | $NCH_3$ | S | 4-Cl | B1 |

Table 1 (continued)

| Compound No. | $R^1$ | $R^2$ | X | Y | $W_n$ | B |
|---|---|---|---|---|---|---|
| 173 | $CH_3$ | $CH_3$ | $NC_2H_5$ | S | 4-Cl | B1 |
| 174 | $CH_3$ | $CH_3$ | $NC_3H_7$ | S | 4-Cl | B1 |
| 175 | $CH_3$ | $CH_3$ | $NCH_2OCH_3$ | S | 4-Cl | B1 |
| 176 | $CH_3$ | $CH_3$ | $NCH_2CH_2OCH_3$ | S | 4-Cl | B1 |
| 177 | $CH_3$ | $CH_3$ | $NCH_2CH=CH_2$ | S | 4-Cl | B1 |
| 178 | $CH_3$ | $CH_3$ | $NCH_2C\equiv CH$ | S | 4-Cl | B1 |
| 179 | $CH_3$ | $CH_3$ | $NCH_2COCH_3$ | S | 4-Cl | B1 |
| 180 | $CH_3$ | $CH_3$ | $NCH_2COOCH_3$ | S | 4-Cl | B1 |
| 181 | $CH_3$ | $CH_3$ | $NCH_2COOC_2H_5$ | S | 4-Cl | B1 |
| 182 | $CH_3$ | $CH_3$ | $NCH_2CN$ | S | 4-Cl | B1 |

Table 1 (continued)

| Compound No. | $R^1$ | $R^2$ | X | Y | $W_n$ | B |
|---|---|---|---|---|---|---|
| 183 | $CH_3$ | $CH_3$ | $NCH_2Ph$ | S | 4-Cl | B1 |
| 184 | $CH_3$ | $CH_3$ | $NCH_2C_6H_4$-4-Cl | S | 4-Cl | B1 |
| 185 | $CH_3$ | $CH_3$ | $NCH_2C_6H_4$-4-$CH_3$ | S | 4-Cl | B1 |
| 186 | $CH_3$ | $CH_3$ | $NCH_2CH_2Ph$ | S | 4-Cl | B1 |
| 187 | $CH_3$ | $CH_3$ | $NSO_2CH_3$ | S | 4-Cl | B1 |
| 188 | $CH_3$ | $CH_3$ | $NSO_2N(CH_3)_2$ | S | 4-Cl | B1 |
| 189 | $CH_3$ | $CH_3$ | NCHO | S | 4-Cl | B2 |
| 190 | $CH_3$ | $CH_3$ | $NCH_3$ | S | 4-Cl | B2 |
| 191 | $CH_3$ | H | NH | S | 4-Cl | B1 |
| 192 | $CH_3$ | H | NH | S | 2-Cl, 4-Cl | B1 |

Table 1 (continued)

| Compound No. | $R^1$ | $R^2$ | X | Y | $W_n$ | B |
|---|---|---|---|---|---|---|
| 193 | $CH_3$ | H | NH | S | 3-Cl, 4-$CH_3$ | B1 |
| 194 | $CH_3$ | $C_2H_5$ | NH | S | 4-Cl | B1 |
| 195 | $CH_3$ | $C_2H_5$ | NH | S | 4-Br | B1 |
| 196 | $CH_3$ | $C_2H_5$ | NH | S | 2-Cl, 4-Cl | B1 |
| 197 | $CH_3$ | $C_2H_5$ | NH | S | 2-Cl, 4-$CH_3$ | B1 |
| 198 | $CH_3$ | i-$C_3H_7$ | NH | S | 4-Cl | B1 |
| 199 | $CH_3$ | i-$C_3H_7$ | NH | S | 4-Br | B1 |
| 200 | $CH_3$ | i-$C_3H_7$ | NH | S | 2-Cl, 4-Cl | B1 |
| 201 | $CH_3$ | i-$C_3H_7$ | NH | S | 2-Cl, 4-$CH_3$ | B1 |
| 202 | $CH_3$ | t-$C_4H_9$ | NH | S | 4-Cl | B1 |

Table 1 (continued)

| Compound No. | $R^1$ | $R^2$ | X | Y | $W_n$ | B |
|---|---|---|---|---|---|---|
| 203 | $CH_3$ | $t-C_4H_9$ | NH | S | 2-Cl, 4-Cl | B1 |
| 204 | $CH_3$ | $t-C_4H_9$ | NH | S | 3-Cl, 4-$CH_3$ | B1 |
| 205 | $CH_3$ | $CF_3$ | NH | S | 4-Cl | B1 |
| 206 | $CH_3$ | $CF_3$ | NH | S | 4-Br | B1 |
| 207 | $CH_3$ | $CF_3$ | NH | S | 2-Cl, 4-Cl | B1 |
| 208 | $CH_3$ | $CF_3$ | NH | S | 3-Cl, 4-$CH_3$ | B1 |
| 209 | $CH_3$ | H | NH | S | 4-Cl | B2 |
| 210 | $CH_3$ | H | NH | S | 2-Cl, 4-Cl | B2 |
| 211 | $CH_3$ | $C_2H_5$ | NH | S | 4-Cl | B2 |
| 212 | $CH_3$ | $i-C_3H_7$ | NH | S | 4-Cl | B2 |

Table 1 (continued)

| Compound No. | $R^1$ | $R^2$ | X | Y | $W_n$ | B |
|---|---|---|---|---|---|---|
| 213 | $CH_3$ | $t-C_4H_9$ | NH | S | 4-Cl | B2 |
| 214 | $CH_3$ | $CF_3$ | NH | S | 2-Cl, 4-Cl | B2 |
| 215 | $CH_3$ | $CH_2Ph$ | NH | S | 4-Cl | B1 |
| 216 | $CH_3$ | $CH_2Ph$ | NH | S | 2-Cl, 4-Cl | B1 |
| 217 | $CH_3$ | $CH_2Ph$ | NCHO | S | 2-Cl, 4-Cl | B1 |
| 218 | $CH_3$ | CHO | NH | S | 4-Cl | B1 |
| 219 | $CH_3$ | CHO | NCHO | S | 4-Cl | B1 |
| 220 | $CH_3$ | CHO | NH | S | 2-Cl, 4-Cl | B1 |
| 221 | $CH_3$ | CHO | NCHO | S | 2-Cl, 4-Cl | B1 |
| 222 | $CH_3$ | $COCH_3$ | NH | S | 4-Cl | B1 |

Table 1 (continued)

| Compound No. | $R^1$ | $R^2$ | X | Y | $W_n$ | B |
|---|---|---|---|---|---|---|
| 223 | $CH_3$ | $COCH_3$ | NH | S | 4-Br | B1 |
| 224 | $CH_3$ | $COCH_3$ | NH | S | 2-Cl, 4-Cl | B1 |
| 225 | $CH_3$ | $COCH_3$ | NCHO | S | 2-Cl, 4-Cl | B1 |
| 226 | $CH_3$ | $SO_2CH_3$ | NH | S | 4-Cl | B1 |
| 227 | $CH_3$ | $SO_2CH_3$ | NH | S | 2-Cl, 4-Cl | B1 |
| 228 | $CH_3$ | $SO_2CH_3$ | NH | S | 3-Cl, 4-$CH_3$ | B1 |
| 229 | $CH_3$ | $CONHCH_3$ | NH | S | 4-Cl | B1 |
| 230 | $CH_3$ | $CONHCH_3$ | NH | S | 4-Br | B1 |
| 231 | $CH_3$ | $CONHCH_3$ | NH | S | 2-Cl, 4-Cl | B1 |
| 232 | $CH_3$ | $CONHCH_3$ | NH | S | 3-Cl, 4-$CH_3$ | B1 |

Table 1 (continued)

| Compound No. | $R^1$ | $R^2$ | X | Y | $W_n$ | B |
|---|---|---|---|---|---|---|
| 233 | $CH_3$ | $CH_2Ph$ | NH | S | 4-Cl | B2 |
| 234 | $CH_3$ | $CH_2Ph$ | NH | S | 2-Cl, 4-Cl | B2 |
| 235 | $CH_3$ | $COCH_3$ | NH | S | 4-Cl | B2 |
| 236 | $CH_3$ | $CONHCH_3$ | NH | S | 4-Cl | B2 |
| 237 | $CH_3$ | $SO_2CH_3$ | NH | S | 4-Cl | B2 |
| 238 | $CH_3$ | $SO_2CH_3$ | NH | S | 2-Cl, 4-Cl | B2 |
| 239 | H | $CH_3$ | NH | S | 4-Cl | B1 |
| 240 | H | $CH_3$ | NH | S | 2-Cl, 4-Cl | B1 |
| 241 | H | $CH_3$ | NH | S | 3-Cl, 4-$CH_3$ | B1 |
| 242 | $CF_3$ | $CH_3$ | NH | S | 4-Cl | B1 |

Table 1 (continued)

| Compound No. | $R^1$ | $R^2$ | X | Y | $W_n$ | B |
|---|---|---|---|---|---|---|
| 243 | $CF_3$ | $CH_3$ | NH | S | 4-Br | B1 |
| 244 | $CF_3$ | $CH_3$ | NH | S | 2-Cl, 4-Cl | B1 |
| 245 | $CF_3$ | $CH_3$ | NH | S | 2-Cl, 4-$CH_3$ | B1 |
| 246 | $C_2H_5$ | $CH_3$ | NH | S | 4-Cl | B1 |
| 247 | $C_2H_5$ | $CH_3$ | NH | S | 4-Br | B1 |
| 248 | $C_2H_5$ | $CH_3$ | NH | S | 2-Cl, 4-Cl | B1 |
| 249 | $C_2H_5$ | $CH_3$ | NH | S | 2-Cl, 4-$CH_3$ | B1 |
| 250 | i-$C_3H_7$ | $CH_3$ | NH | S | 4-Cl | B1 |
| 251 | i-$C_3H_7$ | $CH_3$ | NH | S | 2-Cl, 4-Cl | B1 |
| 252 | i-$C_3H_7$ | $CH_3$ | NH | S | 3-Cl, 4-$CH_3$ | B1 |

Table 1 (continued)

| Compound No. | $R^1$ | $R^2$ | X | Y | $W_n$ | B |
|---|---|---|---|---|---|---|
| 253 | t-$C_4H_9$ | $CH_3$ | NH | S | 4-Cl | B1 |
| 254 | t-$C_4H_9$ | $CH_3$ | NH | S | 4-Br | B1 |
| 255 | t-$C_4H_9$ | $CH_3$ | NH | S | 2-Cl, 4-Cl | B1 |
| 256 | t-$C_4H_9$ | $CH_3$ | NH | S | 3-Cl, 4-$CH_3$ | B1 |
| 257 | H | $CH_3$ | NH | S | 4-Cl | B2 |
| 258 | H | $CH_3$ | NH | S | 2-Cl, 4-Cl | B2 |
| 259 | $C_2H_5$ | $CH_3$ | NH | S | 4-Cl | B2 |
| 260 | i-$C_3H_7$ | $CH_3$ | NH | S | 4-Cl | B2 |
| 261 | $CF_3$ | $CH_3$ | NH | S | 4-Cl | B2 |
| 262 | $CF_3$ | $CH_3$ | NH | S | 2-Cl, 4-Cl | B2 |

Table 1 (continued)

| Compound No. | $R^1$ | $R^2$ | X | Y | $W_n$ | B |
|---|---|---|---|---|---|---|
| 263 | Cl | $CH_3$ | NH | S | 4-Cl | B1 |
| 264 | Cl | $CH_3$ | NH | S | 2-Cl, 4-Cl | B1 |
| 265 | Cl | $CH_3$ | NH | S | 3-Cl, 4-$CH_3$ | B1 |
| 266 | Cl | $CH_3$ | NCHO | S | 4-Cl | B1 |
| 267 | Cl | $CH_3$ | NH | S | 4-Br | B1 |
| 268 | Cl | $CH_3$ | NCHO | S | 2-Cl, 4-Cl | B1 |
| 269 | Cl | $CH_3$ | NCHO | S | 2-Cl, 4-$CH_3$ | B1 |
| 270 | $CH_3O$ | $CH_3$ | NH | S | 4-Cl | B1 |
| 271 | $CH_3O$ | $CH_3$ | NH | S | 4-Br | B1 |
| 272 | $CH_3O$ | $CH_3$ | NH | S | 2-Cl, 4-Cl | B1 |

Table 1 (continued)

| Compound No. | $R^1$ | $R^2$ | X | Y | $W_n$ | B |
|---|---|---|---|---|---|---|
| 273 | $CH_3O$ | $CH_3$ | NH | S | 2-Cl, 4-$CH_3$ | B1 |
| 274 | $CH_3O$ | $CH_3$ | NCHO | S | 4-Cl | B1 |
| 275 | $CH_3O$ | $CH_3$ | NCHO | S | 2-Cl, 4-Cl | B1 |
| 276 | $CH_3O$ | $CH_3$ | NCHO | S | 3-Cl, 4-$CH_3$ | B1 |
| 277 | $CH_3S$ | $CH_3$ | NH | S | 4-Cl | B1 |
| 278 | $CH_3S$ | $CH_3$ | NH | S | 4-Br | B1 |
| 279 | $CH_3S$ | $CH_3$ | NH | S | 2-Cl, 4-Cl | B1 |
| 280 | $CH_3S$ | $CH_3$ | NH | S | 3-Cl, 4-$CH_3$ | B1 |
| 281 | $CH_3S$ | $CH_3$ | NCHO | S | 4-Cl | B1 |
| 282 | $CH_3S$ | $CH_3$ | NCHO | S | 2-Cl, 4-Cl | B1 |

Table 1 (continued)

| Compound No. | $R^1$ | $R^2$ | X | Y | $W_n$ | B |
|---|---|---|---|---|---|---|
| 283 | Cl | $CH_3$ | NH | S | 4-Cl | B2 |
| 284 | Cl | $CH_3$ | NH | S | 2-Cl, 4-Cl | B2 |
| 285 | $CH_3O$ | $CH_3$ | NH | S | 2-Cl, 4-Cl | B2 |
| 286 | $CH_3S$ | $CH_3$ | NH | S | 2-Cl, 4-Cl | B2 |
| 287 | $CH_3$ | $CH_3$ | SO | S | 4-Cl | B1 |
| 288 | $CH_3$ | $CH_3$ | $SO_2$ | S | 4-Cl | B1 |
| 289 | $CH_3$ | $CH_3$ | NH | SO | 4-Cl | B1 |
| 290 | $CH_3$ | $CH_3$ | NH | $SO_2$ | 4-Cl | B1 |
| 291 | $CH_3$ | $CH_3$ | NH | SO | 2-Cl, 4-Cl | B1 |
| 292 | $CH_3$ | $CH_3$ | NH | $SO_2$ | 2-Cl, 4-Cl | B1 |

Table 1 (continued)

| Compound No. | $R^1$ | $R^2$ | X | Y | $W_n$ | B |
|---|---|---|---|---|---|---|
| 293 | $CH_3$ | $CH_3$ | NH | SO | 3-Cl, 4-$CH_3$ | B1 |
| 294 | $CH_3$ | $CH_3$ | NH | $SO_2$ | 3-Cl, 4-$CH_3$ | B1 |
| 295 | $CH_3$ | $CH_3$ | S | O | 4-Cl | B1 |
| 296 | $CH_3$ | $CH_3$ | SO | O | 4-Cl | B1 |
| 297 | $CH_3$ | $CH_3$ | $SO_2$ | O | 4-Cl | B1 |
| 298 | $CH_3$ | $CH_3$ | S | O | 2-Cl, 4-Cl | B1 |
| 299 | $CH_3$ | $CH_3$ | SO | O | 2-Cl, 4-Cl | B1 |
| 300 | $CH_3$ | $CH_3$ | $SO_2$ | O | 2-Cl, 4-Cl | B1 |
| 301 | $CH_3$ | $CH_3$ | NH | O | 4-Cl | B1 |
| 302 | $CH_3$ | $CH_3$ | NH | O | 4-$CH_3$ | B1 |

Table 1 (continued)

| Compound No. | $R^1$ | $R^2$ | X | Y | $W_n$ | B |
|---|---|---|---|---|---|---|
| 303 | $CH_3$ | $CH_3$ | NH | O | 2-Cl, 4-Cl | B1 |
| 304 | $CH_3$ | $CH_3$ | NH | O | 2-Cl, 4-$CH_3$ | B1 |
| 305 | $CH_3$ | $CH_3$ | NH | O | 3-Cl, 4-$CH_3$ | B1 |
| 306 | $CH_3$ | $CH_3$ | NH | O | 2-F, 4-Cl | B1 |
| 307 | $CH_3$ | $CH_3$ | NCHO | O | 4-Cl | B1 |
| 308 | $CH_3$ | $CH_3$ | $NCH_3$ | O | 4-Cl | B1 |
| 309 | $CH_3$ | $CH_3$ | NH | O | 4-Cl | B2 |
| 310 | $CH_3$ | $CH_3$ | NH | O | 2-Cl, 4-Cl | B2 |
| 311 | $CH_3$ | $CH_3$ | NH | S | 4-F | B2 |
| 312 | $CH_3$ | $CH_3$ | NH | S | 4-Cl | B2 |

Table 1 (continued)

| Compound No. | $R^1$ | $R^2$ | X | Y | $W_n$ | B |
|---|---|---|---|---|---|---|
| 313 | $CH_3$ | $CH_3$ | NH | S | 4-$CH_3$ | B2 |
| 314 | $CH_3$ | $CH_3$ | NH | S | 2-Cl, 4-Cl | B2 |
| 315 | $CH_3$ | $CH_3$ | NH | S | 3-Cl, 4-Cl | B2 |
| 316 | $CH_3$ | $CH_3$ | NH | S | 2-Cl, 4-$CH_3$ | B2 |
| 317 | $CH_3$ | $CH_3$ | NH | S | 3-Cl, 4-$CH_3$ | B2 |
| 318 | $CH_3$ | $CH_3$ | NH | S | 2-F, 4-F | B2 |
| 319 | $CH_3$ | $CH_3$ | NH | S | 2-F, 4-Cl | B2 |
| 320 | $CH_3$ | $CH_3$ | NH | S | 2-F, 4-Br | B2 |
| 321 | $CH_3$ | $CH_3$ | NH | S | 2-F, 4-$CH_3$ | B2 |
| 322 | $CH_3$ | $CH_3$ | NH | S | 3-F, 4-$CH_3$ | B2 |

Table 1 (continued)

| Compound No. | $R^1$ | $R^2$ | X | Y | $W_n$ | B |
|---|---|---|---|---|---|---|
| 323 | $CH_3$ | $CH_3$ | NCHO | S | 4-Cl | B2 |
| 324 | $CH_3$ | $CH_3$ | NCHO | S | 2-Cl, 4-Cl | B2 |
| 325 | $CH_3$ | $CH_3$ | NCHO | S | 3-Cl, 4-$CH_3$ | B2 |
| 326 | $CH_3$ | $CH_3$ | NH | S | 4-Cl | B3 |
| 327 | $CH_3$ | $CH_3$ | NH | S | 4-Br | B3 |
| 328 | $CH_3$ | $CH_3$ | NH | S | 2-Cl, 4-Cl | B3 |
| 329 | $CH_3$ | $CH_3$ | NH | S | 2-Cl, 4-$CH_3$ | B3 |
| 330 | $CH_3$ | $CH_3$ | NH | S | 3-Cl, 4-$CH_3$ | B3 |
| 331 | $CH_3$ | $CH_3$ | NH | S | 4-Cl | B4 |
| 332 | $CH_3$ | $CH_3$ | NH | S | 2-Cl, 4-Cl | B4 |

Table 1 (continued)

| Compound No. | $R^1$ | $R^2$ | X | Y | $W_n$ | B |
|---|---|---|---|---|---|---|
| 333 | $CH_3$ | $CH_3$ | NH | S | 4-Cl | B5 |
| 334 | $CH_3$ | $CH_3$ | NH | S | 2-Cl, 4-Cl | B5 |
| 335 | $CH_3$ | $CH_3$ | NH | S | 4-Cl | B6 |
| 336 | $CH_3$ | $CH_3$ | NH | S | 2-Cl, 4-Cl | B6 |
| 337 | $CH_3$ | $CH_3$ | NH | S | 4-Cl | B7 |
| 338 | $CH_3$ | $CH_3$ | NH | S | 2-Cl, 4-$CH_3$ | B7 |
| 339 | $CH_3$ | $CH_3$ | NH | S | 4-Cl | B8 |
| 340 | $CH_3$ | $CH_3$ | NH | S | 2-Cl, 4-Cl | B8 |
| 341 | $CH_3$ | $CH_3$ | NH | S | 4-Cl | B9 |
| 342 | $CH_3$ | $CH_3$ | NH | S | 2-Cl, 4-$CH_3$ | B9 |

Table 1 (continued)

| Compound No. | $R^1$ | $R^2$ | X | Y | $W_n$ | B |
|---|---|---|---|---|---|---|
| 343 | $CH_3$ | $CH_3$ | NH | S | 4-Cl | B10 |
| 344 | $CH_3$ | $CH_3$ | NH | S | 2-Cl, 4-Cl | B10 |
| 345 | $CH_3$ | $CH_3$ | NH | S | 3-Cl, 4-Cl | B10 |
| 346 | $CH_3$ | $CH_3$ | NH | S | 2-F, 4-$CH_3$ | B10 |
| 347 | $CH_3$ | $CH_3$ | NH | S | 3-F, 4-$CH_3$ | B10 |
| 348 | $CH_3$ | $CH_3$ | NH | S | 2-F, 4-Cl | B10 |
| 349 | $CH_3$ | $CH_3$ | NH | S | 4-Cl | B11 |
| 350 | $CH_3$ | $CH_3$ | NH | S | 2-Cl, 4-$CH_3$ | B11 |
| 351 | $CH_3$ | $CH_3$ | NH | S | 4-Cl | B12 |
| 352 | $CH_3$ | $CH_3$ | NH | S | 2-Cl, 4-Cl | B12 |

Table 1 (continued)

| Compound No. | $R^1$ | $R^2$ | X | Y | $W_n$ | B |
|---|---|---|---|---|---|---|
| 353 | $CH_3$ | $CH_3$ | NH | S | 2-F, 4-$CH_3$ | B12 |
| 354 | $CH_3$ | $CH_3$ | NH | S | 3-F, 4-$CH_3$ | B12 |
| 355 | $CH_3$ | $CH_3$ | NH | S | 2-Cl, 4-$CH_3$ | B12 |
| 356 | $CH_3$ | $CH_3$ | NH | S | 3-Cl, 4-$CH_3$ | B12 |
| 357 | $CH_3$ | $CH_3$ | NCHO | S | 4-Cl | B12 |
| 358 | $CH_3$ | $CH_3$ | NCHO | S | 2-Cl, 4-Cl | B12 |
| 359 | $CH_3$ | $CH_3$ | NH | S | 4-Cl | B13 |
| 360 | $CH_3$ | $CH_3$ | NH | S | 2-Cl, 4-Cl | B13 |
| 361 | $CH_3$ | $CH_3$ | NH | S | 2-Cl, 4-$CH_3$ | B13 |

Table 1 (continued)

| Compound No. | $R^1$ | $R^2$ | X | Y | $W_n$ | B |
|---|---|---|---|---|---|---|
| 362 | $CH_3$ | $CH_3$ | NH | S | 2-F, 4-$CH_3$ | B13 |
| 363 | $CH_3$ | $CH_3$ | NH | S | 4-Cl | B14 |
| 364 | $CH_3$ | $CH_3$ | NH | S | 2-Cl, 4-Cl | B14 |
| 365 | $CH_3$ | $CH_3$ | NH | S | 2-Cl, 4-$CH_3$ | B14 |
| 366 | $CH_3$ | $CH_3$ | NH | S | 2-F, 4-$CH_3$ | B14 |
| 367 | $CH_3$ | $CH_3$ | NH | S | 4-Cl | B15 |
| 368 | $CH_3$ | $CH_3$ | NH | S | 2-Cl, 4-Cl | B15 |
| 369 | $CH_3$ | $CH_3$ | NH | S | 2-Cl, 4-$CH_3$ | B15 |
| 370 | $CH_3$ | $CH_3$ | NH | S | 2-F, 4-$CH_3$ | B15 |
| 371 | $CH_3$ | $CH_3$ | NH | S | 4-Cl | B16 |

Table 1 (continued)

| Compound No. | $R^1$ | $R^2$ | X | Y | $W_n$ | B |
|---|---|---|---|---|---|---|
| 372 | $CH_3$ | $CH_3$ | NH | S | 2-Cl, 4-Cl | B16 |
| 373 | $CH_3$ | $CH_3$ | NH | S | 2-Cl, 4-$CH_3$ | B16 |
| 374 | $CH_3$ | $CH_3$ | NH | S | 2-F, 4-$CH_3$ | B16 |
| 375 | $CH_3$ | $CH_3$ | NH | S | 4-Cl | B17 |
| 376 | $CH_3$ | $CH_3$ | NH | S | 2-Cl, 4-Cl | B17 |
| 377 | $CH_3$ | $CH_3$ | NH | S | 2-Cl, 4-$CH_3$ | B17 |
| 378 | $CH_3$ | $CH_3$ | NH | S | 2-F, 4-$CH_3$ | B17 |
| 379 | $CH_3$ | $CH_3$ | NH | S | 4-Cl | B18 |
| 380 | $CH_3$ | $CH_3$ | NH | S | 2-Cl, 4-Cl | B18 |
| 381 | $CH_3$ | $CH_3$ | NH | S | 2-Cl, 4-$CH_3$ | B18 |

Table 1 (continued)

| Compound No. | $R^1$ | $R^2$ | X | Y | $W_n$ | B |
|---|---|---|---|---|---|---|
| 382 | $CH_3$ | $CH_3$ | NH | S | 2-F, 4-$CH_3$ | B18 |
| 383 | $CH_3$ | $CH_3$ | NCHO | S | 2-Cl, 4-Cl | B1 |
| 384 | $CH_3$ | $CH_3$ | NCHO | S | 3-Cl, 4-Cl | B1 |
| 385 | $CH_3$ | $CH_3$ | NCHO | S | 2-F, 4-Cl | B1 |
| 386 | $CH_3$ | $CH_3$ | NCHO | S | 2-F, 4-$CH_3$ | B1 |
| 387 | $CH_3$ | $CH_3$ | NCHO | S | 3-F, 4-Cl | B1 |
| 388 | $CH_3$ | $CH_3$ | NCHO | S | 3-F, 4-$CH_3$ | B1 |
| 389 | $CH_3$ | $CH_3$ | NCHO | S | 2-Cl, 4-$CH_3$ | B1 |
| 390 | $CH_3$ | $CH_3$ | NCHO | S | 3-Cl, 4-$CH_3$ | B1 |
| 391 | $CH_3$ | $CH_3$ | NCHO | S | 2-Cl, 3-Cl, 4-Cl | B1 |

Table 1 (continued)

| Compound No. | $R^1$ | $R^2$ | X | Y | $W_n$ | B |
|---|---|---|---|---|---|---|
| 392 | $CH_3$ | $CH_3$ | NCHO | S | 2-Cl, 4-Cl, 5-Cl | B 1 |
| 393 | $CH_3$ | $CH_3$ | $NCOCH_3$ | S | 2-Cl, 4-Cl | B 1 |
| 394 | $CH_3$ | $CH_3$ | $NCOCH_3$ | S | 3-Cl, 4-Cl | B 1 |
| 395 | $CH_3$ | $CH_3$ | $NCOCH_3$ | S | 2-F, 4-$CH_3$ | B 1 |
| 396 | $CH_3$ | $CH_3$ | $NCOCH_3$ | S | 2-Cl, 3-Cl, 4-Cl | B 1 |
| 397 | $CH_3$ | $CH_3$ | $NCH_3$ | S | 2-Cl, 4-Cl | B 1 |
| 398 | $CH_3$ | $CH_3$ | $NCH_3$ | S | 3-Cl, 4-$CH_3$ | B 1 |
| 399 | $CH_3$ | $CH_3$ | NNO | S | 2-Cl, 4-Cl | B 1 |
| 400 | $CH_3$ | $CH_3$ | NNO | S | 3-Cl, 4-$CH_3$ | B 1 |
| 401 | $CH_3$ | $CH_3$ | $NNH_2$ | S | 4-Cl | B 1 |

Table 1 (continued)

| Compound No. | $R^1$ | $R^2$ | X | Y | $W_n$ | B |
|---|---|---|---|---|---|---|
| 402 | $CH_3$ | $CH_3$ | $NNH_2$ | S | 2-Cl, 4-Cl | B1 |
| 403 | $CH_3$ | $CH_3$ | $NNH_2$ | S | 3-Cl, 4-$CH_3$ | B1 |
| 404 | $CH_3$ | $CH_3$ | $NSO_2CH_3$ | S | 2-Cl, 4-Cl | B1 |
| 405 | $CH_3$ | $CH_3$ | $NSO_2CH_3$ | S | 2-F, 4-$CH_3$ | B1 |
| 406 | $CH_3$ | $CH_3$ | $NSO_2CH_3$ | S | 2-Cl, 3-Cl, 4-Cl | B1 |
| 407 | $CH_3$ | $CH_3$ | $CH_2$ | S | 4-Cl | B1 |
| 408 | $CH_3$ | $CH_3$ | $CH_2$ | S | 2-Cl, 4-Cl | B1 |
| 409 | $CH_3$ | $CH_3$ | $CH_2$ | S | 2-Cl, 4-$CH_3$ | B1 |
| 410 | $CH_3$ | $CH_3$ | $CH_2$ | S | 2-F, 4-$CH_3$ | B1 |
| 411 | $CH_3$ | $CH_3$ | CH(OH) | S | 4-Cl | B1 |

Table 1 (continued)

| Compound No. | $R^1$ | $R^2$ | X | Y | $W_n$ | B |
|---|---|---|---|---|---|---|
| 412 | $CH_3$ | $CH_3$ | CH(OH) | S | 2-Cl, 4-Cl | B1 |
| 413 | $CH_3$ | $CH_3$ | CH(OH) | S | 2-Cl, 4-$CH_3$ | B1 |
| 414 | $CH_3$ | $CH_3$ | CH(OH) | S | 3-Cl, 4-$CH_3$ | B1 |
| 415 | $CH_3$ | $CH_3$ | CH(OH) | S | 2-F, 4-$CH_3$ | B1 |
| 416 | $CH_3$ | $CH_3$ | CH(OH) | S | 3-F, 4-$CH_3$ | B1 |
| 417 | $CH_3$ | $CH_3$ | $CH(OCH_3)$ | S | 4-Cl | B1 |
| 418 | $CH_3$ | $CH_3$ | $CH(OCH_3)$ | S | 2-Cl, 4-Cl | B1 |
| 419 | $CH_3$ | $CH_3$ | $CH(OCOCH_3)$ | S | 4-Cl | B1 |
| 420 | $CH_3$ | $CH_3$ | $CH(OCOCH_3)$ | S | 2-Cl, 4-Cl | B1 |
| 421 | $CH_3$ | $CH_3$ | $CH(OCOCH_3)$ | S | 2-Cl, 4-$CH_3$ | B1 |

Table 1 (continued)

| Compound No. | $R^1$ | $R^2$ | X | Y | $W_n$ | B |
|---|---|---|---|---|---|---|
| 422 | $CH_3$ | $CH_3$ | $CH(OCOCH_3)$ | S | 2-F, 4-$CH_3$ | B1 |
| 423 | $CH_3$ | $CH_3$ | CH(F) | S | 4-Cl | B1 |
| 424 | $CH_3$ | $CH_3$ | CH(F) | S | 2-Cl, 4-Cl | B1 |
| 425 | $CH_3$ | $CH_3$ | CH(Cl) | S | 4-Cl | B1 |
| 426 | $CH_3$ | $CH_3$ | CH(Cl) | S | 2-Cl, 4-Cl | B1 |
| 427 | $CH_3$ | $CH_3$ | C=O | S | 4-Cl | B1 |
| 428 | $CH_3$ | $CH_3$ | C=O | S | 2-Cl, 4-Cl | B1 |
| 429 | $CH_3$ | $CH_3$ | C=O | S | 2-Cl, 4-$CH_3$ | B1 |
| 430 | $CH_3$ | $CH_3$ | C=O | S | 2-F, 4-$CH_3$ | B1 |
| 431 | $CH_3$ | $CH_3$ | $CH_2$ | S | 4-Cl | B2 |

Table 1 (continued)

| Compound No. | $R^1$ | $R^2$ | X | Y | $W_n$ | B |
|---|---|---|---|---|---|---|
| 432 | $CH_3$ | $CH_3$ | $CH_2$ | S | 2-Cl, 4-Cl | B2 |
| 433 | $CH_3$ | $CH_3$ | $CH_2$ | S | 2-Cl, 4-$CH_3$ | B2 |
| 434 | $CH_3$ | $CH_3$ | $CH_2$ | S | 2-F, 4-$CH_3$ | B2 |
| 435 | $CH_3$ | $CH_3$ | CH(OH) | S | 4-Cl | B2 |
| 436 | $CH_3$ | $CH_3$ | CH(OH) | S | 2-Cl, 4-Cl | B2 |
| 437 | $CH_3$ | $CH_3$ | CH(OH) | S | 2-Cl, 4-$CH_3$ | B2 |
| 438 | $CH_3$ | $CH_3$ | CH(OH) | S | 3-Cl, 4-$CH_3$ | B2 |
| 439 | $CH_3$ | $CH_3$ | CH(OH) | S | 2-F, 4-$CH_3$ | B2 |
| 440 | $CH_3$ | $CH_3$ | CH(OH) | S | 3-F, 4-$CH_3$ | B2 |
| 441 | $CH_3$ | $CH_3$ | $CH(OCH_3)$ | S | 4-Cl | B2 |

Table 1 (continued)

| Compound No. | $R^1$ | $R^2$ | X | Y | $W_n$ | B |
|---|---|---|---|---|---|---|
| 442 | $CH_3$ | $CH_3$ | $CH(OCH_3)$ | S | 2-Cl, 4-Cl | B 2 |
| 443 | $CH_3$ | $CH_3$ | $CH(OCOCH_3)$ | S | 4-Cl | B 2 |
| 444 | $CH_3$ | $CH_3$ | $CH(OCOCH_3)$ | S | 2-Cl, 4-Cl | B 2 |
| 445 | $CH_3$ | $CH_3$ | $CH(OCOCH_3)$ | S | 2-Cl, 4-$CH_3$ | B 2 |
| 446 | $CH_3$ | $CH_3$ | $CH(OCOCH_3)$ | S | 2-F, 4-$CH_3$ | B 2 |
| 447 | $CH_3$ | $CH_3$ | CH(F) | S | 4-Cl | B 2 |
| 448 | $CH_3$ | $CH_3$ | CH(F) | S | 2-Cl, 4-Cl | B 2 |
| 449 | $CH_3$ | $CH_3$ | CH(Cl) | S | 4-Cl | B 2 |
| 450 | $CH_3$ | $CH_3$ | CH(Cl) | S | 2-Cl, 4-Cl | B 2 |
| 451 | $CH_3$ | $CH_3$ | C=O | S | 4-Cl | B 2 |

Table 1 (continued)

| Compound No. | $R^1$ | $R^2$ | X | Y | $W_n$ | B |
|---|---|---|---|---|---|---|
| 452 | $CH_3$ | $CH_3$ | C=O | S | 2-Cl, 4-Cl | B2 |
| 453 | $CH_3$ | $CH_3$ | C=O | S | 2-Cl, 4-$CH_3$ | B2 |
| 454 | $CH_3$ | $CH_3$ | C=O | S | 2-F, 4-$CH_3$ | B2 |
| 455 | $CH_3$ | $CH_3$ | $C(CH_3)(OH)$ | S | 4-Cl | B2 |
| 456 | $CH_3$ | $CH_3$ | $C(CH_3)(OH)$ | S | 2-Cl, 4-Cl | B2 |
| 457 | $CH_3$ | $CH_3$ | $C(CH_3)(OCOCH_3)$ | S | 4-Cl | B2 |
| 458 | $CH_3$ | $CH_3$ | $C(CH_3)(OCOCH_3)$ | S | 2-Cl, 4-Cl | B2 |
| 459 | $CH_3$ | $CH_3$ | $C(CH_3)(F)$ | S | 4-Cl | B2 |
| 460 | $CH_3$ | $CH_3$ | $C(CH_3)(F)$ | S | 2-Cl, 4-Cl | B2 |
| 461 | $CH_3$ | $CH_3$ | $C(C_2H_5)(OH)$ | S | 4-Cl | B2 |

Table 1 (continued)

| Compound No. | $R^1$ | $R^2$ | X | Y | $W_n$ | B |
|---|---|---|---|---|---|---|
| 462 | $CH_3$ | $CH_3$ | $C(C_2H_5)(OH)$ | S | 2-Cl, 4-Cl | B2 |
| 463 | $CH_3$ | $CH_3$ | $C(i\text{-}C_3H_7)(OH)$ | S | 4-Cl | B2 |
| 464 | $CH_3$ | $CH_3$ | $C(i\text{-}C_3H_7)(OH)$ | S | 2-Cl, 4-Cl | B2 |
| 465 | $CH_3$ | $CH_3$ | $C(i\text{-}C_3H_7)(OH)$ | S | 2-Cl, 4-$CH_3$ | B2 |
| 466 | $CH_3$ | $CH_3$ | $C(i\text{-}C_3H_7)(OH)$ | S | 2-F, 4-$CH_3$ | B2 |
| 467 | $CH_3$ | $CH_3$ | $CH(CH_3)$ | S | 4-Cl | B2 |
| 468 | $CH_3$ | $CH_3$ | $CH(CH_3)$ | S | 2-Cl, 4-Cl | B2 |
| 469 | $CH_3$ | $CH_3$ | $CH(CH_3)$ | S | 2-Cl, 4-$CH_3$ | B2 |
| 470 | $CH_3$ | $CH_3$ | $CH(CH_3)$ | S | 2-F, 4-$CH_3$ | B2 |
| 471 | Cl | $CH_3$ | $CH_2$ | S | 4-Cl | B2 |

Table 1 (continued)

| Compound No. | $R^1$ | $R^2$ | X | Y | $W_n$ | B |
|---|---|---|---|---|---|---|
| 472 | Cl | $CH_3$ | $CH_2$ | S | 2-Cl, 4-Cl | B2 |
| 473 | Cl | $CH_3$ | $CH_2$ | S | 2-Cl, 4-$CH_3$ | B2 |
| 474 | Cl | $CH_3$ | $CH_2$ | S | 2-F, 4-$CH_3$ | B2 |
| 475 | $CH_3O$ | $CH_3$ | $CH_2$ | S | 4-Cl | B2 |
| 476 | $CH_3O$ | $CH_3$ | $CH_2$ | S | 2-Cl, 4-Cl | B2 |
| 477 | $CH_3O$ | $CH_3$ | $CH_2$ | S | 2-Cl, 4-$CH_3$ | B2 |
| 478 | $CH_3O$ | $CH_3$ | $CH_2$ | S | 2-F, 4-$CH_3$ | B2 |

wherein B1 to B18 are as defined below:

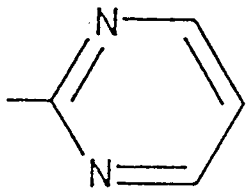

B1 ,

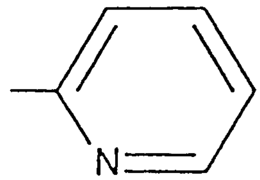

B2 ,

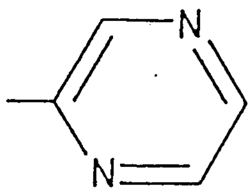

B3 ,

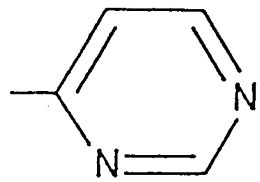

B4 ,

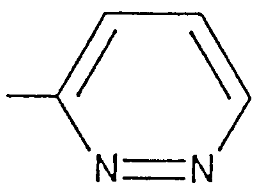

B5 ,

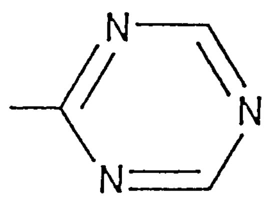

B6 ,

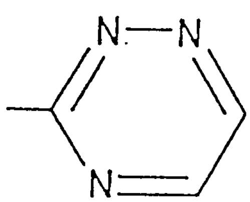

B7 ,

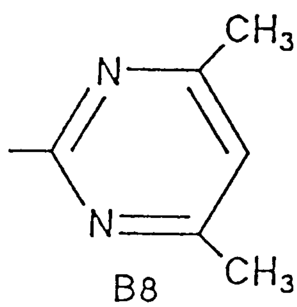

B8 ,

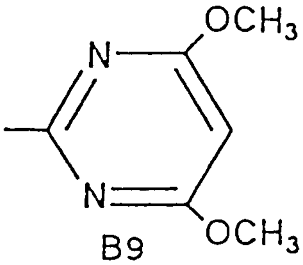
B9 ,

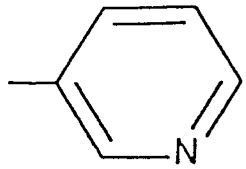
B10 ,

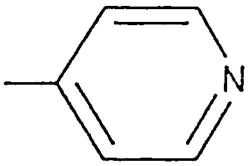
B11 ,

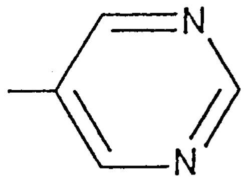
B12

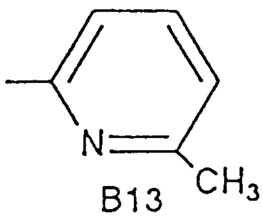
B13 ,

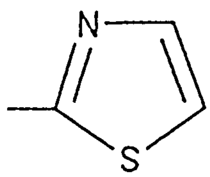
B14 ,

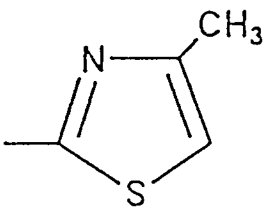
B15 ,

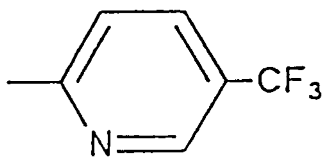
B16 ,

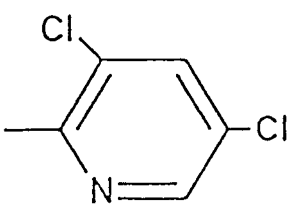
B17 ,

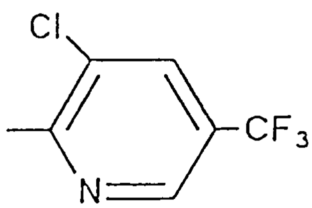
B18 ,

or wherein the substituted pyrazole derivatives are definied by the formula [1b]

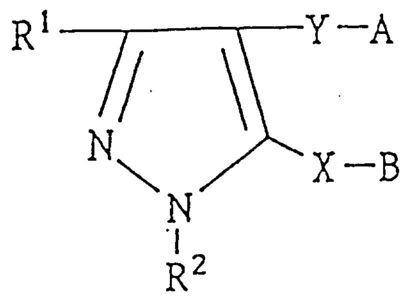

[1b]

having the following meanings of substituents

Table 2

| Compound No. | $R^1$ | $R^2$ | X | Y | A | B |
|---|---|---|---|---|---|---|
| 479 | $CH_3$ | $CH_3$ | NH | S | A1 | B1 |
| 480 | $CH_3$ | $CH_3$ | NH | S | A1 | B2 |
| 481 | $CH_3$ | $CH_3$ | NH | S | A2 | B1 |

Table 2 (continued)

| Compound No. | $R^1$ | $R^2$ | X | Y | $W_n$ | B |
|---|---|---|---|---|---|---|
| 482 | $CH_3$ | $CH_3$ | NH | S | A2 | B2 |
| 483 | $CH_3$ | $CH_3$ | NH | S | A3 | B1 |
| 484 | $CH_3$ | $CH_3$ | NH | S | A3 | B2 |
| 485 | $CH_3$ | $CH_3$ | NH | S | A4 | B1 |
| 486 | $CH_3$ | $CH_3$ | NH | S | A4 | B2 |
| 487 | $CH_3$ | $CH_3$ | NH | S | A5 | B1 |
| 488 | $CH_3$ | $CH_3$ | NH | S | A5 | B2 |
| 489 | $CH_3$ | $CH_3$ | NH | S | A6 | B1 |
| 490 | $CH_3$ | $CH_3$ | NH | S | A6 | B2 |
| 491 | $CH_3$ | $CH_3$ | NH | S | A7 | B1 |

Table 2 (continued)

| Compound No. | $R^1$ | $R^2$ | X | Y | $W_n$ | B |
|---|---|---|---|---|---|---|
| 492 | $CH_3$ | $CH_3$ | NH | S | A7 | B2 |
| 493 | $CH_3$ | $CH_3$ | NH | S | A8 | B1 |
| 494 | $CH_3$ | $CH_3$ | NH | S | A8 | B2 |
| 495 | $CH_3$ | $CH_3$ | NH | S | A9 | B1 |
| 496 | $CH_3$ | $CH_3$ | NH | S | A9 | B2 |
| 497 | $CH_3$ | $CH_3$ | NH | S | A10 | B1 |
| 498 | $CH_3$ | $CH_3$ | NH | S | A10 | B2 |
| 499 | $CH_3$ | $CH_3$ | NH | S | A11 | B1 |
| 500 | $CH_3$ | $CH_3$ | NH | S | A11 | B2 |

wherein A1 to A11 are as defined below

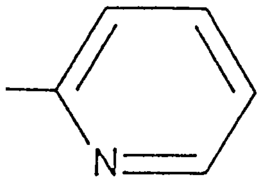
A1 ,

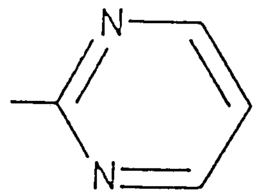
A2 ,

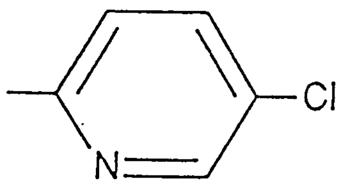
A3 ,

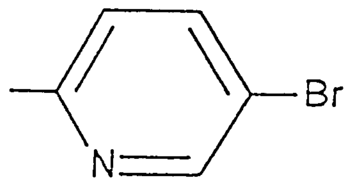
A4 ,

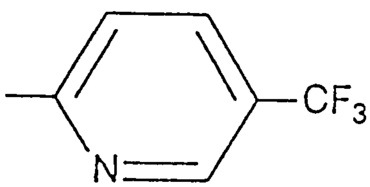
A5 ,

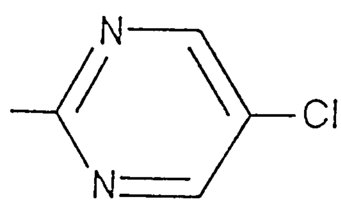
A6 ,

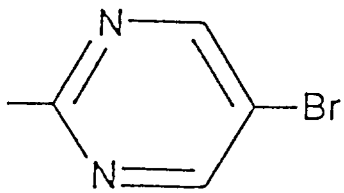
A7 ,

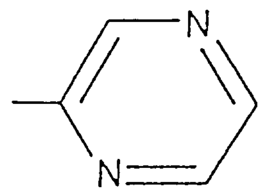
A8 ,

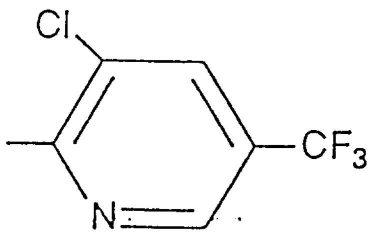
A9 ,

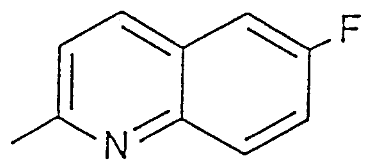
A10 ,

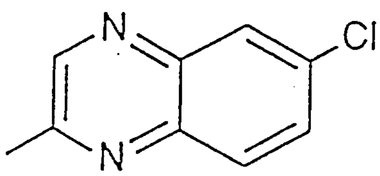
A11 ,

and B1 and B2 are as defined above,

and also to fungicides for agricultural and horticultural use containing the derivative(s) as an active ingredient.

The compound number is referred to in the following description herein. In the tables, Ph indicates a phenyl group, i indicates iso-, and t indicates tertiary.

Next, methods of producing compounds of the present invention are shown below by way of the reaction schemes, which will be explained hereunder.

Reaction Schemes

(Method 1)

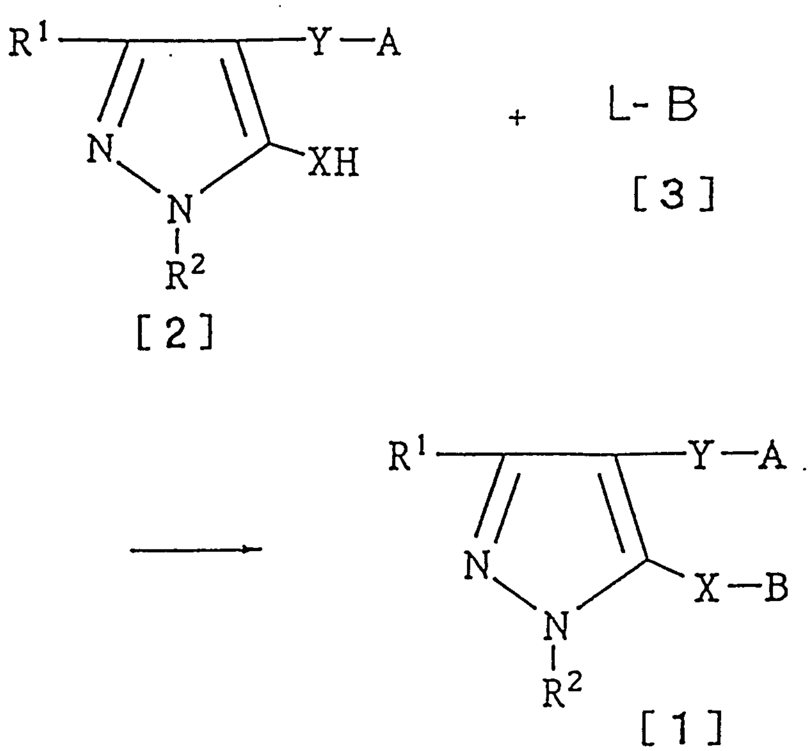

[2]

[3]

[1]

(Method 2)

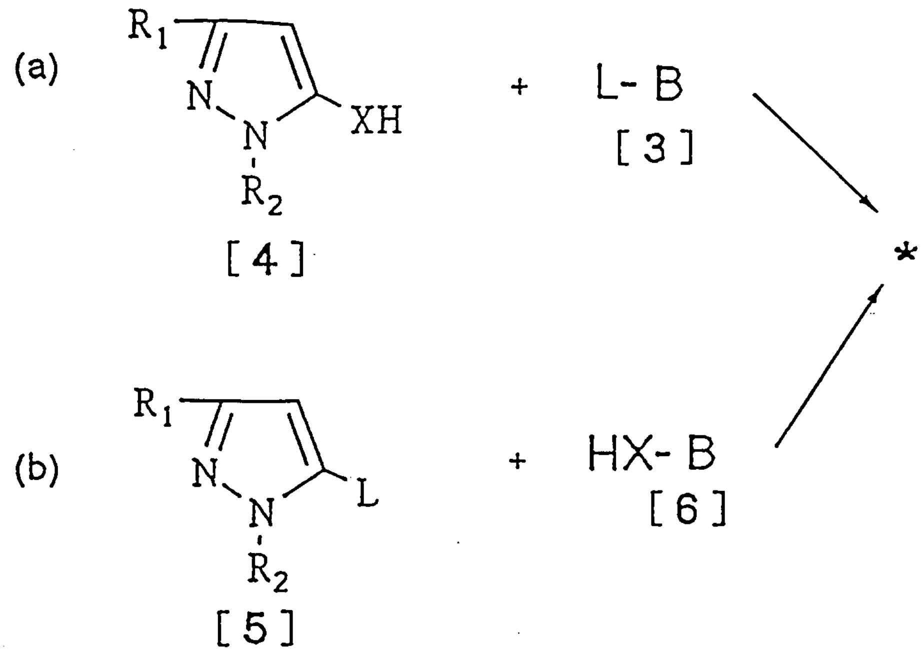

(a) [4] [3]

(b) [5] [6]

When Y ≠ 0:

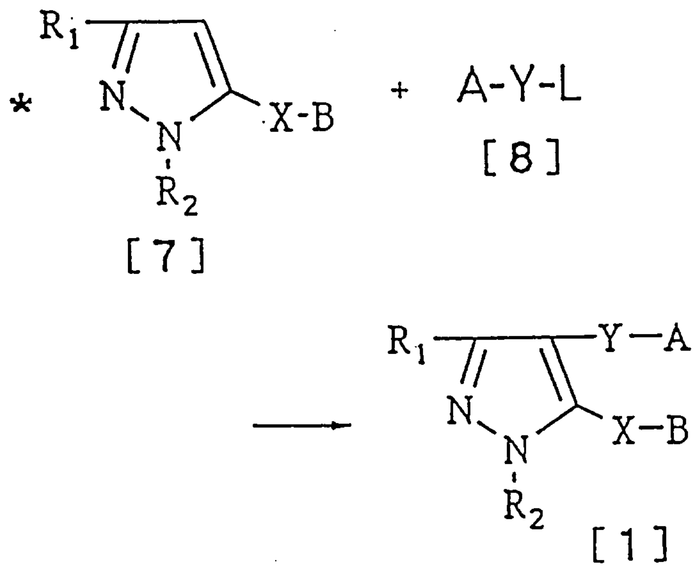

[7] [8]

[1]

(Method 3)

When X = N-$R^3$, $R^3 \neq H$:

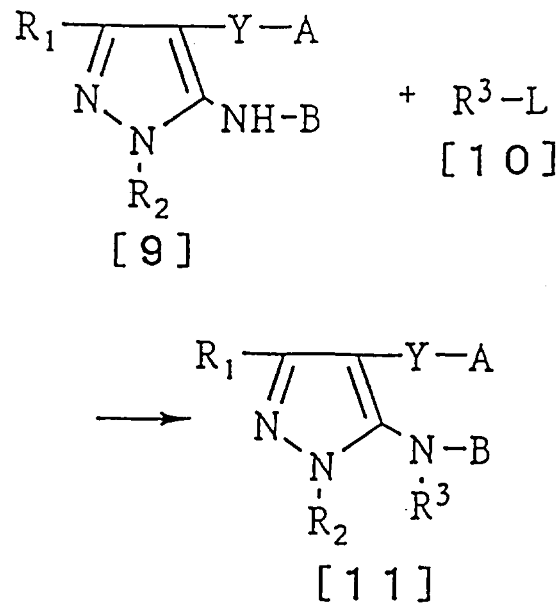

[9] [10] [11]

(Method 1)

Compounds of the present invention are produced by reacting a substituted pyrazole of a general formula [2]:

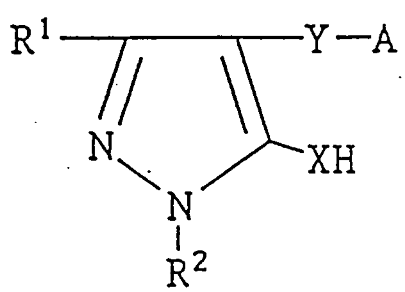

[2]

where $R^1$, $R^2$, X, Y and A have the same meanings as mentioned above, and a heterocyclic compound of a general formula [3]:

L - B [3]

where L represents a leaving group such as a halogen atom or the like; and B has the same meaning as mentioned above. In the case, where X is -$NCOR^4$ or -$NSO_2R^5$, the product may be hydrolyzed by post-treatment or the like to give a compound where X is -NH.

The above-mentioned reaction does not always need a solvent. If needed, however, the solvent may selected from, for example, hydrocarbons such as toluene, xylene, chlorobenzene and the like, halogenated hydrocarbons such as dichloroethane and the like, ethers such as diisopropyl ether, dioxane and the like,

esters such as ethyl acetate and the like, nitriles such as acetonitrile and the like, and polar solvents such as dimethylsulfoxide, dimethylformamide and the like.

If desired, an organic base (e.g., pyridine, triethylamine, etc.) or an inorganic base (e.g., potassium carbonate, sodium hydride, etc.) may be added to the reaction system.

If desired, a copper salt or a copper complex may also be added thereto as a catalyst.

Regarding the amounts of the reactants of the above-mentioned reaction, the heterocyclic compound of formula [3] is within the range of from 1 to 5 equivalents to one equivalent of the substituted pyrazole of formula [2].

The reaction temperature of the reaction is not defined, but in general, it is preferably from room temperature to 200 ° C or the reflux temperature of the solvent used.

After the reaction, the intended product may be obtained by ordinary treatment of the reaction mixture.

(Method 2)

(a) A compound of a general formula [7]:

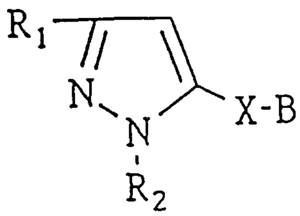

[7]

where $R^1$,$R^2$,X and B have the same meanings as mentioned above, is produced by reacting a pyrazole of a general formula [4]:

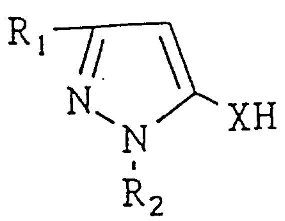

[4]

where $R^1$, $R^2$ and X have the same meanings as mentioned above, and a heterocyclic compound of formula [3] optionally in the presence of a suitable solvent and a suitable base. In the case, where X is $-NCOR^4$ or $-NSO_2R^5$, the product may be hydrolyzed by post-treatment or the like to give a compound where X is -NH.

The solvent to be, if any, in the above-mentioned reaction includes, for example, hydrocarbons such as toluene, xylene, chlorobenzene and the like, halogenated hydrocarbons such as dichloroethane and the like, ethers such as diisopropyl ether, dioxane and the like, esters such as ethyl acetate and the like, nitriles such as acetonitrile and the like, and polar solvents such as dimethylsulfoxide, dimethylformamide and the like.

The base to be, if any, in the same includes, for example, potassium carbonate, sodium hydride and the like.

If desired, a copper salt or a copper complex may be added to the reaction system as a catalyst.

The reaction temperature of the reaction is not defined, but in general, it is preferably from room temperature to 200 ° C or the reflux temperature of the solvent used.

(b) A compound of a general formula [7]:

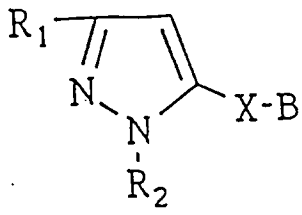

[7]

where $R^1$, $R^2$, X and B have the same meanings as mentioned above is produced by reacting a pyrazole of a general formula [5]:

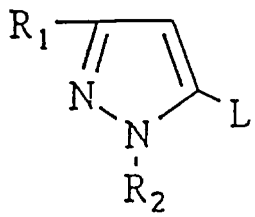

[5]

where $R^1$ and $R^2$ have the same meanings as mentioned above, and L represents a leaving group such as a halogen atom or the like, and a heterocyclic compound of a general formula [6]:

HX - B [6]

where X and B have the same meanings as mentioned above, optionally in the presence of a suitable solvent and a suitable base. In the case, where X is $-NCOR^4$ or $-NSO_2R^5$, the product may be hydrolyzed by post-treatment or the like to give a compound where X is -NH.

The solvent to be, if any, in the above-mentioned reaction includes, for example, hydrocarbons such as toluene, xylene, chlorobenzene and the like, halogenated hydrocarbons such as dichloroethane and the like, ethers such as diisopropyl ether, dioxane and the like, esters such as ethyl acetate and the like, nitriles such as acetonitrile and the like, and polar solvents such as dimethylsulfoxide, dimethylformamide and the like.

The base to be, if any, in the same includes, for example, potassium carbonate, sodium hydride and the like.

If desired, a copper salt or a copper complex may be added to the reaction system as a catalyst.

The reaction temperature of the reaction is not defined, but in general, it is preferably from room temperature to 200 °C or the reflux temperature of the solvent used.

Next, the pyrazole of formula [7] as obtained in the above-mentioned reaction (a) or (b) is reacted with a compound of a general formula [8]:

A - Y - L [8]

where A has the same meaning as mentioned above, Y has the same meaning as mentioned above, except oxygen atom, and L represents a leaving group such as a halogen atom or the like, optionally in the presence of a suitable solvent and a suitable base, to give a compound of the present invention.

The solvent to be, if any, in the above-mentioned reaction includes, for example, hydrocarbons such as toluene, xylene, chlorobenzene and the like, halogenated hydrocarbons such as dichloroethane, chloroform, carbon tetrachloride and the like, ethers such as diisopropyl ether, dioxane and the like, esters such as ethyl acetate and the like, nitriles such as acetonitrile and the like, and polar solvents such as dimethylsulfoxide, dimethylformamide and the like.

The base to be, if any, in the same includes, for example, pyridine, triethylamine, potassium carbonate and the like.

The reaction temperature of the reaction is not defined, but in general, it is preferably from 0 °C to 100 °C.

(Method 3) When X = N-$R^3$, $R^3 \neq$ H:

A compound of the present invention is produced by reacting a pyrazole of a general formula [9]:

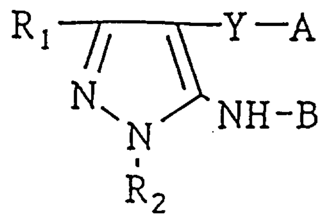

[9]

where $R^1$, $R^2$, Y, A and B have the same meanings as mentioned above, and a compound of a general formula [10]:

$R^3$ - L [10]

where $R^3$ has the same meaning as mentioned above except hydrogen atom, and L represents a leaving group such as a halogen atom or the like, optionally in the presence of a suitable solvent and a suitable base.

The solvent to be, if any, in the above-mentioned reaction includes, for example, hydrocarbons such as benzene, toluene, xylene and the like, halogenated hydrocarbons such as dichloroethane, chloroform, carbon tetrachloride and the like, ethers such as diisopropyl ether, tetrahydrofuran, dioxane and the like, esters such as ethyl acetate and the like, nitriles such as acetonitrile and the like, and polar solvents such as dimethylsulfoxide, dimethylformamide and the like.

The base to be, if any, in the same includes, for example, organic bases such as pyridine, triethylamine and the like, and inorganic bases such as potassium carbonate, sodium hydride and the like.

The reaction temperature of the reaction is not defined, but in general, it is preferably from 0 °C to 100 °C.

EXAMPLES

Next, concrete production examples are mentioned below.

Preparation Example 1 (preparation of compound No. 5 of the invention)

1.4 g of 4-(4-chlorophenylthio)-1,3-dimethyl-5-mercaptopyrazole and 1.2 g of 2-chloropyridine were stirred under heat at 120 °C for 1.5 hours. After cooled, 60 ml of ethyl acetate was added thereto and stirred, and the insoluble components were taken out by filtration. The filtrate was concentrated and then purified by silica gel column chromatography (developing solution; chloroform/ethyl acetate = 9/1), to give 0.6 g of 4-(4-chlorophenylthio)-1,3-dimethyl-5-(2-pyrimidylthio) pyrazole. Oily product. $n_D^{21.0}$ = 1.6465.

Preparation Example 2 (preparation of compound No. 49 of the invention)

① Preparation of N-(1,3-dimehtyl-5-pyrazolyl)formamide:

20 g of 5-amino-1,3-dimethylpyrazole was dissolved in 29 g of formic acid (85 %), and 55 g of acetic anhydride was dropwise added thereto under cooling with ice. After stirred for 3 days at room temperature, the reaction mixture was concentrated under reduced pressure and then purified by silica gel column chromatography (developing solution; chloroform) to give 12.8 g of N-(1,3-dimethyl-5-pyrazolyl)formamide.

② Preparation of 1,3-dimethyl-5-(2-pyridylamino)pyrazole:

A mixed solution comprising 4.1 g (29 mmol) of N-(1,3-dimethyl-5-pyrazolyl)formamide and 10 ml of N,N-dimethylformamide was dropwise added to a suspension of 70 ml of N,N-dimethylformamide containing 1.6 g of sodium hydride (55 %), under cooling with ice. After this was stirred for 2 hours at room temperature, a mixed solution comprising 3.4 g (30 mmol) of 2-chloropyrimidine and 10 ml of N,N-dimethylformamide was added thereto. After this was stirred under heat at 100 °C for further 2 days, the solvent was removed therefrom by distillation under reduced pressure and water was added thereto. Then, this was extracted with chloroform, washed with water and dried with anhydrous sodium sulfate. After this was filtered, the solvent was removed by distillation under reduced pressure, and the residue was purified by silica gel column chromatography to give 2.4 g of 1,3-dimethyl-5-(2-pyrimidylamino)pyrazole. m.p. 179.0 to 182.0 °C.

③ Preparation of compound No. 49 of the invention:

1.1 g of 1,3-dimethyl-5-(2-pyrimidylamino)pyrazole was dissolved in 30 ml of chloroform. 0.5 g of 4-chlorophenylsulfenyl chloride was dropwise added to the resulting solution at room temperature and reacted for one hour with stirring. The organic layer was washed with 30 ml of water and then dried with anhydrous sodium sulfate.

The solvent was removed by distillation under reduced pressure, and the residue was purified by silica gel column chromatography (developing solution; chloroform) to give 0.8 g of 4-(4-chlorophenylthio)-1,3-dimethyl-5-(2-pyrimidylamino)pyrazole. m.p. 158.0 to 159.0 °C.

Preparation Example 3 (preparation of compound No. 172 of the invention)

① Preparation of 1,3-dimethyl-5-(N-(2-pyrimidyl)-N-methylamino)pyrazole:

1.0 g of 1,3-dimethyl-5-(2-pyrimidylamino)pyrazole was added to a suspension of 10 ml of THF containing 0.25 g of sodium hydride (55 %), little by little under cooling with ice and then stirred for one hour at 60 °C. The solution was cooled to room temperature, and 4.1 g of methyl iodide was added thereto and refluxed gently for 2 hours. After cooled, 10 ml of water was added thereto. Then, this was extracted three times each with 30 ml of diethyl ether. The ether layer was dried with anhydrous sodium sulfate, the solvent was removed by distillation under reduced pressure, and the residue was purified by silica gel column chromatography (developing solution; chloroform) to give 0.5 g of 1,3-dimethyl-5-(N-(2-pyrimidyl)-N-methylamino)pyrazole. Yellow oily product.

② Preparation of compound No. 172 of the invention:

0.14 g of p-chlorophenylsulfenyl chloride was dropwise added to a solution of 10 ml of chloroform containing 0.2 g of 1,3-dimethyl-5-(N-(2-pyrimidyl)-N-methylamino)pyrazole and stirred for 15 hours at room temperature.

The solvent was removed by distillation under reduced pressure, and the residue was purified by silica gel column chromatography (developing solution; chloroform) to give 0.25 g of 4-(4-chlorophenylthio)-1,3-dimethyl-5-(N-(2-pyrimldyl)-N-methylamino)pyrazole. m.p. 77.0 to 78.0 °C.

Preparation Example 4 (preparation of compound No. 314 of the invention)

① Preparation of 1,3-dimethyl-5-(2-pyridylamino)pyrazole:

9.9 g of anhydrous potassium carbonate and 1 g of copper(II) acetylacetonate were added to a mixed solution of 60 ml of N,N-dimethylformamide containing 10 g of N-(1,3-dimethyl-5-pyrazolyl)formamide and 10.2 g of 2-bromopyridine and heated under reflux for 3 hours. After the solvent was removed by distillation under reduced pressure, water was added to the reaction mixture, which was then extracted with chloroform. The organic layer was washed with water and dried with anhydrous sodium sulfate.

The solvent was removed by distillation under reduced pressure, and the residue was purified by silica gel column chromatography (developing solution; chloroform/ethyl acetate) to give 4.6 g of 1,3-dimethyl-5-(2-pyridylamino)pyrazole. m.p. 113.0 to 115.0 °C.

② Preparation of compound No. 314 of the invention:

1.27 g of 1,3-dimethyl-5-(2-pyridylamino)pyrazole was dissolved in 50 ml of chloroform and cooled with ice water. 1.55 g of 2,4-dichlorophenylsulfenyl chloride was dropwise added to the solution and stirred for 15 hours at room temperature.

The solution was washed with an aqueous sodium hydrogencarbonate solution and then with water and dried with anhydrous sodium sulfate. The solvent was removed by distillation under reduced pressure, and the residue was purified by silica gel column chromatography (developing solution; chloroform) to give 1.75 g of 4-(2,4-dichlorophenylthio)-1,3-dimethyl-5-(2-pyridylamino)pyrazole. m.p. 144.0 to 145.0 °C.

Preparation Example 5 (preparation of compound No. 435 of the invention)

① Preparation of 2-pyridyl-(1,3-dimethyl-5-pyrazolyl)methanol:

300 ml of dry tetrahydrofuran solution containing 5.5 g of 2-bromopyridine was cooled to -78 °C, and 5.45 g of n-butyl lithium hexane solution (15 w/w %) was dropwise added thereto and stirred for 30 minutes. Then, 4.2 g of 1,3-dimethyl-5-formylpyrazole was dropwise added thereto. Afterwards, this was gradually heated up to room temperature and stirred for 15 hours.

The solution was neutralized by adding 2 N hydrochloric acid thereto, and then extracted three times each with 150 ml of ethyl acetate. The organic layer was dried with anhydrous sodium sulfate, the solvent was removed by distillation under reduced pressure, and the residue was purified by silica gel column chromatography (developing solution; chloroform) to give 5.2 g of 2-pyridyl-(1,3-dimethyl-5-pyrazolyl)-methanol as a brown oily product.

② Preparation of compound No. 435 of the invention:

3.4 g of p-chlorophenylsulfenyl chloride was dropwise added to 60 ml of dry chloroform solution containing 3 g of 2-pyridyl-(1,3-dimethyl-5-pyrazolyl)methanol at room temperature and stirred for 12 hours. After the solvent was removed by distillation, 50 ml of ethyl acetate and 50 ml of aqueous 10 % sodium hydrogencarbonate solution were added to this and stirred for 30 minutes. The organic layer was separated, and the aqueous layer was extracted three times each with 50 ml of ethyl acetate. The combined organic layers were dried with anhydrous sodium sulfate. The solvent was removed by distillation under reduced pressure, and the residue was purified by silica gel column chromatography (developing solution; chloroform) to give 1.2 g of 2-pyridyl-4-(4-chloropehnylthio)-1,3-dimehtyl-5-pyrazolyl)methanol as white crystals. m.p. 90.0 to 91.0 °C.

Preparation Example 6 (preparation of compound No. 451 of the invention)

1.2 g of compound No. 435 of the invention as obtained in Production Example 5 was dissolved in 50 ml of dry dichloromethane, and 1.5 g of manganese oxide was added thereto at room temperature and stirred for 2 hours. The insoluble component was removed by filtration with Celite, the solvent was removed by distillation under reduced pressure, and the residue was crystallized from diisopropyl ether to give 1.0 g of 2-pyridyl-(4-(4-chlorophenylthio)-1,3-dimethyl-5-pyrazolyl)ketone as white crystals. m.p. 111.0 to 113.0 °C.

Physical properties of compounds as produced in accordance with these methods are shown in Table 3 below.

Table 3

| Compound No. | Physical properties | $^1$H-NMR δ (ppm, $CDCl_3$) 、 standard substance TMS |
|---|---|---|
| 5 | oily | $n_D^{21.0}$ = 1.6465 |
| 47 | m.p. 150.0～151.0 ℃ | |
| 49 | m.p. 158.0～159.0 ℃ | |
| 52 | m.p. 170.0～171.0 ℃ | |
| 55 | m.p. 150.0～152.0 ℃ | |
| 56 | m.p. 181.0～182.0 ℃ | |
| 57 | m.p. 190.0～193.0 ℃ | |
| 58 | m.p. 159.0～162.0 ℃ | |

Table 3

| Compound No. | Physical properties | $^1$H-NMR δ (ppm, $CDCl_3$), standard substance TMS |
|---|---|---|
| 59 | m.p. 168.5~171.5 ℃ | |
| 60 | m.p. 157.0~158.0 ℃ | |
| 61 | m.p. 170.5~172.5 ℃ | |
| 63 | m.p. 160.0~162.0 ℃ | |
| 64 | m.p. 156.0~157.0 ℃ | |
| 66 | m.p. 180.0~183.0 ℃ | |
| 67 | m.p. 125.5~127.0 ℃ | |
| 68 | m.p. 154.0~157.0 ℃ | |
| 69 | m.p. 126.0~127.0 ℃ | |

Table 3 (continued)

| Compound No. | Physical properties | $^1$H-NMR δ (ppm, $CDCl_3$), standard substance TMS |
|---|---|---|
| 70 | m.p. 178.0～179.0 ℃ | |
| 71 | m.p. 163.5～167.5 ℃ | |
| 72 | m.p. 174.0～176.0 ℃ | |
| 73 | m.p. 132.5～135.5 ℃ | |
| 76 | m.p. 148.0～151.0 ℃ | |
| 79 | m.p. 127.5～128.5 ℃ | |
| 81 | m.p. 180.0～184.0 ℃ | |
| 83 | m.p. 175.0～177.0 ℃ | |

Table 3 (continued)

| Compound No. | Physical properties | $^1H$-NMR δ (ppm, $CDCl_3$), standard substance TMS |
|---|---|---|
| 95 | m.p. 160.0～162.0 ℃ | |
| 96 | m.p. 180.0～181.5 ℃ | |
| 97 | m.p. 186.0～188.5 ℃ | |
| 98 | m.p. 201.0～205.0 ℃ | |
| 99 | m.p. 166.0～168.0 ℃ | |
| 100 | m.p. 164.5～166.5 ℃ | |
| 102 | m.p. 161.0～163.0 ℃ | |
| 105 | m.p. 148.5～150.0 ℃ | |
| 106 | m.p. 134.5～137.0 ℃ | |

Table 3 (continued)

| Compound No. | Physical properties | $^1$H-NMR δ (ppm, $CDCl_3$), standard substance TMS |
|---|---|---|
| 108 | m.p. 189.0～191.0 ℃ | |
| 110 | m.p. 135.0～137.0 ℃ | |
| 113 | m.p. 178.0～180.0 ℃ | |
| 116 | m.p. 158.0～160.0 ℃ | |
| 120 | m.p. 164.0～166.0 ℃ | |
| 124 | m.p. 140.0～141.0 ℃ | |
| 126 | m.p. 159.0～161.0 ℃ | |
| 129 | m.p. 147.0～148.0 ℃ | |
| 132 | m.p. 181.0～183.0 ℃ | |

Table 3 (continued)

| Compound No. | Physical properties | $^1$H-NMR δ (ppm, $CDCl_3$)、standard substance TMS |
|---|---|---|
| 133 | m.p. 196.0～198.0 ℃ | |
| 142 | m.p. 162.0～164.0 ℃ | |
| 144 | m.p. 143.0～145.0 ℃ | |
| 146 | m.p. 151.5～153.5 ℃ | |
| 149 | m.p. 214.0～216.0 ℃ | |
| 154 | m.p. 139.0～140.0 ℃ | |
| 157 | m.p. 192.5～195.5 ℃ | |
| 159 | m.p. 190.5～193.5 ℃ | |
| 167 | m.p. 123.0～124.0 ℃ | |

Table 3 (continued)

| Compound No. | Physical properties | $^1$H-NMR δ (ppm, $CDCl_3$), standard substance TMS |
|---|---|---|
| 168 | m.p. 152.0～154.0 ℃ | |
| 169 | semi-crystalline | 2.20(s, 3H), 3.65(s, 3H), 3.69(s, 3H), 6.60～7.00(m, 5H), 8.35(d, 2H, J=5Hz) |
| 170 | m.p. 183.0～184.0 ℃ | |
| 172 | m.p. 77.0～ 78.0 ℃ | |
| 175 | m.p. 70.0～ 72.0 ℃ | |
| 176 | oily $n_D^{21.0}=1.5957$ | |
| 177 | m.p. 84.0～ 87.0 ℃ | |
| 178 | m.p. 83.0～ 85.0 ℃ | |

Table 3 (continued)

| Compound No. | Physical properties | $^1$H-NMR δ (ppm, $CDCl_3$)、standard substance TMS |
|---|---|---|
| 179 | oily | 2.08(s, 3H), 2.19(s, 3H), 3.79(s, 3H), 4.09(d, 1H, J=18Hz), 5.10(d, 1H, J=18Hz) 6.54(t, 1H, J=5Hz), 6.90(s, 4H), 8.13(d, 2H, J=5Hz) |
| 180 | m.p. 98.0～100.0 ℃ | |
| 182 | m.p. 128.0～129.0 ℃ | |
| 184 | oily | 2.20(s, 3H), 3.21(s, 3H), 4.50(d, 1H, J=14Hz), 5.52(d, 1H, J=14Hz), 6.58(t, 1H, J=5Hz), 6.91(s, 4H), 7.08(s, 4H), 8.18(d, 2H, J=5Hz) |
| 187 | m.p. 164.0～166.0 ℃ | |

Table 3 (continued)

| Compound No. | Physical properties | $^1H$-NMR δ (ppm, $CDCl_3$ ), standard substance TMS |
|---|---|---|
| 188 | m.p. 189.0～191.0 ℃ | |
| 194 | m.p. 142.0～143.5 ℃ | |
| 195 | m.p. 140.5～141.5 ℃ | |
| 197 | m.p. 149.0～152.0 ℃ | |
| 198 | m.p. 168.5～169.5 ℃ | |
| 199 | m.p. 164.5～167.0 ℃ | |
| 201 | m.p. 183.0～187.0 ℃ | |
| 216 | m.p. 205.0～207.0 ℃ | |
| 291 | m.p. 194.0～196.0 ℃ | |

Table 3 (continued)

| Compound No. | Physical properties | $^1$H-NMR δ (ppm, $CDCl_3$), standard substance TMS |
|---|---|---|
| 292 | m.p. 186.0~188.0 ℃ | |
| 301 | m.p. 148.0~150.0 ℃ | |
| 303 | m.p. 165.0~166.0 ℃ | |
| 311 | m.p. 127.0~128.0 ℃ | |
| 312 | m.p. 167.0~168.0 ℃ | |
| 314 | m.p. 144.0~145.0 ℃ | |
| 315 | m.p. 126.0~128.0 ℃ | |
| 316 | m.p. 145.0~147.0 ℃ | |
| 317 | m.p. 118.0~120.0 ℃ | |

Table 3 (continued)

| Compound No. | Physical properties | $^1$H-NMR δ (ppm, $CDCl_3$)、standard substance TMS |
|---|---|---|
| 318 | m.p. 112.0～114.0 ℃ | |
| 319 | m.p. 99.0～100.0 ℃ | |
| 320 | m.p. 99.0～100.0 ℃ | |
| 321 | m.p. 122.0～124.0 ℃ | |
| 322 | m.p. 107.0～108.0 ℃ | |
| 326 | m.p. 158.0～160.0 ℃ | |
| 328 | m.p. 168.0～169.0 ℃ | |
| 339 | m.p. 147.0～148.0 ℃ | |
| 341 | m.p. 128.0～130.0 ℃ | |

Table 3 (continued)

| Compound No. | Physical properties | $^1$H-NMR δ (ppm, $CDCl_3$)、standard substance TMS |
|---|---|---|
| 343 | m.p. 128.0～131.0 ℃ | |
| 344 | m.p. 180.0～181.0 ℃ | |
| 345 | m.p. 301.0～302.0 ℃ | |
| 346 | m.p. 185.0～186.0 ℃ | |
| 347 | m.p. 106.0～107.0 ℃ | |
| 351 | m.p. 160.0～161.0 ℃ | |
| 352 | m.p. 161.0～163.0 ℃ | |
| 354 | m.p. 142.0～144.0 ℃ | |
| 355 | m.p. 128.0～130.0 ℃ | |

Table 3 (continued)

| Compound No. | Physical properties | $^1H$-NMR δ (ppm, $CDCl_3$)、standard substance TMS |
|---|---|---|
| 363 | m.p. 148.0～149.0 ℃ | |
| 365 | m.p. 172.0～174.0 ℃ | |
| 367 | m.p. 132.0～133.0 ℃ | |
| 391 | m.p. 140.0～141.0 ℃ | |
| 402 | resinous | 2.15(s, 3H), 3.70(s, 3H), 4.62(bs, 2H), 6.50～7.20(m, 4H), 8.16(d, 2H, J=5Hz) |
| 435 | m.p. 90.0～ 91.0 ℃ | |
| 436 | m.p. 142.0～145.0 ℃ | |

Table 3 (continued)

| Compound No. | Physical properties | $^1$H-NMR δ (ppm, $CDCl_3$)、standard substance TMS |
|---|---|---|
| 440 | resinous | 2.20(s, 6H), 3.55(s, 3H), 5.10(bs, 1H), 6.05(s, 1H), 6.50～7.60(m, 6H), 8.40(d, 1H, J=5Hz) |
| 441 | oily | 2.17(s, 3H), 3.30(s, 3H), 3.68(s, 3H), 5.68(s, 1H), 6.80～7.70(m, 7H), 8.30～8.50(m, 1H) |
| 443 | oily | 2.10(s, 3H), 2.25(s, 3H), 4.05(s, 3H), 6.80～8.70(m, 9H), |

Table 3 (continued)

| Compound No. | Physical properties | $^1$H-NMR δ (ppm, $CDCl_3$ ), standard substance TMS |
|---|---|---|
| 447 | oily | 2.19(s, 3H), 3.77(s, 3H), 6.67(d, 1H, J=45Hz), 6.90～7.70(m, 7H), 8.51(d, 1H, J=5Hz) |
| 451 | m.p. 111.0～113.0 ℃ | |
| 455 | oily | 2.03(s, 3H), 2.16(s, 3H), 3.69(s, 3H), 6.00(bs, 1H), 6.80～8.51(m, 8H) |
| 463 | resinous | 0.50(d, 3H, J=7Hz), 1.00(d, 3H, J=7Hz), 2.02(s, 3H), 3.21～3.65(m, 1H), 3.87(s, 3H), 6.00(bs, 3H), 6.61～8.35(m, 8H) |

Table 3 (continued)

| Compound No. | Physical properties | $^1H$-NMR δ (ppm, $CDCl_3$), standard substance TMS |
|---|---|---|
| 481 | semi-crystalline | 2.26(s, 3H), 3.72(s, 3H), 6.62(t, 1H, J=5Hz), 6.88(t, 1H, J=5Hz), 7.61(bs, 1H), 8.23(d, 2H, J=5Hz), 8.34(d, 2H, J=5Hz) |
| 483 | m.p. 167.0~169.0 °C | |
| 485 | m.p. 187.0~189.0 °C | |
| 497 | m.p. 204.0~206.0 °C | |
| 499 | m.p. 159.0~161.0 °C | |

Where compounds of the present invention are used as a fungicide for agricultural and horticultural use, in general, they may be mixed with a suitable carrier, for example, a solid carrier such as clay, talc, bentonite, diatomaceous earth or the like, or a liquid carrier such as water, alcohols (e.g., methanol, ethanol, etc.), aromatic hydrocarbons (e.g., benzene, toluene, xylene, etc.), chlorinated hydrocarbons, ethers, ketones, esters (e.g., ethyl acetate, etc.), acid, amides (e.g., dimethylformamide, etc.) or the like. If desired, they may be blended with an emulsifier, a dispersing agent, a suspending agent, a penetrating agent, a

spreader, a stabilizer and the like to be formed into various practical formulations of liquid, oil, emulsion, wettable powder, powder, granules, flowable or the like.

If desired, they may also be combined with any other herbicides, various insecticides, fungicides , plant growth regulators, synergists and others, in forming them into formulations or in actually sprinkling them onto plants.

The amount of the compound of the present invention to be applied to plants varies, depending upon the place, time, method, plant diseases, growing crops and other conditions. In general, the effective amount is suitably from 0.005 to 50 kg or so per ha (hectare).

Next, some examples of fungicidal formulations of containing the compound of the present invention as an active ingredient are shown below, which, however, are not limitative. In the following examples, "parts" are by weight.

Formulation Example 1: Emulsion

| | |
|---|---|
| Compound of the invention | 20 parts |
| Xylene | 55 parts |
| N,N-dimethylformamide | 20 parts |
| Sorpol 2680 (trade name by Toho Chemical Industry Co.; mixture of nonionic surfactant and anionic surfactant) | 5 parts |

The above ingredients are uniformly blended to form an emulsion. Before use, the emulsion is diluted to from 1/50 to 1/20000, and the diluted emulsion is sprayed over a crop field in an amount of from 0.005 to 50 kg, as the active ingredient, per ha.

Formulation Example 2: Wettable powder

| | |
|---|---|
| Compound of the invention | 25 parts |
| Zieklite PFP (trade name by Zieklite Industry Co.; mixture of kaolinite and sericite) | 66 parts |
| Sorpol 5039 (trade name by Toho Chemical Industry Co.; anionic surfactant) | 4 parts |
| Carplex #80 (trade name by Shionogi & Co., Ltd.; white carbon) | 3 parts |
| Calcium lignin sulfonate | 2 parts |

The above ingredients are uniformly blended and milled to form a wettable powder. Before use, the powder is diluted with water to from 1/50 to 1/20000, and the diluted liquid is sprayed over a crop field in an amount of from 0.005 to 50 kg, as the active ingredient, per ha.

Formulation Example 3: Oil

| | |
|---|---|
| Compound of the invention | 10 parts |
| Methyl Cellosolve | 90 parts |

The above ingredients are uniformly blended to form an oil. For use, this is sprayed over a crop field in an amount of from 0.005 to 50 kg, as the active ingredient, per ha.

Formulation Example 4: Powder

| | |
|---|---|
| Compound of the invention<br>Carplex #80 (trade name by Shionogi & Co., Ltd.; white carbon)<br>Clay<br>Diisopropyl phosphate | 3.0 parts<br>0.5 parts<br>95 parts<br>1.5 parts |

The above ingredients are uniformly blended and milled to form a powder. For use, this is sprayed over a crop filed in an amount of from 0.005 to 50 kg, as the active ingredient, per ha.

Formulation Example 5: Granules

| | |
|---|---|
| Compound of the invention<br>Bentonite<br>Talc<br>Calcium lignin sulfonate | 5 parts<br>54 parts<br>40 parts<br>1 part |

The above ingredients are uniformly blended and milled , and a small amount of water is added thereto and mixed with stirring. The mixture is granulated through an granulating extruder and dried to form granules. For use, the granules are sprayed over a crop field in an amount of from 0.005 to 50 kg, as the active ingredient, per ha.

Formulation Example 6: Flowable

| | |
|---|---|
| Compound of the invention<br>Sorpol 3353 (trade name by Toho Chemical Industry Co.; nonionic surfactant)<br>Lunox 1000C (trade name by Toho Chemical Industry Co.; anionic surfactant)<br>1 % Zanthan gum aqueous solution (natural polymer)<br>Water | 25 parts<br>10 parts<br>0.5 parts<br>20 parts<br>44.5 parts |

The above ingredients except the active ingredient are uniformly mixed , and the compound of the invention is added thereto and well stirred. The resulting blend is then wet-milled in a sand mill to obtain a flowable. Before use, this is diluted to from 1/50 to 1/20000, and the diluted liquid is sprayed over a crop field in an amount of from 0.005 to 50 kg, as the active ingredient, per ha.

Compounds of the present invention are effective for protecting plants from various plant diseases caused by, for example, Pyricularia oryzae, Cochliobolus miyabeanus, Rhizoctonia solani, Erysiphe graminis f. sp. hordei, f. sp. tritici, Pyrenophora graminea, Pyrenophora teres, Gibberella zeae, Puccinia striiformis, P. graminis, P. recondita, P. hordei, Typhula sp., Micronectriella nivais, Ustilago tritici, U. nuda, Pseudocercosporella herpotrichoides, Rhynchosporium secalis, Septoria tritici, Leptosphaeria nodorum, Diaporthe citri, Elsinoe fawcetti, Penicillium digitatum, P. italicum, Sclerotinia mali, Valsa mali, Podosphaera leucotricha, Alternaria mali, Venturia inaequalis, Venturia nashicola, Alternaria kikuchiana, Gymnosporangium haraeanum, Sclerotinia cinerea, Cladosporium carpophilum, Phomopsis sp., Plasmopara viticola, Elsinoe ampelina, Glomerella cingulata, Uncinula necator, Phakopsora ampelopsidis, Gloeosporium kaki, Cercospora kaki, Mycosphaerella nawae, Pseudoperenospora cubensis, Colletotrichum lagenarium, Sphaerotheca fuliginea, Mycosphaerella melonis, Phytophthora infestans, Alternaria solani, Cladosporium fulvam, Phomopsis vexans, Erysiphe cichoracoarum, Alternaria japonica, Cerocosporella brassicae, Puccinia allii, Cercospora kikuchii, Elsinoe glycines, Diaporthe phaseololum, Colletotrichum lindemuthianum, Mycosphaerella personatum, Cercospora arachidicola, Erysiphe pisi, Alternaria solani, Sphaerotheca humuli, Exobasidium reticulatum, Elsinoe leucospila, Alternaria longipes, Erysiphe cichoracearum, Colletotrichum tabacum, Cercospora beticola, Diplocarpon rosae, Sphaerotheca pannosa, Septoria chrysanthemiindici, Puccinia horiana, Botrytis cineria, Sclerotinia sclerotiorum, etc.

The effectiveness of the compounds of the present invention is concretely explained by way of the following test examples, which, however, are not limitative.

Test Example 1: Test for controlling gray mold (Botrytis cineria)

An emulsion of the compound of the present invention was diluted with water to have a concentration of 500 ppm. This was sprayed over two- or three-leave-stage tomato plants (variety; Fukuju) as grown in a pot having a diameter of 7 cm with a spray gun, in an amount of 20 ml/pot.

On the next day, a suspension of spores of Botrytis cinerea (containing 1.0 % of glucose and 2.5 % yeast extract; 40 spores/visible area (× 150)) was sprayed over the plants, which were then put in a cultivation box having a temperature of 25 °C and a humidity of 95 % or more for 5 days. The region of the infected and spotted leaves to all the treated leaves was measured, and the preventive value of the compound was calculated out from the following equation.

Preventive Value
= [1-(region of infected leaves in treated group/region of infected leaves in control group)] x 100

As a result, the following compounds of the present invention showed the preventive value of being 100.

Compounds No. 5, NO. 49, No. 52, NO. 56, No. 58, No. 59, No. 63, No. 67, No. 68, No. 95, No. 96, No. 99, No. 105, No. 108, No. 110, No. 116, No. 120, No. 124, No. 126, No. 129, No. 132, No. 133, No. 142, No. 154, No. 157, No. 167, No. 172, No. 301, No. 311, No. 312, No. 314, No. 315, No. 316, No. 317, No. 318, No. 319, No. 320, No. 321, No. 322, No. 326, No. 328, No. 343, No. 383, No. 391, No. 402, No. 447.

Text Example 2: Test for controlling sheath blight (Rhizoctonia solani)

An emulsion of the compound of the present invention was diluted with water to have a concentration of 500 ppm. 5 ml/pot of this was applied to three- or four-leave-stage rice plant (variety; Nihonbare ) as grown in a pot having a diameter of 5 cm, near the roots of them, and immediately thereafter, 15 ml/pot of this was sprayed over them.

Three days after the treatment, rice hulls as infected with Rhizoctonia solani were put near the roots of the plants and the plants were inoculated.

Then, the pots were put in a cultivation box having a temperature of 28 °C and a humidity of 95 % or more. Five days after the inoculation, the height of the infected and spotted leaves of the plants from the earth was measured, and the preventive value of the compound was calculated out from the following equation.

Preventive Value
= [1-(height of infected leaves in treated group/height of infected leaves in control group)] × 100

As a result, the following compounds of the present invention showed the preventive value of being 100.

Compounds No. 5, No. 47, No. 49, No. 52, No. 55, No. 56, No. 57, No. 59, No. 68, No. 69, No. 70, No. 71, No. 72, No. 79, No. 81, No. 96, No. 97, No. 99, No. 100, No. 102, No. 105, No. 108, No. 110, No. 113, No. 124, No. 126, No. 129, No. 142, No. 144, No. 146, No. 154, No. 157, No. 167, No. 168, No. 169, No. 172, No. 175, No. 178, No. 179, No. 182, No. 188, No. 194, No. 291, No. 292, No. 301, No. 312, No. 314, No. 316, No. 317, No. 318, No. 319, No. 320, No. 321, No. 322, No. 326, No. 328, No. 363, No. 365, No. 367, No. 383, No. 391, No. 402, No. 435, No. 436, No. 440, No. 441, No. 447, No. 451, No. 481, No. 483, No. 485, No. 499.

**Claims**

**Claims for the following Contracting States : AT, BE, CH, DE, DK, FR, GB, GR, IT, LI, LU, NL, SE**

**1.** Substituted pyrazole derivatives, wherein the substituted pyrazole derivatives are defined by a general formula [1]:

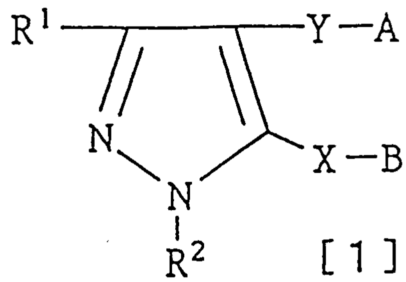

[1]

where $R^1$ represents a hydrogen atom, a halogen atom, an alkyl group, an alkoxy group, an alkylthio group or a haloalkyl group;

$R^2$ represents a hydrogen atom, an alkyl group, a haloalkyl group, a phenylalkyl group, $-COR^6$ or $-SO_2R^7$;

X represents -S-, -SO-, $-SO_2-$, $-N(R^3)-$, -CO- or - $C(R^4)(R^5)-$;

$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, a phenylalkyl group, $-COR^6$ or $-SO_2R^7$;

$R^4$ and $R^5$ independently represent a hydrogen atom, a halogen atom, an alkyl group, a haloalkyl group, an alkenyl group, an alkynyl group or $-OR^8$;

$R^8$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a phenylalkyl group, $-COR^6$ or $-SO_2R^7$;

$R^6$ represents a hydrogen atom, an alkyl group, a haloalkyl group, a phenyl group, a phenylalkyl group, an alkoxy group or

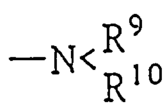 ;

$R^7$ represents an alkyl group, a haloalkyl group, a phenyl group or

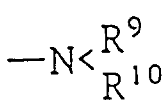 ;

$R^9$ and $R^{10}$ independently represent a hydrogen atom, an alkyl group or a phenyl group;

Y represents an oxygen atom, -S-, -SO-, or $-SO_2-$;

A represents a phenyl group;

B represents

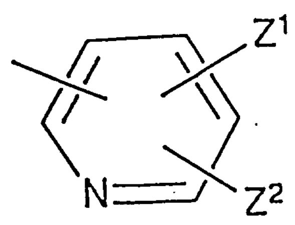
,
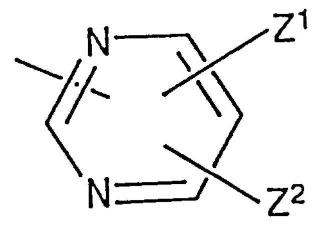
,
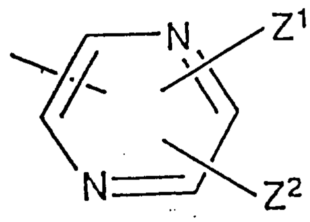
,
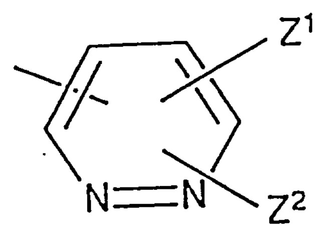
,
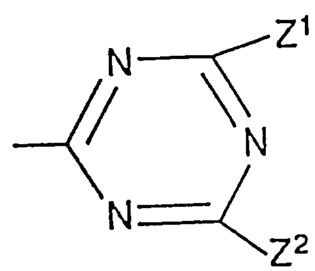
,
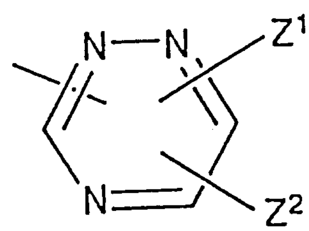

or

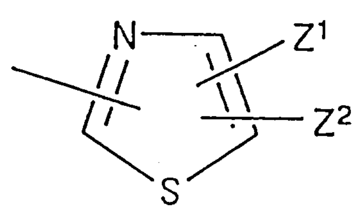
;

$Z^1$ and $Z^2$ independently represent a hydrogen atom, a halogen atom, an alkyl group, an alkoxy group or a haloalkyl group,
wherein the substituted pyrazole derivatives of the general formula [1] include compounds of the formula [1a]

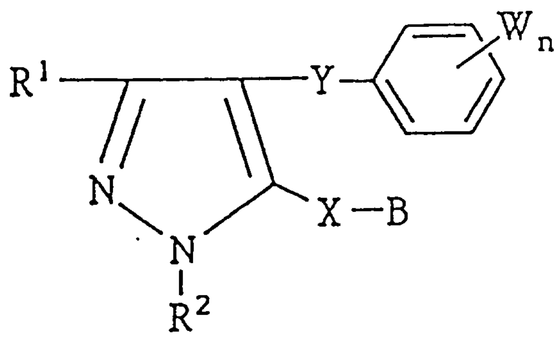

[1a]

having the following meanings of substituents

| $R^1$ | $R^2$ | X | Y | $W_n$ | B |
|---|---|---|---|---|---|
| H | $CH_3$ | S | S | 4-Cl | B 1 |
| $CH_3$ | $CH_3$ | S | S | 4-Cl | B 1 |
| $CF_3$ | $CH_3$ | S | S | 4-Cl | B 1 |
| $CH_3$ | $CH_3$ | S | S | 4-$CH_3$ | B 1 |
| $CF_3$ | $CH_3$ | S | S | 4-$CH_3$ | B 1 |
| $CH_3$ | H | S | S | 4-Cl | B 1 |
| $CH_3$ | H | S | S | 4-$CH_3$ | B 1 |
| $CH_3$ | $CF_3$ | S | S | 4-Cl | B 1 |

| $R^1$ | $R^2$ | X | Y | $W_n$ | B |
|---|---|---|---|---|---|
| $CH_3$ | $CF_3$ | S | S | 4-$CH_3$ | B 1 |
| $CH_3$ | $CH_3$ | S | S | 4-$CF_3$ | B 1 |
| $CH_3$ | $CH_3$ | S | S | 4-Br | B 1 |
| $CH_3$ | $CH_3$ | S | S | 4-$NO_2$ | B 1 |
| $CH_3$ | $CH_3$ | S | S | 4-$OCH_3$ | B 1 |
| $CH_3$ | $CH_3$ | S | S | 4-$C_2H_5$ | B 1 |
| $CH_3$ | $CH_3$ | S | S | 2-Cl | B 1 |
| $CH_3$ | $CH_3$ | S | S | 2-Cl, 4-Cl | B 1 |
| $CH_3$ | $CH_3$ | S | S | 2-Cl, 4-$CH_3$ | B 1 |
| $CH_3$ | $CH_3$ | S | S | 4-Cl | B 2 |
| $CF_3$ | $CH_3$ | S | S | 4-Cl | B 2 |

| $R^1$ | $R^2$ | X | Y | $W_n$ | B |
|---|---|---|---|---|---|
| $CH_3$ | $CH_3$ | S | S | 4-$CH_3$ | B 2 |
| $CF_3$ | $CH_3$ | S | S | 4-$CH_3$ | B 2 |
| $CH_3$ | $CH_3$ | S | S | 2-Cl, 4-Cl | B 2 |
| $CH_3$ | $CH_3$ | S | S | 2-Cl, 4-$CH_3$ | B 2 |
| $CH_3$ | $CH_3$ | S | O | 4-Cl | B 2 |
| $CH_3$ | $CH_3$ | S | O | 4-$CH_3$ | B 2 |
| $CF_3$ | $CH_3$ | S | O | 4-$CH_3$ | B 2 |
| $CH_3$ | $CH_3$ | S | O | 3-Cl, 4-$CH_3$ | B 2 |
| $C_2H_5$ | $CH_3$ | S | S | H | B 1 |
| $C_2H_5$ | $CH_3$ | S | S | 4-Cl | B 1 |

| $R^1$ | $R^2$ | X | Y | $W_n$ | B |
|---|---|---|---|---|---|
| $C_2H_5$ | $CH_3$ | S | S | 4-$CH_3$ | B 1 |
| $C_2H_5$ | $CH_3$ | S | S | 4-$OCH_3$ | B 1 |
| $C_2H_5$ | $CH_3$ | S | S | 3-$CF_3$ | B 1 |
| $C_2H_5$ | $CH_3$ | S | S | 2-Cl, 4-Cl | B 1 |
| $C_2H_5$ | $CH_3$ | S | S | 3-Cl, 4-Cl | B 1 |
| $C_2H_5$ | $CH_3$ | S | S | 2-Cl, 4-$CH_3$ | B 1 |
| i-$C_3H_7$ | $CH_3$ | S | S | 4-Cl | B 1 |
| i-$C_3H_7$ | $CH_3$ | S | S | 4-$CH_3$ | B 1 |
| i-$C_3H_7$ | $CH_3$ | S | S | 4-$OCH_3$ | B 1 |
| t-$C_4H_9$ | $CH_3$ | S | S | 4-Cl | B 1 |

| $R^1$ | $R^2$ | X | Y | $W_n$ | B |
|---|---|---|---|---|---|
| $t-C_4H_9$ | $CH_3$ | S | S | $4-CH_3$ | B 1 |
| $t-C_4H_9$ | $CH_3$ | S | S | $4-OCH_3$ | B 1 |
| $CH_3$ | $C_2H_5$ | S | S | 4-Cl | B 1 |
| $CH_3$ | $C_3H_7$ | S | S | 4-Cl | B 1 |
| $CH_3$ | $CH_3$ | NH | S | H | B 1 |
| $CF_3$ | $CH_3$ | NH | S | H | B 1 |
| $CH_3$ | $CH_3$ | NH | S | 4-Cl | B 1 |
| $CF_3$ | $CH_3$ | NH | S | 4-Cl | B 1 |
| H | $CH_3$ | NH | S | 4-Cl | B 1 |
| $CH_3$ | $CH_3$ | NH | S | $4-CH_3$ | B 1 |

| $R^1$ | $R^2$ | X | Y | $W_n$ | B |
|---|---|---|---|---|---|
| $CF_3$ | $CH_3$ | NH | S | 4-$CH_3$ | B 1 |
| H | $CH_3$ | NH | S | 4-$CH_3$ | B 1 |
| $CH_3$ | $CH_3$ | NH | S | 4-F | B 1 |
| $CH_3$ | $CH_3$ | NH | S | 4-Br | B 1 |
| $CH_3$ | $CH_3$ | NH | S | 4-I | B 1 |
| $CH_3$ | $CH_3$ | NH | S | 2-F | B 1 |
| $CH_3$ | $CH_3$ | NH | S | 2-Cl | B 1 |
| $CH_3$ | $CH_3$ | NH | S | 2-Br | B 1 |
| $CH_3$ | $CH_3$ | NH | S | 2-I | B 1 |
| $CH_3$ | $CH_3$ | NH | S | 3-F | B 1 |

| $R^1$ | $R^2$ | X | Y | $W_n$ | B |
|---|---|---|---|---|---|
| $CH_3$ | $CH_3$ | NH | S | 3-Cl | B 1 |
| $CH_3$ | $CH_3$ | NH | S | 3-Br | B 1 |
| $CH_3$ | $CH_3$ | NH | S | 3-I | B 1 |
| $CH_3$ | $CH_3$ | NH | S | $2-OCH_3$ | B 1 |
| $CH_3$ | $CH_3$ | NH | S | $3-OCH_3$ | B 1 |
| $CH_3$ | $CH_3$ | NH | S | $4-OCH_3$ | B 1 |
| $CH_3$ | $CH_3$ | NH | S | $2-CF_3$ | B 1 |
| $CH_3$ | $CH_3$ | NH | S | $3-CF_3$ | B 1 |
| $CH_3$ | $CH_3$ | NH | S | $4-CF_3$ | B 1 |
| $CH_3$ | $CH_3$ | NH | S | $4-C_2H_5$ | B 1 |

| $R^1$ | $R^2$ | X | Y | $W_n$ | B |
|---|---|---|---|---|---|
| $CH_3$ | $CH_3$ | NH | S | 4-$C_3H_7$ | B 1 |
| $CH_3$ | $CH_3$ | NH | S | 4-$C_4H_9$ | B 1 |
| $CH_3$ | $CH_3$ | NH | S | 4-i-$C_3H_7$ | B 1 |
| $CH_3$ | $CH_3$ | NH | S | 4-t-$C_4H_9$ | B 1 |
| $CH_3$ | $CH_3$ | NH | S | 2-$OCF_3$ | B 1 |
| $CH_3$ | $CH_3$ | NH | S | 3-$OCF_3$ | B 1 |
| $CH_3$ | $CH_3$ | NH | S | 4-$OCF_3$ | B 1 |
| $CH_3$ | $CH_3$ | NH | S | 3-$NO_2$ | B 1 |
| $CH_3$ | $CH_3$ | NH | S | 4-$NO_2$ | B 1 |
| $CH_3$ | $CH_3$ | NH | S | 3-$NH_2$ | B 1 |

| $R^1$ | $R^2$ | X | Y | $W_n$ | B |
|---|---|---|---|---|---|
| $CH_3$ | $CH_3$ | NH | S | 4-$NH_2$ | B1 |
| $CH_3$ | $CH_3$ | NH | S | 4-$NHCOCH_3$ | B1 |
| $CH_3$ | $CH_3$ | NH | S | 4-$NHSO_2CH_3$ | B1 |
| $CH_3$ | $CH_3$ | NH | S | 4-$NHCOCF_3$ | B1 |
| $CH_3$ | $CH_3$ | NH | S | 4-$NHSO_2CF_3$ | B1 |
| $CH_3$ | $CH_3$ | NH | S | 4-Ph | B1 |
| $CH_3$ | $CH_3$ | NH | S | 4-OPh | B1 |
| $CH_3$ | $CH_3$ | NH | S | 4-$OCF_2CF_2H$ | B1 |
| $CH_3$ | $CH_3$ | NH | S | 4-$COCH_3$ | B1 |
| $CH_3$ | $CH_3$ | NH | S | 4-NHCOPh | B1 |

| $R^1$ | $R^2$ | X | Y | $W_n$ | B |
|---|---|---|---|---|---|
| $CH_3$ | $CH_3$ | NH | S | 4-$NHCOOCH_3$ | B 1 |
| $CH_3$ | $CH_3$ | NH | S | 4-$NHCON(CH_3)_2$ | B 1 |
| $CH_3$ | $CH_3$ | NH | S | 2-Cl, 3-Cl | B 1 |
| $CH_3$ | $CH_3$ | NH | S | 2-Cl, 4-Cl | B 1 |
| $CH_3$ | $CH_3$ | NH | S | 2-Cl, 5-Cl | B 1 |
| $CH_3$ | $CH_3$ | NH | S | 2-Cl, 6-Cl | B 1 |
| $CH_3$ | $CH_3$ | NH | S | 3-Cl, 4-Cl | B 1 |
| $CH_3$ | $CH_3$ | NH | S | 3-Cl, 5-Cl | B 1 |
| $CH_3$ | $CH_3$ | NH | S | 2-$CH_3$, 3-$CH_3$ | B 1 |
| $CH_3$ | $CH_3$ | NH | S | 2-$CH_3$, 4-$CH_3$ | B 1 |

| $R^1$ | $R^2$ | X | Y | $W_n$ | B |
|---|---|---|---|---|---|
| $CH_3$ | $CH_3$ | NH | S | 2-$CH_3$, 5-$CH_3$ | B 1 |
| $CH_3$ | $CH_3$ | NH | S | 2-$CH_3$, 6-$CH_3$ | B 1 |
| $CH_3$ | $CH_3$ | NH | S | 3-$CH_3$, 4-$CH_3$ | B 1 |
| $CH_3$ | $CH_3$ | NH | S | 3-$CH_3$, 5-$CH_3$ | B 1 |
| $CH_3$ | $CH_3$ | NH | S | 2-Cl, 3-$CH_3$ | B 1 |
| $CH_3$ | $CH_3$ | NH | S | 2-Cl, 4-$CH_3$ | B 1 |
| $CH_3$ | $CH_3$ | NH | S | 2-Cl, 5-$CH_3$ | B 1 |
| $CH_3$ | $CH_3$ | NH | S | 3-Cl, 4-$CH_3$ | B 1 |
| $CH_3$ | $CH_3$ | NH | S | 3-Cl, 5-$CH_3$ | B 1 |
| $CH_3$ | $CH_3$ | NH | S | 2-$CH_3$, 3-Cl | B 1 |

| $R^1$ | $R^2$ | X | Y | $W_n$ | B |
|---|---|---|---|---|---|
| $CH_3$ | $CH_3$ | NH | S | 2-$CH_3$, 4-Cl | B 1 |
| $CH_3$ | $CH_3$ | NH | S | 2-$CH_3$, 5-Cl | B 1 |
| $CH_3$ | $CH_3$ | NH | S | 2-F, 3-F | B 1 |
| $CH_3$ | $CH_3$ | NH | S | 2-F, 4-F | B 1 |
| $CH_3$ | $CH_3$ | NH | S | 2-F, 5-F | B 1 |
| $CH_3$ | $CH_3$ | NH | S | 2-F, 6-F | B 1 |
| $CH_3$ | $CH_3$ | NH | S | 2-F, 3-Cl | B 1 |
| $CH_3$ | $CH_3$ | NH | S | 2-F, 4-Cl | B 1 |
| $CH_3$ | $CH_3$ | NH | S | 2-F, 5-Cl | B 1 |
| $CH_3$ | $CH_3$ | NH | S | 2-F, 6-Cl | B 1 |

| $R^1$ | $R^2$ | X | Y | $W_n$ | B |
|---|---|---|---|---|---|
| $CH_3$ | $CH_3$ | NH | S | 2-F, 3-$CH_3$ | B 1 |
| $CH_3$ | $CH_3$ | NH | S | 2-F, 4-$CH_3$ | B 1 |
| $CH_3$ | $CH_3$ | NH | S | 2-F, 3-Br | B 1 |
| $CH_3$ | $CH_3$ | NH | S | 2-F, 4-Br | B 1 |
| $CH_3$ | $CH_3$ | NH | S | 3-F, 4-Cl | B 1 |
| $CH_3$ | $CH_3$ | NH | S | 3-F, 5-Cl | B 1 |
| $CH_3$ | $CH_3$ | NH | S | 3-F, 4-$CH_3$ | B 1 |
| $CH_3$ | $CH_3$ | NH | S | 3-F, 5-$CH_3$ | B 1 |
| $CH_3$ | $CH_3$ | NH | S | 2-Br, 4-Cl | B 1 |
| $CH_3$ | $CH_3$ | NH | S | 2-Br, 4-Br | B 1 |

| $R^1$ | $R^2$ | X | Y | $W_n$ | B |
|---|---|---|---|---|---|
| $CH_3$ | $CH_3$ | NH | S | 2-Br, 4-$CH_3$ | B 1 |
| $CH_3$ | $CH_3$ | NH | S | 3-Br, 4-Cl | B 1 |
| $CH_3$ | $CH_3$ | NH | S | 3-Br, 4-Br | B 1 |
| $CH_3$ | $CH_3$ | NH | S | 3-Br, 4-$CH_3$ | B 1 |
| $CH_3$ | $CH_3$ | NH | S | 2-Cl, 4-Br | B 1 |
| $CH_3$ | $CH_3$ | NH | S | 3-Cl, 4-Br | B 1 |
| $CH_3$ | $CH_3$ | NH | S | 2-Cl, 4-I | B 1 |
| $CH_3$ | $CH_3$ | NH | S | 3-Cl, 4-I | B 1 |
| $CH_3$ | $CH_3$ | NH | S | 3-F, 4-Br | B 1 |
| $CH_3$ | $CH_3$ | NH | S | 3, 4-$OCH_2O$- | B 1 |

| $R^1$ | $R^2$ | X | Y | $W_n$ | B |
|---|---|---|---|---|---|
| $CH_3$ | $CH_3$ | NH | S | 2-$CF_3$, 3-Cl | B 1 |
| $CH_3$ | $CH_3$ | NH | S | 2-$CF_3$, 4-Cl | B 1 |
| $CH_3$ | $CH_3$ | NH | S | 2-$CF_3$, 3-Br | B 1 |
| $CH_3$ | $CH_3$ | NH | S | 2-$CF_3$, 4-Br | B 1 |
| $CH_3$ | $CH_3$ | NH | S | 2-F, 4-$NO_2$ | B 1 |
| $CH_3$ | $CH_3$ | NH | S | 3-F, 4-$NO_2$ | B 1 |
| $CH_3$ | $CH_3$ | NH | S | 2-Cl, 4-$NO_2$ | B 1 |
| $CH_3$ | $CH_3$ | NH | S | 3-Cl, 4-$NO_2$ | B 1 |
| $CH_3$ | $CH_3$ | NH | S | 2-F, 4-$OCH_3$ | B 1 |
| $CH_3$ | $CH_3$ | NH | S | 3-F, 4-$OCH_3$ | B 1 |

| $R^1$ | $R^2$ | X | Y | $W_n$ | B |
|---|---|---|---|---|---|
| $CH_3$ | $CH_3$ | NH | S | 2-Cl, 4-$OCH_3$ | B 1 |
| $CH_3$ | $CH_3$ | NH | S | 3-Cl, 4-$OCH_3$ | B 1 |
| $CH_3$ | $CH_3$ | NH | S | 2-$OCH_3$, 4-$OCH_3$ | B 1 |
| $CH_3$ | $CH_3$ | NH | S | 3-$OCH_3$, 4-$OCH_3$ | B 1 |
| $CH_3$ | $CH_3$ | NH | S | 2-Cl, 3-Cl, 4-Cl | B 1 |
| $CH_3$ | $CH_3$ | NH | S | 2-Cl, 4-Cl, 5-Cl | B 1 |
| $CH_3$ | $CH_3$ | NH | S | 2-Cl, 4-Cl, 6-Cl | B 1 |
| $CH_3$ | $CH_3$ | NH | S | 3-Cl, 4-Cl, 5-Cl | B 1 |
| $CH_3$ | $CH_3$ | NH | S | 2-Cl, 3-Cl, 4-$CH_3$ | B 1 |
| $CH_3$ | $CH_3$ | NH | S | 2-Cl, 3-Cl, 4-Br | B 1 |

| $R^1$ | $R^2$ | X | Y | $W_n$ | B |
|---|---|---|---|---|---|
| $CH_3$ | $CH_3$ | NH | S | 2-Cl, 3-Cl, 4-I | B 1 |
| $CH_3$ | $CH_3$ | NH | S | 2-Cl, 4-Br, 6-Cl | B 1 |
| $CH_3$ | $CH_3$ | NH | S | 2-Cl, 4-$CH_3$, 6-Cl | B 1 |
| $CH_3$ | $CH_3$ | NH | S | 2-Br, 4-Cl, 6-Cl | B 1 |
| $CH_3$ | $CH_3$ | NCHO | S | 4-Cl | B 1 |
| $CH_3$ | $CH_3$ | $NCOCH_3$ | S | 4-Cl | B 1 |
| $CH_3$ | $CH_3$ | $NCOOCH_3$ | S | 4-Cl | B 1 |
| $CH_3$ | $CH_3$ | $NCON(CH_3)_2$ | S | 4-Cl | B 1 |
| $CH_3$ | $CH_3$ | NCONHPh | S | 4-Cl | B 1 |
| $CH_3$ | $CH_3$ | $NCH_3$ | S | 4-Cl | B 1 |

| $R^1$ | $R^2$ | X | Y | $W_n$ | B |
|---|---|---|---|---|---|
| $CH_3$ | $CH_3$ | $NC_2H_5$ | S | 4-Cl | B 1 |
| $CH_3$ | $CH_3$ | $NC_3H_7$ | S | 4-Cl | B 1 |
| $CH_3$ | $CH_3$ | $NCH_2OCH_3$ | S | 4-Cl | B 1 |
| $CH_3$ | $CH_3$ | $NCH_2CH_2OCH_3$ | S | 4-Cl | B 1 |
| $CH_3$ | $CH_3$ | $NCH_2CH=CH_2$ | S | 4-Cl | B 1 |
| $CH_3$ | $CH_3$ | $NCH_2C\equiv CH$ | S | 4-Cl | B 1 |
| $CH_3$ | $CH_3$ | $NCH_2COCH_3$ | S | 4-Cl | B 1 |
| $CH_3$ | $CH_3$ | $NCH_2COOCH_3$ | S | 4-Cl | B 1 |
| $CH_3$ | $CH_3$ | $NCH_2COOC_2H_5$ | S | 4-Cl | B 1 |
| $CH_3$ | $CH_3$ | $NCH_2CN$ | S | 4-Cl | B 1 |

| $R^1$ | $R^2$ | X | Y | $W_n$ | B |
|---|---|---|---|---|---|
| $CH_3$ | $CH_3$ | $NCH_2Ph$ | S | 4-Cl | B 1 |
| $CH_3$ | $CH_3$ | $NCH_2C_6H_4$-4-Cl | S | 4-Cl | B 1 |
| $CH_3$ | $CH_3$ | $NCH_2C_6H_4$-4-$CH_3$ | S | 4-Cl | B 1 |
| $CH_3$ | $CH_3$ | $NCH_2CH_2Ph$ | S | 4-Cl | B 1 |
| $CH_3$ | $CH_3$ | $NSO_2CH_3$ | S | 4-Cl | B 1 |
| $CH_3$ | $CH_3$ | $NSO_2N(CH_3)_2$ | S | 4-Cl | B 1 |
| $CH_3$ | $CH_3$ | NCHO | S | 4-Cl | B 2 |
| $CH_3$ | $CH_3$ | $NCH_3$ | S | 4-Cl | B 2 |
| $CH_3$ | H | NH | S | 4-Cl | B 1 |
| $CH_3$ | H | NH | S | 2-Cl, 4-Cl | B 1 |

| $R^1$ | $R^2$ | X | Y | $W_n$ | B |
|---|---|---|---|---|---|
| $CH_3$ | H | NH | S | 3-Cl, 4-$CH_3$ | B 1 |
| $CH_3$ | $C_2H_5$ | NH | S | 4-Cl | B 1 |
| $CH_3$ | $C_2H_5$ | NH | S | 4-Br | B 1 |
| $CH_3$ | $C_2H_5$ | NH | S | 2-Cl, 4-Cl | B 1 |
| $CH_3$ | $C_2H_5$ | NH | S | 2-Cl, 4-$CH_3$ | B 1 |
| $CH_3$ | i-$C_3H_7$ | NH | S | 4-Cl | B 1 |
| $CH_3$ | i-$C_3H_7$ | NH | S | 4-Br | B 1 |
| $CH_3$ | i-$C_3H_7$ | NH | S | 2-Cl, 4-Cl | B 1 |
| $CH_3$ | i-$C_3H_7$ | NH | S | 2-Cl, 4-$CH_3$ | B 1 |
| $CH_3$ | t-$C_4H_9$ | NH | S | 4-Cl | B 1 |

| $R^1$ | $R^2$ | X | Y | $W_n$ | B |
|---|---|---|---|---|---|
| $CH_3$ | $t-C_4H_9$ | NH | S | 2-Cl, 4-Cl | B 1 |
| $CH_3$ | $t-C_4H_9$ | NH | S | 3-Cl, 4-$CH_3$ | B 1 |
| $CH_3$ | $CF_3$ | NH | S | 4-Cl | B 1 |
| $CH_3$ | $CF_3$ | NH | S | 4-Br | B 1 |
| $CH_3$ | $CF_3$ | NH | S | 2-Cl, 4-Cl | B 1 |
| $CH_3$ | $CF_3$ | NH | S | 3-Cl, 4-$CH_3$ | B 1 |
| $CH_3$ | H | NH | S | 4-Cl | B 2 |
| $CH_3$ | H | NH | S | 2-Cl, 4-Cl | B 2 |
| $CH_3$ | $C_2H_5$ | NH | S | 4-Cl | B 2 |
| $CH_3$ | $i-C_3H_7$ | NH | S | 4-Cl | B 2 |

| $R^1$ | $R^2$ | X | Y | $W_n$ | B |
|---|---|---|---|---|---|
| $CH_3$ | $t-C_4H_9$ | NH | S | 4-Cl | B 2 |
| $CH_3$ | $CF_3$ | NH | S | 2-Cl, 4-Cl | B 2 |
| $CH_3$ | $CH_2Ph$ | NH | S | 4-Cl | B 1 |
| $CH_3$ | $CH_2Ph$ | NH | S | 2-Cl, 4-Cl | B 1 |
| $CH_3$ | $CH_2Ph$ | NCHO | S | 2-Cl, 4-Cl | B 1 |
| $CH_3$ | CHO | NH | S | 4-Cl | B 1 |
| $CH_3$ | CHO | NCHO | S | 4-Cl | B 1 |
| $CH_3$ | CHO | NH | S | 2-Cl, 4-Cl | B 1 |
| $CH_3$ | CHO | NCHO | S | 2-Cl, 4-Cl | B 1 |
| $CH_3$ | $COCH_3$ | NH | S | 4-Cl | B 1 |

| $R^1$ | $R^2$ | X | Y | $W_n$ | B |
|---|---|---|---|---|---|
| $CH_3$ | $COCH_3$ | NH | S | 4-Br | B 1 |
| $CH_3$ | $COCH_3$ | NH | S | 2-Cl, 4-Cl | B 1 |
| $CH_3$ | $COCH_3$ | NCHO | S | 2-Cl, 4-Cl | B 1 |
| $CH_3$ | $SO_2CH_3$ | NH | S | 4-Cl | B 1 |
| $CH_3$ | $SO_2CH_3$ | NH | S | 2-Cl, 4-Cl | B 1 |
| $CH_3$ | $SO_2CH_3$ | NH | S | 3-Cl, 4-$CH_3$ | B 1 |
| $CH_3$ | $CONHCH_3$ | NH | S | 4-Cl | B 1 |
| $CH_3$ | $CONHCH_3$ | NH | S | 4-Br | B 1 |
| $CH_3$ | $CONHCH_3$ | NH | S | 2-Cl, 4-Cl | B 1 |
| $CH_3$ | $CONHCH_3$ | NH | S | 3-Cl, 4-$CH_3$ | B 1 |

| $R^1$ | $R^2$ | X | Y | $W_n$ | B |
|---|---|---|---|---|---|
| $CH_3$ | $CH_2Ph$ | NH | S | 4-Cl | B2 |
| $CH_3$ | $CH_2Ph$ | NH | S | 2-Cl, 4-Cl | B2 |
| $CH_3$ | $COCH_3$ | NH | S | 4-Cl | B2 |
| $CH_3$ | $CONHCH_3$ | NH | S | 4-Cl | B2 |
| $CH_3$ | $SO_2CH_3$ | NH | S | 4-Cl | B2 |
| $CH_3$ | $SO_2CH_3$ | NH | S | 2-Cl, 4-Cl | B2 |
| H | $CH_3$ | NH | S | 4-Cl | B1 |
| H | $CH_3$ | NH | S | 2-Cl, 4-Cl | B1 |
| H | $CH_3$ | NH | S | 3-Cl, 4-$CH_3$ | B1 |
| $CF_3$ | $CH_3$ | NH | S | 4-Cl | B1 |

| $R^1$ | $R^2$ | X | Y | $W_n$ | B |
|---|---|---|---|---|---|
| $CF_3$ | $CH_3$ | NH | S | 4-Br | B 1 |
| $CF_3$ | $CH_3$ | NH | S | 2-Cl, 4-Cl | B 1 |
| $CF_3$ | $CH_3$ | NH | S | 2-Cl, 4-$CH_3$ | B 1 |
| $C_2H_5$ | $CH_3$ | NH | S | 4-Cl | B 1 |
| $C_2H_5$ | $CH_3$ | NH | S | 4-Br | B 1 |
| $C_2H_5$ | $CH_3$ | NH | S | 2-Cl, 4-Cl | B 1 |
| $C_2H_5$ | $CH_3$ | NH | S | 2-Cl, 4-$CH_3$ | B 1 |
| i-$C_3H_7$ | $CH_3$ | NH | S | 4-Cl | B 1 |
| i-$C_3H_7$ | $CH_3$ | NH | S | 2-Cl, 4-Cl | B 1 |
| i-$C_3H_7$ | $CH_3$ | NH | S | 3-Cl, 4-$CH_3$ | B 1 |

| $R^1$ | $R^2$ | X | Y | $W_n$ | B |
|---|---|---|---|---|---|
| $t-C_4H_9$ | $CH_3$ | NH | S | 4-Cl | B1 |
| $t-C_4H_9$ | $CH_3$ | NH | S | 4-Br | B1 |
| $t-C_4H_9$ | $CH_3$ | NH | S | 2-Cl, 4-Cl | B1 |
| $t-C_4H_9$ | $CH_3$ | NH | S | 3-Cl, 4-$CH_3$ | B1 |
| H | $CH_3$ | NH | S | 4-Cl | B2 |
| H | $CH_3$ | NH | S | 2-Cl, 4-Cl | B2 |
| $C_2H_5$ | $CH_3$ | NH | S | 4-Cl | B2 |
| $i-C_3H_7$ | $CH_3$ | NH | S | 4-Cl | B2 |
| $CF_3$ | $CH_3$ | NH | S | 4-Cl | B2 |
| $CF_3$ | $CH_3$ | NH | S | 2-Cl, 4-Cl | B2 |

| $R^1$ | $R^2$ | X | Y | $W_n$ | B |
|---|---|---|---|---|---|
| Cl | $CH_3$ | NH | S | 4-Cl | B1 |
| Cl | $CH_3$ | NH | S | 2-Cl, 4-Cl | B1 |
| Cl | $CH_3$ | NH | S | 3-Cl, 4-$CH_3$ | B1 |
| Cl | $CH_3$ | NCHO | S | 4-Cl | B1 |
| Cl | $CH_3$ | NH | S | 4-Br | B1 |
| Cl | $CH_3$ | NCHO | S | 2-Cl, 4-Cl | B1 |
| Cl | $CH_3$ | NCHO | S | 2-Cl, 4-$CH_3$ | B1 |
| $CH_3O$ | $CH_3$ | NH | S | 4-Cl | B1 |
| $CH_3O$ | $CH_3$ | NH | S | 4-Br | B1 |
| $CH_3O$ | $CH_3$ | NH | S | 2-Cl, 4-Cl | B1 |

| $R^1$ | $R^2$ | X | Y | $W_n$ | B |
|---|---|---|---|---|---|
| $CH_3O$ | $CH_3$ | NH | S | 2-Cl, 4-$CH_3$ | B 1 |
| $CH_3O$ | $CH_3$ | NCHO | S | 4-Cl | B 1 |
| $CH_3O$ | $CH_3$ | NCHO | S | 2-Cl, 4-Cl | B 1 |
| $CH_3O$ | $CH_3$ | NCHO | S | 3-Cl, 4-$CH_3$ | B 1 |
| $CH_3S$ | $CH_3$ | NH | S | 4-Cl | B 1 |
| $CH_3S$ | $CH_3$ | NH | S | 4-Br | B 1 |
| $CH_3S$ | $CH_3$ | NH | S | 2-Cl, 4-Cl | B 1 |
| $CH_3S$ | $CH_3$ | NH | S | 3-Cl, 4-$CH_3$ | B 1 |
| $CH_3S$ | $CH_3$ | NCHO | S | 4-Cl | B 1 |
| $CH_3S$ | $CH_3$ | NCHO | S | 2-Cl, 4-Cl | B 1 |

| $R^1$ | $R^2$ | X | Y | $W_n$ | B |
|---|---|---|---|---|---|
| Cl | $CH_3$ | NH | S | 4-Cl | B 2 |
| Cl | $CH_3$ | NH | S | 2-Cl, 4-Cl | B 2 |
| $CH_3O$ | $CH_3$ | NH | S | 2-Cl, 4-Cl | B 2 |
| $CH_3S$ | $CH_3$ | NH | S | 2-Cl, 4-Cl | B 2 |
| $CH_3$ | $CH_3$ | SO | S | 4-Cl | B 1 |
| $CH_3$ | $CH_3$ | $SO_2$ | S | 4-Cl | B 1 |
| $CH_3$ | $CH_3$ | NH | SO | 4-Cl | B 1 |
| $CH_3$ | $CH_3$ | NH | $SO_2$ | 4-Cl | B 1 |
| $CH_3$ | $CH_3$ | NH | SO | 2-Cl, 4-Cl | B 1 |
| $CH_3$ | $CH_3$ | NH | $SO_2$ | 2-Cl, 4-Cl | B 1 |

| $R^1$ | $R^2$ | X | Y | $W_n$ | B |
|---|---|---|---|---|---|
| $CH_3$ | $CH_3$ | NH | SO | 3-Cl, 4-$CH_3$ | B 1 |
| $CH_3$ | $CH_3$ | NH | $SO_2$ | 3-Cl, 4-$CH_3$ | B 1 |
| $CH_3$ | $CH_3$ | S | O | 4-Cl | B 1 |
| $CH_3$ | $CH_3$ | SO | O | 4-Cl | B 1 |
| $CH_3$ | $CH_3$ | $SO_2$ | O | 4-Cl | B 1 |
| $CH_3$ | $CH_3$ | S | O | 2-Cl, 4-Cl | B 1 |
| $CH_3$ | $CH_3$ | SO | O | 2-Cl, 4-Cl | B 1 |
| $CH_3$ | $CH_3$ | $SO_2$ | O | 2-Cl, 4-Cl | B 1 |
| $CH_3$ | $CH_3$ | NH | O | 4-Cl | B 1 |
| $CH_3$ | $CH_3$ | NH | O | 4-$CH_3$ | B 1 |

| $R^1$ | $R^2$ | X | Y | $W_n$ | B |
|---|---|---|---|---|---|
| $CH_3$ | $CH_3$ | NH | O | 2-Cl, 4-Cl | B1 |
| $CH_3$ | $CH_3$ | NH | O | 2-Cl, 4-$CH_3$ | B1 |
| $CH_3$ | $CH_3$ | NH | O | 3-Cl, 4-$CH_3$ | B1 |
| $CH_3$ | $CH_3$ | NH | O | 2-F, 4-Cl | B1 |
| $CH_3$ | $CH_3$ | NCHO | O | 4-Cl | B1 |
| $CH_3$ | $CH_3$ | $NCH_3$ | O | 4-Cl | B1 |
| $CH_3$ | $CH_3$ | NH | O | 4-Cl | B2 |
| $CH_3$ | $CH_3$ | NH | O | 2-Cl, 4-Cl | B2 |
| $CH_3$ | $CH_3$ | NH | S | 4-F | B2 |
| $CH_3$ | $CH_3$ | NH | S | 4-Cl | B2 |

| $R^1$ | $R^2$ | X | Y | $W_n$ | B |
|---|---|---|---|---|---|
| $CH_3$ | $CH_3$ | NH | S | 4-$CH_3$ | B 2 |
| $CH_3$ | $CH_3$ | NH | S | 2-Cl, 4-Cl | B 2 |
| $CH_3$ | $CH_3$ | NH | S | 3-Cl, 4-Cl | B 2 |
| $CH_3$ | $CH_3$ | NH | S | 2-Cl, 4-$CH_3$ | B 2 |
| $CH_3$ | $CH_3$ | NH | S | 3-Cl, 4-$CH_3$ | B 2 |
| $CH_3$ | $CH_3$ | NH | S | 2-F, 4-F | B 2 |
| $CH_3$ | $CH_3$ | NH | S | 2-F, 4-Cl | B 2 |
| $CH_3$ | $CH_3$ | NH | S | 2-F, 4-Br | B 2 |
| $CH_3$ | $CH_3$ | NH | S | 2-F, 4-$CH_3$ | B 2 |
| $CH_3$ | $CH_3$ | NH | S | 3-F, 4-$CH_3$ | B 2 |

| $R^1$ | $R^2$ | X | Y | $W_n$ | B |
|---|---|---|---|---|---|
| $CH_3$ | $CH_3$ | NCHO | S | 4-Cl | B2 |
| $CH_3$ | $CH_3$ | NCHO | S | 2-Cl, 4-Cl | B2 |
| $CH_3$ | $CH_3$ | NCHO | S | 3-Cl, 4-$CH_3$ | B2 |
| $CH_3$ | $CH_3$ | NH | S | 4-Cl | B3 |
| $CH_3$ | $CH_3$ | NH | S | 4-Br | B3 |
| $CH_3$ | $CH_3$ | NH | S | 2-Cl, 4-Cl | B3 |
| $CH_3$ | $CH_3$ | NH | S | 2-Cl, 4-$CH_3$ | B3 |
| $CH_3$ | $CH_3$ | NH | S | 3-Cl, 4-$CH_3$ | B3 |
| $CH_3$ | $CH_3$ | NH | S | 4-Cl | B4 |
| $CH_3$ | $CH_3$ | NH | S | 2-Cl, 4-Cl | B4 |

| $R^1$ | $R^2$ | X | Y | $W_n$ | B |
|---|---|---|---|---|---|
| $CH_3$ | $CH_3$ | NH | S | 4-Cl | B5 |
| $CH_3$ | $CH_3$ | NH | S | 2-Cl, 4-Cl | B5 |
| $CH_3$ | $CH_3$ | NH | S | 4-Cl | B6 |
| $CH_3$ | $CH_3$ | NH | S | 2-Cl, 4-Cl | B6 |
| $CH_3$ | $CH_3$ | NH | S | 4-Cl | B7 |
| $CH_3$ | $CH_3$ | NH | S | 2-Cl, 4-$CH_3$ | B7 |
| $CH_3$ | $CH_3$ | NH | S | 4-Cl | B8 |
| $CH_3$ | $CH_3$ | NH | S | 2-Cl, 4-Cl | B8 |
| $CH_3$ | $CH_3$ | NH | S | 4-Cl | B9 |
| $CH_3$ | $CH_3$ | NH | S | 2-Cl, 4-$CH_3$ | B9 |

| $R^1$ | $R^2$ | X | Y | $W_n$ | B |
|---|---|---|---|---|---|
| $CH_3$ | $CH_3$ | NH | S | 4-Cl | B10 |
| $CH_3$ | $CH_3$ | NH | S | 2-Cl, 4-Cl | B10 |
| $CH_3$ | $CH_3$ | NH | S | 3-Cl, 4-Cl | B10 |
| $CH_3$ | $CH_3$ | NH | S | 2-F, 4-$CH_3$ | B10 |
| $CH_3$ | $CH_3$ | NH | S | 3-F, 4-$CH_3$ | B10 |
| $CH_3$ | $CH_3$ | NH | S | 2-F, 4-Cl | B10 |
| $CH_3$ | $CH_3$ | NH | S | 4-Cl | B11 |
| $CH_3$ | $CH_3$ | NH | S | 2-Cl, 4-$CH_3$ | B11 |
| $CH_3$ | $CH_3$ | NH | S | 4-Cl | B12 |
| $CH_3$ | $CH_3$ | NH | S | 2-Cl, 4-Cl | B12 |

| $R^1$ | $R^2$ | X | Y | $W_n$ | B |
|---|---|---|---|---|---|
| $CH_3$ | $CH_3$ | NH | S | 2-F, 4-$CH_3$ | B12 |
| $CH_3$ | $CH_3$ | NH | S | 3-F, 4-$CH_3$ | B12 |
| $CH_3$ | $CH_3$ | NH | S | 2-Cl, 4-$CH_3$ | B12 |
| $CH_3$ | $CH_3$ | NH | S | 3-Cl, 4-$CH_3$ | B12 |
| $CH_3$ | $CH_3$ | NCHO | S | 4-Cl | B12 |
| $CH_3$ | $CH_3$ | NCHO | S | 2-Cl, 4-Cl | B12 |
| $CH_3$ | $CH_3$ | NH | S | 4-Cl | B13 |
| $CH_3$ | $CH_3$ | NH | S | 2-Cl, 4-Cl | B13 |
| $CH_3$ | $CH_3$ | NH | S | 2-Cl, 4-$CH_3$ | B13 |

| $R^1$ | $R^2$ | X | Y | $W_n$ | B |
|---|---|---|---|---|---|
| $CH_3$ | $CH_3$ | NH | S | 2-F, 4-$CH_3$ | B 1 3 |
| $CH_3$ | $CH_3$ | NH | S | 4-Cl | B 1 4 |
| $CH_3$ | $CH_3$ | NH | S | 2-Cl, 4-Cl | B 1 4 |
| $CH_3$ | $CH_3$ | NH | S | 2-Cl, 4-$CH_3$ | B 1 4 |
| $CH_3$ | $CH_3$ | NH | S | 2-F, 4-$CH_3$ | B 1 4 |
| $CH_3$ | $CH_3$ | NH | S | 4-Cl | B 1 5 |
| $CH_3$ | $CH_3$ | NH | S | 2-Cl, 4-Cl | B 1 5 |
| $CH_3$ | $CH_3$ | NH | S | 2-Cl, 4-$CH_3$ | B 1 5 |
| $CH_3$ | $CH_3$ | NH | S | 2-F, 4-$CH_3$ | B 1 5 |
| $CH_3$ | $CH_3$ | NH | S | 4-Cl | B 1 6 |

| $R^1$ | $R^2$ | X | Y | $W_n$ | B |
|---|---|---|---|---|---|
| $CH_3$ | $CH_3$ | NH | S | 2-Cl, 4-Cl | B 16 |
| $CH_3$ | $CH_3$ | NH | S | 2-Cl, 4-$CH_3$ | B 16 |
| $CH_3$ | $CH_3$ | NH | S | 2-F, 4-$CH_3$ | B 16 |
| $CH_3$ | $CH_3$ | NH | S | 4-Cl | B 17 |
| $CH_3$ | $CH_3$ | NH | S | 2-Cl, 4-Cl | B 17 |
| $CH_3$ | $CH_3$ | NH | S | 2-Cl, 4-$CH_3$ | B 17 |
| $CH_3$ | $CH_3$ | NH | S | 2-F, 4-$CH_3$ | B 17 |
| $CH_3$ | $CH_3$ | NH | S | 4-Cl | B 18 |
| $CH_3$ | $CH_3$ | NH | S | 2-Cl, 4-Cl | B 18 |
| $CH_3$ | $CH_3$ | NH | S | 2-Cl, 4-$CH_3$ | B 18 |

| $R^1$ | $R^2$ | X | Y | $W_n$ | B |
|---|---|---|---|---|---|
| $CH_3$ | $CH_3$ | NH | S | 2-F, 4-$CH_3$ | B 1 8 |
| $CH_3$ | $CH_3$ | NCHO | S | 2-Cl, 4-Cl | B 1 |
| $CH_3$ | $CH_3$ | NCHO | S | 3-Cl, 4-Cl | B 1 |
| $CH_3$ | $CH_3$ | NCHO | S | 2-F, 4-Cl | B 1 |
| $CH_3$ | $CH_3$ | NCHO | S | 2-F, 4-$CH_3$ | B 1 |
| $CH_3$ | $CH_3$ | NCHO | S | 3-F, 4-Cl | B 1 |
| $CH_3$ | $CH_3$ | NCHO | S | 3-F, 4-$CH_3$ | B 1 |
| $CH_3$ | $CH_3$ | NCHO | S | 2-Cl, 4-$CH_3$ | B 1 |
| $CH_3$ | $CH_3$ | NCHO | S | 3-Cl, 4-$CH_3$ | B 1 |
| $CH_3$ | $CH_3$ | NCHO | S | 2-Cl, 3-Cl, 4-Cl | B 1 |

| $R^1$ | $R^2$ | X | Y | $W_n$ | B |
|---|---|---|---|---|---|
| $CH_3$ | $CH_3$ | NCHO | S | 2-Cl, 4-Cl, 5-Cl | B 1 |
| $CH_3$ | $CH_3$ | $NCOCH_3$ | S | 2-Cl, 4-Cl | B 1 |
| $CH_3$ | $CH_3$ | $NCOCH_3$ | S | 3-Cl, 4-Cl | B 1 |
| $CH_3$ | $CH_3$ | $NCOCH_3$ | S | 2-F, 4-$CH_3$ | B 1 |
| $CH_3$ | $CH_3$ | $NCOCH_3$ | S | 2-Cl, 3-Cl, 4-Cl | B 1 |
| $CH_3$ | $CH_3$ | $NCH_3$ | S | 2-Cl, 4-Cl | B 1 |
| $CH_3$ | $CH_3$ | $NCH_3$ | S | 3-Cl, 4-$CH_3$ | B 1 |
| $CH_3$ | $CH_3$ | NNO | S | 2-Cl, 4-Cl | B 1 |
| $CH_3$ | $CH_3$ | NNO | S | 3-Cl, 4-$CH_3$ | B 1 |
| $CH_3$ | $CH_3$ | $NNH_2$ | S | 4-Cl | B 1 |

| $R^1$ | $R^2$ | X | Y | $W_n$ | B |
|---|---|---|---|---|---|
| $CH_3$ | $CH_3$ | $NNH_2$ | S | 2-Cl, 4-Cl | B1 |
| $CH_3$ | $CH_3$ | $NNH_2$ | S | 3-Cl, 4-$CH_3$ | B1 |
| $CH_3$ | $CH_3$ | $NSO_2CH_3$ | S | 2-Cl, 4-Cl | B1 |
| $CH_3$ | $CH_3$ | $NSO_2CH_3$ | S | 2-F, 4-$CH_3$ | B1 |
| $CH_3$ | $CH_3$ | $NSO_2CH_3$ | S | 2-Cl, 3-Cl, 4-Cl | B1 |
| $CH_3$ | $CH_3$ | $CH_2$ | S | 4-Cl | B1 |
| $CH_3$ | $CH_3$ | $CH_2$ | S | 2-Cl, 4-Cl | B1 |
| $CH_3$ | $CH_3$ | $CH_2$ | S | 2-Cl, 4-$CH_3$ | B1 |
| $CH_3$ | $CH_3$ | $CH_2$ | S | 2-F, 4-$CH_3$ | B1 |
| $CH_3$ | $CH_3$ | CH(OH) | S | 4-Cl | B1 |

| $R^1$ | $R^2$ | X | Y | $W_n$ | B |
|---|---|---|---|---|---|
| $CH_3$ | $CH_3$ | CH(OH) | S | 2-Cl, 4-Cl | B 1 |
| $CH_3$ | $CH_3$ | CH(OH) | S | 2-Cl, 4-$CH_3$ | B 1 |
| $CH_3$ | $CH_3$ | CH(OH) | S | 3-Cl, 4-$CH_3$ | B 1 |
| $CH_3$ | $CH_3$ | CH(OH) | S | 2-F, 4-$CH_3$ | B 1 |
| $CH_3$ | $CH_3$ | CH(OH) | S | 3-F, 4-$CH_3$ | B 1 |
| $CH_3$ | $CH_3$ | CH($OCH_3$) | S | 4-Cl | B 1 |
| $CH_3$ | $CH_3$ | CH($OCH_3$) | S | 2-Cl, 4-Cl | B 1 |
| $CH_3$ | $CH_3$ | CH($OCOCH_3$) | S | 4-Cl | B 1 |
| $CH_3$ | $CH_3$ | CH($OCOCH_3$) | S | 2-Cl, 4-Cl | B 1 |
| $CH_3$ | $CH_3$ | CH($OCOCH_3$) | S | 2-Cl, 4-$CH_3$ | B 1 |

| $R^1$ | $R^2$ | X | Y | $W_n$ | B |
|---|---|---|---|---|---|
| $CH_3$ | $CH_3$ | $CH(OCOCH_3)$ | S | 2-F, 4-$CH_3$ | B 1 |
| $CH_3$ | $CH_3$ | CH(F) | S | 4-Cl | B 1 |
| $CH_3$ | $CH_3$ | CH(F) | S | 2-Cl, 4-Cl | B 1 |
| $CH_3$ | $CH_3$ | CH(Cl) | S | 4-Cl | B 1 |
| $CH_3$ | $CH_3$ | CH(Cl) | S | 2-Cl, 4-Cl | B 1 |
| $CH_3$ | $CH_3$ | C=O | S | 4-Cl | B 1 |
| $CH_3$ | $CH_3$ | C=O | S | 2-Cl, 4-Cl | B 1 |
| $CH_3$ | $CH_3$ | C=O | S | 2-Cl, 4-$CH_3$ | B 1 |
| $CH_3$ | $CH_3$ | C=O | S | 2-F, 4-$CH_3$ | B 1 |
| $CH_3$ | $CH_3$ | $CH_2$ | S | 4-Cl | B 2 |

| $R^1$ | $R^2$ | X | Y | $W_n$ | B |
|---|---|---|---|---|---|
| $CH_3$ | $CH_3$ | $CH_2$ | S | 2-Cl, 4-Cl | B 2 |
| $CH_3$ | $CH_3$ | $CH_2$ | S | 2-Cl, 4-$CH_3$ | B 2 |
| $CH_3$ | $CH_3$ | $CH_2$ | S | 2-F, 4-$CH_3$ | B 2 |
| $CH_3$ | $CH_3$ | CH(OH) | S | 4-Cl | B 2 |
| $CH_3$ | $CH_3$ | CH(OH) | S | 2-Cl, 4-Cl | B 2 |
| $CH_3$ | $CH_3$ | CH(OH) | S | 2-Cl, 4-$CH_3$ | B 2 |
| $CH_3$ | $CH_3$ | CH(OH) | S | 3-Cl, 4-$CH_3$ | B 2 |
| $CH_3$ | $CH_3$ | CH(OH) | S | 2-F, 4-$CH_3$ | B 2 |
| $CH_3$ | $CH_3$ | CH(OH) | S | 3-F, 4-$CH_3$ | B 2 |
| $CH_3$ | $CH_3$ | CH($OCH_3$) | S | 4-Cl | B 2 |

| $R^1$ | $R^2$ | X | Y | $W_n$ | B |
|---|---|---|---|---|---|
| $CH_3$ | $CH_3$ | $CH(OCH_3)$ | S | 2-Cl, 4-Cl | B 2 |
| $CH_3$ | $CH_3$ | $CH(OCOCH_3)$ | S | 4-Cl | B 2 |
| $CH_3$ | $CH_3$ | $CH(OCOCH_3)$ | S | 2-Cl, 4-Cl | B 2 |
| $CH_3$ | $CH_3$ | $CH(OCOCH_3)$ | S | 2-Cl, 4-$CH_3$ | B 2 |
| $CH_3$ | $CH_3$ | $CH(OCOCH_3)$ | S | 2-F, 4-$CH_3$ | B 2 |
| $CH_3$ | $CH_3$ | CH(F) | S | 4-Cl | B 2 |
| $CH_3$ | $CH_3$ | CH(F) | S | 2-Cl, 4-Cl | B 2 |
| $CH_3$ | $CH_3$ | CH(Cl) | S | 4-Cl | B 2 |
| $CH_3$ | $CH_3$ | CH(Cl) | S | 2-Cl, 4-Cl | B 2 |
| $CH_3$ | $CH_3$ | C=O | S | 4-Cl | B 2 |

| $R^1$ | $R^2$ | X | Y | $W_n$ | B |
|---|---|---|---|---|---|
| $CH_3$ | $CH_3$ | C=O | S | 2-Cl, 4-Cl | B 2 |
| $CH_3$ | $CH_3$ | C=O | S | 2-Cl, 4-$CH_3$ | B 2 |
| $CH_3$ | $CH_3$ | C=O | S | 2-F, 4-$CH_3$ | B 2 |
| $CH_3$ | $CH_3$ | $C(CH_3)(OH)$ | S | 4-Cl | B 2 |
| $CH_3$ | $CH_3$ | $C(CH_3)(OH)$ | S | 2-Cl, 4-Cl | B 2 |
| $CH_3$ | $CH_3$ | $C(CH_3)(OCOCH_3)$ | S | 4-Cl | B 2 |
| $CH_3$ | $CH_3$ | $C(CH_3)(OCOCH_3)$ | S | 2-Cl, 4-Cl | B 2 |
| $CH_3$ | $CH_3$ | $C(CH_3)(F)$ | S | 4-Cl | B 2 |
| $CH_3$ | $CH_3$ | $C(CH_3)(F)$ | S | 2-Cl, 4-Cl | B 2 |
| $CH_3$ | $CH_3$ | $C(C_2H_5)(OH)$ | S | 4-Cl | B 2 |

| $R^1$ | $R^2$ | X | Y | $W_n$ | B |
|---|---|---|---|---|---|
| $CH_3$ | $CH_3$ | $C(C_2H_5)(OH)$ | S | 2-Cl, 4-Cl | B 2 |
| $CH_3$ | $CH_3$ | $C(i-C_3H_7)(OH)$ | S | 4-Cl | B 2 |
| $CH_3$ | $CH_3$ | $C(i-C_3H_7)(OH)$ | S | 2-Cl, 4-Cl | B 2 |
| $CH_3$ | $CH_3$ | $C(i-C_3H_7)(OH)$ | S | 2-Cl, 4-$CH_3$ | B 2 |
| $CH_3$ | $CH_3$ | $C(i-C_3H_7)(OH)$ | S | 2-F, 4-$CH_3$ | B 2 |
| $CH_3$ | $CH_3$ | $CH(CH_3)$ | S | 4-Cl | B 2 |
| $CH_3$ | $CH_3$ | $CH(CH_3)$ | S | 2-Cl, 4-Cl | B 2 |
| $CH_3$ | $CH_3$ | $CH(CH_3)$ | S | 2-Cl, 4-$CH_3$ | B 2 |
| $CH_3$ | $CH_3$ | $CH(CH_3)$ | S | 2-F, 4-$CH_3$ | B 2 |
| Cl | $CH_3$ | $CH_2$ | S | 4-Cl | B 2 |

| $R^1$ | $R^2$ | X | Y | $W_n$ | B |
|---|---|---|---|---|---|
| Cl | $CH_3$ | $CH_2$ | S | 2-Cl, 4-Cl | B2 |
| Cl | $CH_3$ | $CH_2$ | S | 2-Cl, 4-$CH_3$ | B2 |
| Cl | $CH_3$ | $CH_2$ | S | 2-F, 4-$CH_3$ | B2 |
| $CH_3O$ | $CH_3$ | $CH_2$ | S | 4-Cl | B2 |
| $CH_3O$ | $CH_3$ | $CH_2$ | S | 2-Cl, 4-Cl | B2 |
| $CH_3O$ | $CH_3$ | $CH_2$ | S | 2-Cl, 4-$CH_3$ | B2 |
| $CH_3O$ | $CH_3$ | $CH_2$ | S | 2-F, 4-$CH_3$ | B2 |

wherein B1 to B18 are as defined below:

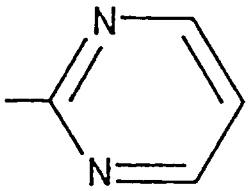

,

B1

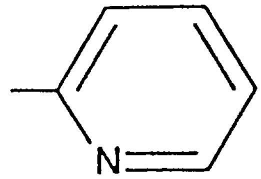

,

B2

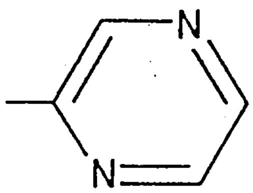

,

B3

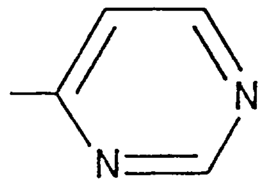

,

B4

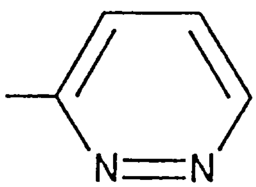

,

B5

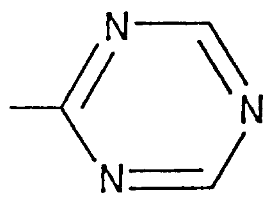

,

B6

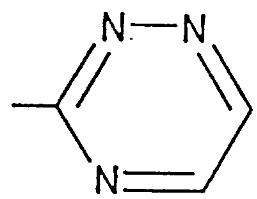

,

B7

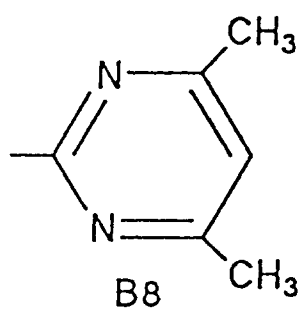

,

B8

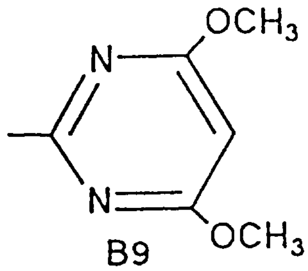
B9 ,

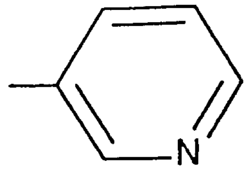
B10 ,

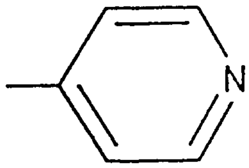
B11 ,

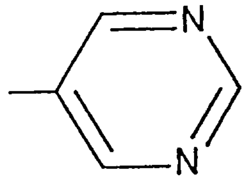
B12

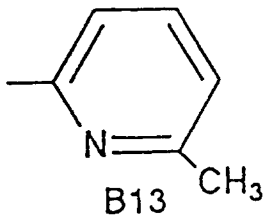
B13 ,

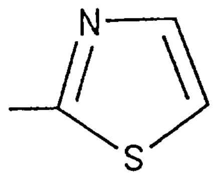
B14 ,

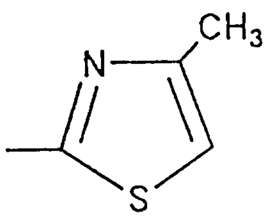
B15 ,

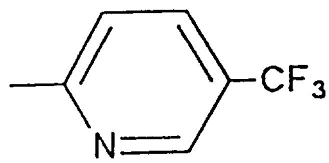
B16 ,

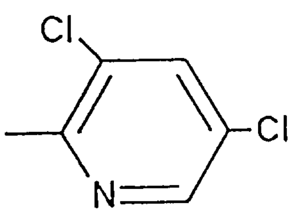
B17 ,

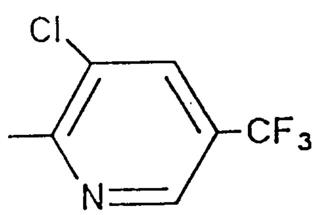
B18 ,

or wherein the substituted pyrazole derivatives are definied by the formula [1b]

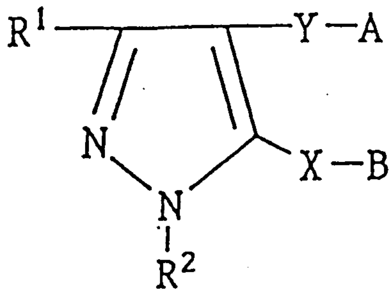

[1b]

having the following meanings of substituents

| $R^1$ | $R^2$ | X | Y | A | B |
|---|---|---|---|---|---|
| $CH_3$ | $CH_3$ | NH | S | A 1 | B 1 |
| $CH_3$ | $CH_3$ | NH | S | A 1 | B 2 |
| $CH_3$ | $CH_3$ | NH | S | A 2 | B 1 |

| $R^1$ | $R^2$ | X | Y | A | B |
|---|---|---|---|---|---|
| $CH_3$ | $CH_3$ | NH | S | A 2 | B 2 |
| $CH_3$ | $CH_3$ | NH | S | A 3 | B 1 |
| $CH_3$ | $CH_3$ | NH | S | A 3 | B 2 |
| $CH_3$ | $CH_3$ | NH | S | A 4 | B 1 |
| $CH_3$ | $CH_3$ | NH | S | A 4 | B 2 |
| $CH_3$ | $CH_3$ | NH | S | A 5 | B 1 |
| $CH_3$ | $CH_3$ | NH | S | A 5 | B 2 |
| $CH_3$ | $CH_3$ | NH | S | A 6 | B 1 |
| $CH_3$ | $CH_3$ | NH | S | A 6 | B 2 |
| $CH_3$ | $CH_3$ | NH | S | A 7 | B 1 |
| $CH_3$ | $CH_3$ | NH | S | A 7 | B 2 |
| $CH_3$ | $CH_3$ | NH | S | A 8 | B 1 |
| $CH_3$ | $CH_3$ | NH | S | A 8 | B 2 |
| $CH_3$ | $CH_3$ | NH | S | A 9 | B 1 |
| $CH_3$ | $CH_3$ | NH | S | A 9 | B 2 |
| $CH_3$ | $CH_3$ | NH | S | A 10 | B 1 |
| $CH_3$ | $CH_3$ | NH | S | A 10 | B 2 |
| $CH_3$ | $CH_3$ | NH | S | A 11 | B 1 |
| $CH_3$ | $CH_3$ | NH | S | A 11 | B 2 |

wherein A1 to A11 are as defined below

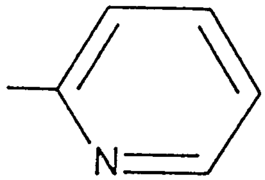

A1

,

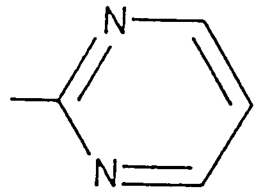

A2

,

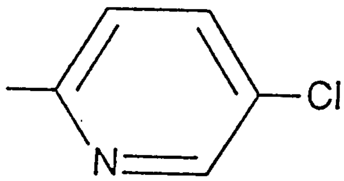

A3

,

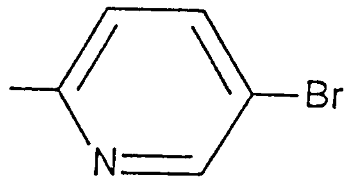

A4

,

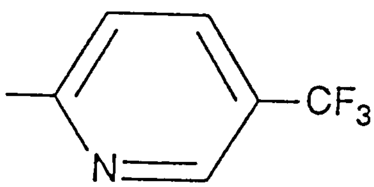

A5

,

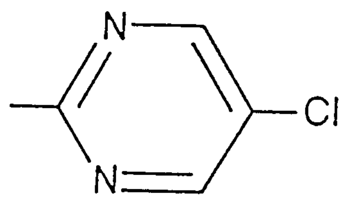

A6

,

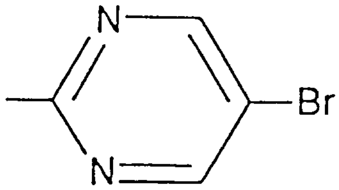

A7 ,

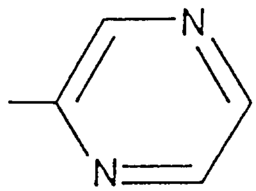

A8 ,

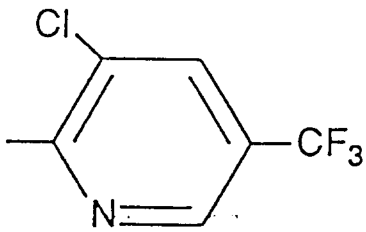

A9 ,

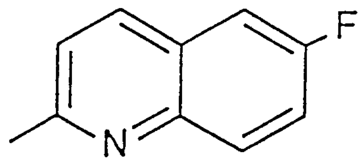

A10 ,

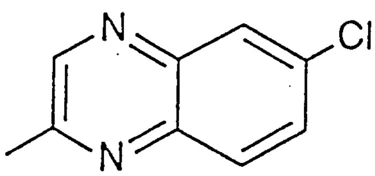

A11 ,

and B1 and B2 are as defined above.

2. Substituted pyrazole derivatives as claimed in claim 1, which are compounds of formula [1a].

3. Substituted pyrazole derivatives as claimed in claim 1, in which X is -N($R^3$)-.

4. Substituted pyrazole derivatives as claimed in claim 2, in which Y is -S-.

5. Substituted pyrazole derivatives as claimed in claim 1, which are compounds of the general formula [1a], in which $R^1$ and $R^2$ each are an alkyl group, X is -N($R^3$)-, Y is -S- and B is B1, B2, B4, B10, B11 or B12.

6. A fungicide for agricultural and horticultural use, containing one or more substituted pyrazoles as claimed in claim 1, as an active ingredient.

7. Use of a substituted pyrazole derivative as claimed in claim 1 as a fungicide for agricultural and horticultural use.

**Claims for the following Contracting State : ES**

1. A method for preparing substituted pyrazole derivatives of the general formula [1]:

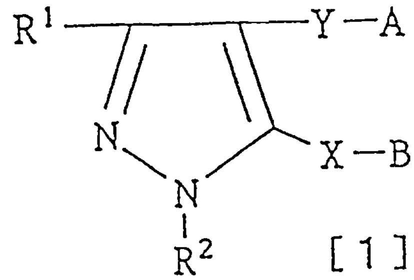

[1]

where $R^1$ represents a hydrogen atom, a halogen atom, an alkyl group, an alkoxy group, an alkylthio group or a haloalkyl group;
$R^2$ represents a hydrogen atom, an alkyl group, a haloalkyl group, a phenylalkyl group, $-COR^6$ or $-SO_2R^7$;
X represents -S-, -SO-, $-SO_2-$, $-N(R^3)-$, -CO- or - $C(R^4)(R^5)-$;
$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, a phenylalkyl group, $-COR^6$ or $-SO_2R^7$;
$R^4$ and $R^5$ independently represent a hydrogen atom, a halogen atom, an alkyl group, a haloalkyl group, an alkenyl group, an alkynyl group or $-OR^8$;
$R^8$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a phenylalkyl group, $-COR^6$ or $-SO_2R^7$;
$R^6$ represents a hydrogen atom, an alkyl group, a haloalkyl group, a phenyl group, a phenylalkyl group, an alkoxy group or

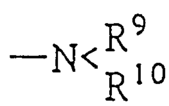 ;

$R^7$ represents an alkyl group, a haloalkyl group, a phenyl group or

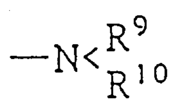 ;

$R^9$ and $R^{10}$ independently represent a hydrogen atom, an alkyl group or a phenyl group;
Y represents an oxygen atom, -S-, -SO-, or $-SO_2-$;
A represents a phenyl group
B represents

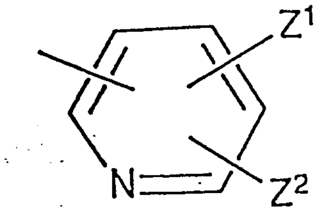 , 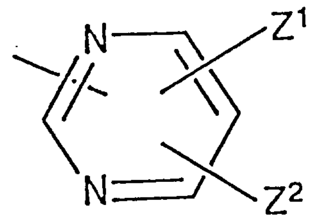 ,

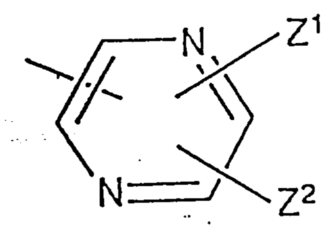 , 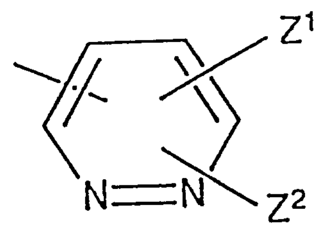 ,

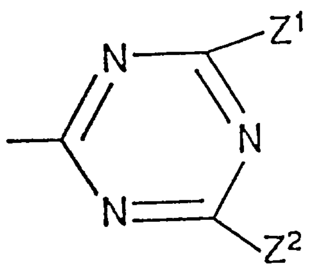 , 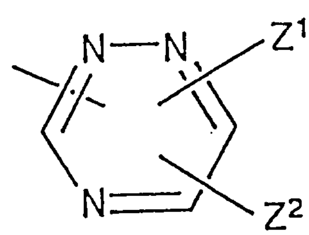

or

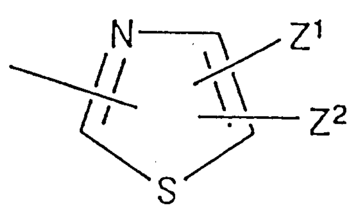 ;

$Z^1$ and $Z^2$ independently represent a hydrogen atom, a halogen atom, an alkyl group, an alkoxy group or a haloalkyl group,

wherein the substituted pyrazole derivatives of the general formula [1] include compounds of the formula [1a]

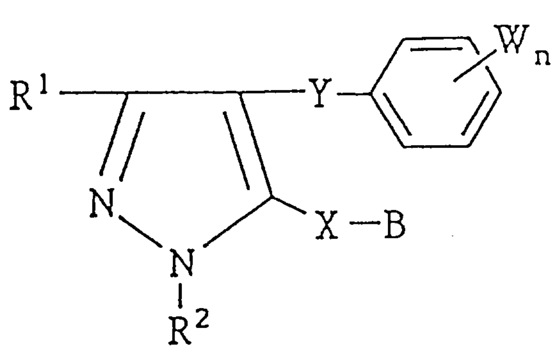 [1a]

having the following meanings of substituents

| $R^1$ | $R^2$ | X | Y | $W_n$ | B |
|---|---|---|---|---|---|
| H | $CH_3$ | S | S | 4-Cl | B 1 |
| $CH_3$ | $CH_3$ | S | S | 4-Cl | B 1 |
| $CF_3$ | $CH_3$ | S | S | 4-Cl | B 1 |
| $CH_3$ | $CH_3$ | S | S | 4-$CH_3$ | B 1 |
| $CF_3$ | $CH_3$ | S | S | 4-$CH_3$ | B 1 |
| $CH_3$ | H | S | S | 4-Cl | B 1 |
| $CH_3$ | H | S | S | 4-$CH_3$ | B 1 |
| $CH_3$ | $CF_3$ | S | S | 4-Cl | B 1 |

| $R^1$ | $R^2$ | X | Y | $W_n$ | B |
|---|---|---|---|---|---|
| $CH_3$ | $CF_3$ | S | S | 4-$CH_3$ | B 1 |
| $CH_3$ | $CH_3$ | S | S | 4-$CF_3$ | B 1 |
| $CH_3$ | $CH_3$ | S | S | 4-Br | B 1 |
| $CH_3$ | $CH_3$ | S | S | 4-$NO_2$ | B 1 |
| $CH_3$ | $CH_3$ | S | S | 4-$OCH_3$ | B 1 |
| $CH_3$ | $CH_3$ | S | S | 4-$C_2H_5$ | B 1 |
| $CH_3$ | $CH_3$ | S | S | 2-Cl | B 1 |
| $CH_3$ | $CH_3$ | S | S | 2-Cl, 4-Cl | B 1 |
| $CH_3$ | $CH_3$ | S | S | 2-Cl, 4-$CH_3$ | B 1 |
| $CH_3$ | $CH_3$ | S | S | 4-Cl | B 2 |
| $CF_3$ | $CH_3$ | S | S | 4-Cl | B 2 |

| $R^1$ | $R^2$ | X | Y | $W_n$ | B |
|---|---|---|---|---|---|
| $CH_3$ | $CH_3$ | S | S | 4-$CH_3$ | B 2 |
| $CF_3$ | $CH_3$ | S | S | 4-$CH_3$ | B 2 |
| $CH_3$ | $CH_3$ | S | S | 2-Cl, 4-Cl | B 2 |
| $CH_3$ | $CH_3$ | S | S | 2-Cl, 4-$CH_3$ | B 2 |
| $CH_3$ | $CH_3$ | S | O | 4-Cl | B 2 |
| $CH_3$ | $CH_3$ | S | O | 4-$CH_3$ | B 2 |
| $CF_3$ | $CH_3$ | S | O | 4-$CH_3$ | B 2 |
| $CH_3$ | $CH_3$ | S | O | 3-Cl, 4-$CH_3$ | B 2 |
| $C_2H_5$ | $CH_3$ | S | S | H | B 1 |
| $C_2H_5$ | $CH_3$ | S | S | 4-Cl | B 1 |

| $R^1$ | $R^2$ | X | Y | $W_n$ | B |
|---|---|---|---|---|---|
| $C_2H_5$ | $CH_3$ | S | S | 4-$CH_3$ | B 1 |
| $C_2H_5$ | $CH_3$ | S | S | 4-$OCH_3$ | B 1 |
| $C_2H_5$ | $CH_3$ | S | S | 3-$CF_3$ | B 1 |
| $C_2H_5$ | $CH_3$ | S | S | 2-Cl, 4-Cl | B 1 |
| $C_2H_5$ | $CH_3$ | S | S | 3-Cl, 4-Cl | B 1 |
| $C_2H_5$ | $CH_3$ | S | S | 2-Cl, 4-$CH_3$ | B 1 |
| i-$C_3H_7$ | $CH_3$ | S | S | 4-Cl | B 1 |
| i-$C_3H_7$ | $CH_3$ | S | S | 4-$CH_3$ | B 1 |
| i-$C_3H_7$ | $CH_3$ | S | S | 4-$OCH_3$ | B 1 |
| t-$C_4H_9$ | $CH_3$ | S | S | 4-Cl | B 1 |

| $R^1$ | $R^2$ | X | Y | $W_n$ | B |
|---|---|---|---|---|---|
| t-$C_4H_9$ | $CH_3$ | S | S | 4-$CH_3$ | B 1 |
| t-$C_4H_9$ | $CH_3$ | S | S | 4-$OCH_3$ | B 1 |
| $CH_3$ | $C_2H_5$ | S | S | 4-Cl | B 1 |
| $CH_3$ | $C_3H_7$ | S | S | 4-Cl | B 1 |
| $CH_3$ | $CH_3$ | NH | S | H | B 1 |
| $CF_3$ | $CH_3$ | NH | S | H | B 1 |
| $CH_3$ | $CH_3$ | NH | S | 4-Cl | B 1 |
| $CF_3$ | $CH_3$ | NH | S | 4-Cl | B 1 |
| H | $CH_3$ | NH | S | 4-Cl | B 1 |
| $CH_3$ | $CH_3$ | NH | S | 4-$CH_3$ | B 1 |

| $R^1$ | $R^2$ | X | Y | $W_n$ | B |
|---|---|---|---|---|---|
| $CF_3$ | $CH_3$ | NH | S | 4-$CH_3$ | B1 |
| H | $CH_3$ | NH | S | 4-$CH_3$ | B1 |
| $CH_3$ | $CH_3$ | NH | S | 4-F | B1 |
| $CH_3$ | $CH_3$ | NH | S | 4-Br | B1 |
| $CH_3$ | $CH_3$ | NH | S | 4-I | B1 |
| $CH_3$ | $CH_3$ | NH | S | 2-F | B1 |
| $CH_3$ | $CH_3$ | NH | S | 2-Cl | B1 |
| $CH_3$ | $CH_3$ | NH | S | 2-Br | B1 |
| $CH_3$ | $CH_3$ | NH | S | 2-I | B1 |
| $CH_3$ | $CH_3$ | NH | S | 3-F | B1 |

| $R^1$ | $R^2$ | X | Y | $W_n$ | B |
|---|---|---|---|---|---|
| $CH_3$ | $CH_3$ | NH | S | 3-Cl | B1 |
| $CH_3$ | $CH_3$ | NH | S | 3-Br | B1 |
| $CH_3$ | $CH_3$ | NH | S | 3-I | B1 |
| $CH_3$ | $CH_3$ | NH | S | 2-$OCH_3$ | B1 |
| $CH_3$ | $CH_3$ | NH | S | 3-$OCH_3$ | B1 |
| $CH_3$ | $CH_3$ | NH | S | 4-$OCH_3$ | B1 |
| $CH_3$ | $CH_3$ | NH | S | 2-$CF_3$ | B1 |
| $CH_3$ | $CH_3$ | NH | S | 3-$CF_3$ | B1 |
| $CH_3$ | $CH_3$ | NH | S | 4-$CF_3$ | B1 |
| $CH_3$ | $CH_3$ | NH | S | 4-$C_2H_5$ | B1 |

| $R^1$ | $R^2$ | X | Y | $W_n$ | B |
|---|---|---|---|---|---|
| $CH_3$ | $CH_3$ | NH | S | $4-C_3H_7$ | B 1 |
| $CH_3$ | $CH_3$ | NH | S | $4-C_4H_9$ | B 1 |
| $CH_3$ | $CH_3$ | NH | S | $4-i-C_3H_7$ | B 1 |
| $CH_3$ | $CH_3$ | NH | S | $4-t-C_4H_9$ | B 1 |
| $CH_3$ | $CH_3$ | NH | S | $2-OCF_3$ | B 1 |
| $CH_3$ | $CH_3$ | NH | S | $3-OCF_3$ | B 1 |
| $CH_3$ | $CH_3$ | NH | S | $4-OCF_3$ | B 1 |
| $CH_3$ | $CH_3$ | NH | S | $3-NO_2$ | B 1 |
| $CH_3$ | $CH_3$ | NH | S | $4-NO_2$ | B 1 |
| $CH_3$ | $CH_3$ | NH | S | $3-NH_2$ | B 1 |

| $R^1$ | $R^2$ | X | Y | $W_n$ | B |
|---|---|---|---|---|---|
| $CH_3$ | $CH_3$ | NH | S | 4-$NH_2$ | B 1 |
| $CH_3$ | $CH_3$ | NH | S | 4-$NHCOCH_3$ | B 1 |
| $CH_3$ | $CH_3$ | NH | S | 4-$NHSO_2CH_3$ | B 1 |
| $CH_3$ | $CH_3$ | NH | S | 4-$NHCOCF_3$ | B 1 |
| $CH_3$ | $CH_3$ | NH | S | 4-$NHSO_2CF_3$ | B 1 |
| $CH_3$ | $CH_3$ | NH | S | 4-Ph | B 1 |
| $CH_3$ | $CH_3$ | NH | S | 4-OPh | B 1 |
| $CH_3$ | $CH_3$ | NH | S | 4-$OCF_2CF_2H$ | B 1 |
| $CH_3$ | $CH_3$ | NH | S | 4-$COCH_3$ | B 1 |
| $CH_3$ | $CH_3$ | NH | S | 4-NHCOPh | B 1 |

| $R^1$ | $R^2$ | X | Y | $W_n$ | B |
|---|---|---|---|---|---|
| $CH_3$ | $CH_3$ | NH | S | 4-$NHCOOCH_3$ | B 1 |
| $CH_3$ | $CH_3$ | NH | S | 4-$NHCON(CH_3)_2$ | B 1 |
| $CH_3$ | $CH_3$ | NH | S | 2-Cl, 3-Cl | B 1 |
| $CH_3$ | $CH_3$ | NH | S | 2-Cl, 4-Cl | B 1 |
| $CH_3$ | $CH_3$ | NH | S | 2-Cl, 5-Cl | B 1 |
| $CH_3$ | $CH_3$ | NH | S | 2-Cl, 6-Cl | B 1 |
| $CH_3$ | $CH_3$ | NH | S | 3-Cl, 4-Cl | B 1 |
| $CH_3$ | $CH_3$ | NH | S | 3-Cl, 5-Cl | B 1 |
| $CH_3$ | $CH_3$ | NH | S | 2-$CH_3$, 3-$CH_3$ | B 1 |
| $CH_3$ | $CH_3$ | NH | S | 2-$CH_3$, 4-$CH_3$ | B 1 |

| $R^1$ | $R^2$ | X | Y | $W_n$ | B |
|---|---|---|---|---|---|
| $CH_3$ | $CH_3$ | NH | S | 2-$CH_3$, 5-$CH_3$ | B 1 |
| $CH_3$ | $CH_3$ | NH | S | 2-$CH_3$, 6-$CH_3$ | B 1 |
| $CH_3$ | $CH_3$ | NH | S | 3-$CH_3$, 4-$CH_3$ | B 1 |
| $CH_3$ | $CH_3$ | NH | S | 3-$CH_3$, 5-$CH_3$ | B 1 |
| $CH_3$ | $CH_3$ | NH | S | 2-Cl, 3-$CH_3$ | B 1 |
| $CH_3$ | $CH_3$ | NH | S | 2-Cl, 4-$CH_3$ | B 1 |
| $CH_3$ | $CH_3$ | NH | S | 2-Cl, 5-$CH_3$ | B 1 |
| $CH_3$ | $CH_3$ | NH | S | 3-Cl, 4-$CH_3$ | B 1 |
| $CH_3$ | $CH_3$ | NH | S | 3-Cl, 5-$CH_3$ | B 1 |
| $CH_3$ | $CH_3$ | NH | S | 2-$CH_3$, 3-Cl | B 1 |

| $R^1$ | $R^2$ | X | Y | $W_n$ | B |
|---|---|---|---|---|---|
| $CH_3$ | $CH_3$ | NH | S | 2-$CH_3$, 4-Cl | B 1 |
| $CH_3$ | $CH_3$ | NH | S | 2-$CH_3$, 5-Cl | B 1 |
| $CH_3$ | $CH_3$ | NH | S | 2-F, 3-F | B 1 |
| $CH_3$ | $CH_3$ | NH | S | 2-F, 4-F | B 1 |
| $CH_3$ | $CH_3$ | NH | S | 2-F, 5-F | B 1 |
| $CH_3$ | $CH_3$ | NH | S | 2-F, 6-F | B 1 |
| $CH_3$ | $CH_3$ | NH | S | 2-F, 3-Cl | B 1 |
| $CH_3$ | $CH_3$ | NH | S | 2-F, 4-Cl | B 1 |
| $CH_3$ | $CH_3$ | NH | S | 2-F, 5-Cl | B 1 |
| $CH_3$ | $CH_3$ | NH | S | 2-F, 6-Cl | B 1 |

| $R^1$ | $R^2$ | X | Y | $W_n$ | B |
|---|---|---|---|---|---|
| $CH_3$ | $CH_3$ | NH | S | 2-F, 3-$CH_3$ | B 1 |
| $CH_3$ | $CH_3$ | NH | S | 2-F, 4-$CH_3$ | B 1 |
| $CH_3$ | $CH_3$ | NH | S | 2-F, 3-Br | B 1 |
| $CH_3$ | $CH_3$ | NH | S | 2-F, 4-Br | B 1 |
| $CH_3$ | $CH_3$ | NH | S | 3-F, 4-Cl | B 1 |
| $CH_3$ | $CH_3$ | NH | S | 3-F, 5-Cl | B 1 |
| $CH_3$ | $CH_3$ | NH | S | 3-F, 4-$CH_3$ | B 1 |
| $CH_3$ | $CH_3$ | NH | S | 3-F, 5-$CH_3$ | B 1 |
| $CH_3$ | $CH_3$ | NH | S | 2-Br, 4-Cl | B 1 |
| $CH_3$ | $CH_3$ | NH | S | 2-Br, 4-Br | B 1 |

| $R^1$ | $R^2$ | X | Y | $W_n$ | B |
|---|---|---|---|---|---|
| $CH_3$ | $CH_3$ | NH | S | 2-Br, 4-$CH_3$ | B 1 |
| $CH_3$ | $CH_3$ | NH | S | 3-Br, 4-Cl | B 1 |
| $CH_3$ | $CH_3$ | NH | S | 3-Br, 4-Br | B 1 |
| $CH_3$ | $CH_3$ | NH | S | 3-Br, 4-$CH_3$ | B 1 |
| $CH_3$ | $CH_3$ | NH | S | 2-Cl, 4-Br | B 1 |
| $CH_3$ | $CH_3$ | NH | S | 3-Cl, 4-Br | B 1 |
| $CH_3$ | $CH_3$ | NH | S | 2-Cl, 4-I | B 1 |
| $CH_3$ | $CH_3$ | NH | S | 3-Cl, 4-I | B 1 |
| $CH_3$ | $CH_3$ | NH | S | 3-F, 4-Br | B 1 |
| $CH_3$ | $CH_3$ | NH | S | 3, 4-$OCH_2O$- | B 1 |

| $R^1$ | $R^2$ | X | Y | $W_n$ | B |
|---|---|---|---|---|---|
| $CH_3$ | $CH_3$ | NH | S | 2-$CF_3$, 3-Cl | B1 |
| $CH_3$ | $CH_3$ | NH | S | 2-$CF_3$, 4-Cl | B1 |
| $CH_3$ | $CH_3$ | NH | S | 2-$CF_3$, 3-Br | B1 |
| $CH_3$ | $CH_3$ | NH | S | 2-$CF_3$, 4-Br | B1 |
| $CH_3$ | $CH_3$ | NH | S | 2-F, 4-$NO_2$ | B1 |
| $CH_3$ | $CH_3$ | NH | S | 3-F, 4-$NO_2$ | B1 |
| $CH_3$ | $CH_3$ | NH | S | 2-Cl, 4-$NO_2$ | B1 |
| $CH_3$ | $CH_3$ | NH | S | 3-Cl, 4-$NO_2$ | B1 |
| $CH_3$ | $CH_3$ | NH | S | 2-F, 4-$OCH_3$ | B1 |
| $CH_3$ | $CH_3$ | NH | S | 3-F, 4-$OCH_3$ | B1 |

| $R^1$ | $R^2$ | X | Y | $W_n$ | B |
|---|---|---|---|---|---|
| $CH_3$ | $CH_3$ | NH | S | 2-Cl, 4-$OCH_3$ | B 1 |
| $CH_3$ | $CH_3$ | NH | S | 3-Cl, 4-$OCH_3$ | B 1 |
| $CH_3$ | $CH_3$ | NH | S | 2-$OCH_3$, 4-$OCH_3$ | B 1 |
| $CH_3$ | $CH_3$ | NH | S | 3-$OCH_3$, 4-$OCH_3$ | B 1 |
| $CH_3$ | $CH_3$ | NH | S | 2-Cl, 3-Cl, 4-Cl | B 1 |
| $CH_3$ | $CH_3$ | NH | S | 2-Cl, 4-Cl, 5-Cl | B 1 |
| $CH_3$ | $CH_3$ | NH | S | 2-Cl, 4-Cl, 6-Cl | B 1 |
| $CH_3$ | $CH_3$ | NH | S | 3-Cl, 4-Cl, 5-Cl | B 1 |
| $CH_3$ | $CH_3$ | NH | S | 2-Cl, 3-Cl, 4-$CH_3$ | B 1 |
| $CH_3$ | $CH_3$ | NH | S | 2-Cl, 3-Cl, 4-Br | B 1 |

| $R^1$ | $R^2$ | X | Y | $W_n$ | B |
|---|---|---|---|---|---|
| $CH_3$ | $CH_3$ | NH | S | 2-Cl, 3-Cl, 4-I | B1 |
| $CH_3$ | $CH_3$ | NH | S | 2-Cl, 4-Br, 6-Cl | B1 |
| $CH_3$ | $CH_3$ | NH | S | 2-Cl, 4-$CH_3$, 6-Cl | B1 |
| $CH_3$ | $CH_3$ | NH | S | 2-Br, 4-Cl, 6-Cl | B1 |
| $CH_3$ | $CH_3$ | NCHO | S | 4-Cl | B1 |
| $CH_3$ | $CH_3$ | $NCOCH_3$ | S | 4-Cl | B1 |
| $CH_3$ | $CH_3$ | $NCOOCH_3$ | S | 4-Cl | B1 |
| $CH_3$ | $CH_3$ | $NCON(CH_3)_2$ | S | 4-Cl | B1 |
| $CH_3$ | $CH_3$ | NCONHPh | S | 4-Cl | B1 |
| $CH_3$ | $CH_3$ | $NCH_3$ | S | 4-Cl | B1 |

| $R^1$ | $R^2$ | X | Y | $W_n$ | B |
|---|---|---|---|---|---|
| $CH_3$ | $CH_3$ | $NC_2H_5$ | S | 4-Cl | B1 |
| $CH_3$ | $CH_3$ | $NC_3H_7$ | S | 4-Cl | B1 |
| $CH_3$ | $CH_3$ | $NCH_2OCH_3$ | S | 4-Cl | B1 |
| $CH_3$ | $CH_3$ | $NCH_2CH_2OCH_3$ | S | 4-Cl | B1 |
| $CH_3$ | $CH_3$ | $NCH_2CH=CH_2$ | S | 4-Cl | B1 |
| $CH_3$ | $CH_3$ | $NCH_2C\equiv CH$ | S | 4-Cl | B1 |
| $CH_3$ | $CH_3$ | $NCH_2COCH_3$ | S | 4-Cl | B1 |
| $CH_3$ | $CH_3$ | $NCH_2COOCH_3$ | S | 4-Cl | B1 |
| $CH_3$ | $CH_3$ | $NCH_2COOC_2H_5$ | S | 4-Cl | B1 |
| $CH_3$ | $CH_3$ | $NCH_2CN$ | S | 4-Cl | B1 |

| $R^1$ | $R^2$ | X | Y | $W_n$ | B |
|---|---|---|---|---|---|
| $CH_3$ | $CH_3$ | $NCH_2Ph$ | S | 4-Cl | B 1 |
| $CH_3$ | $CH_3$ | $NCH_2C_6H_4$-4-Cl | S | 4-Cl | B 1 |
| $CH_3$ | $CH_3$ | $NCH_2C_6H_4$-4-$CH_3$ | S | 4-Cl | B 1 |
| $CH_3$ | $CH_3$ | $NCH_2CH_2Ph$ | S | 4-Cl | B 1 |
| $CH_3$ | $CH_3$ | $NSO_2CH_3$ | S | 4-Cl | B 1 |
| $CH_3$ | $CH_3$ | $NSO_2N(CH_3)_2$ | S | 4-Cl | B 1 |
| $CH_3$ | $CH_3$ | NCHO | S | 4-Cl | B 2 |
| $CH_3$ | $CH_3$ | $NCH_3$ | S | 4-Cl | B 2 |
| $CH_3$ | H | NH | S | 4-Cl | B 1 |
| $CH_3$ | H | NH | S | 2-Cl, 4-Cl | B 1 |

| $R^1$ | $R^2$ | X | Y | $W_n$ | B |
|---|---|---|---|---|---|
| $CH_3$ | H | NH | S | 3-Cl, 4-$CH_3$ | B 1 |
| $CH_3$ | $C_2H_5$ | NH | S | 4-Cl | B 1 |
| $CH_3$ | $C_2H_5$ | NH | S | 4-Br | B 1 |
| $CH_3$ | $C_2H_5$ | NH | S | 2-Cl, 4-Cl | B 1 |
| $CH_3$ | $C_2H_5$ | NH | S | 2-Cl, 4-$CH_3$ | B 1 |
| $CH_3$ | i-$C_3H_7$ | NH | S | 4-Cl | B 1 |
| $CH_3$ | i-$C_3H_7$ | NH | S | 4-Br | B 1 |
| $CH_3$ | i-$C_3H_7$ | NH | S | 2-Cl, 4-Cl | B 1 |
| $CH_3$ | i-$C_3H_7$ | NH | S | 2-Cl, 4-$CH_3$ | B 1 |
| $CH_3$ | t-$C_4H_9$ | NH | S | 4-Cl | B 1 |

| $R^1$ | $R^2$ | X | Y | $W_n$ | B |
|---|---|---|---|---|---|
| $CH_3$ | $t-C_4H_9$ | NH | S | 2-Cl, 4-Cl | B 1 |
| $CH_3$ | $t-C_4H_9$ | NH | S | 3-Cl, 4-$CH_3$ | B 1 |
| $CH_3$ | $CF_3$ | NH | S | 4-Cl | B 1 |
| $CH_3$ | $CF_3$ | NH | S | 4-Br | B 1 |
| $CH_3$ | $CF_3$ | NH | S | 2-Cl, 4-Cl | B 1 |
| $CH_3$ | $CF_3$ | NH | S | 3-Cl, 4-$CH_3$ | B 1 |
| $CH_3$ | H | NH | S | 4-Cl | B 2 |
| $CH_3$ | H | NH | S | 2-Cl, 4-Cl | B 2 |
| $CH_3$ | $C_2H_5$ | NH | S | 4-Cl | B 2 |
| $CH_3$ | $i-C_3H_7$ | NH | S | 4-Cl | B 2 |

| $R^1$ | $R^2$ | X | Y | $W_n$ | B |
|---|---|---|---|---|---|
| $CH_3$ | t-$C_4H_9$ | NH | S | 4-Cl | B 2 |
| $CH_3$ | $CF_3$ | NH | S | 2-Cl, 4-Cl | B 2 |
| $CH_3$ | $CH_2Ph$ | NH | S | 4-Cl | B 1 |
| $CH_3$ | $CH_2Ph$ | NH | S | 2-Cl, 4-Cl | B 1 |
| $CH_3$ | $CH_2Ph$ | NCHO | S | 2-Cl, 4-Cl | B 1 |
| $CH_3$ | CHO | NH | S | 4-Cl | B 1 |
| $CH_3$ | CHO | NCHO | S | 4-Cl | B 1 |
| $CH_3$ | CHO | NH | S | 2-Cl, 4-Cl | B 1 |
| $CH_3$ | CHO | NCHO | S | 2-Cl, 4-Cl | B 1 |
| $CH_3$ | $COCH_3$ | NH | S | 4-Cl | B 1 |

| $R^1$ | $R^2$ | X | Y | $W_n$ | B |
|---|---|---|---|---|---|
| $CH_3$ | $COCH_3$ | NH | S | 4-Br | B 1 |
| $CH_3$ | $COCH_3$ | NH | S | 2-Cl, 4-Cl | B 1 |
| $CH_3$ | $COCH_3$ | NCHO | S | 2-Cl, 4-Cl | B 1 |
| $CH_3$ | $SO_2CH_3$ | NH | S | 4-Cl | B 1 |
| $CH_3$ | $SO_2CH_3$ | NH | S | 2-Cl, 4-Cl | B 1 |
| $CH_3$ | $SO_2CH_3$ | NH | S | 3-Cl, 4-$CH_3$ | B 1 |
| $CH_3$ | $CONHCH_3$ | NH | S | 4-Cl | B 1 |
| $CH_3$ | $CONHCH_3$ | NH | S | 4-Br | B 1 |
| $CH_3$ | $CONHCH_3$ | NH | S | 2-Cl, 4-Cl | B 1 |
| $CH_3$ | $CONHCH_3$ | NH | S | 3-Cl, 4-$CH_3$ | B 1 |

| $R^1$ | $R^2$ | X | Y | $W_n$ | B |
|---|---|---|---|---|---|
| $CH_3$ | $CH_2Ph$ | NH | S | 4-Cl | B 2 |
| $CH_3$ | $CH_2Ph$ | NH | S | 2-Cl, 4-Cl | B 2 |
| $CH_3$ | $COCH_3$ | NH | S | 4-Cl | B 2 |
| $CH_3$ | $CONHCH_3$ | NH | S | 4-Cl | B 2 |
| $CH_3$ | $SO_2CH_3$ | NH | S | 4-Cl | B 2 |
| $CH_3$ | $SO_2CH_3$ | NH | S | 2-Cl, 4-Cl | B 2 |
| H | $CH_3$ | NH | S | 4-Cl | B 1 |
| H | $CH_3$ | NH | S | 2-Cl, 4-Cl | B 1 |
| H | $CH_3$ | NH | S | 3-Cl, 4-$CH_3$ | B 1 |
| $CF_3$ | $CH_3$ | NH | S | 4-Cl | B 1 |

| $R^1$ | $R^2$ | X | Y | $W_n$ | B |
|---|---|---|---|---|---|
| $CF_3$ | $CH_3$ | NH | S | 4-Br | B 1 |
| $CF_3$ | $CH_3$ | NH | S | 2-Cl, 4-Cl | B 1 |
| $CF_3$ | $CH_3$ | NH | S | 2-Cl, 4-$CH_3$ | B 1 |
| $C_2H_5$ | $CH_3$ | NH | S | 4-Cl | B 1 |
| $C_2H_5$ | $CH_3$ | NH | S | 4-Br | B 1 |
| $C_2H_5$ | $CH_3$ | NH | S | 2-Cl, 4-Cl | B 1 |
| $C_2H_5$ | $CH_3$ | NH | S | 2-Cl, 4-$CH_3$ | B 1 |
| i-$C_3H_7$ | $CH_3$ | NH | S | 4-Cl | B 1 |
| i-$C_3H_7$ | $CH_3$ | NH | S | 2-Cl, 4-Cl | B 1 |
| i-$C_3H_7$ | $CH_3$ | NH | S | 3-Cl, 4-$CH_3$ | B 1 |

| $R^1$ | $R^2$ | X | Y | $W_n$ | B |
|---|---|---|---|---|---|
| t-$C_4H_9$ | $CH_3$ | NH | S | 4-Cl | B 1 |
| t-$C_4H_9$ | $CH_3$ | NH | S | 4-Br | B 1 |
| t-$C_4H_9$ | $CH_3$ | NH | S | 2-Cl, 4-Cl | B 1 |
| t-$C_4H_9$ | $CH_3$ | NH | S | 3-Cl, 4-$CH_3$ | B 1 |
| H | $CH_3$ | NH | S | 4-Cl | B 2 |
| H | $CH_3$ | NH | S | 2-Cl, 4-Cl | B 2 |
| $C_2H_5$ | $CH_3$ | NH | S | 4-Cl | B 2 |
| i-$C_3H_7$ | $CH_3$ | NH | S | 4-Cl | B 2 |
| $CF_3$ | $CH_3$ | NH | S | 4-Cl | B 2 |
| $CF_3$ | $CH_3$ | NH | S | 2-Cl, 4-Cl | B 2 |

| $R^1$ | $R^2$ | X | Y | $W_n$ | B |
|---|---|---|---|---|---|
| Cl | $CH_3$ | NH | S | 4-Cl | B 1 |
| Cl | $CH_3$ | NH | S | 2-Cl, 4-Cl | B 1 |
| Cl | $CH_3$ | NH | S | 3-Cl, 4-$CH_3$ | B 1 |
| Cl | $CH_3$ | NCHO | S | 4-Cl | B 1 |
| Cl | $CH_3$ | NH | S | 4-Br | B 1 |
| Cl | $CH_3$ | NCHO | S | 2-Cl, 4-Cl | B 1 |
| Cl | $CH_3$ | NCHO | S | 2-Cl, 4-$CH_3$ | B 1 |
| $CH_3O$ | $CH_3$ | NH | S | 4-Cl | B 1 |
| $CH_3O$ | $CH_3$ | NH | S | 4-Br | B 1 |
| $CH_3O$ | $CH_3$ | NH | S | 2-Cl, 4-Cl | B 1 |

| $R^1$ | $R^2$ | X | Y | $W_n$ | B |
|---|---|---|---|---|---|
| $CH_3O$ | $CH_3$ | NH | S | 2-Cl, 4-$CH_3$ | B 1 |
| $CH_3O$ | $CH_3$ | NCHO | S | 4-Cl | B 1 |
| $CH_3O$ | $CH_3$ | NCHO | S | 2-Cl, 4-Cl | B 1 |
| $CH_3O$ | $CH_3$ | NCHO | S | 3-Cl, 4-$CH_3$ | B 1 |
| $CH_3S$ | $CH_3$ | NH | S | 4-Cl | B 1 |
| $CH_3S$ | $CH_3$ | NH | S | 4-Br | B 1 |
| $CH_3S$ | $CH_3$ | NH | S | 2-Cl, 4-Cl | B 1 |
| $CH_3S$ | $CH_3$ | NH | S | 3-Cl, 4-$CH_3$ | B 1 |
| $CH_3S$ | $CH_3$ | NCHO | S | 4-Cl | B 1 |
| $CH_3S$ | $CH_3$ | NCHO | S | 2-Cl, 4-Cl | B 1 |

| $R^1$ | $R^2$ | X | Y | $W_n$ | B |
|---|---|---|---|---|---|
| Cl | $CH_3$ | NH | S | 4-Cl | B2 |
| Cl | $CH_3$ | NH | S | 2-Cl, 4-Cl | B2 |
| $CH_3O$ | $CH_3$ | NH | S | 2-Cl, 4-Cl | B2 |
| $CH_3S$ | $CH_3$ | NH | S | 2-Cl, 4-Cl | B2 |
| $CH_3$ | $CH_3$ | SO | S | 4-Cl | B1 |
| $CH_3$ | $CH_3$ | $SO_2$ | S | 4-Cl | B1 |
| $CH_3$ | $CH_3$ | NH | SO | 4-Cl | B1 |
| $CH_3$ | $CH_3$ | NH | $SO_2$ | 4-Cl | B1 |
| $CH_3$ | $CH_3$ | NH | SO | 2-Cl, 4-Cl | B1 |
| $CH_3$ | $CH_3$ | NH | $SO_2$ | 2-Cl, 4-Cl | B1 |

| $R^1$ | $R^2$ | X | Y | $W_n$ | B |
|---|---|---|---|---|---|
| $CH_3$ | $CH_3$ | NH | SO | 3-Cl, 4-$CH_3$ | B 1 |
| $CH_3$ | $CH_3$ | NH | $SO_2$ | 3-Cl, 4-$CH_3$ | B 1 |
| $CH_3$ | $CH_3$ | S | O | 4-Cl | B 1 |
| $CH_3$ | $CH_3$ | SO | O | 4-Cl | B 1 |
| $CH_3$ | $CH_3$ | $SO_2$ | O | 4-Cl | B 1 |
| $CH_3$ | $CH_3$ | S | O | 2-Cl, 4-Cl | B 1 |
| $CH_3$ | $CH_3$ | SO | O | 2-Cl, 4-Cl | B 1 |
| $CH_3$ | $CH_3$ | $SO_2$ | O | 2-Cl, 4-Cl | B 1 |
| $CH_3$ | $CH_3$ | NH | O | 4-Cl | B 1 |
| $CH_3$ | $CH_3$ | NH | O | 4-$CH_3$ | B 1 |

| $R^1$ | $R^2$ | X | Y | $W_n$ | B |
|---|---|---|---|---|---|
| $CH_3$ | $CH_3$ | NH | O | 2-Cl, 4-Cl | B 1 |
| $CH_3$ | $CH_3$ | NH | O | 2-Cl, 4-$CH_3$ | B 1 |
| $CH_3$ | $CH_3$ | NH | O | 3-Cl, 4-$CH_3$ | B 1 |
| $CH_3$ | $CH_3$ | NH | O | 2-F, 4-Cl | B 1 |
| $CH_3$ | $CH_3$ | NCHO | O | 4-Cl | B 1 |
| $CH_3$ | $CH_3$ | $NCH_3$ | O | 4-Cl | B 1 |
| $CH_3$ | $CH_3$ | NH | O | 4-Cl | B 2 |
| $CH_3$ | $CH_3$ | NH | O | 2-Cl, 4-Cl | B 2 |
| $CH_3$ | $CH_3$ | NH | S | 4-F | B 2 |
| $CH_3$ | $CH_3$ | NH | S | 4-Cl | B 2 |

| $R^1$ | $R^2$ | X | Y | $W_n$ | B |
|---|---|---|---|---|---|
| $CH_3$ | $CH_3$ | NH | S | 4-$CH_3$ | B2 |
| $CH_3$ | $CH_3$ | NH | S | 2-Cl, 4-Cl | B2 |
| $CH_3$ | $CH_3$ | NH | S | 3-Cl, 4-Cl | B2 |
| $CH_3$ | $CH_3$ | NH | S | 2-Cl, 4-$CH_3$ | B2 |
| $CH_3$ | $CH_3$ | NH | S | 3-Cl, 4-$CH_3$ | B2 |
| $CH_3$ | $CH_3$ | NH | S | 2-F, 4-F | B2 |
| $CH_3$ | $CH_3$ | NH | S | 2-F, 4-Cl | B2 |
| $CH_3$ | $CH_3$ | NH | S | 2-F, 4-Br | B2 |
| $CH_3$ | $CH_3$ | NH | S | 2-F, 4-$CH_3$ | B2 |
| $CH_3$ | $CH_3$ | NH | S | 3-F, 4-$CH_3$ | B2 |

| $R^1$ | $R^2$ | X | Y | $W_n$ | B |
|---|---|---|---|---|---|
| $CH_3$ | $CH_3$ | NCHO | S | 4-Cl | B 2 |
| $CH_3$ | $CH_3$ | NCHO | S | 2-Cl, 4-Cl | B 2 |
| $CH_3$ | $CH_3$ | NCHO | S | 3-Cl, 4-$CH_3$ | B 2 |
| $CH_3$ | $CH_3$ | NH | S | 4-Cl | B 3 |
| $CH_3$ | $CH_3$ | NH | S | 4-Br | B 3 |
| $CH_3$ | $CH_3$ | NH | S | 2-Cl, 4-Cl | B 3 |
| $CH_3$ | $CH_3$ | NH | S | 2-Cl, 4-$CH_3$ | B 3 |
| $CH_3$ | $CH_3$ | NH | S | 3-Cl, 4-$CH_3$ | B 3 |
| $CH_3$ | $CH_3$ | NH | S | 4-Cl | B 4 |
| $CH_3$ | $CH_3$ | NH | S | 2-Cl, 4-Cl | B 4 |

| $R^1$ | $R^2$ | X | Y | $W_n$ | B |
|---|---|---|---|---|---|
| $CH_3$ | $CH_3$ | NH | S | 4-Cl | B5 |
| $CH_3$ | $CH_3$ | NH | S | 2-Cl, 4-Cl | B5 |
| $CH_3$ | $CH_3$ | NH | S | 4-Cl | B6 |
| $CH_3$ | $CH_3$ | NH | S | 2-Cl, 4-Cl | B6 |
| $CH_3$ | $CH_3$ | NH | S | 4-Cl | B7 |
| $CH_3$ | $CH_3$ | NH | S | 2-Cl, 4-$CH_3$ | B7 |
| $CH_3$ | $CH_3$ | NH | S | 4-Cl | B8 |
| $CH_3$ | $CH_3$ | NH | S | 2-Cl, 4-Cl | B8 |
| $CH_3$ | $CH_3$ | NH | S | 4-Cl | B9 |
| $CH_3$ | $CH_3$ | NH | S | 2-Cl, 4-$CH_3$ | B9 |

| $R^1$ | $R^2$ | X | Y | $W_n$ | B |
|---|---|---|---|---|---|
| $CH_3$ | $CH_3$ | NH | S | 4-Cl | B10 |
| $CH_3$ | $CH_3$ | NH | S | 2-Cl, 4-Cl | B10 |
| $CH_3$ | $CH_3$ | NH | S | 3-Cl, 4-Cl | B10 |
| $CH_3$ | $CH_3$ | NH | S | 2-F, 4-$CH_3$ | B10 |
| $CH_3$ | $CH_3$ | NH | S | 3-F, 4-$CH_3$ | B10 |
| $CH_3$ | $CH_3$ | NH | S | 2-F, 4-Cl | B10 |
| $CH_3$ | $CH_3$ | NH | S | 4-Cl | B11 |
| $CH_3$ | $CH_3$ | NH | S | 2-Cl, 4-$CH_3$ | B11 |
| $CH_3$ | $CH_3$ | NH | S | 4-Cl | B12 |
| $CH_3$ | $CH_3$ | NH | S | 2-Cl, 4-Cl | B12 |

| $R^1$ | $R^2$ | X | Y | $W_n$ | B |
|---|---|---|---|---|---|
| $CH_3$ | $CH_3$ | NH | S | 2-F, 4-$CH_3$ | B 1 2 |
| $CH_3$ | $CH_3$ | NH | S | 3-F, 4-$CH_3$ | B 1 2 |
| $CH_3$ | $CH_3$ | NH | S | 2-Cl, 4-$CH_3$ | B 1 2 |
| $CH_3$ | $CH_3$ | NH | S | 3-Cl, 4-$CH_3$ | B 1 2 |
| $CH_3$ | $CH_3$ | NCHO | S | 4-Cl | B 1 2 |
| $CH_3$ | $CH_3$ | NCHO | S | 2-Cl, 4-Cl | B 1 2 |
| $CH_3$ | $CH_3$ | NH | S | 4-Cl | B 1 3 |
| $CH_3$ | $CH_3$ | NH | S | 2-Cl, 4-Cl | B 1 3 |
| $CH_3$ | $CH_3$ | NH | S | 2-Cl, 4-$CH_3$ | B 1 3 |

| $R^1$ | $R^2$ | X | Y | $W_n$ | B |
|---|---|---|---|---|---|
| $CH_3$ | $CH_3$ | NH | S | 2-F, 4-$CH_3$ | B13 |
| $CH_3$ | $CH_3$ | NH | S | 4-Cl | B14 |
| $CH_3$ | $CH_3$ | NH | S | 2-Cl, 4-Cl | B14 |
| $CH_3$ | $CH_3$ | NH | S | 2-Cl, 4-$CH_3$ | B14 |
| $CH_3$ | $CH_3$ | NH | S | 2-F, 4-$CH_3$ | B14 |
| $CH_3$ | $CH_3$ | NH | S | 4-Cl | B15 |
| $CH_3$ | $CH_3$ | NH | S | 2-Cl, 4-Cl | B15 |
| $CH_3$ | $CH_3$ | NH | S | 2-Cl, 4-$CH_3$ | B15 |
| $CH_3$ | $CH_3$ | NH | S | 2-F, 4-$CH_3$ | B15 |
| $CH_3$ | $CH_3$ | NH | S | 4-Cl | B16 |

| $R^1$ | $R^2$ | X | Y | $W_n$ | B |
|---|---|---|---|---|---|
| $CH_3$ | $CH_3$ | NH | S | 2-Cl, 4-Cl | B16 |
| $CH_3$ | $CH_3$ | NH | S | 2-Cl, 4-$CH_3$ | B16 |
| $CH_3$ | $CH_3$ | NH | S | 2-F, 4-$CH_3$ | B16 |
| $CH_3$ | $CH_3$ | NH | S | 4-Cl | B17 |
| $CH_3$ | $CH_3$ | NH | S | 2-Cl, 4-Cl | B17 |
| $CH_3$ | $CH_3$ | NH | S | 2-Cl, 4-$CH_3$ | B17 |
| $CH_3$ | $CH_3$ | NH | S | 2-F, 4-$CH_3$ | B17 |
| $CH_3$ | $CH_3$ | NH | S | 4-Cl | B18 |
| $CH_3$ | $CH_3$ | NH | S | 2-Cl, 4-Cl | B18 |
| $CH_3$ | $CH_3$ | NH | S | 2-Cl, 4-$CH_3$ | B18 |

| $R^1$ | $R^2$ | X | Y | $W_n$ | B |
|---|---|---|---|---|---|
| $CH_3$ | $CH_3$ | NH | S | 2-F, 4-$CH_3$ | B 18 |
| $CH_3$ | $CH_3$ | NCHO | S | 2-Cl, 4-Cl | B 1 |
| $CH_3$ | $CH_3$ | NCHO | S | 3-Cl, 4-Cl | B 1 |
| $CH_3$ | $CH_3$ | NCHO | S | 2-F, 4-Cl | B 1 |
| $CH_3$ | $CH_3$ | NCHO | S | 2-F, 4-$CH_3$ | B 1 |
| $CH_3$ | $CH_3$ | NCHO | S | 3-F, 4-Cl | B 1 |
| $CH_3$ | $CH_3$ | NCHO | S | 3-F, 4-$CH_3$ | B 1 |
| $CH_3$ | $CH_3$ | NCHO | S | 2-Cl, 4-$CH_3$ | B 1 |
| $CH_3$ | $CH_3$ | NCHO | S | 3-Cl, 4-$CH_3$ | B 1 |
| $CH_3$ | $CH_3$ | NCHO | S | 2-Cl, 3-Cl, 4-Cl | B 1 |

| $R^1$ | $R^2$ | X | Y | $W_n$ | B |
|---|---|---|---|---|---|
| $CH_3$ | $CH_3$ | NCHO | S | 2-Cl, 4-Cl, 5-Cl | B 1 |
| $CH_3$ | $CH_3$ | $NCOCH_3$ | S | 2-Cl, 4-Cl | B 1 |
| $CH_3$ | $CH_3$ | $NCOCH_3$ | S | 3-Cl, 4-Cl | B 1 |
| $CH_3$ | $CH_3$ | $NCOCH_3$ | S | 2-F, 4-$CH_3$ | B 1 |
| $CH_3$ | $CH_3$ | $NCOCH_3$ | S | 2-Cl, 3-Cl, 4-Cl | B 1 |
| $CH_3$ | $CH_3$ | $NCH_3$ | S | 2-Cl, 4-Cl | B 1 |
| $CH_3$ | $CH_3$ | $NCH_3$ | S | 3-Cl, 4-$CH_3$ | B 1 |
| $CH_3$ | $CH_3$ | NNO | S | 2-Cl, 4-Cl | B 1 |
| $CH_3$ | $CH_3$ | NNO | S | 3-Cl, 4-$CH_3$ | B 1 |
| $CH_3$ | $CH_3$ | $NNH_2$ | S | 4-Cl | B 1 |

| $R^1$ | $R^2$ | X | Y | $W_n$ | B |
|---|---|---|---|---|---|
| $CH_3$ | $CH_3$ | $NNH_2$ | S | 2-Cl, 4-Cl | B 1 |
| $CH_3$ | $CH_3$ | $NNH_2$ | S | 3-Cl, 4-$CH_3$ | B 1 |
| $CH_3$ | $CH_3$ | $NSO_2CH_3$ | S | 2-Cl, 4-Cl | B 1 |
| $CH_3$ | $CH_3$ | $NSO_2CH_3$ | S | 2-F, 4-$CH_3$ | B 1 |
| $CH_3$ | $CH_3$ | $NSO_2CH_3$ | S | 2-Cl, 3-Cl, 4-Cl | B 1 |
| $CH_3$ | $CH_3$ | $CH_2$ | S | 4-Cl | B 1 |
| $CH_3$ | $CH_3$ | $CH_2$ | S | 2-Cl, 4-Cl | B 1 |
| $CH_3$ | $CH_3$ | $CH_2$ | S | 2-Cl, 4-$CH_3$ | B 1 |
| $CH_3$ | $CH_3$ | $CH_2$ | S | 2-F, 4-$CH_3$ | B 1 |
| $CH_3$ | $CH_3$ | CH(OH) | S | 4-Cl | B 1 |

| $R^1$ | $R^2$ | X | Y | $W_n$ | B |
|---|---|---|---|---|---|
| $CH_3$ | $CH_3$ | CH(OH) | S | 2-Cl, 4-Cl | B 1 |
| $CH_3$ | $CH_3$ | CH(OH) | S | 2-Cl, 4-$CH_3$ | B 1 |
| $CH_3$ | $CH_3$ | CH(OH) | S | 3-Cl, 4-$CH_3$ | B 1 |
| $CH_3$ | $CH_3$ | CH(OH) | S | 2-F, 4-$CH_3$ | B 1 |
| $CH_3$ | $CH_3$ | CH(OH) | S | 3-F, 4-$CH_3$ | B 1 |
| $CH_3$ | $CH_3$ | CH($OCH_3$) | S | 4-Cl | B 1 |
| $CH_3$ | $CH_3$ | CH($OCH_3$) | S | 2-Cl, 4-Cl | B 1 |
| $CH_3$ | $CH_3$ | CH($OCOCH_3$) | S | 4-Cl | B 1 |
| $CH_3$ | $CH_3$ | CH($OCOCH_3$) | S | 2-Cl, 4-Cl | B 1 |
| $CH_3$ | $CH_3$ | CH($OCOCH_3$) | S | 2-Cl, 4-$CH_3$ | B 1 |

| $R^1$ | $R^2$ | X | Y | $W_n$ | B |
|---|---|---|---|---|---|
| $CH_3$ | $CH_3$ | $CH(OCOCH_3)$ | S | 2-F, 4-$CH_3$ | B1 |
| $CH_3$ | $CH_3$ | CH(F) | S | 4-Cl | B1 |
| $CH_3$ | $CH_3$ | CH(F) | S | 2-Cl, 4-Cl | B1 |
| $CH_3$ | $CH_3$ | CH(Cl) | S | 4-Cl | B1 |
| $CH_3$ | $CH_3$ | CH(Cl) | S | 2-Cl, 4-Cl | B1 |
| $CH_3$ | $CH_3$ | C=O | S | 4-Cl | B1 |
| $CH_3$ | $CH_3$ | C=O | S | 2-Cl, 4-Cl | B1 |
| $CH_3$ | $CH_3$ | C=O | S | 2-Cl, 4-$CH_3$ | B1 |
| $CH_3$ | $CH_3$ | C=O | S | 2-F, 4-$CH_3$ | B1 |
| $CH_3$ | $CH_3$ | $CH_2$ | S | 4-Cl | B2 |

| $R^1$ | $R^2$ | X | Y | $W_n$ | B |
|---|---|---|---|---|---|
| $CH_3$ | $CH_3$ | $CH_2$ | S | 2-Cl, 4-Cl | B 2 |
| $CH_3$ | $CH_3$ | $CH_2$ | S | 2-Cl, 4-$CH_3$ | B 2 |
| $CH_3$ | $CH_3$ | $CH_2$ | S | 2-F, 4-$CH_3$ | B 2 |
| $CH_3$ | $CH_3$ | CH(OH) | S | 4-Cl | B 2 |
| $CH_3$ | $CH_3$ | CH(OH) | S | 2-Cl, 4-Cl | B 2 |
| $CH_3$ | $CH_3$ | CH(OH) | S | 2-Cl, 4-$CH_3$ | B 2 |
| $CH_3$ | $CH_3$ | CH(OH) | S | 3-Cl, 4-$CH_3$ | B 2 |
| $CH_3$ | $CH_3$ | CH(OH) | S | 2-F, 4-$CH_3$ | B 2 |
| $CH_3$ | $CH_3$ | CH(OH) | S | 3-F, 4-$CH_3$ | B 2 |
| $CH_3$ | $CH_3$ | CH($OCH_3$) | S | 4-Cl | B 2 |

| $R^1$ | $R^2$ | X | Y | $W_n$ | B |
|---|---|---|---|---|---|
| $CH_3$ | $CH_3$ | $CH(OCH_3)$ | S | 2-Cl, 4-Cl | B 2 |
| $CH_3$ | $CH_3$ | $CH(OCOCH_3)$ | S | 4-Cl | B 2 |
| $CH_3$ | $CH_3$ | $CH(OCOCH_3)$ | S | 2-Cl, 4-Cl | B 2 |
| $CH_3$ | $CH_3$ | $CH(OCOCH_3)$ | S | 2-Cl, 4-$CH_3$ | B 2 |
| $CH_3$ | $CH_3$ | $CH(OCOCH_3)$ | S | 2-F, 4-$CH_3$ | B 2 |
| $CH_3$ | $CH_3$ | CH(F) | S | 4-Cl | B 2 |
| $CH_3$ | $CH_3$ | CH(F) | S | 2-Cl, 4-Cl | B 2 |
| $CH_3$ | $CH_3$ | CH(Cl) | S | 4-Cl | B 2 |
| $CH_3$ | $CH_3$ | CH(Cl) | S | 2-Cl, 4-Cl | B 2 |
| $CH_3$ | $CH_3$ | C=O | S | 4-Cl | B 2 |

| $R^1$ | $R^2$ | X | Y | $W_n$ | B |
|---|---|---|---|---|---|
| $CH_3$ | $CH_3$ | C=O | S | 2-Cl, 4-Cl | B 2 |
| $CH_3$ | $CH_3$ | C=O | S | 2-Cl, 4-$CH_3$ | B 2 |
| $CH_3$ | $CH_3$ | C=O | S | 2-F, 4-$CH_3$ | B 2 |
| $CH_3$ | $CH_3$ | $C(CH_3)(OH)$ | S | 4-Cl | B 2 |
| $CH_3$ | $CH_3$ | $C(CH_3)(OH)$ | S | 2-Cl, 4-Cl | B 2 |
| $CH_3$ | $CH_3$ | $C(CH_3)(OCOCH_3)$ | S | 4-Cl | B 2 |
| $CH_3$ | $CH_3$ | $C(CH_3)(OCOCH_3)$ | S | 2-Cl, 4-Cl | B 2 |
| $CH_3$ | $CH_3$ | $C(CH_3)(F)$ | S | 4-Cl | B 2 |
| $CH_3$ | $CH_3$ | $C(CH_3)(F)$ | S | 2-Cl, 4-Cl | B 2 |
| $CH_3$ | $CH_3$ | $C(C_2H_5)(OH)$ | S | 4-Cl | B 2 |

| $R^1$ | $R^2$ | X | Y | $W_n$ | B |
|---|---|---|---|---|---|
| $CH_3$ | $CH_3$ | $C(C_2H_5)(OH)$ | S | 2-Cl, 4-Cl | B 2 |
| $CH_3$ | $CH_3$ | $C(i-C_3H_7)(OH)$ | S | 4-Cl | B 2 |
| $CH_3$ | $CH_3$ | $C(i-C_3H_7)(OH)$ | S | 2-Cl, 4-Cl | B 2 |
| $CH_3$ | $CH_3$ | $C(i-C_3H_7)(OH)$ | S | 2-Cl, 4-$CH_3$ | B 2 |
| $CH_3$ | $CH_3$ | $C(i-C_3H_7)(OH)$ | S | 2-F, 4-$CH_3$ | B 2 |
| $CH_3$ | $CH_3$ | $CH(CH_3)$ | S | 4-Cl | B 2 |
| $CH_3$ | $CH_3$ | $CH(CH_3)$ | S | 2-Cl, 4-Cl | B 2 |
| $CH_3$ | $CH_3$ | $CH(CH_3)$ | S | 2-Cl, 4-$CH_3$ | B 2 |
| $CH_3$ | $CH_3$ | $CH(CH_3)$ | S | 2-F, 4-$CH_3$ | B 2 |
| Cl | $CH_3$ | $CH_2$ | S | 4-Cl | B 2 |

| $R^1$ | $R^2$ | X | Y | $W_n$ | B |
|---|---|---|---|---|---|
| Cl | $CH_3$ | $CH_2$ | S | 2-Cl, 4-Cl | B2 |
| Cl | $CH_3$ | $CH_2$ | S | 2-Cl, 4-$CH_3$ | B2 |
| Cl | $CH_3$ | $CH_2$ | S | 2-F, 4-$CH_3$ | B2 |
| $CH_3O$ | $CH_3$ | $CH_2$ | S | 4-Cl | B2 |
| $CH_3O$ | $CH_3$ | $CH_2$ | S | 2-Cl, 4-Cl | B2 |
| $CH_3O$ | $CH_3$ | $CH_2$ | S | 2-Cl, 4-$CH_3$ | B2 |
| $CH_3O$ | $CH_3$ | $CH_2$ | S | 2-F, 4-$CH_3$ | B2 |

wherein B1 to B18 are as defined below:

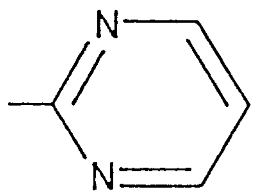

B1

,

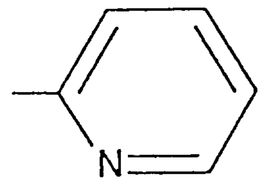

B2

,

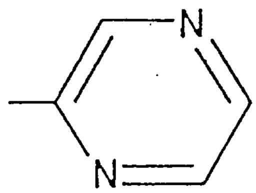

B3

,

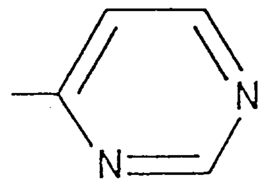

B4

,

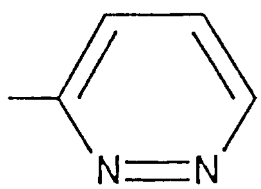

B5

,

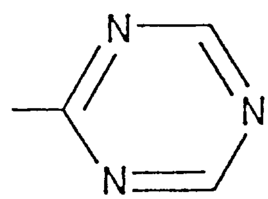

B6

,

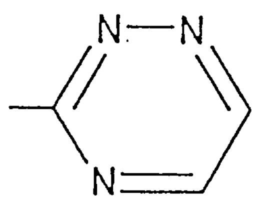

B7

,

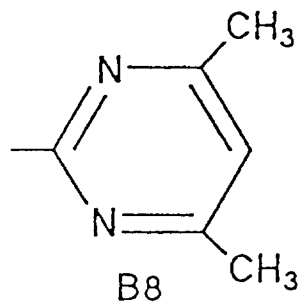

B8

,

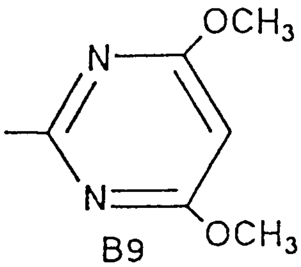
B9 ,

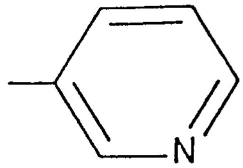
B10 ,

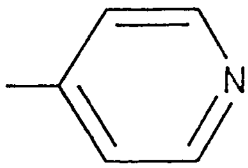
B11 ,

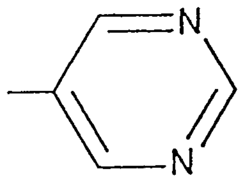
B12

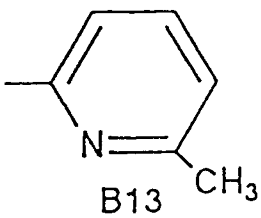
B13 ,

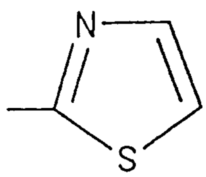
B14 ,

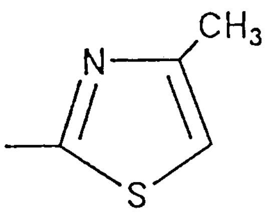
B15 ,

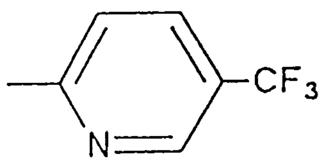
B16 ,

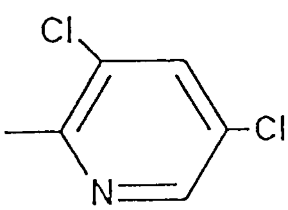
B17 ,

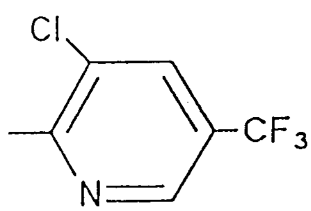
B18 ,

or wherein the substituted pyrazole derivatives are definied by the formula [1b]

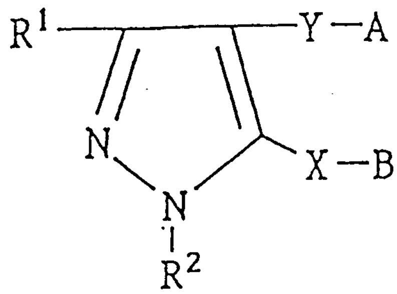

[1b]

having the following meanings of substituents

| $R^1$ | $R^2$ | X | Y | A | B |
|---|---|---|---|---|---|
| $CH_3$ | $CH_3$ | NH | S | A 1 | B 1 |
| $CH_3$ | $CH_3$ | NH | S | A 1 | B 2 |
| $CH_3$ | $CH_3$ | NH | S | A 2 | B 1 |

| $R^1$ | $R^2$ | X | Y | A | B |
|---|---|---|---|---|---|
| $CH_3$ | $CH_3$ | NH | S | A2 | B2 |
| $CH_3$ | $CH_3$ | NH | S | A3 | B1 |
| $CH_3$ | $CH_3$ | NH | S | A3 | B2 |
| $CH_3$ | $CH_3$ | NH | S | A4 | B1 |
| $CH_3$ | $CH_3$ | NH | S | A4 | B2 |
| $CH_3$ | $CH_3$ | NH | S | A5 | B1 |
| $CH_3$ | $CH_3$ | NH | S | A5 | B2 |
| $CH_3$ | $CH_3$ | NH | S | A6 | B1 |
| $CH_3$ | $CH_3$ | NH | S | A6 | B2 |
| $CH_3$ | $CH_3$ | NH | S | A7 | B1 |
| $CH_3$ | $CH_3$ | NH | S | A7 | B2 |
| $CH_3$ | $CH_3$ | NH | S | A8 | B1 |
| $CH_3$ | $CH_3$ | NH | S | A8 | B2 |
| $CH_3$ | $CH_3$ | NH | S | A9 | B1 |
| $CH_3$ | $CH_3$ | NH | S | A9 | B2 |
| $CH_3$ | $CH_3$ | NH | S | A10 | B1 |
| $CH_3$ | $CH_3$ | NH | S | A10 | B2 |
| $CH_3$ | $CH_3$ | NH | S | A11 | B1 |
| $CH_3$ | $CH_3$ | NH | S | A11 | B2 |

wherein A1 to A11 are as defined below

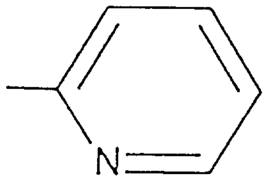
A1
,

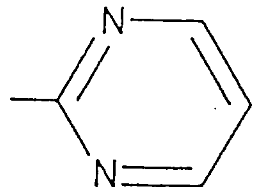
A2
,

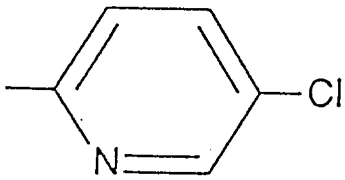
A3
,

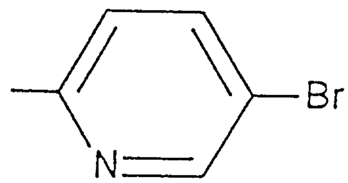
A4
,

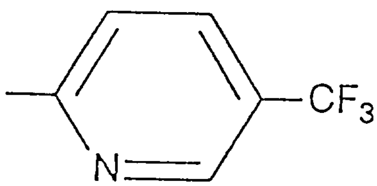
A5
,

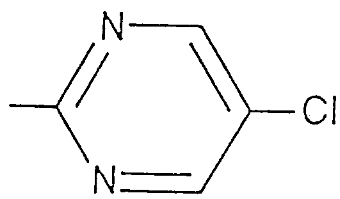
A6
,

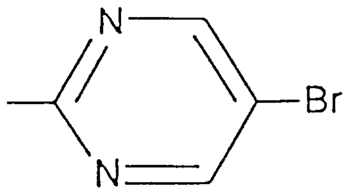
A7 ,
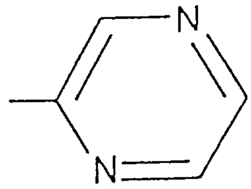
A8 ,

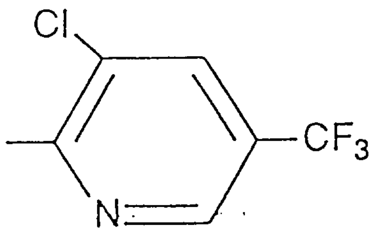
A9 ,
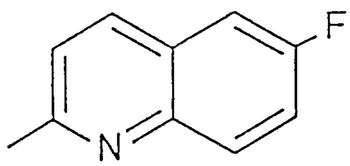
A10 ,

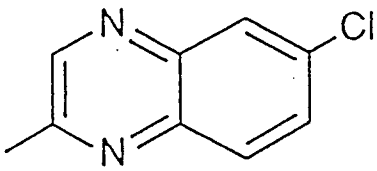
,
A11

and B1 and B2 are as defined above,
according to the following reaction schemes:

(Method 1)

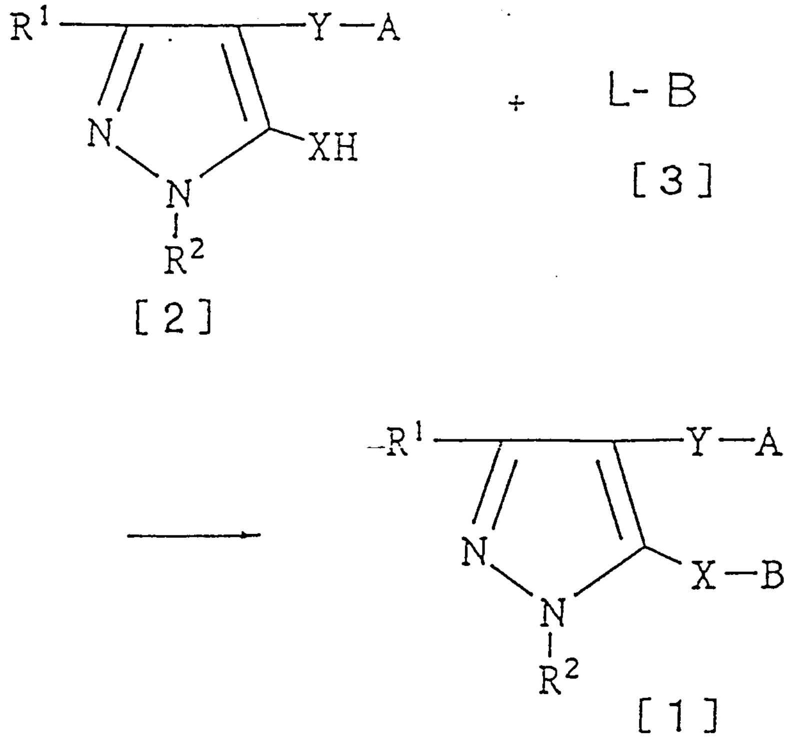

[2] + L-B [3] → [1]

wherein $R^1$, $R^2$, X, Y and A have the same meanings as mentioned above, L represents a leaving group and B has the same meaning as mentioned above,

(Method 2)

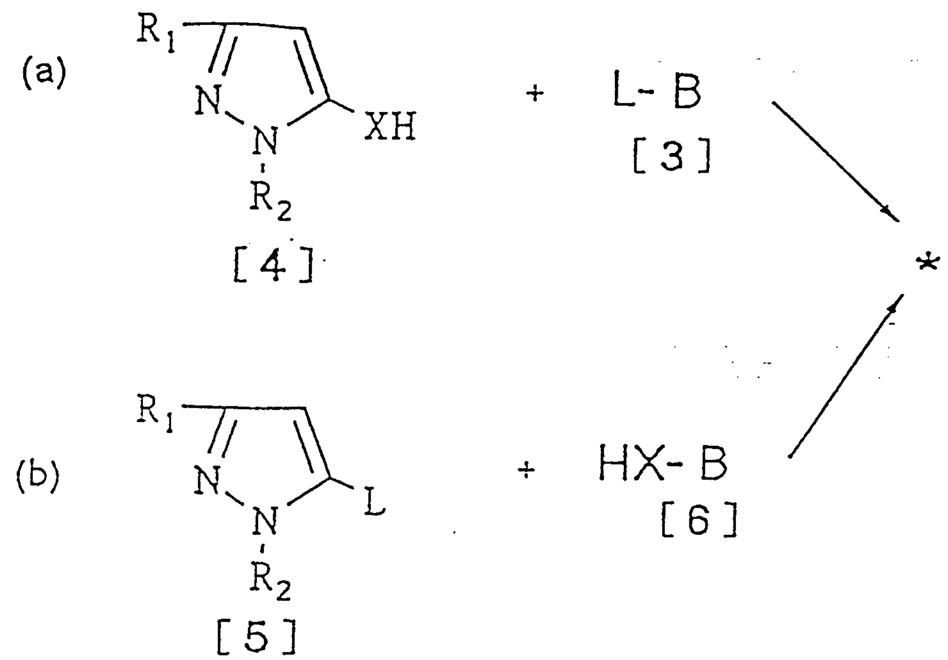

(a) [4] + L-B [3]

(b) [5] + HX-B [6]

When Y ≠ 0:

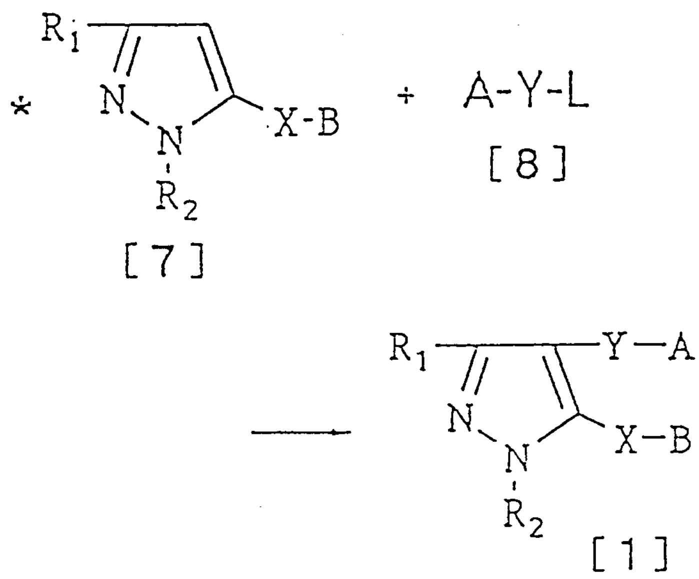

[7] + A-Y-L [8] [1]

wherein $R^1$, $R^2$, B, X and A have the same meanings as mentioned above and L represents a leaving group, and Y has the same meaning as mentioned above except oxygen atom,

(Method 3)

When X = N-$R^3$, $R^3 \neq$ H:

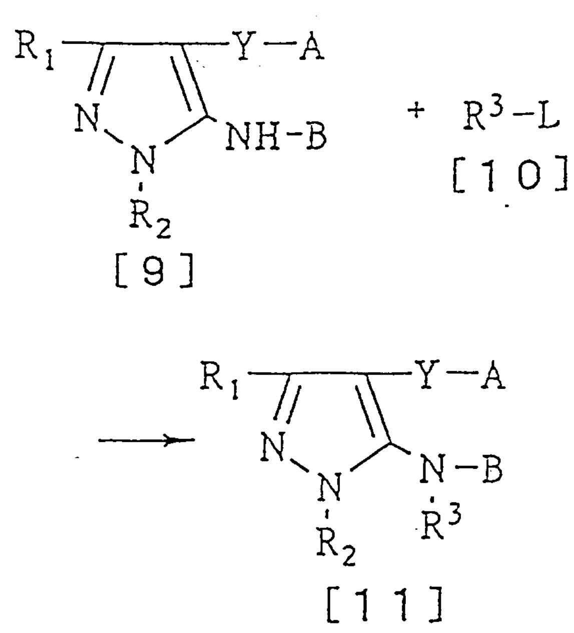

[9] [10] [11]

wherein $R^1$, $R^2$, A, B and Y have the same meaning as mentioned above, L represents a leaving group and $R^3$ has the same meaning as mentioned above except hydrogen atom.

2. A method of preparing substituted pyrazole derivatives as claimed in claim 1, wherein said substituted pyrazole derivatives are compounds of formula [1a].

3. A method of preparing substituted pyrazole derivatives as claimed in claim 1, in which X is -N($R^3$)-.

4. A method of preparing substituted pyrazole derivatives as claimed in claim 2, wherein Y is -S-.

5. A method of preparing substituted pyrazole derivatives as claimed in claim 1, which are compounds of the general formula [1a], wherein $R^1$ and $R^2$ each are an alkyl group, X is -N($R^3$)-, Y is -S- and B is B1, B2, B4, B 10, B11 or B12.

6. A fungicide for agricultural and horticultural use, containing one or more substituted pyrazoles as claimed in claim 1, as an active ingredient.

7. A method of preparing a fungicide for agricultural and horticultural use by preparing a fungicide containing one or more substituted pyrazoles as defined in claim 1 as an active ingredient.

**Patentansprüche**

**Patentansprüche für folgende Vertragsstaaten : AT, BE, CH, DE, DK, FR, GB, GR, IT, LI, LU, NL, SE**

1. Substituierte Pyrazolderivate, wobei diese substituierten Pyrazolderivate definiert sind durch eine allgemeinen Formel (1):

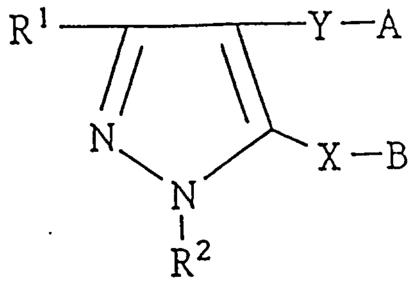

(1)

worin $R^1$ ein Wasserstoffatom, ein Halogenatom, eine Alkylgruppe, eine Alkoxygruppe, eine Alkylthiogruppe oder eine Haloalkylgruppe darstellt;
$R^2$ ein Wasserstoffatom, eine Alkylgruppe, eine Haloalkylgruppe, eine Phenylalkylgruppe, $-COR^6$ oder $-SO_2R^7$ darstellt;
X -S-, -SO-, $-SO_2-$, $-N(R^3)-$, -CO- oder $-C(R^4)(R^5)$-darstellt;
$R^3$ ein Wasserstoffatom, eine Alkylgruppe, eine Haloalkylgruppe, eine Alkenylgruppe, eine Alkinylgruppe, eine Alkoxyalkylgruppe, eine Cyanoalkylgruppe, eine Alkylcarbonylalkylgruppe, eine Alkoxycarbonylalkylgruppe, eine Nitrosogruppe, eine Aminogruppe, eine Phenylalkylgruppe, $-COR^6$ oder $-SO_2R^7$ darstellt;
$R^4$ und $R^5$ unabhängig ein Wasserstoffatom, ein Halogenatom, eine Alkylgruppe, eine Haloalkylgruppe, eine Alkenylgruppe, eine Alkinylgruppe oder $-OR^8$ darstellen;
$R^8$ ein Wasserstoffatom, eine Alkylgruppe, eine Haloalkylgruppe, eine Alkenylgruppe, eine Alkinylgruppe, eine Alkoxyalkylgruppe, eine Cyanoalkylgruppe, eine Alkylcarbonylalkylgruppe, eine Alkoxycarbonylalkylgruppe, eine Phenylalkylgruppe, $-COR^6$ oder $-SO_2R^7$ darstellt;
$R^6$ ein Wasserstoffatom, eine Alkylgruppe, eine Haloalkylgruppe, eine Phenylgruppe, eine Phenylalkylgruppe, eine Alkoxygruppe oder

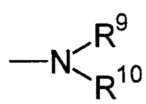

darstellt;
$R^7$ eine Alkylgruppe, eine Haloalkylgruppe, eine Phenylgruppe oder

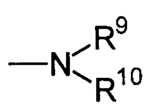

darstellt;
$R^9$ und $R^{10}$ unabhängig ein Wasserstoffatom, eine Alkylgruppe oder eine Phenylgruppe darstellen;
Y ein Sauerstoffatom, -S-, -SO- oder $-SO_2-$ darstellt;
A eine Phenylgruppe darstellt;
B darstellt:

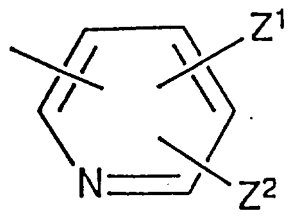

,

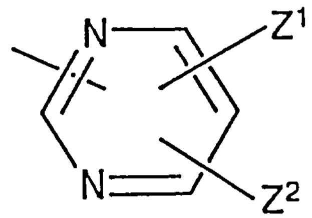

,

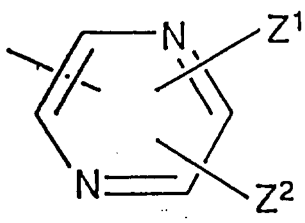

,

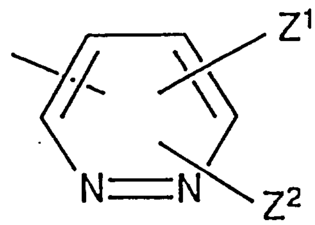

,

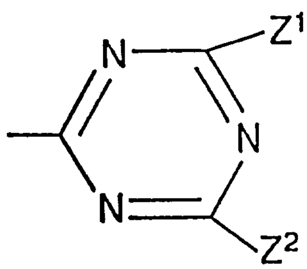

,

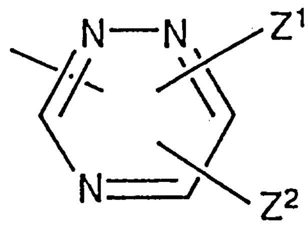

oder

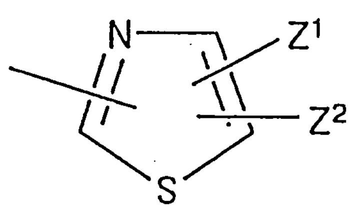

$Z^1$ und $Z^2$ unabhängig ein Wasserstoffatom, ein Halogenatom, eine Alkylgruppe, eine Alkoxygruppe oder eine Haloalkylgruppe darstellen,
wobei die substituierten Pyrazolderivate der allgemeinen Formel (1) Verbindungen der Formel (1a) einschliessen:

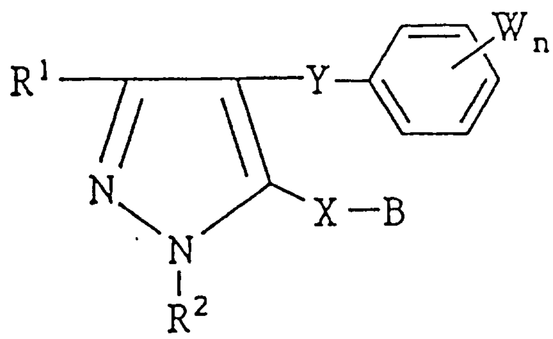

(1a)

mit den folgenden Substituentenbedeutungen:

| $R^1$ | $R^2$ | X | Y | $W_n$ | B |
|---|---|---|---|---|---|
| H | $CH_3$ | S | S | 4-Cl | B1 |
| $CH_3$ | $CH_3$ | S | S | 4-Cl | B1 |
| $CF_3$ | $CH_3$ | S | S | 4-Cl | B1 |
| $CH_3$ | $CH_3$ | S | S | 4-$CH_3$ | B1 |
| $CF_3$ | $CH_3$ | S | S | 4-$CH_3$ | B1 |
| $CH_3$ | H | S | S | 4-Cl | B1 |
| $CH_3$ | H | S | S | 4-$CH_3$ | B1 |
| $CH_3$ | $CF_3$ | S | S | 4-Cl | B1 |

| $R^1$ | $R^2$ | X | Y | $W_n$ | B |
|---|---|---|---|---|---|
| $CH_3$ | $CF_3$ | S | S | 4-$CH_3$ | B 1 |
| $CH_3$ | $CH_3$ | S | S | 4-$CF_3$ | B 1 |
| $CH_3$ | $CH_3$ | S | S | 4-Br | B 1 |
| $CH_3$ | $CH_3$ | S | S | 4-$NO_2$ | B 1 |
| $CH_3$ | $CH_3$ | S | S | 4-$OCH_3$ | B 1 |
| $CH_3$ | $CH_3$ | S | S | 4-$C_2H_5$ | B 1 |
| $CH_3$ | $CH_3$ | S | S | 2-Cl | B 1 |
| $CH_3$ | $CH_3$ | S | S | 2-Cl, 4-Cl | B 1 |
| $CH_3$ | $CH_3$ | S | S | 2-Cl, 4-$CH_3$ | B 1 |
| $CH_3$ | $CH_3$ | S | S | 4-Cl | B 2 |
| $CF_3$ | $CH_3$ | S | S | 4-Cl | B 2 |

| $R^1$ | $R^2$ | X | Y | $W_n$ | B |
|---|---|---|---|---|---|
| $CH_3$ | $CH_3$ | S | S | 4-$CH_3$ | B2 |
| $CF_3$ | $CH_3$ | S | S | 4-$CH_3$ | B2 |
| $CH_3$ | $CH_3$ | S | S | 2-Cl, 4-Cl | B2 |
| $CH_3$ | $CH_3$ | S | S | 2-Cl, 4-$CH_3$ | B2 |
| $CH_3$ | $CH_3$ | S | O | 4-Cl | B2 |
| $CH_3$ | $CH_3$ | S | O | 4-$CH_3$ | B2 |
| $CF_3$ | $CH_3$ | S | O | 4-$CH_3$ | B2 |
| $CH_3$ | $CH_3$ | S | O | 3-Cl, 4-$CH_3$ | B2 |
| $C_2H_5$ | $CH_3$ | S | S | H | B1 |
| $C_2H_5$ | $CH_3$ | S | S | 4-Cl | B1 |

| $R^1$ | $R^2$ | X | Y | $W_n$ | B |
|---|---|---|---|---|---|
| $C_2H_5$ | $CH_3$ | S | S | 4-$CH_3$ | B 1 |
| $C_2H_5$ | $CH_3$ | S | S | 4-$OCH_3$ | B 1 |
| $C_2H_5$ | $CH_3$ | S | S | 3-$CF_3$ | B 1 |
| $C_2H_5$ | $CH_3$ | S | S | 2-Cl, 4-Cl | B 1 |
| $C_2H_5$ | $CH_3$ | S | S | 3-Cl, 4-Cl | B 1 |
| $C_2H_5$ | $CH_3$ | S | S | 2-Cl, 4-$CH_3$ | B 1 |
| i-$C_3H_7$ | $CH_3$ | S | S | 4-Cl | B 1 |
| i-$C_3H_7$ | $CH_3$ | S | S | 4-$CH_3$ | B 1 |
| i-$C_3H_7$ | $CH_3$ | S | S | 4-$OCH_3$ | B 1 |
| t-$C_4H_9$ | $CH_3$ | S | S | 4-Cl | B 1 |

| $R^1$ | $R^2$ | X | Y | $W_n$ | B |
|---|---|---|---|---|---|
| t-$C_4H_9$ | $CH_3$ | S | S | 4-$CH_3$ | B 1 |
| t-$C_4H_9$ | $CH_3$ | S | S | 4-$OCH_3$ | B 1 |
| $CH_3$ | $C_2H_5$ | S | S | 4-Cl | B 1 |
| $CH_3$ | $C_3H_7$ | S | S | 4-Cl | B 1 |
| $CH_3$ | $CH_3$ | NH | S | H | B 1 |
| $CF_3$ | $CH_3$ | NH | S | H | B 1 |
| $CH_3$ | $CH_3$ | NH | S | 4-Cl | B 1 |
| $CF_3$ | $CH_3$ | NH | S | 4-Cl | B 1 |
| H | $CH_3$ | NH | S | 4-Cl | B 1 |
| $CH_3$ | $CH_3$ | NH | S | 4-$CH_3$ | B 1 |

| $R^1$ | $R^2$ | X | Y | $W_n$ | B |
|---|---|---|---|---|---|
| $CF_3$ | $CH_3$ | NH | S | 4-$CH_3$ | B 1 |
| H | $CH_3$ | NH | S | 4-$CH_3$ | B 1 |
| $CH_3$ | $CH_3$ | NH | S | 4-F | B 1 |
| $CH_3$ | $CH_3$ | NH | S | 4-Br | B 1 |
| $CH_3$ | $CH_3$ | NH | S | 4-I | B 1 |
| $CH_3$ | $CH_3$ | NH | S | 2-F | B 1 |
| $CH_3$ | $CH_3$ | NH | S | 2-Cl | B 1 |
| $CH_3$ | $CH_3$ | NH | S | 2-Br | B 1 |
| $CH_3$ | $CH_3$ | NH | S | 2-I | B 1 |
| $CH_3$ | $CH_3$ | NH | S | 3-F | B 1 |

| $R^1$ | $R^2$ | X | Y | $W_n$ | B |
|---|---|---|---|---|---|
| $CH_3$ | $CH_3$ | NH | S | 3-Cl | B 1 |
| $CH_3$ | $CH_3$ | NH | S | 3-Br | B 1 |
| $CH_3$ | $CH_3$ | NH | S | 3-I | B 1 |
| $CH_3$ | $CH_3$ | NH | S | $2-OCH_3$ | B 1 |
| $CH_3$ | $CH_3$ | NH | S | $3-OCH_3$ | B 1 |
| $CH_3$ | $CH_3$ | NH | S | $4-OCH_3$ | B 1 |
| $CH_3$ | $CH_3$ | NH | S | $2-CF_3$ | B 1 |
| $CH_3$ | $CH_3$ | NH | S | $3-CF_3$ | B 1 |
| $CH_3$ | $CH_3$ | NH | S | $4-CF_3$ | B 1 |
| $CH_3$ | $CH_3$ | NH | S | $4-C_2H_5$ | B 1 |

| $R^1$ | $R^2$ | X | Y | $W_n$ | B |
|---|---|---|---|---|---|
| $CH_3$ | $CH_3$ | NH | S | $4-C_3H_7$ | B 1 |
| $CH_3$ | $CH_3$ | NH | S | $4-C_4H_9$ | B 1 |
| $CH_3$ | $CH_3$ | NH | S | $4-i-C_3H_7$ | B 1 |
| $CH_3$ | $CH_3$ | NH | S | $4-t-C_4H_9$ | B 1 |
| $CH_3$ | $CH_3$ | NH | S | $2-OCF_3$ | B 1 |
| $CH_3$ | $CH_3$ | NH | S | $3-OCF_3$ | B 1 |
| $CH_3$ | $CH_3$ | NH | S | $4-OCF_3$ | B 1 |
| $CH_3$ | $CH_3$ | NH | S | $3-NO_2$ | B 1 |
| $CH_3$ | $CH_3$ | NH | S | $4-NO_2$ | B 1 |
| $CH_3$ | $CH_3$ | NH | S | $3-NH_2$ | B 1 |

| $R^1$ | $R^2$ | X | Y | $W_n$ | B |
|---|---|---|---|---|---|
| $CH_3$ | $CH_3$ | NH | S | 4-$NH_2$ | B1 |
| $CH_3$ | $CH_3$ | NH | S | 4-$NHCOCH_3$ | B1 |
| $CH_3$ | $CH_3$ | NH | S | 4-$NHSO_2CH_3$ | B1 |
| $CH_3$ | $CH_3$ | NH | S | 4-$NHCOCF_3$ | B1 |
| $CH_3$ | $CH_3$ | NH | S | 4-$NHSO_2CF_3$ | B1 |
| $CH_3$ | $CH_3$ | NH | S | 4-Ph | B1 |
| $CH_3$ | $CH_3$ | NH | S | 4-OPh | B1 |
| $CH_3$ | $CH_3$ | NH | S | 4-$OCF_2CF_2H$ | B1 |
| $CH_3$ | $CH_3$ | NH | S | 4-$COCH_3$ | B1 |
| $CH_3$ | $CH_3$ | NH | S | 4-NHCOPh | B1 |

| $R^1$ | $R^2$ | X | Y | $W_n$ | B |
|---|---|---|---|---|---|
| $CH_3$ | $CH_3$ | NH | S | 4-$NHCOOCH_3$ | B 1 |
| $CH_3$ | $CH_3$ | NH | S | 4-$NHCON(CH_3)_2$ | B 1 |
| $CH_3$ | $CH_3$ | NH | S | 2-Cl, 3-Cl | B 1 |
| $CH_3$ | $CH_3$ | NH | S | 2-Cl, 4-Cl | B 1 |
| $CH_3$ | $CH_3$ | NH | S | 2-Cl, 5-Cl | B 1 |
| $CH_3$ | $CH_3$ | NH | S | 2-Cl, 6-Cl | B 1 |
| $CH_3$ | $CH_3$ | NH | S | 3-Cl, 4-Cl | B 1 |
| $CH_3$ | $CH_3$ | NH | S | 3-Cl, 5-Cl | B 1 |
| $CH_3$ | $CH_3$ | NH | S | 2-$CH_3$, 3-$CH_3$ | B 1 |
| $CH_3$ | $CH_3$ | NH | S | 2-$CH_3$, 4-$CH_3$ | B 1 |

| $R^1$ | $R^2$ | X | Y | $W_n$ | B |
|---|---|---|---|---|---|
| $CH_3$ | $CH_3$ | NH | S | 2-$CH_3$, 5-$CH_3$ | B 1 |
| $CH_3$ | $CH_3$ | NH | S | 2-$CH_3$, 6-$CH_3$ | B 1 |
| $CH_3$ | $CH_3$ | NH | S | 3-$CH_3$, 4-$CH_3$ | B 1 |
| $CH_3$ | $CH_3$ | NH | S | 3-$CH_3$, 5-$CH_3$ | B 1 |
| $CH_3$ | $CH_3$ | NH | S | 2-Cl, 3-$CH_3$ | B 1 |
| $CH_3$ | $CH_3$ | NH | S | 2-Cl, 4-$CH_3$ | B 1 |
| $CH_3$ | $CH_3$ | NH | S | 2-Cl, 5-$CH_3$ | B 1 |
| $CH_3$ | $CH_3$ | NH | S | 3-Cl, 4-$CH_3$ | B 1 |
| $CH_3$ | $CH_3$ | NH | S | 3-Cl, 5-$CH_3$ | B 1 |
| $CH_3$ | $CH_3$ | NH | S | 2-$CH_3$, 3-Cl | B 1 |

| $R^1$ | $R^2$ | X | Y | $W_n$ | B |
|---|---|---|---|---|---|
| $CH_3$ | $CH_3$ | NH | S | 2-$CH_3$, 4-Cl | B 1 |
| $CH_3$ | $CH_3$ | NH | S | 2-$CH_3$, 5-Cl | B 1 |
| $CH_3$ | $CH_3$ | NH | S | 2-F, 3-F | B 1 |
| $CH_3$ | $CH_3$ | NH | S | 2-F, 4-F | B 1 |
| $CH_3$ | $CH_3$ | NH | S | 2-F, 5-F | B 1 |
| $CH_3$ | $CH_3$ | NH | S | 2-F, 6-F | B 1 |
| $CH_3$ | $CH_3$ | NH | S | 2-F, 3-Cl | B 1 |
| $CH_3$ | $CH_3$ | NH | S | 2-F, 4-Cl | B 1 |
| $CH_3$ | $CH_3$ | NH | S | 2-F, 5-Cl | B 1 |
| $CH_3$ | $CH_3$ | NH | S | 2-F, 6-Cl | B 1 |

| $R^1$ | $R^2$ | X | Y | $W_n$ | B |
|---|---|---|---|---|---|
| $CH_3$ | $CH_3$ | NH | S | 2-F, 3-$CH_3$ | B 1 |
| $CH_3$ | $CH_3$ | NH | S | 2-F, 4-$CH_3$ | B 1 |
| $CH_3$ | $CH_3$ | NH | S | 2-F, 3-Br | B 1 |
| $CH_3$ | $CH_3$ | NH | S | 2-F, 4-Br | B 1 |
| $CH_3$ | $CH_3$ | NH | S | 3-F, 4-Cl | B 1 |
| $CH_3$ | $CH_3$ | NH | S | 3-F, 5-Cl | B 1 |
| $CH_3$ | $CH_3$ | NH | S | 3-F, 4-$CH_3$ | B 1 |
| $CH_3$ | $CH_3$ | NH | S | 3-F, 5-$CH_3$ | B 1 |
| $CH_3$ | $CH_3$ | NH | S | 2-Br, 4-Cl | B 1 |
| $CH_3$ | $CH_3$ | NH | S | 2-Br, 4-Br | B 1 |

| $R^1$ | $R^2$ | X | Y | $W_n$ | B |
|---|---|---|---|---|---|
| $CH_3$ | $CH_3$ | NH | S | 2-Br, 4-$CH_3$ | B 1 |
| $CH_3$ | $CH_3$ | NH | S | 3-Br, 4-Cl | B 1 |
| $CH_3$ | $CH_3$ | NH | S | 3-Br, 4-Br | B 1 |
| $CH_3$ | $CH_3$ | NH | S | 3-Br, 4-$CH_3$ | B 1 |
| $CH_3$ | $CH_3$ | NH | S | 2-Cl, 4-Br | B 1 |
| $CH_3$ | $CH_3$ | NH | S | 3-Cl, 4-Br | B 1 |
| $CH_3$ | $CH_3$ | NH | S | 2-Cl, 4-I | B 1 |
| $CH_3$ | $CH_3$ | NH | S | 3-Cl, 4-I | B 1 |
| $CH_3$ | $CH_3$ | NH | S | 3-F, 4-Br | B 1 |
| $CH_3$ | $CH_3$ | NH | S | 3, 4-$OCH_2O$- | B 1 |

| $R^1$ | $R^2$ | X | Y | $W_n$ | B |
|---|---|---|---|---|---|
| $CH_3$ | $CH_3$ | NH | S | 2-$CF_3$, 3-Cl | B 1 |
| $CH_3$ | $CH_3$ | NH | S | 2-$CF_3$, 4-Cl | B 1 |
| $CH_3$ | $CH_3$ | NH | S | 2-$CF_3$, 3-Br | B 1 |
| $CH_3$ | $CH_3$ | NH | S | 2-$CF_3$, 4-Br | B 1 |
| $CH_3$ | $CH_3$ | NH | S | 2-F, 4-$NO_2$ | B 1 |
| $CH_3$ | $CH_3$ | NH | S | 3-F, 4-$NO_2$ | B 1 |
| $CH_3$ | $CH_3$ | NH | S | 2-Cl, 4-$NO_2$ | B 1 |
| $CH_3$ | $CH_3$ | NH | S | 3-Cl, 4-$NO_2$ | B 1 |
| $CH_3$ | $CH_3$ | NH | S | 2-F, 4-$OCH_3$ | B 1 |
| $CH_3$ | $CH_3$ | NH | S | 3-F, 4-$OCH_3$ | B 1 |

| $R^1$ | $R^2$ | X | Y | $W_n$ | B |
|---|---|---|---|---|---|
| $CH_3$ | $CH_3$ | NH | S | 2-Cl, 4-$OCH_3$ | B 1 |
| $CH_3$ | $CH_3$ | NH | S | 3-Cl, 4-$OCH_3$ | B 1 |
| $CH_3$ | $CH_3$ | NH | S | 2-$OCH_3$, 4-$OCH_3$ | B 1 |
| $CH_3$ | $CH_3$ | NH | S | 3-$OCH_3$, 4-$OCH_3$ | B 1 |
| $CH_3$ | $CH_3$ | NH | S | 2-Cl, 3-Cl, 4-Cl | B 1 |
| $CH_3$ | $CH_3$ | NH | S | 2-Cl, 4-Cl, 5-Cl | B 1 |
| $CH_3$ | $CH_3$ | NH | S | 2-Cl, 4-Cl, 6-Cl | B 1 |
| $CH_3$ | $CH_3$ | NH | S | 3-Cl, 4-Cl, 5-Cl | B 1 |
| $CH_3$ | $CH_3$ | NH | S | 2-Cl, 3-Cl, 4-$CH_3$ | B 1 |
| $CH_3$ | $CH_3$ | NH | S | 2-Cl, 3-Cl, 4-Br | B 1 |

| $R^1$ | $R^2$ | X | Y | $W_n$ | B |
|---|---|---|---|---|---|
| $CH_3$ | $CH_3$ | NH | S | 2-Cl, 3-Cl, 4-I | B 1 |
| $CH_3$ | $CH_3$ | NH | S | 2-Cl, 4-Br, 6-Cl | B 1 |
| $CH_3$ | $CH_3$ | NH | S | 2-Cl, 4-$CH_3$, 6-Cl | B 1 |
| $CH_3$ | $CH_3$ | NH | S | 2-Br, 4-Cl, 6-Cl | B 1 |
| $CH_3$ | $CH_3$ | NCHO | S | 4-Cl | B 1 |
| $CH_3$ | $CH_3$ | $NCOCH_3$ | S | 4-Cl | B 1 |
| $CH_3$ | $CH_3$ | $NCOOCH_3$ | S | 4-Cl | B 1 |
| $CH_3$ | $CH_3$ | $NCON(CH_3)_2$ | S | 4-Cl | B 1 |
| $CH_3$ | $CH_3$ | NCONHPh | S | 4-Cl | B 1 |
| $CH_3$ | $CH_3$ | $NCH_3$ | S | 4-Cl | B 1 |

| $R^1$ | $R^2$ | X | Y | $W_n$ | B |
|---|---|---|---|---|---|
| $CH_3$ | $CH_3$ | $NC_2H_5$ | S | 4-Cl | B1 |
| $CH_3$ | $CH_3$ | $NC_3H_7$ | S | 4-Cl | B1 |
| $CH_3$ | $CH_3$ | $NCH_2OCH_3$ | S | 4-Cl | B1 |
| $CH_3$ | $CH_3$ | $NCH_2CH_2OCH_3$ | S | 4-Cl | B1 |
| $CH_3$ | $CH_3$ | $NCH_2CH=CH_2$ | S | 4-Cl | B1 |
| $CH_3$ | $CH_3$ | $NCH_2C\equiv CH$ | S | 4-Cl | B1 |
| $CH_3$ | $CH_3$ | $NCH_2COCH_3$ | S | 4-Cl | B1 |
| $CH_3$ | $CH_3$ | $NCH_2COOCH_3$ | S | 4-Cl | B1 |
| $CH_3$ | $CH_3$ | $NCH_2COOC_2H_5$ | S | 4-Cl | B1 |
| $CH_3$ | $CH_3$ | $NCH_2CN$ | S | 4-Cl | B1 |

| $R^1$ | $R^2$ | X | Y | $W_n$ | B |
|---|---|---|---|---|---|
| $CH_3$ | $CH_3$ | $NCH_2Ph$ | S | 4-Cl | B 1 |
| $CH_3$ | $CH_3$ | $NCH_2C_6H_4$-4-Cl | S | 4-Cl | B 1 |
| $CH_3$ | $CH_3$ | $NCH_2C_6H_4$-4-$CH_3$ | S | 4-Cl | B 1 |
| $CH_3$ | $CH_3$ | $NCH_2CH_2Ph$ | S | 4-Cl | B 1 |
| $CH_3$ | $CH_3$ | $NSO_2CH_3$ | S | 4-Cl | B 1 |
| $CH_3$ | $CH_3$ | $NSO_2N(CH_3)_2$ | S | 4-Cl | B 1 |
| $CH_3$ | $CH_3$ | NCHO | S | 4-Cl | B 2 |
| $CH_3$ | $CH_3$ | $NCH_3$ | S | 4-Cl | B 2 |
| $CH_3$ | H | NH | S | 4-Cl | B 1 |
| $CH_3$ | H | NH | S | 2-Cl, 4-Cl | B 1 |

| $R^1$ | $R^2$ | X | Y | $W_n$ | B |
|---|---|---|---|---|---|
| $CH_3$ | H | NH | S | 3-Cl, 4-$CH_3$ | B 1 |
| $CH_3$ | $C_2H_5$ | NH | S | 4-Cl | B 1 |
| $CH_3$ | $C_2H_5$ | NH | S | 4-Br | B 1 |
| $CH_3$ | $C_2H_5$ | NH | S | 2-Cl, 4-Cl | B 1 |
| $CH_3$ | $C_2H_5$ | NH | S | 2-Cl, 4-$CH_3$ | B 1 |
| $CH_3$ | i-$C_3H_7$ | NH | S | 4-Cl | B 1 |
| $CH_3$ | i-$C_3H_7$ | NH | S | 4-Br | B 1 |
| $CH_3$ | i-$C_3H_7$ | NH | S | 2-Cl, 4-Cl | B 1 |
| $CH_3$ | i-$C_3H_7$ | NH | S | 2-Cl, 4-$CH_3$ | B 1 |
| $CH_3$ | t-$C_4H_9$ | NH | S | 4-Cl | B 1 |

| $R^1$ | $R^2$ | X | Y | $W_n$ | B |
|---|---|---|---|---|---|
| $CH_3$ | t-$C_4H_9$ | NH | S | 2-Cl, 4-Cl | B 1 |
| $CH_3$ | t-$C_4H_9$ | NH | S | 3-Cl, 4-$CH_3$ | B 1 |
| $CH_3$ | $CF_3$ | NH | S | 4-Cl | B 1 |
| $CH_3$ | $CF_3$ | NH | S | 4-Br | B 1 |
| $CH_3$ | $CF_3$ | NH | S | 2-Cl, 4-Cl | B 1 |
| $CH_3$ | $CF_3$ | NH | S | 3-Cl, 4-$CH_3$ | B 1 |
| $CH_3$ | H | NH | S | 4-Cl | B 2 |
| $CH_3$ | H | NH | S | 2-Cl, 4-Cl | B 2 |
| $CH_3$ | $C_2H_5$ | NH | S | 4-Cl | B 2 |
| $CH_3$ | i-$C_3H_7$ | NH | S | 4-Cl | B 2 |

| $R^1$ | $R^2$ | X | Y | $W_n$ | B |
|---|---|---|---|---|---|
| $CH_3$ | $t-C_4H_9$ | NH | S | 4-Cl | B 2 |
| $CH_3$ | $CF_3$ | NH | S | 2-Cl, 4-Cl | B 2 |
| $CH_3$ | $CH_2Ph$ | NH | S | 4-Cl | B 1 |
| $CH_3$ | $CH_2Ph$ | NH | S | 2-Cl, 4-Cl | B 1 |
| $CH_3$ | $CH_2Ph$ | NCHO | S | 2-Cl, 4-Cl | B 1 |
| $CH_3$ | CHO | NH | S | 4-Cl | B 1 |
| $CH_3$ | CHO | NCHO | S | 4-Cl | B 1 |
| $CH_3$ | CHO | NH | S | 2-Cl, 4-Cl | B 1 |
| $CH_3$ | CHO | NCHO | S | 2-Cl, 4-Cl | B 1 |
| $CH_3$ | $COCH_3$ | NH | S | 4-Cl | B 1 |

| $R^1$ | $R^2$ | X | Y | $W_n$ | B |
|---|---|---|---|---|---|
| $CH_3$ | $COCH_3$ | NH | S | 4-Br | B 1 |
| $CH_3$ | $COCH_3$ | NH | S | 2-Cl, 4-Cl | B 1 |
| $CH_3$ | $COCH_3$ | NCHO | S | 2-Cl, 4-Cl | B 1 |
| $CH_3$ | $SO_2CH_3$ | NH | S | 4-Cl | B 1 |
| $CH_3$ | $SO_2CH_3$ | NH | S | 2-Cl, 4-Cl | B 1 |
| $CH_3$ | $SO_2CH_3$ | NH | S | 3-Cl, 4-$CH_3$ | B 1 |
| $CH_3$ | $CONHCH_3$ | NH | S | 4-Cl | B 1 |
| $CH_3$ | $CONHCH_3$ | NH | S | 4-Br | B 1 |
| $CH_3$ | $CONHCH_3$ | NH | S | 2-Cl, 4-Cl | B 1 |
| $CH_3$ | $CONHCH_3$ | NH | S | 3-Cl, 4-$CH_3$ | B 1 |

| $R^1$ | $R^2$ | X | Y | $W_n$ | B |
|---|---|---|---|---|---|
| $CH_3$ | $CH_2Ph$ | NH | S | 4-Cl | B2 |
| $CH_3$ | $CH_2Ph$ | NH | S | 2-Cl, 4-Cl | B2 |
| $CH_3$ | $COCH_3$ | NH | S | 4-Cl | B2 |
| $CH_3$ | $CONHCH_3$ | NH | S | 4-Cl | B2 |
| $CH_3$ | $SO_2CH_3$ | NH | S | 4-Cl | B2 |
| $CH_3$ | $SO_2CH_3$ | NH | S | 2-Cl, 4-Cl | B2 |
| H | $CH_3$ | NH | S | 4-Cl | B1 |
| H | $CH_3$ | NH | S | 2-Cl, 4-Cl | B1 |
| H | $CH_3$ | NH | S | 3-Cl, 4-$CH_3$ | B1 |
| $CF_3$ | $CH_3$ | NH | S | 4-Cl | B1 |

| $R^1$ | $R^2$ | X | Y | $W_n$ | B |
|---|---|---|---|---|---|
| $CF_3$ | $CH_3$ | NH | S | 4-Br | B1 |
| $CF_3$ | $CH_3$ | NH | S | 2-Cl, 4-Cl | B1 |
| $CF_3$ | $CH_3$ | NH | S | 2-Cl, 4-$CH_3$ | B1 |
| $C_2H_5$ | $CH_3$ | NH | S | 4-Cl | B1 |
| $C_2H_5$ | $CH_3$ | NH | S | 4-Br | B1 |
| $C_2H_5$ | $CH_3$ | NH | S | 2-Cl, 4-Cl | B1 |
| $C_2H_5$ | $CH_3$ | NH | S | 2-Cl, 4-$CH_3$ | B1 |
| i-$C_3H_7$ | $CH_3$ | NH | S | 4-Cl | B1 |
| i-$C_3H_7$ | $CH_3$ | NH | S | 2-Cl, 4-Cl | B1 |
| i-$C_3H_7$ | $CH_3$ | NH | S | 3-Cl, 4-$CH_3$ | B1 |

| $R^1$ | $R^2$ | X | Y | $W_n$ | B |
|---|---|---|---|---|---|
| t-$C_4H_9$ | $CH_3$ | NH | S | 4-Cl | B 1 |
| t-$C_4H_9$ | $CH_3$ | NH | S | 4-Br | B 1 |
| t-$C_4H_9$ | $CH_3$ | NH | S | 2-Cl, 4-Cl | B 1 |
| t-$C_4H_9$ | $CH_3$ | NH | S | 3-Cl, 4-$CH_3$ | B 1 |
| H | $CH_3$ | NH | S | 4-Cl | B 2 |
| H | $CH_3$ | NH | S | 2-Cl, 4-Cl | B 2 |
| $C_2H_5$ | $CH_3$ | NH | S | 4-Cl | B 2 |
| i-$C_3H_7$ | $CH_3$ | NH | S | 4-Cl | B 2 |
| $CF_3$ | $CH_3$ | NH | S | 4-Cl | B 2 |
| $CF_3$ | $CH_3$ | NH | S | 2-Cl, 4-Cl | B 2 |

| $R^1$ | $R^2$ | X | Y | $W_n$ | B |
|---|---|---|---|---|---|
| Cl | $CH_3$ | NH | S | 4-Cl | B 1 |
| Cl | $CH_3$ | NH | S | 2-Cl, 4-Cl | B 1 |
| Cl | $CH_3$ | NH | S | 3-Cl, 4-$CH_3$ | B 1 |
| Cl | $CH_3$ | NCHO | S | 4-Cl | B 1 |
| Cl | $CH_3$ | NH | S | 4-Br | B 1 |
| Cl | $CH_3$ | NCHO | S | 2-Cl, 4-Cl | B 1 |
| Cl | $CH_3$ | NCHO | S | 2-Cl, 4-$CH_3$ | B 1 |
| $CH_3O$ | $CH_3$ | NH | S | 4-Cl | B 1 |
| $CH_3O$ | $CH_3$ | NH | S | 4-Br | B 1 |
| $CH_3O$ | $CH_3$ | NH | S | 2-Cl, 4-Cl | B 1 |

| $R^1$ | $R^2$ | X | Y | $W_n$ | B |
|---|---|---|---|---|---|
| $CH_3O$ | $CH_3$ | NH | S | 2-Cl, 4-$CH_3$ | B1 |
| $CH_3O$ | $CH_3$ | NCHO | S | 4-Cl | B1 |
| $CH_3O$ | $CH_3$ | NCHO | S | 2-Cl, 4-Cl | B1 |
| $CH_3O$ | $CH_3$ | NCHO | S | 3-Cl, 4-$CH_3$ | B1 |
| $CH_3S$ | $CH_3$ | NH | S | 4-Cl | B1 |
| $CH_3S$ | $CH_3$ | NH | S | 4-Br | B1 |
| $CH_3S$ | $CH_3$ | NH | S | 2-Cl, 4-Cl | B1 |
| $CH_3S$ | $CH_3$ | NH | S | 3-Cl, 4-$CH_3$ | B1 |
| $CH_3S$ | $CH_3$ | NCHO | S | 4-Cl | B1 |
| $CH_3S$ | $CH_3$ | NCHO | S | 2-Cl, 4-Cl | B1 |

| $R^1$ | $R^2$ | X | Y | $W_n$ | B |
|---|---|---|---|---|---|
| Cl | $CH_3$ | NH | S | 4-Cl | B2 |
| Cl | $CH_3$ | NH | S | 2-Cl, 4-Cl | B2 |
| $CH_3O$ | $CH_3$ | NH | S | 2-Cl, 4-Cl | B2 |
| $CH_3S$ | $CH_3$ | NH | S | 2-Cl, 4-Cl | B2 |
| $CH_3$ | $CH_3$ | SO | S | 4-Cl | B1 |
| $CH_3$ | $CH_3$ | $SO_2$ | S | 4-Cl | B1 |
| $CH_3$ | $CH_3$ | NH | SO | 4-Cl | B1 |
| $CH_3$ | $CH_3$ | NH | $SO_2$ | 4-Cl | B1 |
| $CH_3$ | $CH_3$ | NH | SO | 2-Cl, 4-Cl | B1 |
| $CH_3$ | $CH_3$ | NH | $SO_2$ | 2-Cl, 4-Cl | B1 |

| $R^1$ | $R^2$ | X | Y | $W_n$ | B |
|---|---|---|---|---|---|
| $CH_3$ | $CH_3$ | NH | SO | 3-Cl, 4-$CH_3$ | B 1 |
| $CH_3$ | $CH_3$ | NH | $SO_2$ | 3-Cl, 4-$CH_3$ | B 1 |
| $CH_3$ | $CH_3$ | S | O | 4-Cl | B 1 |
| $CH_3$ | $CH_3$ | SO | O | 4-Cl | B 1 |
| $CH_3$ | $CH_3$ | $SO_2$ | O | 4-Cl | B 1 |
| $CH_3$ | $CH_3$ | S | O | 2-Cl, 4-Cl | B 1 |
| $CH_3$ | $CH_3$ | SO | O | 2-Cl, 4-Cl | B 1 |
| $CH_3$ | $CH_3$ | $SO_2$ | O | 2-Cl, 4-Cl | B 1 |
| $CH_3$ | $CH_3$ | NH | O | 4-Cl | B 1 |
| $CH_3$ | $CH_3$ | NH | O | 4-$CH_3$ | B 1 |

| $R^1$ | $R^2$ | X | Y | $W_n$ | B |
|---|---|---|---|---|---|
| $CH_3$ | $CH_3$ | NH | O | 2-Cl, 4-Cl | B 1 |
| $CH_3$ | $CH_3$ | NH | O | 2-Cl, 4-$CH_3$ | B 1 |
| $CH_3$ | $CH_3$ | NH | O | 3-Cl, 4-$CH_3$ | B 1 |
| $CH_3$ | $CH_3$ | NH | O | 2-F, 4-Cl | B 1 |
| $CH_3$ | $CH_3$ | NCHO | O | 4-Cl | B 1 |
| $CH_3$ | $CH_3$ | $NCH_3$ | O | 4-Cl | B 1 |
| $CH_3$ | $CH_3$ | NH | O | 4-Cl | B 2 |
| $CH_3$ | $CH_3$ | NH | O | 2-Cl, 4-Cl | B 2 |
| $CH_3$ | $CH_3$ | NH | S | 4-F | B 2 |
| $CH_3$ | $CH_3$ | NH | S | 4-Cl | B 2 |

| $R^1$ | $R^2$ | X | Y | $W_n$ | B |
|---|---|---|---|---|---|
| $CH_3$ | $CH_3$ | NH | S | $4-CH_3$ | B2 |
| $CH_3$ | $CH_3$ | NH | S | 2-Cl, 4-Cl | B2 |
| $CH_3$ | $CH_3$ | NH | S | 3-Cl, 4-Cl | B2 |
| $CH_3$ | $CH_3$ | NH | S | $2-Cl, 4-CH_3$ | B2 |
| $CH_3$ | $CH_3$ | NH | S | $3-Cl, 4-CH_3$ | B2 |
| $CH_3$ | $CH_3$ | NH | S | 2-F, 4-F | B2 |
| $CH_3$ | $CH_3$ | NH | S | 2-F, 4-Cl | B2 |
| $CH_3$ | $CH_3$ | NH | S | 2-F, 4-Br | B2 |
| $CH_3$ | $CH_3$ | NH | S | $2-F, 4-CH_3$ | B2 |
| $CH_3$ | $CH_3$ | NH | S | $3-F, 4-CH_3$ | B2 |

| $R^1$ | $R^2$ | X | Y | $W_n$ | B |
|---|---|---|---|---|---|
| $CH_3$ | $CH_3$ | NCHO | S | 4-Cl | B 2 |
| $CH_3$ | $CH_3$ | NCHO | S | 2-Cl, 4-Cl | B 2 |
| $CH_3$ | $CH_3$ | NCHO | S | 3-Cl, 4-$CH_3$ | B 2 |
| $CH_3$ | $CH_3$ | NH | S | 4-Cl | B 3 |
| $CH_3$ | $CH_3$ | NH | S | 4-Br | B 3 |
| $CH_3$ | $CH_3$ | NH | S | 2-Cl, 4-Cl | B 3 |
| $CH_3$ | $CH_3$ | NH | S | 2-Cl, 4-$CH_3$ | B 3 |
| $CH_3$ | $CH_3$ | NH | S | 3-Cl, 4-$CH_3$ | B 3 |
| $CH_3$ | $CH_3$ | NH | S | 4-Cl | B 4 |
| $CH_3$ | $CH_3$ | NH | S | 2-Cl, 4-Cl | B 4 |

| $R^1$ | $R^2$ | X | Y | $W_n$ | B |
|---|---|---|---|---|---|
| $CH_3$ | $CH_3$ | NH | S | 4-Cl | B 5 |
| $CH_3$ | $CH_3$ | NH | S | 2-Cl, 4-Cl | B 5 |
| $CH_3$ | $CH_3$ | NH | S | 4-Cl | B 6 |
| $CH_3$ | $CH_3$ | NH | S | 2-Cl, 4-Cl | B 6 |
| $CH_3$ | $CH_3$ | NH | S | 4-Cl | B 7 |
| $CH_3$ | $CH_3$ | NH | S | 2-Cl, 4-$CH_3$ | B 7 |
| $CH_3$ | $CH_3$ | NH | S | 4-Cl | B 8 |
| $CH_3$ | $CH_3$ | NH | S | 2-Cl, 4-Cl | B 8 |
| $CH_3$ | $CH_3$ | NH | S | 4-Cl | B 9 |
| $CH_3$ | $CH_3$ | NH | S | 2-Cl, 4-$CH_3$ | B 9 |

| $R^1$ | $R^2$ | X | Y | $W_n$ | B |
|---|---|---|---|---|---|
| $CH_3$ | $CH_3$ | NH | S | 4-Cl | B10 |
| $CH_3$ | $CH_3$ | NH | S | 2-Cl, 4-Cl | B10 |
| $CH_3$ | $CH_3$ | NH | S | 3-Cl, 4-Cl | B10 |
| $CH_3$ | $CH_3$ | NH | S | 2-F, 4-$CH_3$ | B10 |
| $CH_3$ | $CH_3$ | NH | S | 3-F, 4-$CH_3$ | B10 |
| $CH_3$ | $CH_3$ | NH | S | 2-F, 4-Cl | B10 |
| $CH_3$ | $CH_3$ | NH | S | 4-Cl | B11 |
| $CH_3$ | $CH_3$ | NH | S | 2-Cl, 4-$CH_3$ | B11 |
| $CH_3$ | $CH_3$ | NH | S | 4-Cl | B12 |
| $CH_3$ | $CH_3$ | NH | S | 2-Cl, 4-Cl | B12 |

| $R^1$ | $R^2$ | X | Y | $W_n$ | B |
|---|---|---|---|---|---|
| $CH_3$ | $CH_3$ | NH | S | 2-F, 4-$CH_3$ | B 1 2 |
| $CH_3$ | $CH_3$ | NH | S | 3-F, 4-$CH_3$ | B 1 2 |
| $CH_3$ | $CH_3$ | NH | S | 2-Cl, 4-$CH_3$ | B 1 2 |
| $CH_3$ | $CH_3$ | NH | S | 3-Cl, 4-$CH_3$ | B 1 2 |
| $CH_3$ | $CH_3$ | NCHO | S | 4-Cl | B 1 2 |
| $CH_3$ | $CH_3$ | NCHO | S | 2-Cl, 4-Cl | B 1 2 |
| $CH_3$ | $CH_3$ | NH | S | 4-Cl | B 1 3 |
| $CH_3$ | $CH_3$ | NH | S | 2-Cl, 4-Cl | B 1 3 |
| $CH_3$ | $CH_3$ | NH | S | 2-Cl, 4-$CH_3$ | B 1 3 |

| $R^1$ | $R^2$ | X | Y | $W_n$ | B |
|---|---|---|---|---|---|
| $CH_3$ | $CH_3$ | NH | S | 2-F, 4-$CH_3$ | B 1 3 |
| $CH_3$ | $CH_3$ | NH | S | 4-Cl | B 1 4 |
| $CH_3$ | $CH_3$ | NH | S | 2-Cl, 4-Cl | B 1 4 |
| $CH_3$ | $CH_3$ | NH | S | 2-Cl, 4-$CH_3$ | B 1 4 |
| $CH_3$ | $CH_3$ | NH | S | 2-F, 4-$CH_3$ | B 1 4 |
| $CH_3$ | $CH_3$ | NH | S | 4-Cl | B 1 5 |
| $CH_3$ | $CH_3$ | NH | S | 2-Cl, 4-Cl | B 1 5 |
| $CH_3$ | $CH_3$ | NH | S | 2-Cl, 4-$CH_3$ | B 1 5 |
| $CH_3$ | $CH_3$ | NH | S | 2-F, 4-$CH_3$ | B 1 5 |
| $CH_3$ | $CH_3$ | NH | S | 4-Cl | B 1 6 |

| $R^1$ | $R^2$ | X | Y | $W_n$ | B |
|---|---|---|---|---|---|
| $CH_3$ | $CH_3$ | NH | S | 2-Cl, 4-Cl | B16 |
| $CH_3$ | $CH_3$ | NH | S | 2-Cl, 4-$CH_3$ | B16 |
| $CH_3$ | $CH_3$ | NH | S | 2-F, 4-$CH_3$ | B16 |
| $CH_3$ | $CH_3$ | NH | S | 4-Cl | B17 |
| $CH_3$ | $CH_3$ | NH | S | 2-Cl, 4-Cl | B17 |
| $CH_3$ | $CH_3$ | NH | S | 2-Cl, 4-$CH_3$ | B17 |
| $CH_3$ | $CH_3$ | NH | S | 2-F, 4-$CH_3$ | B17 |
| $CH_3$ | $CH_3$ | NH | S | 4-Cl | B18 |
| $CH_3$ | $CH_3$ | NH | S | 2-Cl, 4-Cl | B18 |
| $CH_3$ | $CH_3$ | NH | S | 2-Cl, 4-$CH_3$ | B18 |

| $R^1$ | $R^2$ | X | Y | $W_n$ | B |
|---|---|---|---|---|---|
| $CH_3$ | $CH_3$ | NH | S | 2-F, 4-$CH_3$ | B 1 8 |
| $CH_3$ | $CH_3$ | NCHO | S | 2-Cl, 4-Cl | B 1 |
| $CH_3$ | $CH_3$ | NCHO | S | 3-Cl, 4-Cl | B 1 |
| $CH_3$ | $CH_3$ | NCHO | S | 2-F, 4-Cl | B 1 |
| $CH_3$ | $CH_3$ | NCHO | S | 2-F, 4-$CH_3$ | B 1 |
| $CH_3$ | $CH_3$ | NCHO | S | 3-F, 4-Cl | B 1 |
| $CH_3$ | $CH_3$ | NCHO | S | 3-F, 4-$CH_3$ | B 1 |
| $CH_3$ | $CH_3$ | NCHO | S | 2-Cl, 4-$CH_3$ | B 1 |
| $CH_3$ | $CH_3$ | NCHO | S | 3-Cl, 4-$CH_3$ | B 1 |
| $CH_3$ | $CH_3$ | NCHO | S | 2-Cl, 3-Cl, 4-Cl | B 1 |

| $R^1$ | $R^2$ | X | Y | $W_n$ | B |
|---|---|---|---|---|---|
| $CH_3$ | $CH_3$ | NCHO | S | 2-Cl, 4-Cl, 5-Cl | B 1 |
| $CH_3$ | $CH_3$ | $NCOCH_3$ | S | 2-Cl, 4-Cl | B 1 |
| $CH_3$ | $CH_3$ | $NCOCH_3$ | S | 3-Cl, 4-Cl | B 1 |
| $CH_3$ | $CH_3$ | $NCOCH_3$ | S | 2-F, 4-$CH_3$ | B 1 |
| $CH_3$ | $CH_3$ | $NCOCH_3$ | S | 2-Cl, 3-Cl, 4-Cl | B 1 |
| $CH_3$ | $CH_3$ | $NCH_3$ | S | 2-Cl, 4-Cl | B 1 |
| $CH_3$ | $CH_3$ | $NCH_3$ | S | 3-Cl, 4-$CH_3$ | B 1 |
| $CH_3$ | $CH_3$ | NNO | S | 2-Cl, 4-Cl | B 1 |
| $CH_3$ | $CH_3$ | NNO | S | 3-Cl, 4-$CH_3$ | B 1 |
| $CH_3$ | $CH_3$ | $NNH_2$ | S | 4-Cl | B 1 |

| $R^1$ | $R^2$ | X | Y | $W_n$ | B |
|---|---|---|---|---|---|
| $CH_3$ | $CH_3$ | $NNH_2$ | S | 2-Cl, 4-Cl | B 1 |
| $CH_3$ | $CH_3$ | $NNH_2$ | S | 3-Cl, 4-$CH_3$ | B 1 |
| $CH_3$ | $CH_3$ | $NSO_2CH_3$ | S | 2-Cl, 4-Cl | B 1 |
| $CH_3$ | $CH_3$ | $NSO_2CH_3$ | S | 2-F, 4-$CH_3$ | B 1 |
| $CH_3$ | $CH_3$ | $NSO_2CH_3$ | S | 2-Cl, 3-Cl, 4-Cl | B 1 |
| $CH_3$ | $CH_3$ | $CH_2$ | S | 4-Cl | B 1 |
| $CH_3$ | $CH_3$ | $CH_2$ | S | 2-Cl, 4-Cl | B 1 |
| $CH_3$ | $CH_3$ | $CH_2$ | S | 2-Cl, 4-$CH_3$ | B 1 |
| $CH_3$ | $CH_3$ | $CH_2$ | S | 2-F, 4-$CH_3$ | B 1 |
| $CH_3$ | $CH_3$ | CH(OH) | S | 4-Cl | B 1 |

| $R^1$ | $R^2$ | X | Y | $W_n$ | B |
|---|---|---|---|---|---|
| $CH_3$ | $CH_3$ | CH(OH) | S | 2-Cl, 4-Cl | B 1 |
| $CH_3$ | $CH_3$ | CH(OH) | S | 2-Cl, 4-$CH_3$ | B 1 |
| $CH_3$ | $CH_3$ | CH(OH) | S | 3-Cl, 4-$CH_3$ | B 1 |
| $CH_3$ | $CH_3$ | CH(OH) | S | 2-F, 4-$CH_3$ | B 1 |
| $CH_3$ | $CH_3$ | CH(OH) | S | 3-F, 4-$CH_3$ | B 1 |
| $CH_3$ | $CH_3$ | CH($OCH_3$) | S | 4-Cl | B 1 |
| $CH_3$ | $CH_3$ | CH($OCH_3$) | S | 2-Cl, 4-Cl | B 1 |
| $CH_3$ | $CH_3$ | CH($OCOCH_3$) | S | 4-Cl | B 1 |
| $CH_3$ | $CH_3$ | CH($OCOCH_3$) | S | 2-Cl, 4-Cl | B 1 |
| $CH_3$ | $CH_3$ | CH($OCOCH_3$) | S | 2-Cl, 4-$CH_3$ | B 1 |

| $R^1$ | $R^2$ | X | Y | $W_n$ | B |
|---|---|---|---|---|---|
| $CH_3$ | $CH_3$ | $CH(OCOCH_3)$ | S | 2-F, 4-$CH_3$ | B 1 |
| $CH_3$ | $CH_3$ | CH(F) | S | 4-Cl | B 1 |
| $CH_3$ | $CH_3$ | CH(F) | S | 2-Cl, 4-Cl | B 1 |
| $CH_3$ | $CH_3$ | CH(Cl) | S | 4-Cl | B 1 |
| $CH_3$ | $CH_3$ | CH(Cl) | S | 2-Cl, 4-Cl | B 1 |
| $CH_3$ | $CH_3$ | C=O | S | 4-Cl | B 1 |
| $CH_3$ | $CH_3$ | C=O | S | 2-Cl, 4-Cl | B 1 |
| $CH_3$ | $CH_3$ | C=O | S | 2-Cl, 4-$CH_3$ | B 1 |
| $CH_3$ | $CH_3$ | C=O | S | 2-F, 4-$CH_3$ | B 1 |
| $CH_3$ | $CH_3$ | $CH_2$ | S | 4-Cl | B 2 |

| $R^1$ | $R^2$ | X | Y | $W_n$ | B |
|---|---|---|---|---|---|
| $CH_3$ | $CH_3$ | $CH_2$ | S | 2-Cl, 4-Cl | B 2 |
| $CH_3$ | $CH_3$ | $CH_2$ | S | 2-Cl, 4-$CH_3$ | B 2 |
| $CH_3$ | $CH_3$ | $CH_2$ | S | 2-F, 4-$CH_3$ | B 2 |
| $CH_3$ | $CH_3$ | CH(OH) | S | 4-Cl | B 2 |
| $CH_3$ | $CH_3$ | CH(OH) | S | 2-Cl, 4-Cl | B 2 |
| $CH_3$ | $CH_3$ | CH(OH) | S | 2-Cl, 4-$CH_3$ | B 2 |
| $CH_3$ | $CH_3$ | CH(OH) | S | 3-Cl, 4-$CH_3$ | B 2 |
| $CH_3$ | $CH_3$ | CH(OH) | S | 2-F, 4-$CH_3$ | B 2 |
| $CH_3$ | $CH_3$ | CH(OH) | S | 3-F, 4-$CH_3$ | B 2 |
| $CH_3$ | $CH_3$ | CH($OCH_3$) | S | 4-Cl | B 2 |

| $R^1$ | $R^2$ | X | Y | $W_n$ | B |
|---|---|---|---|---|---|
| $CH_3$ | $CH_3$ | $CH(OCH_3)$ | S | 2-Cl, 4-Cl | B 2 |
| $CH_3$ | $CH_3$ | $CH(OCOCH_3)$ | S | 4-Cl | B 2 |
| $CH_3$ | $CH_3$ | $CH(OCOCH_3)$ | S | 2-Cl, 4-Cl | B 2 |
| $CH_3$ | $CH_3$ | $CH(OCOCH_3)$ | S | 2-Cl, 4-$CH_3$ | B 2 |
| $CH_3$ | $CH_3$ | $CH(OCOCH_3)$ | S | 2-F, 4-$CH_3$ | B 2 |
| $CH_3$ | $CH_3$ | CH(F) | S | 4-Cl | B 2 |
| $CH_3$ | $CH_3$ | CH(F) | S | 2-Cl, 4-Cl | B 2 |
| $CH_3$ | $CH_3$ | CH(Cl) | S | 4-Cl | B 2 |
| $CH_3$ | $CH_3$ | CH(Cl) | S | 2-Cl, 4-Cl | B 2 |
| $CH_3$ | $CH_3$ | C=O | S | 4-Cl | B 2 |

| $R^1$ | $R^2$ | X | Y | $W_n$ | B |
|---|---|---|---|---|---|
| $CH_3$ | $CH_3$ | C=O | S | 2-Cl, 4-Cl | B 2 |
| $CH_3$ | $CH_3$ | C=O | S | 2-Cl, 4-$CH_3$ | B 2 |
| $CH_3$ | $CH_3$ | C=O | S | 2-F, 4-$CH_3$ | B 2 |
| $CH_3$ | $CH_3$ | $C(CH_3)(OH)$ | S | 4-Cl | B 2 |
| $CH_3$ | $CH_3$ | $C(CH_3)(OH)$ | S | 2-Cl, 4-Cl | B 2 |
| $CH_3$ | $CH_3$ | $C(CH_3)(OCOCH_3)$ | S | 4-Cl | B 2 |
| $CH_3$ | $CH_3$ | $C(CH_3)(OCOCH_3)$ | S | 2-Cl, 4-Cl | B 2 |
| $CH_3$ | $CH_3$ | $C(CH_3)(F)$ | S | 4-Cl | B 2 |
| $CH_3$ | $CH_3$ | $C(CH_3)(F)$ | S | 2-Cl, 4-Cl | B 2 |
| $CH_3$ | $CH_3$ | $C(C_2H_5)(OH)$ | S | 4-Cl | B 2 |

| $R^1$ | $R^2$ | X | Y | $W_n$ | B |
|---|---|---|---|---|---|
| $CH_3$ | $CH_3$ | $C(C_2H_5)(OH)$ | S | 2-Cl, 4-Cl | B 2 |
| $CH_3$ | $CH_3$ | $C(i\text{-}C_3H_7)(OH)$ | S | 4-Cl | B 2 |
| $CH_3$ | $CH_3$ | $C(i\text{-}C_3H_7)(OH)$ | S | 2-Cl, 4-Cl | B 2 |
| $CH_3$ | $CH_3$ | $C(i\text{-}C_3H_7)(OH)$ | S | 2-Cl, 4-$CH_3$ | B 2 |
| $CH_3$ | $CH_3$ | $C(i\text{-}C_3H_7)(OH)$ | S | 2-F, 4-$CH_3$ | B 2 |
| $CH_3$ | $CH_3$ | $CH(CH_3)$ | S | 4-Cl | B 2 |
| $CH_3$ | $CH_3$ | $CH(CH_3)$ | S | 2-Cl, 4-Cl | B 2 |
| $CH_3$ | $CH_3$ | $CH(CH_3)$ | S | 2-Cl, 4-$CH_3$ | B 2 |
| $CH_3$ | $CH_3$ | $CH(CH_3)$ | S | 2-F, 4-$CH_3$ | B 2 |
| Cl | $CH_3$ | $CH_2$ | S | 4-Cl | B 2 |

| $R^1$ | $R^2$ | X | Y | $W_n$ | B |
|---|---|---|---|---|---|
| Cl | $CH_3$ | $CH_2$ | S | 2-Cl, 4-Cl | B 2 |
| Cl | $CH_3$ | $CH_2$ | S | 2-Cl, 4-$CH_3$ | B 2 |
| Cl | $CH_3$ | $CH_2$ | S | 2-F, 4-$CH_3$ | B 2 |
| $CH_3O$ | $CH_3$ | $CH_2$ | S | 4-Cl | B 2 |
| $CH_3O$ | $CH_3$ | $CH_2$ | S | 2-Cl, 4-Cl | B 2 |
| $CH_3O$ | $CH_3$ | $CH_2$ | S | 2-Cl, 4-$CH_3$ | B 2 |
| $CH_3O$ | $CH_3$ | $CH_2$ | S | 2-F, 4-$CH_3$ | B 2 |

wobei B1 bis B18 wie nachstehend definiert sind:

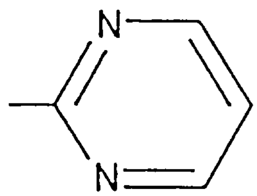

B1

,

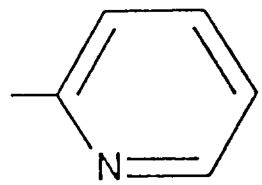

B2

,

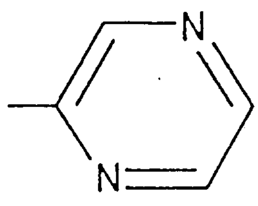

B3

,

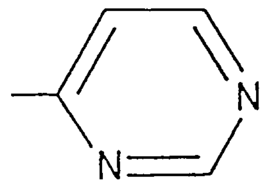

B4

,

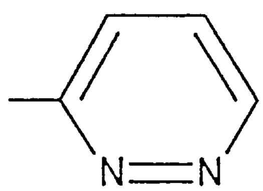

B5

,

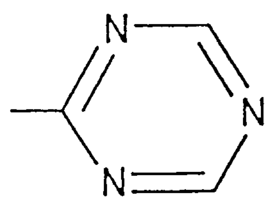

B6

,

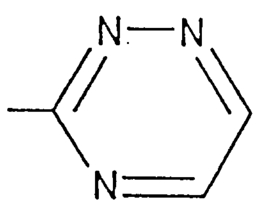

B7

,

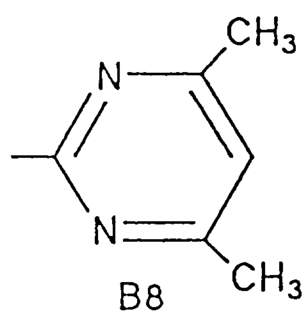

B8

,

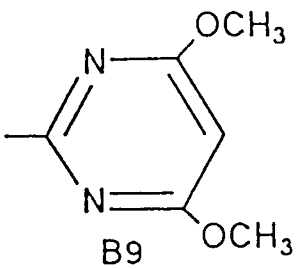

B9 ,

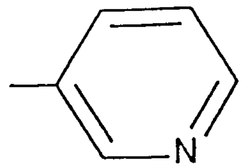

B10 ,

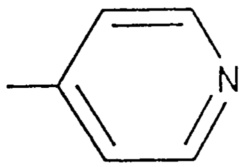

B11 ,

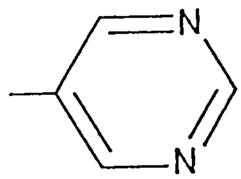

B12

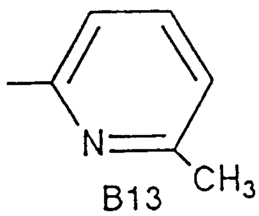

B13 ,

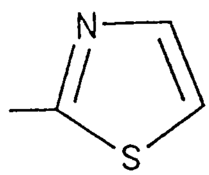

B14 ,

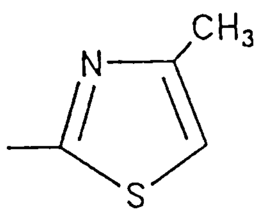

B15 ,

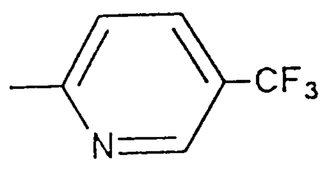

B16 ,

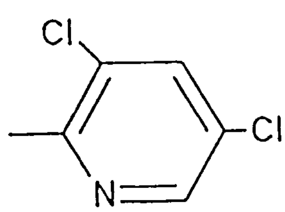

B17 ,

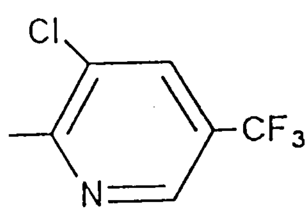

B18 ,

oder wobei die substituierten Pyrazolderivate durch die Formel (1b) definiert sind:

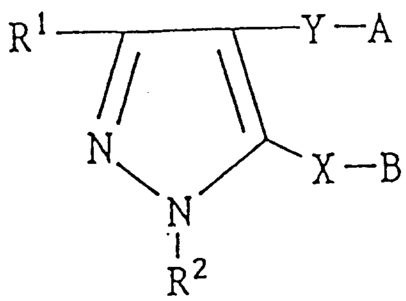

(1b)

wobei die Substituenten die folgenden Bedeutungen haben:

| $R^1$ | $R^2$ | X | Y | A | B |
|---|---|---|---|---|---|
| $CH_3$ | $CH_3$ | NH | S | A 1 | B 1 |
| $CH_3$ | $CH_3$ | NH | S | A 1 | B 2 |
| $CH_3$ | $CH_3$ | NH | S | A 2 | B 1 |

| $R^1$ | $R^2$ | X | Y | A | B |
|---|---|---|---|---|---|
| $CH_3$ | $CH_3$ | NH | S | A 2 | B 2 |
| $CH_3$ | $CH_3$ | NH | S | A 3 | B 1 |
| $CH_3$ | $CH_3$ | NH | S | A 3 | B 2 |
| $CH_3$ | $CH_3$ | NH | S | A 4 | B 1 |
| $CH_3$ | $CH_3$ | NH | S | A 4 | B 2 |
| $CH_3$ | $CH_3$ | NH | S | A 5 | B 1 |
| $CH_3$ | $CH_3$ | NH | S | A 5 | B 2 |
| $CH_3$ | $CH_3$ | NH | S | A 6 | B 1 |
| $CH_3$ | $CH_3$ | NH | S | A 6 | B 2 |
| $CH_3$ | $CH_3$ | NH | S | A 7 | B 1 |
| $CH_3$ | $CH_3$ | NH | S | A 7 | B 2 |
| $CH_3$ | $CH_3$ | NH | S | A 8 | B 1 |
| $CH_3$ | $CH_3$ | NH | S | A 8 | B 2 |
| $CH_3$ | $CH_3$ | NH | S | A 9 | B 1 |
| $CH_3$ | $CH_3$ | NH | S | A 9 | B 2 |
| $CH_3$ | $CH_3$ | NH | S | A 10 | B 1 |
| $CH_3$ | $CH_3$ | NH | S | A 10 | B 2 |
| $CH_3$ | $CH_3$ | NH | S | A 11 | B 1 |
| $CH_3$ | $CH_3$ | NH | S | A 11 | B 2 |

wobei A1 bis A11 wie nachstehend definiert sind:

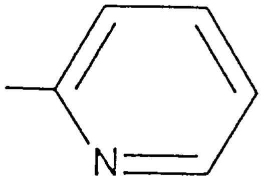
A1
,

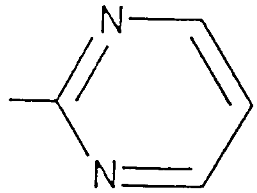
A2
,

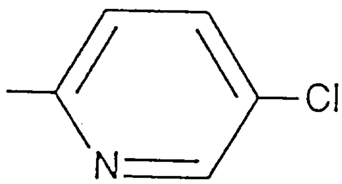
A3
,

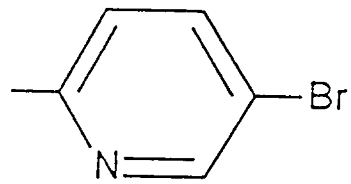
A4
,

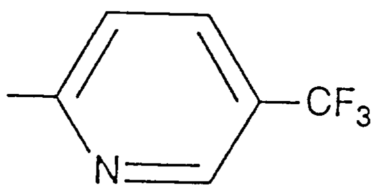
A5
,

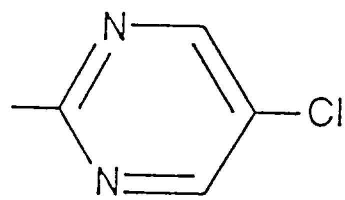
A6
,

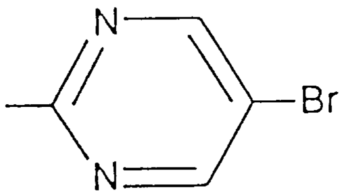

A7 ,

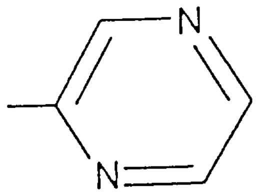

A8 ,

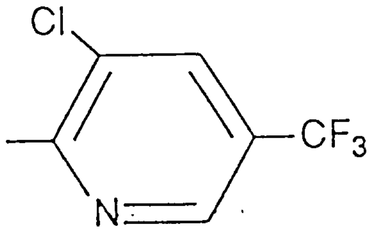

A9 ,

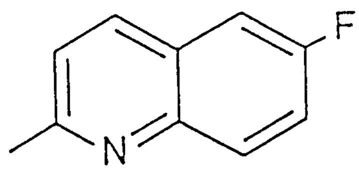

A10 ,

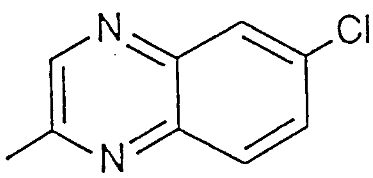

A11 ,

und B1 und B2 wie oben definiert sind.

2. Substituierte Pyrazolderivate gemäss Anspruch 1, die Verbindungen mit der Formel (1a) sind.

3. Substituierte Pyrazolderivate gemäss Anspruch 1, in denen X -N($R^3$)- ist.

4. Substituierte Pyrazolderivate gemäss Anspruch 2, bei denen Y -S- ist.

5. Substituierte Pyrazolderivate gemäss Anspruch 1, die Verbindungen der allgemeinen Formel (1a) sind, bei denen $R^1$ und $R^2$ jeweils eine Alkylgruppe sind, X -N($R^3$)- ist, Y -S- ist und B B1, B2, B4, B10, B11 oder B12 ist.

6. Fungizid zur landwirtschaftlichen und gartenbaulichen Verwendung, enthaltend ein oder mehrere substituierte Pyrazole gemäss Anspruch 1 als Wirkstoff.

7. Verwendung eines substituierten Pyrazolderivats gemäss Anspruch 1 als Fungizid zur landwirtschaftlichen und gartenbaulichen Verwendung.

**Patentansprüche für folgenden Vertragsstaat : ES**

1. Verfahren zur Herstellung von substituierten Pyrazolderivaten der allgemeinen Formel (1):

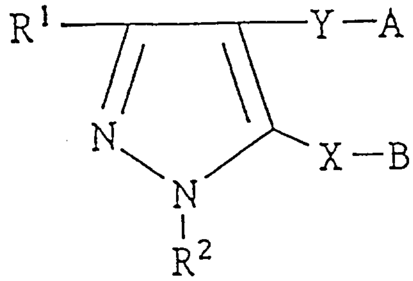

(1)

worin $R^1$ ein Wasserstoffatom, ein Halogenatom, eine Alkylgruppe, eine Alkoxygruppe, eine Alkylthiogruppe oder eine Haloalkylgruppe darstellt;
$R^2$ ein Wasserstoffatom, eine Alkylgruppe, eine Haloalkylgruppe, eine Phenylalkylgruppe, $-COR^6$ oder $-SO_2R^7$ darstellt;
X -S-, -SO-, $-SO_2-$, $-N(R^3)-$, -CO- oder $-C(R^4)(R^5)$-darstellt;
$R^3$ ein Wasserstoffatom, eine Alkylgruppe, eine Haloalkylgruppe, eine Alkenylgruppe, eine Alkinylgruppe, eine Alkoxyalkylgruppe, eine Cyanoalkylgruppe, eine Alkylcarbonylalkylgruppe, eine Alkoxycarbonylalkylgruppe, eine Nitrosogruppe, eine Aminogruppe, eine Phenylalkylgruppe, $-COR^6$ oder $-SO_2R^7$ darstellt;
$R^4$ und $R^5$ unabhängig ein Wasserstoffatom, ein Halogenatom, eine Alkylgruppe, eine Haloalkylgruppe, eine Alkenylgruppe, eine Alkinylgruppe oder $-OR^8$ darstellen;
$R^8$ ein Wasserstoffatom, eine Alkylgruppe, eine Haloalkylgruppe, eine Alkenylgruppe, eine Alkinylgruppe, eine Alkoxyalkylgruppe, eine Cyanoalkylgruppe, eine Alkylcarbonylalkylgruppe, eine Alkoxycarbonylalkylgruppe, eine Phenylalkylgruppe, $-COR^6$ oder $-SO_2R^7$ darstellt;
$R^6$ ein Wasserstoffatom, eine Alkylgruppe, eine Haloalkylgruppe, eine Phenylgruppe, eine Phenylalkylgruppe, eine Alkoxygruppe oder

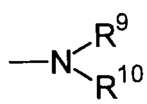

darstellt;
$R^7$ eine Alkylgruppe, eine Haloalkylgruppe, eine Phenylgruppe oder

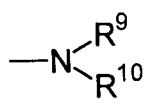

darstellt;
$R^9$ und $R^{10}$ unabhängig ein Wasserstoffatom, eine Alkylgruppe oder eine Phenylgruppe darstellen;
Y ein Sauerstoffatom, -S-, -SO- oder $-SO_2-$ darstellt;
A eine Phenylgruppe darstellt;
B darstellt:

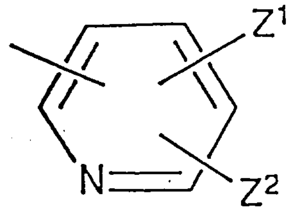 ,

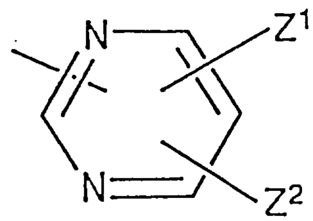 ,

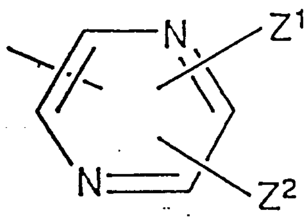 ,

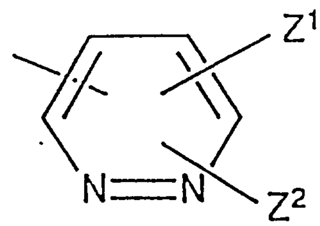 ,

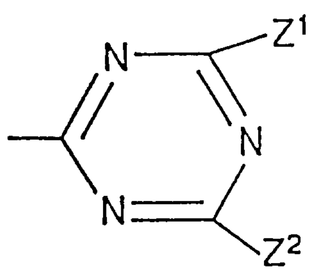 ,

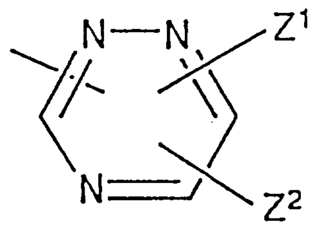

oder

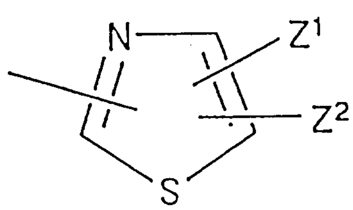

$Z^1$ und $Z^2$ unabhängig ein Wasserstoffatom, ein Halogenatom, eine Alkylgruppe, eine Alkoxygruppe oder eine Haloalkylgruppe darstellen,
wobei die substituierten Pyrazolderivate der allgemeinen Formel (1) Verbindungen der Formel (1a) einschliessen:

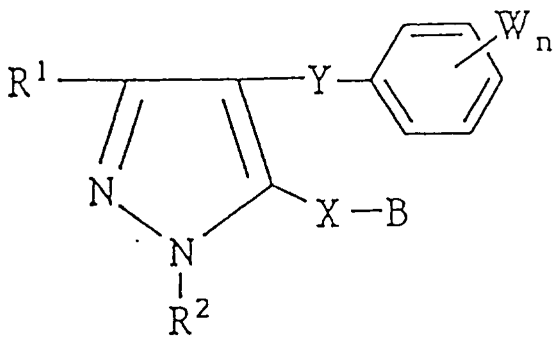

(1a)

mit folgenden Substituentenbedeutungen:

| $R^1$ | $R^2$ | X | Y | $W_n$ | B |
|---|---|---|---|---|---|
| H | $CH_3$ | S | S | 4-Cl | B1 |
| $CH_3$ | $CH_3$ | S | S | 4-Cl | B1 |
| $CF_3$ | $CH_3$ | S | S | 4-Cl | B1 |
| $CH_3$ | $CH_3$ | S | S | 4-$CH_3$ | B1 |
| $CF_3$ | $CH_3$ | S | S | 4-$CH_3$ | B1 |
| $CH_3$ | H | S | S | 4-Cl | B1 |
| $CH_3$ | H | S | S | 4-$CH_3$ | B1 |
| $CH_3$ | $CF_3$ | S | S | 4-Cl | B1 |

| $R^1$ | $R^2$ | X | Y | $W_n$ | B |
|---|---|---|---|---|---|
| $CH_3$ | $CF_3$ | S | S | 4-$CH_3$ | B 1 |
| $CH_3$ | $CH_3$ | S | S | 4-$CF_3$ | B 1 |
| $CH_3$ | $CH_3$ | S | S | 4-Br | B 1 |
| $CH_3$ | $CH_3$ | S | S | 4-$NO_2$ | B 1 |
| $CH_3$ | $CH_3$ | S | S | 4-$OCH_3$ | B 1 |
| $CH_3$ | $CH_3$ | S | S | 4-$C_2H_5$ | B 1 |
| $CH_3$ | $CH_3$ | S | S | 2-Cl | B 1 |
| $CH_3$ | $CH_3$ | S | S | 2-Cl, 4-Cl | B 1 |
| $CH_3$ | $CH_3$ | S | S | 2-Cl, 4-$CH_3$ | B 1 |
| $CH_3$ | $CH_3$ | S | S | 4-Cl | B 2 |
| $CF_3$ | $CH_3$ | S | S | 4-Cl | B 2 |

| $R^1$ | $R^2$ | X | Y | $W_n$ | B |
|---|---|---|---|---|---|
| $CH_3$ | $CH_3$ | S | S | 4-$CH_3$ | B 2 |
| $CF_3$ | $CH_3$ | S | S | 4-$CH_3$ | B 2 |
| $CH_3$ | $CH_3$ | S | S | 2-Cl, 4-Cl | B 2 |
| $CH_3$ | $CH_3$ | S | S | 2-Cl, 4-$CH_3$ | B 2 |
| $CH_3$ | $CH_3$ | S | O | 4-Cl | B 2 |
| $CH_3$ | $CH_3$ | S | O | 4-$CH_3$ | B 2 |
| $CF_3$ | $CH_3$ | S | O | 4-$CH_3$ | B 2 |
| $CH_3$ | $CH_3$ | S | O | 3-Cl, 4-$CH_3$ | B 2 |
| $C_2H_5$ | $CH_3$ | S | S | H | B 1 |
| $C_2H_5$ | $CH_3$ | S | S | 4-Cl | B 1 |

| $R^1$ | $R^2$ | X | Y | $W_n$ | B |
|---|---|---|---|---|---|
| $C_2H_5$ | $CH_3$ | S | S | 4-$CH_3$ | B 1 |
| $C_2H_5$ | $CH_3$ | S | S | 4-$OCH_3$ | B 1 |
| $C_2H_5$ | $CH_3$ | S | S | 3-$CF_3$ | B 1 |
| $C_2H_5$ | $CH_3$ | S | S | 2-Cl, 4-Cl | B 1 |
| $C_2H_5$ | $CH_3$ | S | S | 3-Cl, 4-Cl | B 1 |
| $C_2H_5$ | $CH_3$ | S | S | 2-Cl, 4-$CH_3$ | B 1 |
| i-$C_3H_7$ | $CH_3$ | S | S | 4-Cl | B 1 |
| i-$C_3H_7$ | $CH_3$ | S | S | 4-$CH_3$ | B 1 |
| i-$C_3H_7$ | $CH_3$ | S | S | 4-$OCH_3$ | B 1 |
| t-$C_4H_9$ | $CH_3$ | S | S | 4-Cl | B 1 |

| $R^1$ | $R^2$ | X | Y | $W_n$ | B |
|---|---|---|---|---|---|
| $t-C_4H_9$ | $CH_3$ | S | S | $4-CH_3$ | B 1 |
| $t-C_4H_9$ | $CH_3$ | S | S | $4-OCH_3$ | B 1 |
| $CH_3$ | $C_2H_5$ | S | S | 4-Cl | B 1 |
| $CH_3$ | $C_3H_7$ | S | S | 4-Cl | B 1 |
| $CH_3$ | $CH_3$ | NH | S | H | B 1 |
| $CF_3$ | $CH_3$ | NH | S | H | B 1 |
| $CH_3$ | $CH_3$ | NH | S | 4-Cl | B 1 |
| $CF_3$ | $CH_3$ | NH | S | 4-Cl | B 1 |
| H | $CH_3$ | NH | S | 4-Cl | B 1 |
| $CH_3$ | $CH_3$ | NH | S | $4-CH_3$ | B 1 |

| $R^1$ | $R^2$ | X | Y | $W_n$ | B |
|---|---|---|---|---|---|
| $CF_3$ | $CH_3$ | NH | S | 4-$CH_3$ | B 1 |
| H | $CH_3$ | NH | S | 4-$CH_3$ | B 1 |
| $CH_3$ | $CH_3$ | NH | S | 4-F | B 1 |
| $CH_3$ | $CH_3$ | NH | S | 4-Br | B 1 |
| $CH_3$ | $CH_3$ | NH | S | 4-I | B 1 |
| $CH_3$ | $CH_3$ | NH | S | 2-F | B 1 |
| $CH_3$ | $CH_3$ | NH | S | 2-Cl | B 1 |
| $CH_3$ | $CH_3$ | NH | S | 2-Br | B 1 |
| $CH_3$ | $CH_3$ | NH | S | 2-I | B 1 |
| $CH_3$ | $CH_3$ | NH | S | 3-F | B 1 |

| $R^1$ | $R^2$ | X | Y | $W_n$ | B |
|---|---|---|---|---|---|
| $CH_3$ | $CH_3$ | NH | S | 3-Cl | B 1 |
| $CH_3$ | $CH_3$ | NH | S | 3-Br | B 1 |
| $CH_3$ | $CH_3$ | NH | S | 3-I | B 1 |
| $CH_3$ | $CH_3$ | NH | S | 2-$OCH_3$ | B 1 |
| $CH_3$ | $CH_3$ | NH | S | 3-$OCH_3$ | B 1 |
| $CH_3$ | $CH_3$ | NH | S | 4-$OCH_3$ | B 1 |
| $CH_3$ | $CH_3$ | NH | S | 2-$CF_3$ | B 1 |
| $CH_3$ | $CH_3$ | NH | S | 3-$CF_3$ | B 1 |
| $CH_3$ | $CH_3$ | NH | S | 4-$CF_3$ | B 1 |
| $CH_3$ | $CH_3$ | NH | S | 4-$C_2H_5$ | B 1 |

| $R^1$ | $R^2$ | X | Y | $W_n$ | B |
|---|---|---|---|---|---|
| $CH_3$ | $CH_3$ | NH | S | $4-C_3H_7$ | B 1 |
| $CH_3$ | $CH_3$ | NH | S | $4-C_4H_9$ | B 1 |
| $CH_3$ | $CH_3$ | NH | S | $4-i-C_3H_7$ | B 1 |
| $CH_3$ | $CH_3$ | NH | S | $4-t-C_4H_9$ | B 1 |
| $CH_3$ | $CH_3$ | NH | S | $2-OCF_3$ | B 1 |
| $CH_3$ | $CH_3$ | NH | S | $3-OCF_3$ | B 1 |
| $CH_3$ | $CH_3$ | NH | S | $4-OCF_3$ | B 1 |
| $CH_3$ | $CH_3$ | NH | S | $3-NO_2$ | B 1 |
| $CH_3$ | $CH_3$ | NH | S | $4-NO_2$ | B 1 |
| $CH_3$ | $CH_3$ | NH | S | $3-NH_2$ | B 1 |

| $R^1$ | $R^2$ | X | Y | $W_n$ | B |
|---|---|---|---|---|---|
| $CH_3$ | $CH_3$ | NH | S | 4-$NH_2$ | B 1 |
| $CH_3$ | $CH_3$ | NH | S | 4-$NHCOCH_3$ | B 1 |
| $CH_3$ | $CH_3$ | NH | S | 4-$NHSO_2CH_3$ | B 1 |
| $CH_3$ | $CH_3$ | NH | S | 4-$NHCOCF_3$ | B 1 |
| $CH_3$ | $CH_3$ | NH | S | 4-$NHSO_2CF_3$ | B 1 |
| $CH_3$ | $CH_3$ | NH | S | 4-Ph | B 1 |
| $CH_3$ | $CH_3$ | NH | S | 4-OPh | B 1 |
| $CH_3$ | $CH_3$ | NH | S | 4-$OCF_2CF_2H$ | B 1 |
| $CH_3$ | $CH_3$ | NH | S | 4-$COCH_3$ | B 1 |
| $CH_3$ | $CH_3$ | NH | S | 4-NHCOPh | B 1 |

| $R^1$ | $R^2$ | X | Y | $W_n$ | B |
|---|---|---|---|---|---|
| $CH_3$ | $CH_3$ | NH | S | 4-$NHCOOCH_3$ | B 1 |
| $CH_3$ | $CH_3$ | NH | S | 4-$NHCON(CH_3)_2$ | B 1 |
| $CH_3$ | $CH_3$ | NH | S | 2-Cl, 3-Cl | B 1 |
| $CH_3$ | $CH_3$ | NH | S | 2-Cl, 4-Cl | B 1 |
| $CH_3$ | $CH_3$ | NH | S | 2-Cl, 5-Cl | B 1 |
| $CH_3$ | $CH_3$ | NH | S | 2-Cl, 6-Cl | B 1 |
| $CH_3$ | $CH_3$ | NH | S | 3-Cl, 4-Cl | B 1 |
| $CH_3$ | $CH_3$ | NH | S | 3-Cl, 5-Cl | B 1 |
| $CH_3$ | $CH_3$ | NH | S | 2-$CH_3$, 3-$CH_3$ | B 1 |
| $CH_3$ | $CH_3$ | NH | S | 2-$CH_3$, 4-$CH_3$ | B 1 |

| $R^1$ | $R^2$ | X | Y | $W_n$ | B |
|---|---|---|---|---|---|
| $CH_3$ | $CH_3$ | NH | S | 2-$CH_3$, 5-$CH_3$ | B 1 |
| $CH_3$ | $CH_3$ | NH | S | 2-$CH_3$, 6-$CH_3$ | B 1 |
| $CH_3$ | $CH_3$ | NH | S | 3-$CH_3$, 4-$CH_3$ | B 1 |
| $CH_3$ | $CH_3$ | NH | S | 3-$CH_3$, 5-$CH_3$ | B 1 |
| $CH_3$ | $CH_3$ | NH | S | 2-Cl, 3-$CH_3$ | B 1 |
| $CH_3$ | $CH_3$ | NH | S | 2-Cl, 4-$CH_3$ | B 1 |
| $CH_3$ | $CH_3$ | NH | S | 2-Cl, 5-$CH_3$ | B 1 |
| $CH_3$ | $CH_3$ | NH | S | 3-Cl, 4-$CH_3$ | B 1 |
| $CH_3$ | $CH_3$ | NH | S | 3-Cl, 5-$CH_3$ | B 1 |
| $CH_3$ | $CH_3$ | NH | S | 2-$CH_3$, 3-Cl | B 1 |

| $R^1$ | $R^2$ | X | Y | $W_n$ | B |
|---|---|---|---|---|---|
| $CH_3$ | $CH_3$ | NH | S | 2-$CH_3$, 4-Cl | B1 |
| $CH_3$ | $CH_3$ | NH | S | 2-$CH_3$, 5-Cl | B1 |
| $CH_3$ | $CH_3$ | NH | S | 2-F, 3-F | B1 |
| $CH_3$ | $CH_3$ | NH | S | 2-F, 4-F | B1 |
| $CH_3$ | $CH_3$ | NH | S | 2-F, 5-F | B1 |
| $CH_3$ | $CH_3$ | NH | S | 2-F, 6-F | B1 |
| $CH_3$ | $CH_3$ | NH | S | 2-F, 3-Cl | B1 |
| $CH_3$ | $CH_3$ | NH | S | 2-F, 4-Cl | B1 |
| $CH_3$ | $CH_3$ | NH | S | 2-F, 5-Cl | B1 |
| $CH_3$ | $CH_3$ | NH | S | 2-F, 6-Cl | B1 |

| $R^1$ | $R^2$ | X | Y | $W_n$ | B |
|---|---|---|---|---|---|
| $CH_3$ | $CH_3$ | NH | S | 2-F, 3-$CH_3$ | B 1 |
| $CH_3$ | $CH_3$ | NH | S | 2-F, 4-$CH_3$ | B 1 |
| $CH_3$ | $CH_3$ | NH | S | 2-F, 3-Br | B 1 |
| $CH_3$ | $CH_3$ | NH | S | 2-F, 4-Br | B 1 |
| $CH_3$ | $CH_3$ | NH | S | 3-F, 4-Cl | B 1 |
| $CH_3$ | $CH_3$ | NH | S | 3-F, 5-Cl | B 1 |
| $CH_3$ | $CH_3$ | NH | S | 3-F, 4-$CH_3$ | B 1 |
| $CH_3$ | $CH_3$ | NH | S | 3-F, 5-$CH_3$ | B 1 |
| $CH_3$ | $CH_3$ | NH | S | 2-Br, 4-Cl | B 1 |
| $CH_3$ | $CH_3$ | NH | S | 2-Br, 4-Br | B 1 |

| $R^1$ | $R^2$ | X | Y | $W_n$ | B |
|---|---|---|---|---|---|
| $CH_3$ | $CH_3$ | NH | S | 2-Br, 4-$CH_3$ | B1 |
| $CH_3$ | $CH_3$ | NH | S | 3-Br, 4-Cl | B1 |
| $CH_3$ | $CH_3$ | NH | S | 3-Br, 4-Br | B1 |
| $CH_3$ | $CH_3$ | NH | S | 3-Br, 4-$CH_3$ | B1 |
| $CH_3$ | $CH_3$ | NH | S | 2-Cl, 4-Br | B1 |
| $CH_3$ | $CH_3$ | NH | S | 3-Cl, 4-Br | B1 |
| $CH_3$ | $CH_3$ | NH | S | 2-Cl, 4-I | B1 |
| $CH_3$ | $CH_3$ | NH | S | 3-Cl, 4-I | B1 |
| $CH_3$ | $CH_3$ | NH | S | 3-F, 4-Br | B1 |
| $CH_3$ | $CH_3$ | NH | S | 3, 4-$OCH_2O$- | B1 |

| $R^1$ | $R^2$ | X | Y | $W_n$ | B |
|---|---|---|---|---|---|
| $CH_3$ | $CH_3$ | NH | S | 2-$CF_3$, 3-Cl | B 1 |
| $CH_3$ | $CH_3$ | NH | S | 2-$CF_3$, 4-Cl | B 1 |
| $CH_3$ | $CH_3$ | NH | S | 2-$CF_3$, 3-Br | B 1 |
| $CH_3$ | $CH_3$ | NH | S | 2-$CF_3$, 4-Br | B 1 |
| $CH_3$ | $CH_3$ | NH | S | 2-F, 4-$NO_2$ | B 1 |
| $CH_3$ | $CH_3$ | NH | S | 3-F, 4-$NO_2$ | B 1 |
| $CH_3$ | $CH_3$ | NH | S | 2-Cl, 4-$NO_2$ | B 1 |
| $CH_3$ | $CH_3$ | NH | S | 3-Cl, 4-$NO_2$ | B 1 |
| $CH_3$ | $CH_3$ | NH | S | 2-F, 4-$OCH_3$ | B 1 |
| $CH_3$ | $CH_3$ | NH | S | 3-F, 4-$OCH_3$ | B 1 |

| $R^1$ | $R^2$ | X | Y | $W_n$ | B |
|---|---|---|---|---|---|
| $CH_3$ | $CH_3$ | NH | S | 2-Cl, 4-$OCH_3$ | B 1 |
| $CH_3$ | $CH_3$ | NH | S | 3-Cl, 4-$OCH_3$ | B 1 |
| $CH_3$ | $CH_3$ | NH | S | 2-$OCH_3$, 4-$OCH_3$ | B 1 |
| $CH_3$ | $CH_3$ | NH | S | 3-$OCH_3$, 4-$OCH_3$ | B 1 |
| $CH_3$ | $CH_3$ | NH | S | 2-Cl, 3-Cl, 4-Cl | B 1 |
| $CH_3$ | $CH_3$ | NH | S | 2-Cl, 4-Cl, 5-Cl | B 1 |
| $CH_3$ | $CH_3$ | NH | S | 2-Cl, 4-Cl, 6-Cl | B 1 |
| $CH_3$ | $CH_3$ | NH | S | 3-Cl, 4-Cl, 5-Cl | B 1 |
| $CH_3$ | $CH_3$ | NH | S | 2-Cl, 3-Cl, 4-$CH_3$ | B 1 |
| $CH_3$ | $CH_3$ | NH | S | 2-Cl, 3-Cl, 4-Br | B 1 |

| $R^1$ | $R^2$ | X | Y | $W_n$ | B |
|---|---|---|---|---|---|
| $CH_3$ | $CH_3$ | NH | S | 2-Cl, 3-Cl, 4-I | B 1 |
| $CH_3$ | $CH_3$ | NH | S | 2-Cl, 4-Br, 6-Cl | B 1 |
| $CH_3$ | $CH_3$ | NH | S | 2-Cl, 4-$CH_3$, 6-Cl | B 1 |
| $CH_3$ | $CH_3$ | NH | S | 2-Br, 4-Cl, 6-Cl | B 1 |
| $CH_3$ | $CH_3$ | NCHO | S | 4-Cl | B 1 |
| $CH_3$ | $CH_3$ | $NCOCH_3$ | S | 4-Cl | B 1 |
| $CH_3$ | $CH_3$ | $NCOOCH_3$ | S | 4-Cl | B 1 |
| $CH_3$ | $CH_3$ | $NCON(CH_3)_2$ | S | 4-Cl | B 1 |
| $CH_3$ | $CH_3$ | NCONHPh | S | 4-Cl | B 1 |
| $CH_3$ | $CH_3$ | $NCH_3$ | S | 4-Cl | B 1 |

| $R^1$ | $R^2$ | X | Y | $W_n$ | B |
|---|---|---|---|---|---|
| $CH_3$ | $CH_3$ | $NC_2H_5$ | S | 4-Cl | B 1 |
| $CH_3$ | $CH_3$ | $NC_3H_7$ | S | 4-Cl | B 1 |
| $CH_3$ | $CH_3$ | $NCH_2OCH_3$ | S | 4-Cl | B 1 |
| $CH_3$ | $CH_3$ | $NCH_2CH_2OCH_3$ | S | 4-Cl | B 1 |
| $CH_3$ | $CH_3$ | $NCH_2CH=CH_2$ | S | 4-Cl | B 1 |
| $CH_3$ | $CH_3$ | $NCH_2C\equiv CH$ | S | 4-Cl | B 1 |
| $CH_3$ | $CH_3$ | $NCH_2COCH_3$ | S | 4-Cl | B 1 |
| $CH_3$ | $CH_3$ | $NCH_2COOCH_3$ | S | 4-Cl | B 1 |
| $CH_3$ | $CH_3$ | $NCH_2COOC_2H_5$ | S | 4-Cl | B 1 |
| $CH_3$ | $CH_3$ | $NCH_2CN$ | S | 4-Cl | B 1 |

| $R^1$ | $R^2$ | X | Y | $W_n$ | B |
|---|---|---|---|---|---|
| $CH_3$ | $CH_3$ | $NCH_2Ph$ | S | 4-Cl | B1 |
| $CH_3$ | $CH_3$ | $NCH_2C_6H_4$-4-Cl | S | 4-Cl | B1 |
| $CH_3$ | $CH_3$ | $NCH_2C_6H_4$-4-$CH_3$ | S | 4-Cl | B1 |
| $CH_3$ | $CH_3$ | $NCH_2CH_2Ph$ | S | 4-Cl | B1 |
| $CH_3$ | $CH_3$ | $NSO_2CH_3$ | S | 4-Cl | B1 |
| $CH_3$ | $CH_3$ | $NSO_2N(CH_3)_2$ | S | 4-Cl | B1 |
| $CH_3$ | $CH_3$ | NCHO | S | 4-Cl | B2 |
| $CH_3$ | $CH_3$ | $NCH_3$ | S | 4-Cl | B2 |
| $CH_3$ | H | NH | S | 4-Cl | B1 |
| $CH_3$ | H | NH | S | 2-Cl, 4-Cl | B1 |

| $R^1$ | $R^2$ | X | Y | $W_n$ | B |
|---|---|---|---|---|---|
| $CH_3$ | H | NH | S | 3-Cl, 4-$CH_3$ | B 1 |
| $CH_3$ | $C_2H_5$ | NH | S | 4-Cl | B 1 |
| $CH_3$ | $C_2H_5$ | NH | S | 4-Br | B 1 |
| $CH_3$ | $C_2H_5$ | NH | S | 2-Cl, 4-Cl | B 1 |
| $CH_3$ | $C_2H_5$ | NH | S | 2-Cl, 4-$CH_3$ | B 1 |
| $CH_3$ | i-$C_3H_7$ | NH | S | 4-Cl | B 1 |
| $CH_3$ | i-$C_3H_7$ | NH | S | 4-Br | B 1 |
| $CH_3$ | i-$C_3H_7$ | NH | S | 2-Cl, 4-Cl | B 1 |
| $CH_3$ | i-$C_3H_7$ | NH | S | 2-Cl, 4-$CH_3$ | B 1 |
| $CH_3$ | t-$C_4H_9$ | NH | S | 4-Cl | B 1 |

| $R^1$ | $R^2$ | X | Y | $W_n$ | B |
|---|---|---|---|---|---|
| $CH_3$ | $t-C_4H_9$ | NH | S | 2-Cl, 4-Cl | B 1 |
| $CH_3$ | $t-C_4H_9$ | NH | S | 3-Cl, 4-$CH_3$ | B 1 |
| $CH_3$ | $CF_3$ | NH | S | 4-Cl | B 1 |
| $CH_3$ | $CF_3$ | NH | S | 4-Br | B 1 |
| $CH_3$ | $CF_3$ | NH | S | 2-Cl, 4-Cl | B 1 |
| $CH_3$ | $CF_3$ | NH | S | 3-Cl, 4-$CH_3$ | B 1 |
| $CH_3$ | H | NH | S | 4-Cl | B 2 |
| $CH_3$ | H | NH | S | 2-Cl, 4-Cl | B 2 |
| $CH_3$ | $C_2H_5$ | NH | S | 4-Cl | B 2 |
| $CH_3$ | $i-C_3H_7$ | NH | S | 4-Cl | B 2 |

| $R^1$ | $R^2$ | X | Y | $W_n$ | B |
|---|---|---|---|---|---|
| $CH_3$ | $t-C_4H_9$ | NH | S | 4-Cl | B 2 |
| $CH_3$ | $CF_3$ | NH | S | 2-Cl, 4-Cl | B 2 |
| $CH_3$ | $CH_2Ph$ | NH | S | 4-Cl | B 1 |
| $CH_3$ | $CH_2Ph$ | NH | S | 2-Cl, 4-Cl | B 1 |
| $CH_3$ | $CH_2Ph$ | NCHO | S | 2-Cl, 4-Cl | B 1 |
| $CH_3$ | CHO | NH | S | 4-Cl | B 1 |
| $CH_3$ | CHO | NCHO | S | 4-Cl | B 1 |
| $CH_3$ | CHO | NH | S | 2-Cl, 4-Cl | B 1 |
| $CH_3$ | CHO | NCHO | S | 2-Cl, 4-Cl | B 1 |
| $CH_3$ | $COCH_3$ | NH | S | 4-Cl | B 1 |

| $R^1$ | $R^2$ | X | Y | $W_n$ | B |
|---|---|---|---|---|---|
| $CH_3$ | $COCH_3$ | NH | S | 4-Br | B 1 |
| $CH_3$ | $COCH_3$ | NH | S | 2-Cl, 4-Cl | B 1 |
| $CH_3$ | $COCH_3$ | NCHO | S | 2-Cl, 4-Cl | B 1 |
| $CH_3$ | $SO_2CH_3$ | NH | S | 4-Cl | B 1 |
| $CH_3$ | $SO_2CH_3$ | NH | S | 2-Cl, 4-Cl | B 1 |
| $CH_3$ | $SO_2CH_3$ | NH | S | 3-Cl, 4-$CH_3$ | B 1 |
| $CH_3$ | $CONHCH_3$ | NH | S | 4-Cl | B 1 |
| $CH_3$ | $CONHCH_3$ | NH | S | 4-Br | B 1 |
| $CH_3$ | $CONHCH_3$ | NH | S | 2-Cl, 4-Cl | B 1 |
| $CH_3$ | $CONHCH_3$ | NH | S | 3-Cl, 4-$CH_3$ | B 1 |

| $R^1$ | $R^2$ | X | Y | $W_n$ | B |
|---|---|---|---|---|---|
| $CH_3$ | $CH_2Ph$ | NH | S | 4-Cl | B2 |
| $CH_3$ | $CH_2Ph$ | NH | S | 2-Cl, 4-Cl | B2 |
| $CH_3$ | $COCH_3$ | NH | S | 4-Cl | B2 |
| $CH_3$ | $CONHCH_3$ | NH | S | 4-Cl | B2 |
| $CH_3$ | $SO_2CH_3$ | NH | S | 4-Cl | B2 |
| $CH_3$ | $SO_2CH_3$ | NH | S | 2-Cl, 4-Cl | B2 |
| H | $CH_3$ | NH | S | 4-Cl | B1 |
| H | $CH_3$ | NH | S | 2-Cl, 4-Cl | B1 |
| H | $CH_3$ | NH | S | 3-Cl, 4-$CH_3$ | B1 |
| $CF_3$ | $CH_3$ | NH | S | 4-Cl | B1 |

| $R^1$ | $R^2$ | X | Y | $W_n$ | B |
|---|---|---|---|---|---|
| $CF_3$ | $CH_3$ | NH | S | 4-Br | B 1 |
| $CF_3$ | $CH_3$ | NH | S | 2-Cl, 4-Cl | B 1 |
| $CF_3$ | $CH_3$ | NH | S | 2-Cl, 4-$CH_3$ | B 1 |
| $C_2H_5$ | $CH_3$ | NH | S | 4-Cl | B 1 |
| $C_2H_5$ | $CH_3$ | NH | S | 4-Br | B 1 |
| $C_2H_5$ | $CH_3$ | NH | S | 2-Cl, 4-Cl | B 1 |
| $C_2H_5$ | $CH_3$ | NH | S | 2-Cl, 4-$CH_3$ | B 1 |
| i-$C_3H_7$ | $CH_3$ | NH | S | 4-Cl | B 1 |
| i-$C_3H_7$ | $CH_3$ | NH | S | 2-Cl, 4-Cl | B 1 |
| i-$C_3H_7$ | $CH_3$ | NH | S | 3-Cl, 4-$CH_3$ | B 1 |

| $R^1$ | $R^2$ | X | Y | $W_n$ | B |
|---|---|---|---|---|---|
| t-$C_4H_9$ | $CH_3$ | NH | S | 4-Cl | B 1 |
| t-$C_4H_9$ | $CH_3$ | NH | S | 4-Br | B 1 |
| t-$C_4H_9$ | $CH_3$ | NH | S | 2-Cl, 4-Cl | B 1 |
| t-$C_4H_9$ | $CH_3$ | NH | S | 3-Cl, 4-$CH_3$ | B 1 |
| H | $CH_3$ | NH | S | 4-Cl | B 2 |
| H | $CH_3$ | NH | S | 2-Cl, 4-Cl | B 2 |
| $C_2H_5$ | $CH_3$ | NH | S | 4-Cl | B 2 |
| i-$C_3H_7$ | $CH_3$ | NH | S | 4-Cl | B 2 |
| $CF_3$ | $CH_3$ | NH | S | 4-Cl | B 2 |
| $CF_3$ | $CH_3$ | NH | S | 2-Cl, 4-Cl | B 2 |

| $R^1$ | $R^2$ | X | Y | $W_n$ | B |
|---|---|---|---|---|---|
| Cl | $CH_3$ | NH | S | 4-Cl | B 1 |
| Cl | $CH_3$ | NH | S | 2-Cl, 4-Cl | B 1 |
| Cl | $CH_3$ | NH | S | 3-Cl, 4-$CH_3$ | B 1 |
| Cl | $CH_3$ | NCHO | S | 4-Cl | B 1 |
| Cl | $CH_3$ | NH | S | 4-Br | B 1 |
| Cl | $CH_3$ | NCHO | S | 2-Cl, 4-Cl | B 1 |
| Cl | $CH_3$ | NCHO | S | 2-Cl, 4-$CH_3$ | B 1 |
| $CH_3O$ | $CH_3$ | NH | S | 4-Cl | B 1 |
| $CH_3O$ | $CH_3$ | NH | S | 4-Br | B 1 |
| $CH_3O$ | $CH_3$ | NH | S | 2-Cl, 4-Cl | B 1 |

| $R^1$ | $R^2$ | X | Y | $W_n$ | B |
|---|---|---|---|---|---|
| $CH_3O$ | $CH_3$ | NH | S | 2-Cl, 4-$CH_3$ | B 1 |
| $CH_3O$ | $CH_3$ | NCHO | S | 4-Cl | B 1 |
| $CH_3O$ | $CH_3$ | NCHO | S | 2-Cl, 4-Cl | B 1 |
| $CH_3O$ | $CH_3$ | NCHO | S | 3-Cl, 4-$CH_3$ | B 1 |
| $CH_3S$ | $CH_3$ | NH | S | 4-Cl | B 1 |
| $CH_3S$ | $CH_3$ | NH | S | 4-Br | B 1 |
| $CH_3S$ | $CH_3$ | NH | S | 2-Cl, 4-Cl | B 1 |
| $CH_3S$ | $CH_3$ | NH | S | 3-Cl, 4-$CH_3$ | B 1 |
| $CH_3S$ | $CH_3$ | NCHO | S | 4-Cl | B 1 |
| $CH_3S$ | $CH_3$ | NCHO | S | 2-Cl, 4-Cl | B 1 |

| $R^1$ | $R^2$ | X | Y | $W_n$ | B |
|---|---|---|---|---|---|
| Cl | $CH_3$ | NH | S | 4-Cl | B2 |
| Cl | $CH_3$ | NH | S | 2-Cl, 4-Cl | B2 |
| $CH_3O$ | $CH_3$ | NH | S | 2-Cl, 4-Cl | B2 |
| $CH_3S$ | $CH_3$ | NH | S | 2-Cl, 4-Cl | B2 |
| $CH_3$ | $CH_3$ | SO | S | 4-Cl | B1 |
| $CH_3$ | $CH_3$ | $SO_2$ | S | 4-Cl | B1 |
| $CH_3$ | $CH_3$ | NH | SO | 4-Cl | B1 |
| $CH_3$ | $CH_3$ | NH | $SO_2$ | 4-Cl | B1 |
| $CH_3$ | $CH_3$ | NH | SO | 2-Cl, 4-Cl | B1 |
| $CH_3$ | $CH_3$ | NH | $SO_2$ | 2-Cl, 4-Cl | B1 |

| $R^1$ | $R^2$ | X | Y | $W_n$ | B |
|---|---|---|---|---|---|
| $CH_3$ | $CH_3$ | NH | SO | 3-Cl, 4-$CH_3$ | B 1 |
| $CH_3$ | $CH_3$ | NH | $SO_2$ | 3-Cl, 4-$CH_3$ | B 1 |
| $CH_3$ | $CH_3$ | S | O | 4-Cl | B 1 |
| $CH_3$ | $CH_3$ | SO | O | 4-Cl | B 1 |
| $CH_3$ | $CH_3$ | $SO_2$ | O | 4-Cl | B 1 |
| $CH_3$ | $CH_3$ | S | O | 2-Cl, 4-Cl | B 1 |
| $CH_3$ | $CH_3$ | SO | O | 2-Cl, 4-Cl | B 1 |
| $CH_3$ | $CH_3$ | $SO_2$ | O | 2-Cl, 4-Cl | B 1 |
| $CH_3$ | $CH_3$ | NH | O | 4-Cl | B 1 |
| $CH_3$ | $CH_3$ | NH | O | 4-$CH_3$ | B 1 |

| $R^1$ | $R^2$ | X | Y | $W_n$ | B |
|---|---|---|---|---|---|
| $CH_3$ | $CH_3$ | NH | O | 2-Cl, 4-Cl | B 1 |
| $CH_3$ | $CH_3$ | NH | O | 2-Cl, 4-$CH_3$ | B 1 |
| $CH_3$ | $CH_3$ | NH | O | 3-Cl, 4-$CH_3$ | B 1 |
| $CH_3$ | $CH_3$ | NH | O | 2-F, 4-Cl | B 1 |
| $CH_3$ | $CH_3$ | NCHO | O | 4-Cl | B 1 |
| $CH_3$ | $CH_3$ | $NCH_3$ | O | 4-Cl | B 1 |
| $CH_3$ | $CH_3$ | NH | O | 4-Cl | B 2 |
| $CH_3$ | $CH_3$ | NH | O | 2-Cl, 4-Cl | B 2 |
| $CH_3$ | $CH_3$ | NH | S | 4-F | B 2 |
| $CH_3$ | $CH_3$ | NH | S | 4-Cl | B 2 |

| $R^1$ | $R^2$ | X | Y | $W_n$ | B |
|---|---|---|---|---|---|
| $CH_3$ | $CH_3$ | NH | S | 4-$CH_3$ | B2 |
| $CH_3$ | $CH_3$ | NH | S | 2-Cl, 4-Cl | B2 |
| $CH_3$ | $CH_3$ | NH | S | 3-Cl, 4-Cl | B2 |
| $CH_3$ | $CH_3$ | NH | S | 2-Cl, 4-$CH_3$ | B2 |
| $CH_3$ | $CH_3$ | NH | S | 3-Cl, 4-$CH_3$ | B2 |
| $CH_3$ | $CH_3$ | NH | S | 2-F, 4-F | B2 |
| $CH_3$ | $CH_3$ | NH | S | 2-F, 4-Cl | B2 |
| $CH_3$ | $CH_3$ | NH | S | 2-F, 4-Br | B2 |
| $CH_3$ | $CH_3$ | NH | S | 2-F, 4-$CH_3$ | B2 |
| $CH_3$ | $CH_3$ | NH | S | 3-F, 4-$CH_3$ | B2 |

| $R^1$ | $R^2$ | X | Y | $W_n$ | B |
|---|---|---|---|---|---|
| $CH_3$ | $CH_3$ | NCHO | S | 4-Cl | B 2 |
| $CH_3$ | $CH_3$ | NCHO | S | 2-Cl, 4-Cl | B 2 |
| $CH_3$ | $CH_3$ | NCHO | S | 3-Cl, 4-$CH_3$ | B 2 |
| $CH_3$ | $CH_3$ | NH | S | 4-Cl | B 3 |
| $CH_3$ | $CH_3$ | NH | S | 4-Br | B 3 |
| $CH_3$ | $CH_3$ | NH | S | 2-Cl, 4-Cl | B 3 |
| $CH_3$ | $CH_3$ | NH | S | 2-Cl, 4-$CH_3$ | B 3 |
| $CH_3$ | $CH_3$ | NH | S | 3-Cl, 4-$CH_3$ | B 3 |
| $CH_3$ | $CH_3$ | NH | S | 4-Cl | B 4 |
| $CH_3$ | $CH_3$ | NH | S | 2-Cl, 4-Cl | B 4 |

| $R^1$ | $R^2$ | X | Y | $W_n$ | B |
|---|---|---|---|---|---|
| $CH_3$ | $CH_3$ | NH | S | 4-Cl | B5 |
| $CH_3$ | $CH_3$ | NH | S | 2-Cl, 4-Cl | B5 |
| $CH_3$ | $CH_3$ | NH | S | 4-Cl | B6 |
| $CH_3$ | $CH_3$ | NH | S | 2-Cl, 4-Cl | B6 |
| $CH_3$ | $CH_3$ | NH | S | 4-Cl | B7 |
| $CH_3$ | $CH_3$ | NH | S | 2-Cl, 4-$CH_3$ | B7 |
| $CH_3$ | $CH_3$ | NH | S | 4-Cl | B8 |
| $CH_3$ | $CH_3$ | NH | S | 2-Cl, 4-Cl | B8 |
| $CH_3$ | $CH_3$ | NH | S | 4-Cl | B9 |
| $CH_3$ | $CH_3$ | NH | S | 2-Cl, 4-$CH_3$ | B9 |

| $R^1$ | $R^2$ | X | Y | $W_n$ | B |
|---|---|---|---|---|---|
| $CH_3$ | $CH_3$ | NH | S | 4-Cl | B10 |
| $CH_3$ | $CH_3$ | NH | S | 2-Cl, 4-Cl | B10 |
| $CH_3$ | $CH_3$ | NH | S | 3-Cl, 4-Cl | B10 |
| $CH_3$ | $CH_3$ | NH | S | 2-F, 4-$CH_3$ | B10 |
| $CH_3$ | $CH_3$ | NH | S | 3-F, 4-$CH_3$ | B10 |
| $CH_3$ | $CH_3$ | NH | S | 2-F, 4-Cl | B10 |
| $CH_3$ | $CH_3$ | NH | S | 4-Cl | B11 |
| $CH_3$ | $CH_3$ | NH | S | 2-Cl, 4-$CH_3$ | B11 |
| $CH_3$ | $CH_3$ | NH | S | 4-Cl | B12 |
| $CH_3$ | $CH_3$ | NH | S | 2-Cl, 4-Cl | B12 |

| $R^1$ | $R^2$ | X | Y | $W_n$ | B |
|---|---|---|---|---|---|
| $CH_3$ | $CH_3$ | NH | S | 2-F, 4-$CH_3$ | B12 |
| $CH_3$ | $CH_3$ | NH | S | 3-F, 4-$CH_3$ | B12 |
| $CH_3$ | $CH_3$ | NH | S | 2-Cl, 4-$CH_3$ | B12 |
| $CH_3$ | $CH_3$ | NH | S | 3-Cl, 4-$CH_3$ | B12 |
| $CH_3$ | $CH_3$ | NCHO | S | 4-Cl | B12 |
| $CH_3$ | $CH_3$ | NCHO | S | 2-Cl, 4-Cl | B12 |
| $CH_3$ | $CH_3$ | NH | S | 4-Cl | B13 |
| $CH_3$ | $CH_3$ | NH | S | 2-Cl, 4-Cl | B13 |
| $CH_3$ | $CH_3$ | NH | S | 2-Cl, 4-$CH_3$ | B13 |

| $R^1$ | $R^2$ | X | Y | $W_n$ | B |
|---|---|---|---|---|---|
| $CH_3$ | $CH_3$ | NH | S | 2-F, 4-$CH_3$ | B 13 |
| $CH_3$ | $CH_3$ | NH | S | 4-Cl | B 14 |
| $CH_3$ | $CH_3$ | NH | S | 2-Cl, 4-Cl | B 14 |
| $CH_3$ | $CH_3$ | NH | S | 2-Cl, 4-$CH_3$ | B 14 |
| $CH_3$ | $CH_3$ | NH | S | 2-F, 4-$CH_3$ | B 14 |
| $CH_3$ | $CH_3$ | NH | S | 4-Cl | B 15 |
| $CH_3$ | $CH_3$ | NH | S | 2-Cl, 4-Cl | B 15 |
| $CH_3$ | $CH_3$ | NH | S | 2-Cl, 4-$CH_3$ | B 15 |
| $CH_3$ | $CH_3$ | NH | S | 2-F, 4-$CH_3$ | B 15 |
| $CH_3$ | $CH_3$ | NH | S | 4-Cl | B 16 |

| $R^1$ | $R^2$ | X | Y | $W_n$ | B |
|---|---|---|---|---|---|
| $CH_3$ | $CH_3$ | NH | S | 2-Cl, 4-Cl | B16 |
| $CH_3$ | $CH_3$ | NH | S | 2-Cl, 4-$CH_3$ | B16 |
| $CH_3$ | $CH_3$ | NH | S | 2-F, 4-$CH_3$ | B16 |
| $CH_3$ | $CH_3$ | NH | S | 4-Cl | B17 |
| $CH_3$ | $CH_3$ | NH | S | 2-Cl, 4-Cl | B17 |
| $CH_3$ | $CH_3$ | NH | S | 2-Cl, 4-$CH_3$ | B17 |
| $CH_3$ | $CH_3$ | NH | S | 2-F, 4-$CH_3$ | B17 |
| $CH_3$ | $CH_3$ | NH | S | 4-Cl | B18 |
| $CH_3$ | $CH_3$ | NH | S | 2-Cl, 4-Cl | B18 |
| $CH_3$ | $CH_3$ | NH | S | 2-Cl, 4-$CH_3$ | B18 |

| $R^1$ | $R^2$ | X | Y | $W_n$ | B |
|---|---|---|---|---|---|
| $CH_3$ | $CH_3$ | NH | S | 2-F, 4-$CH_3$ | B 1 8 |
| $CH_3$ | $CH_3$ | NCHO | S | 2-Cl, 4-Cl | B 1 |
| $CH_3$ | $CH_3$ | NCHO | S | 3-Cl, 4-Cl | B 1 |
| $CH_3$ | $CH_3$ | NCHO | S | 2-F, 4-Cl | B 1 |
| $CH_3$ | $CH_3$ | NCHO | S | 2-F, 4-$CH_3$ | B 1 |
| $CH_3$ | $CH_3$ | NCHO | S | 3-F, 4-Cl | B 1 |
| $CH_3$ | $CH_3$ | NCHO | S | 3-F, 4-$CH_3$ | B 1 |
| $CH_3$ | $CH_3$ | NCHO | S | 2-Cl, 4-$CH_3$ | B 1 |
| $CH_3$ | $CH_3$ | NCHO | S | 3-Cl, 4-$CH_3$ | B 1 |
| $CH_3$ | $CH_3$ | NCHO | S | 2-Cl, 3-Cl, 4-Cl | B 1 |

| $R^1$ | $R^2$ | X | Y | $W_n$ | B |
|---|---|---|---|---|---|
| $CH_3$ | $CH_3$ | NCHO | S | 2-Cl, 4-Cl, 5-Cl | B 1 |
| $CH_3$ | $CH_3$ | $NCOCH_3$ | S | 2-Cl, 4-Cl | B 1 |
| $CH_3$ | $CH_3$ | $NCOCH_3$ | S | 3-Cl, 4-Cl | B 1 |
| $CH_3$ | $CH_3$ | $NCOCH_3$ | S | 2-F, 4-$CH_3$ | B 1 |
| $CH_3$ | $CH_3$ | $NCOCH_3$ | S | 2-Cl, 3-Cl, 4-Cl | B 1 |
| $CH_3$ | $CH_3$ | $NCH_3$ | S | 2-Cl, 4-Cl | B 1 |
| $CH_3$ | $CH_3$ | $NCH_3$ | S | 3-Cl, 4-$CH_3$ | B 1 |
| $CH_3$ | $CH_3$ | NNO | S | 2-Cl, 4-Cl | B 1 |
| $CH_3$ | $CH_3$ | NNO | S | 3-Cl, 4-$CH_3$ | B 1 |
| $CH_3$ | $CH_3$ | $NNH_2$ | S | 4-Cl | B 1 |

| $R^1$ | $R^2$ | X | Y | $W_n$ | B |
|---|---|---|---|---|---|
| $CH_3$ | $CH_3$ | $NNH_2$ | S | 2-Cl, 4-Cl | B1 |
| $CH_3$ | $CH_3$ | $NNH_2$ | S | 3-Cl, 4-$CH_3$ | B1 |
| $CH_3$ | $CH_3$ | $NSO_2CH_3$ | S | 2-Cl, 4-Cl | B1 |
| $CH_3$ | $CH_3$ | $NSO_2CH_3$ | S | 2-F, 4-$CH_3$ | B1 |
| $CH_3$ | $CH_3$ | $NSO_2CH_3$ | S | 2-Cl, 3-Cl, 4-Cl | B1 |
| $CH_3$ | $CH_3$ | $CH_2$ | S | 4-Cl | B1 |
| $CH_3$ | $CH_3$ | $CH_2$ | S | 2-Cl, 4-Cl | B1 |
| $CH_3$ | $CH_3$ | $CH_2$ | S | 2-Cl, 4-$CH_3$ | B1 |
| $CH_3$ | $CH_3$ | $CH_2$ | S | 2-F, 4-$CH_3$ | B1 |
| $CH_3$ | $CH_3$ | CH(OH) | S | 4-Cl | B1 |

| $R^1$ | $R^2$ | X | Y | $W_n$ | B |
|---|---|---|---|---|---|
| $CH_3$ | $CH_3$ | CH(OH) | S | 2-Cl, 4-Cl | B 1 |
| $CH_3$ | $CH_3$ | CH(OH) | S | 2-Cl, 4-$CH_3$ | B 1 |
| $CH_3$ | $CH_3$ | CH(OH) | S | 3-Cl, 4-$CH_3$ | B 1 |
| $CH_3$ | $CH_3$ | CH(OH) | S | 2-F, 4-$CH_3$ | B 1 |
| $CH_3$ | $CH_3$ | CH(OH) | S | 3-F, 4-$CH_3$ | B 1 |
| $CH_3$ | $CH_3$ | $CH(OCH_3)$ | S | 4-Cl | B 1 |
| $CH_3$ | $CH_3$ | $CH(OCH_3)$ | S | 2-Cl, 4-Cl | B 1 |
| $CH_3$ | $CH_3$ | $CH(OCOCH_3)$ | S | 4-Cl | B 1 |
| $CH_3$ | $CH_3$ | $CH(OCOCH_3)$ | S | 2-Cl, 4-Cl | B 1 |
| $CH_3$ | $CH_3$ | $CH(OCOCH_3)$ | S | 2-Cl, 4-$CH_3$ | B 1 |

| $R^1$ | $R^2$ | X | Y | $W_n$ | B |
|---|---|---|---|---|---|
| $CH_3$ | $CH_3$ | $CH(OCOCH_3)$ | S | 2-F, 4-$CH_3$ | B 1 |
| $CH_3$ | $CH_3$ | CH(F) | S | 4-Cl | B 1 |
| $CH_3$ | $CH_3$ | CH(F) | S | 2-Cl, 4-Cl | B 1 |
| $CH_3$ | $CH_3$ | CH(Cl) | S | 4-Cl | B 1 |
| $CH_3$ | $CH_3$ | CH(Cl) | S | 2-Cl, 4-Cl | B 1 |
| $CH_3$ | $CH_3$ | C=O | S | 4-Cl | B 1 |
| $CH_3$ | $CH_3$ | C=O | S | 2-Cl, 4-Cl | B 1 |
| $CH_3$ | $CH_3$ | C=O | S | 2-Cl, 4-$CH_3$ | B 1 |
| $CH_3$ | $CH_3$ | C=O | S | 2-F, 4-$CH_3$ | B 1 |
| $CH_3$ | $CH_3$ | $CH_2$ | S | 4-Cl | B 2 |

| $R^1$ | $R^2$ | X | Y | $W_n$ | B |
|---|---|---|---|---|---|
| $CH_3$ | $CH_3$ | $CH_2$ | S | 2-Cl, 4-Cl | B 2 |
| $CH_3$ | $CH_3$ | $CH_2$ | S | 2-Cl, 4-$CH_3$ | B 2 |
| $CH_3$ | $CH_3$ | $CH_2$ | S | 2-F, 4-$CH_3$ | B 2 |
| $CH_3$ | $CH_3$ | CH(OH) | S | 4-Cl | B 2 |
| $CH_3$ | $CH_3$ | CH(OH) | S | 2-Cl, 4-Cl | B 2 |
| $CH_3$ | $CH_3$ | CH(OH) | S | 2-Cl, 4-$CH_3$ | B 2 |
| $CH_3$ | $CH_3$ | CH(OH) | S | 3-Cl, 4-$CH_3$ | B 2 |
| $CH_3$ | $CH_3$ | CH(OH) | S | 2-F, 4-$CH_3$ | B 2 |
| $CH_3$ | $CH_3$ | CH(OH) | S | 3-F, 4-$CH_3$ | B 2 |
| $CH_3$ | $CH_3$ | CH($OCH_3$) | S | 4-Cl | B 2 |

| $R^1$ | $R^2$ | X | Y | $W_n$ | B |
|---|---|---|---|---|---|
| $CH_3$ | $CH_3$ | $CH(OCH_3)$ | S | 2-Cl, 4-Cl | B 2 |
| $CH_3$ | $CH_3$ | $CH(OCOCH_3)$ | S | 4-Cl | B 2 |
| $CH_3$ | $CH_3$ | $CH(OCOCH_3)$ | S | 2-Cl, 4-Cl | B 2 |
| $CH_3$ | $CH_3$ | $CH(OCOCH_3)$ | S | 2-Cl, 4-$CH_3$ | B 2 |
| $CH_3$ | $CH_3$ | $CH(OCOCH_3)$ | S | 2-F, 4-$CH_3$ | B 2 |
| $CH_3$ | $CH_3$ | CH(F) | S | 4-Cl | B 2 |
| $CH_3$ | $CH_3$ | CH(F) | S | 2-Cl, 4-Cl | B 2 |
| $CH_3$ | $CH_3$ | CH(Cl) | S | 4-Cl | B 2 |
| $CH_3$ | $CH_3$ | CH(Cl) | S | 2-Cl, 4-Cl | B 2 |
| $CH_3$ | $CH_3$ | C=O | S | 4-Cl | B 2 |

| $R^1$ | $R^2$ | X | Y | $W_n$ | B |
|---|---|---|---|---|---|
| $CH_3$ | $CH_3$ | C=O | S | 2-Cl, 4-Cl | B 2 |
| $CH_3$ | $CH_3$ | C=O | S | 2-Cl, 4-$CH_3$ | B 2 |
| $CH_3$ | $CH_3$ | C=O | S | 2-F, 4-$CH_3$ | B 2 |
| $CH_3$ | $CH_3$ | $C(CH_3)(OH)$ | S | 4-Cl | B 2 |
| $CH_3$ | $CH_3$ | $C(CH_3)(OH)$ | S | 2-Cl, 4-Cl | B 2 |
| $CH_3$ | $CH_3$ | $C(CH_3)(OCOCH_3)$ | S | 4-Cl | B 2 |
| $CH_3$ | $CH_3$ | $C(CH_3)(OCOCH_3)$ | S | 2-Cl, 4-Cl | B 2 |
| $CH_3$ | $CH_3$ | $C(CH_3)(F)$ | S | 4-Cl | B 2 |
| $CH_3$ | $CH_3$ | $C(CH_3)(F)$ | S | 2-Cl, 4-Cl | B 2 |
| $CH_3$ | $CH_3$ | $C(C_2H_5)(OH)$ | S | 4-Cl | B 2 |

| $R^1$ | $R^2$ | X | Y | $W_n$ | B |
|---|---|---|---|---|---|
| $CH_3$ | $CH_3$ | $C(C_2H_5)(OH)$ | S | 2-Cl, 4-Cl | B 2 |
| $CH_3$ | $CH_3$ | $C(i-C_3H_7)(OH)$ | S | 4-Cl | B 2 |
| $CH_3$ | $CH_3$ | $C(i-C_3H_7)(OH)$ | S | 2-Cl, 4-Cl | B 2 |
| $CH_3$ | $CH_3$ | $C(i-C_3H_7)(OH)$ | S | 2-Cl, 4-$CH_3$ | B 2 |
| $CH_3$ | $CH_3$ | $C(i-C_3H_7)(OH)$ | S | 2-F, 4-$CH_3$ | B 2 |
| $CH_3$ | $CH_3$ | $CH(CH_3)$ | S | 4-Cl | B 2 |
| $CH_3$ | $CH_3$ | $CH(CH_3)$ | S | 2-Cl, 4-Cl | B 2 |
| $CH_3$ | $CH_3$ | $CH(CH_3)$ | S | 2-Cl, 4-$CH_3$ | B 2 |
| $CH_3$ | $CH_3$ | $CH(CH_3)$ | S | 2-F, 4-$CH_3$ | B 2 |
| Cl | $CH_3$ | $CH_2$ | S | 4-Cl | B 2 |

| $R^1$ | $R^2$ | X | Y | $W_n$ | B |
|---|---|---|---|---|---|
| Cl | $CH_3$ | $CH_2$ | S | 2-Cl, 4-Cl | B2 |
| Cl | $CH_3$ | $CH_2$ | S | 2-Cl, 4-$CH_3$ | B2 |
| Cl | $CH_3$ | $CH_2$ | S | 2-F, 4-$CH_3$ | B2 |
| $CH_3O$ | $CH_3$ | $CH_2$ | S | 4-Cl | B2 |
| $CH_3O$ | $CH_3$ | $CH_2$ | S | 2-Cl, 4-Cl | B2 |
| $CH_3O$ | $CH_3$ | $CH_2$ | S | 2-Cl, 4-$CH_3$ | B2 |
| $CH_3O$ | $CH_3$ | $CH_2$ | S | 2-F, 4-$CH_3$ | B2 |

wobei B1 bis B18 wie nachstehend definiert sind:

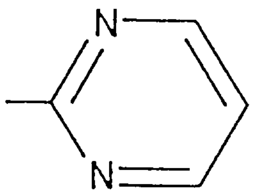

B1

,

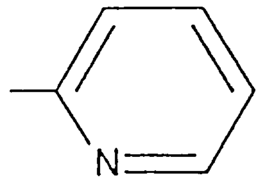

B2

,

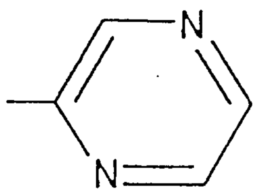

B3

,

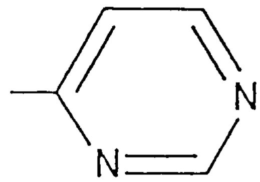

B4

,

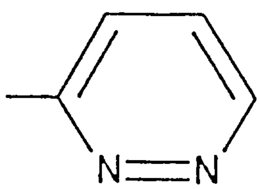

B5

,

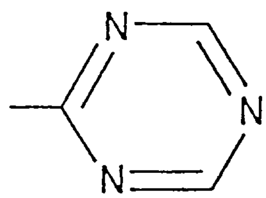

B6

,

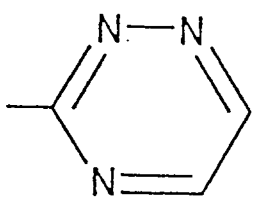

B7

,

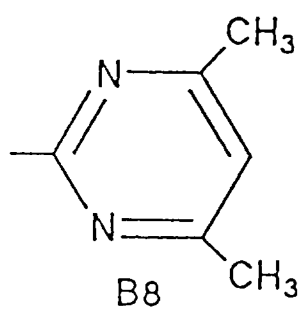

B8

,

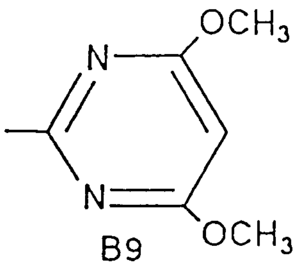
B9 ,

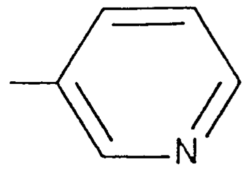
B10 ,

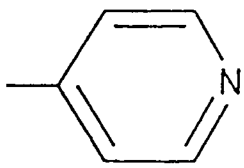
B11 ,

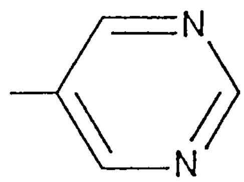
B12

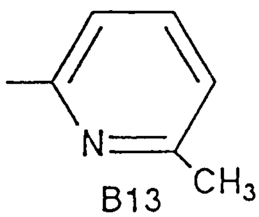
B13 ,

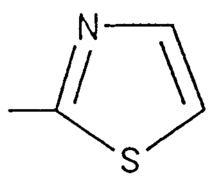
B14 ,

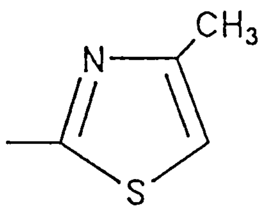
B15 ,

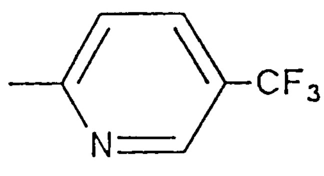
B16 ,

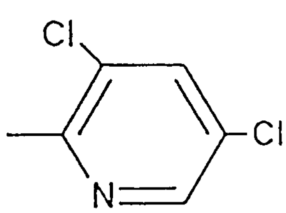
B17 ,

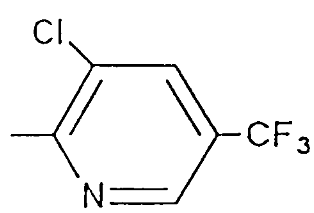
B18 ,

oder wobei die substituierten Pyrazolderivate durch die Formel (1b) definiert sind:

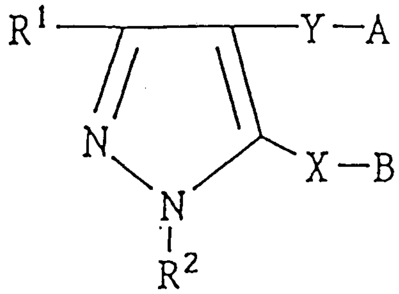

(1b)

wobei die Substituenten die folgenden Bedeutungen haben:

| $R^1$ | $R^2$ | X | Y | A | B |
|---|---|---|---|---|---|
| $CH_3$ | $CH_3$ | NH | S | A 1 | B 1 |
| $CH_3$ | $CH_3$ | NH | S | A 1 | B 2 |
| $CH_3$ | $CH_3$ | NH | S | A 2 | B 1 |

| $R^1$ | $R^2$ | X | Y | A | B |
|---|---|---|---|---|---|
| $CH_3$ | $CH_3$ | NH | S | A 2 | B 2 |
| $CH_3$ | $CH_3$ | NH | S | A 3 | B 1 |
| $CH_3$ | $CH_3$ | NH | S | A 3 | B 2 |
| $CH_3$ | $CH_3$ | NH | S | A 4 | B 1 |
| $CH_3$ | $CH_3$ | NH | S | A 4 | B 2 |
| $CH_3$ | $CH_3$ | NH | S | A 5 | B 1 |
| $CH_3$ | $CH_3$ | NH | S | A 5 | B 2 |
| $CH_3$ | $CH_3$ | NH | S | A 6 | B 1 |
| $CH_3$ | $CH_3$ | NH | S | A 6 | B 2 |
| $CH_3$ | $CH_3$ | NH | S | A 7 | B 1 |
| $CH_3$ | $CH_3$ | NH | S | A 7 | B 2 |
| $CH_3$ | $CH_3$ | NH | S | A 8 | B 1 |
| $CH_3$ | $CH_3$ | NH | S | A 8 | B 2 |
| $CH_3$ | $CH_3$ | NH | S | A 9 | B 1 |
| $CH_3$ | $CH_3$ | NH | S | A 9 | B 2 |
| $CH_3$ | $CH_3$ | NH | S | A 10 | B 1 |
| $CH_3$ | $CH_3$ | NH | S | A 10 | B 2 |
| $CH_3$ | $CH_3$ | NH | S | A 11 | B 1 |
| $CH_3$ | $CH_3$ | NH | S | A 11 | B 2 |

wobei A1 bis A11 wie nachstehend definiert sind:

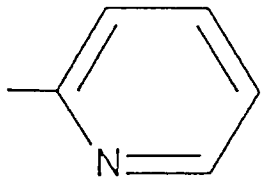
A1
,
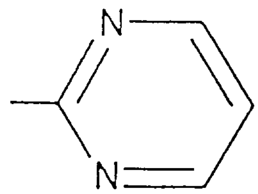
A2
,

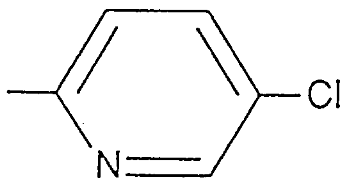
A3
,
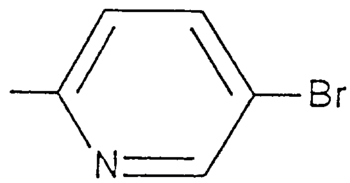
A4
,

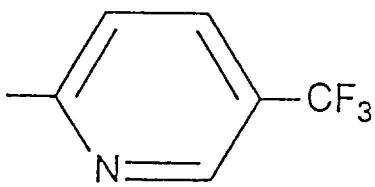
A5
,
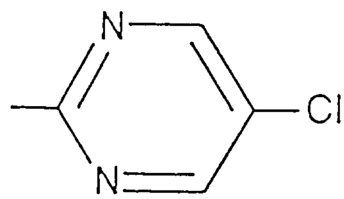
A6
,

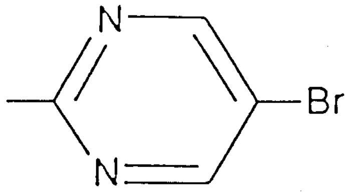

A7 ,

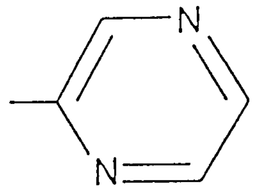

A8 ,

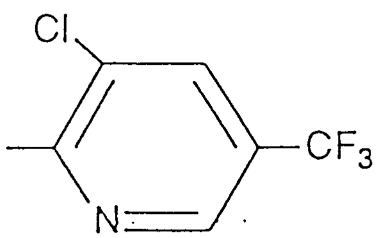

A9 ,

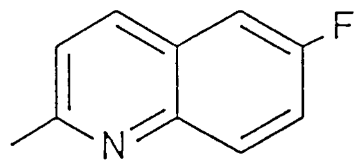

A10 ,

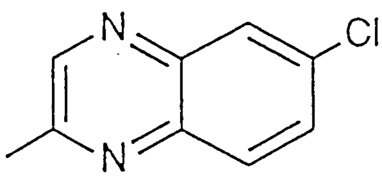

A11 ,

und B1 und B2 wie oben definiert sind, und zwar nach den folgenden Reaktionsschemata:

(Verfahren 1)

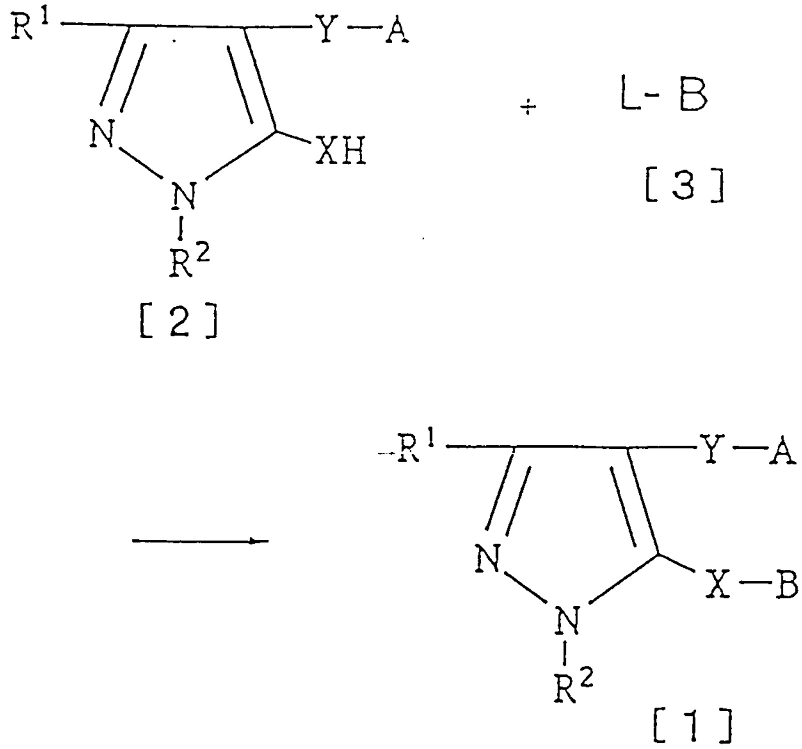

[2] [3] [1]

worin $R^1$, $R^2$, X, Y und A die selben Bedeutungen wie oben haben, L eine Abgangsgruppe darstellt und B die selbe Bedeutung wie oben hat,

(Verfahren 2)

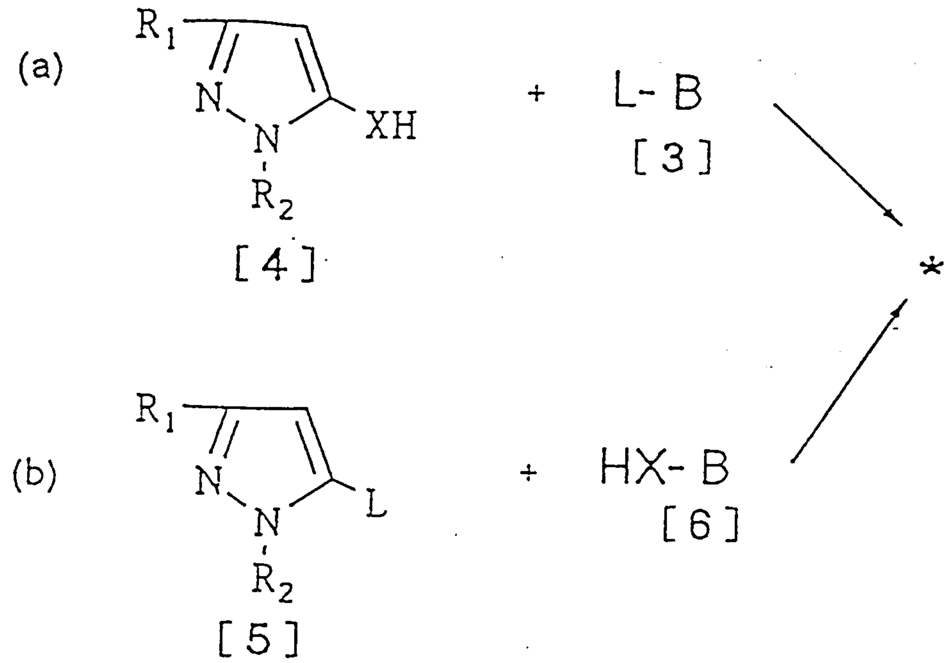

(a) [4] [3]

(b) [5] [6]

Wenn Y ≠ 0

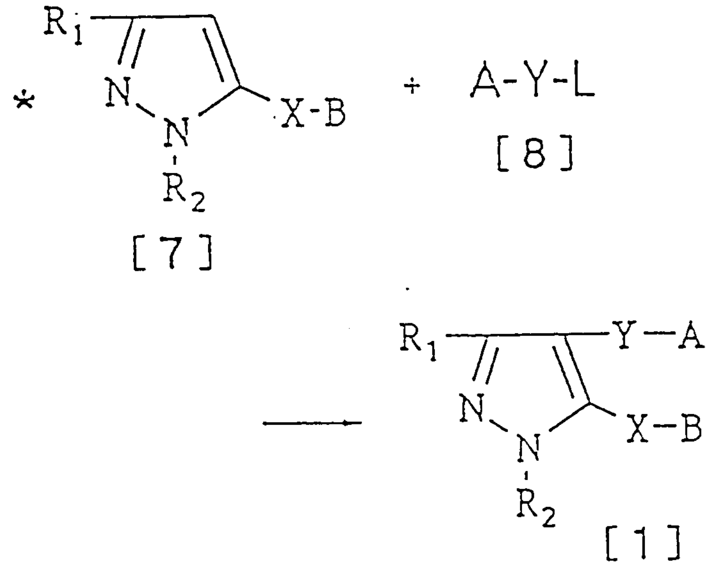

[7] [8] [1]

wobei $R^1$, $R^2$, B, X und A die selben Bedeutungen wie oben haben und L eine Abgangsgruppe darstellt, und Y die selben Bedeutungen wie oben, mit Ausnahme eines Sauerstoffatoms, hat,

(Verfahren 3)

Wenn X = N-$R^3$, R3 ≠ H:

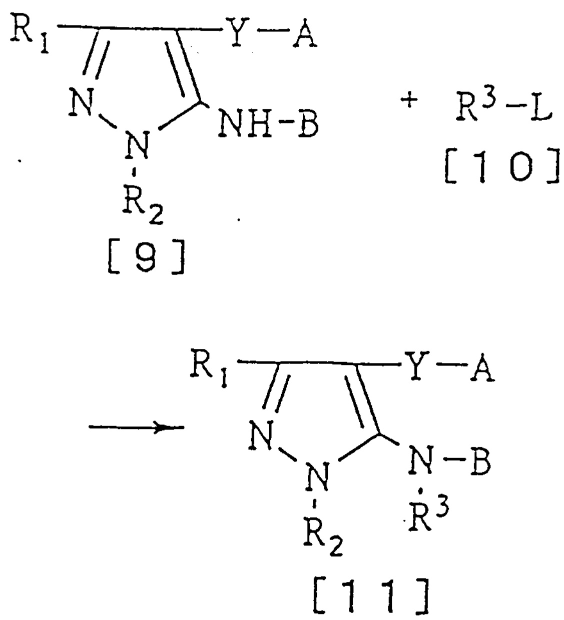

[9] [10] [11]

worin $R^1$, $R^2$, A, B und Y die selben Bedeutungen wie oben haben, L eine Abgangsgruppe darstellt und $R^3$ die selbe Bedeutung wie oben, mit Ausnahme eines Wasserstoffatoms, hat.

**2.** Verfahren zur Herstellung von substituierten Pyrazolderivaten gemäss Anspruch 1, wobei die substituierten Pyrazolderivate Verbindungen der Formel (1a) sind.

**3.** Verfahren zur Herstellung von substituierten Pyrazolderivaten gemäss Anspruch 1, bei denen X -N($R^3$)- ist.

4. Verfahren zur Herstellung von substituierten Pyrazolderivaten gemäss Anspruch 2, in denen Y -S-ist.

5. Verfahren zur Herstellung von substituierten Pyrazolderivaten gemäss Anspruch 1, die Verbindungen der allgemeinen Formel (1a) sind, wobei $R^1$ und $R^2$ jeweils eine Alkylgruppe sind, X -N($R^3$)- ist, Y -S-ist und B B1, B2, B4, B10, B11 oder B12 ist.

6. Fungizid zur landwirtschaftlichen und gartenbaulichen Verwendung, enthaltend ein oder mehrere substituierte Pyrazole gemäss Anspruch 1 als Wirkstoff.

7. Verfahren zur Herstellung eines Fungizids zur landwirtschaftlichen und gartenbaulichen Verwendung durch Herstellung eines Fungizids, das ein oder mehrere substituierte Pyrazole gemäss Anspruch 1 als Wirkstoff enthält.

**Revendications**

**Revendications pour les Etats contractants suivants : AT, BE, CH, DE, DK, FR, GB, GR, IT, LI, LU, NL, SE**

1. Dérivés du pyrazole substitués, selon lesquels les dérivés du pyrazole substitués sont définis par une formule générale [1] :

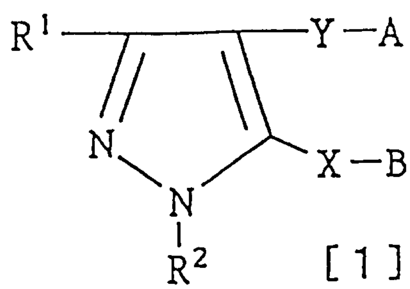

[1]

dans laquelle $R^1$ représente un atome d'hydrogène, un atome d'halogène un groupe alkyle, un groupe alcoxy, un groupe alkylthio ou un groupe haloalkyle ;
$R^2$ représente un atome d'hydrogène, un groupe alkyle, un groupe haloalkyle, un groupe phénylalkyle, -$COR^6$ ou -$SO_2R^7$ ;
X représente -S-, -SO-, -$SO_2$-, -N($R^3$)-, -CO- ou -C($R^4$)($R^5$)- ;
$R^3$ représente un atome d'hydrogène, un groupe alkyle, un groupe haloalkyle, un groupe alcényle, un groupe alcynyle, un groupe alcoxyalkyle, un groupe cyanoalkyle, un groupe alkylcarbonylalkyle, un groupe alcoxycarbonylalkyle, un groupe nitroso, un groupe amino, un groupe phénylalkyle, -$COR^6$ ou -$SO_2R^7$ ;
$R^4$ et $R^5$ représentent indépendamment un atome d'hydrogène, un atome d'halogène, un groupe alkyle, un groupe haloalkyle, un groupe alcényle, un groupe alcynyle ou -$OR^8$ ;
$R^8$ représente un atome d'hydrogène, un groupe alkyle, un groupe haloalkyle, un groupe alcényle, un groupe alcynyle, un groupe alcoxyalkyle, un groupe cyanoalkyle, un groupe alkylcarbonylalkyle, un groupe alcoxycarbonylalkyle, un groupe phénylalkyle, -$COR^6$ ou -$SO_2R^7$ ;
$R^6$ représente un atome d'hydrogène, un groupe alkyle, un groupe haloalkyle, un groupe phényle, un groupe phénylalkyle, un groupe alcoxy ou

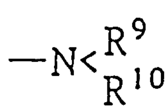 ;

$R^7$ représente un groupe alkyle, un groupe haloalkyle, un groupe phényle ou

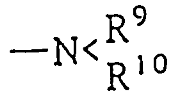

;

$R^9$ et $R^{10}$ représentent indépendamment un atome d'hydrogène, un groupe alkyle ou un groupe phényle ;
Y représente un atome d'oxygène, -S-, -SO- ou -$SO_2$-;
A représente un groupe phényle ;
B représente

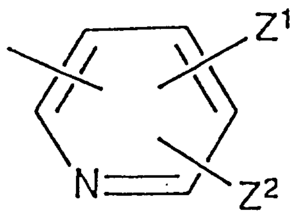

,

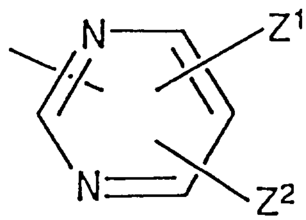

,

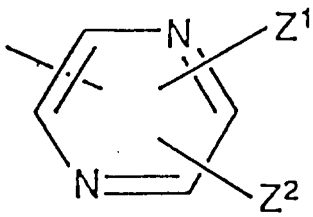

,

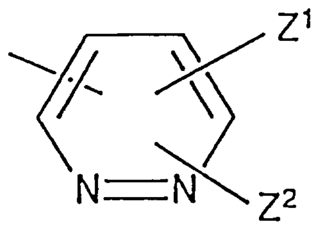

,

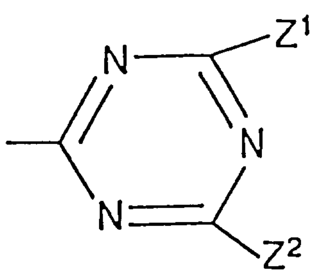

,

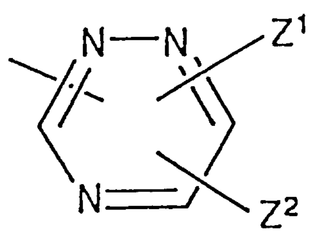

ou

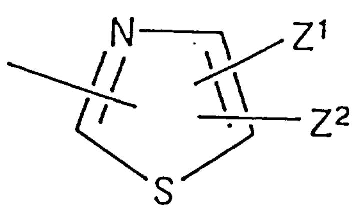

;

$Z^1$ et $Z^2$ représentent indépendamment un atome d'hydrogène, un atome d'halogène, un groupe alkyle,

un groupe alcoxy ou un groupe haloalkyle,
et les dérivés du pyrazole substitués de formule générale [1] incluent les composés de formule [1a]

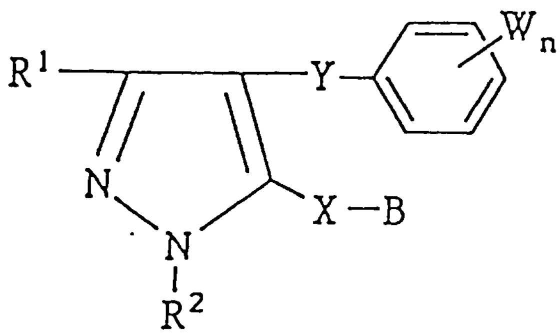

[1a]

ayant les significations des substituants suivantes

| $R^1$ | $R^2$ | X | Y | $W_n$ | B |
|---|---|---|---|---|---|
| H | $CH_3$ | S | S | 4-Cl | B 1 |
| $CH_3$ | $CH_3$ | S | S | 4-Cl | B 1 |
| $CF_3$ | $CH_3$ | S | S | 4-Cl | B 1 |
| $CH_3$ | $CH_3$ | S | S | 4-$CH_3$ | B 1 |
| $CF_3$ | $CH_3$ | S | S | 4-$CH_3$ | B 1 |
| $CH_3$ | H | S | S | 4-Cl | B 1 |
| $CH_3$ | H | S | S | 4-$CH_3$ | B 1 |
| $CH_3$ | $CF_3$ | S | S | 4-Cl | B 1 |
| $CH_3$ | $CF_3$ | S | S | 4-$CH_3$ | B 1 |
| $CH_3$ | $CH_3$ | S | S | 4-$CF_3$ | B 1 |
| $CH_3$ | $CH_3$ | S | S | 4-Br | B 1 |
| $CH_3$ | $CH_3$ | S | S | 4-$NO_2$ | B 1 |
| $CH_3$ | $CH_3$ | S | S | 4-$OCH_3$ | B 1 |
| $CH_3$ | $CH_3$ | S | S | 4-$C_2H_5$ | B 1 |
| $CH_3$ | $CH_3$ | S | S | 2-Cl | B 1 |
| $CH_3$ | $CH_3$ | S | S | 2-Cl, 4-Cl | B 1 |
| $CH_3$ | $CH_3$ | S | S | 2-Cl, 4-$CH_3$ | B 1 |
| $CH_3$ | $CH_3$ | S | S | 4-Cl | B 2 |
| $CF_3$ | $CH_3$ | S | S | 4-Cl | B 2 |

| $R^1$ | $R^2$ | X | Y | $W_n$ | B |
|---|---|---|---|---|---|
| $CH_3$ | $CH_3$ | S | S | 4-$CH_3$ | B 2 |
| $CF_3$ | $CH_3$ | S | S | 4-$CH_3$ | B 2 |
| $CH_3$ | $CH_3$ | S | S | 2-Cl, 4-Cl | B 2 |
| $CH_3$ | $CH_3$ | S | S | 2-Cl, 4-$CH_3$ | B 2 |
| $CH_3$ | $CH_3$ | S | O | 4-Cl | B 2 |
| $CH_3$ | $CH_3$ | S | O | 4-$CH_3$ | B 2 |
| $CF_3$ | $CH_3$ | S | O | 4-$CH_3$ | B 2 |
| $CH_3$ | $CH_3$ | S | O | 3-Cl, 4-$CH_3$ | B 2 |
| $C_2H_5$ | $CH_3$ | S | S | H | B 1 |
| $C_2H_5$ | $CH_3$ | S | S | 4-Cl | B 1 |
| $C_2H_5$ | $CH_3$ | S | S | 4-$CH_3$ | B 1 |
| $C_2H_5$ | $CH_3$ | S | S | 4-$OCH_3$ | B 1 |
| $C_2H_5$ | $CH_3$ | S | S | 3-$CF_3$ | B 1 |
| $C_2H_5$ | $CH_3$ | S | S | 2-Cl, 4-Cl | B 1 |
| $C_2H_5$ | $CH_3$ | S | S | 3-Cl, 4-Cl | B 1 |
| $C_2H_5$ | $CH_3$ | S | S | 2-Cl, 4-$CH_3$ | B 1 |
| i-$C_3H_7$ | $CH_3$ | S | S | 4-Cl | B 1 |
| i-$C_3H_7$ | $CH_3$ | S | S | 4-$CH_3$ | B 1 |
| i-$C_3H_7$ | $CH_3$ | S | S | 4-$OCH_3$ | B 1 |
| t-$C_4H_9$ | $CH_3$ | S | S | 4-Cl | B 1 |

| $R^1$ | $R^2$ | X | Y | $W_n$ | B |
|---|---|---|---|---|---|
| t-$C_4H_9$ | $CH_3$ | S | S | 4-$CH_3$ | B 1 |
| t-$C_4H_9$ | $CH_3$ | S | S | 4-$OCH_3$ | B 1 |
| $CH_3$ | $C_2H_5$ | S | S | 4-Cl | B 1 |
| $CH_3$ | $C_3H_7$ | S | S | 4-Cl | B 1 |
| $CH_3$ | $CH_3$ | NH | S | H | B 1 |
| $CF_3$ | $CH_3$ | NH | S | H | B 1 |
| $CH_3$ | $CH_3$ | NH | S | 4-Cl | B 1 |
| $CF_3$ | $CH_3$ | NH | S | 4-Cl | B 1 |
| H | $CH_3$ | NH | S | 4-Cl | B 1 |
| $CH_3$ | $CH_3$ | NH | S | 4-$CH_3$ | B 1 |
| $CF_3$ | $CH_3$ | NH | S | 4-$CH_3$ | B 1 |
| H | $CH_3$ | NH | S | 4-$CH_3$ | B 1 |
| $CH_3$ | $CH_3$ | NH | S | 4-F | B 1 |
| $CH_3$ | $CH_3$ | NH | S | 4-Br | B 1 |
| $CH_3$ | $CH_3$ | NH | S | 4-I | B 1 |
| $CH_3$ | $CH_3$ | NH | S | 2-F | B 1 |
| $CH_3$ | $CH_3$ | NH | S | 2-Cl | B 1 |
| $CH_3$ | $CH_3$ | NH | S | 2-Br | B 1 |
| $CH_3$ | $CH_3$ | NH | S | 2-I | B 1 |
| $CH_3$ | $CH_3$ | NH | S | 3-F | B 1 |

| $R^1$ | $R^2$ | X | Y | $W_n$ | B |
|---|---|---|---|---|---|
| $CH_3$ | $CH_3$ | NH | S | 3-Cl | B 1 |
| $CH_3$ | $CH_3$ | NH | S | 3-Br | B 1 |
| $CH_3$ | $CH_3$ | NH | S | 3-I | B 1 |
| $CH_3$ | $CH_3$ | NH | S | 2-$OCH_3$ | B 1 |
| $CH_3$ | $CH_3$ | NH | S | 3-$OCH_3$ | B 1 |
| $CH_3$ | $CH_3$ | NH | S | 4-$OCH_3$ | B 1 |
| $CH_3$ | $CH_3$ | NH | S | 2-$CF_3$ | B 1 |
| $CH_3$ | $CH_3$ | NH | S | 3-$CF_3$ | B 1 |
| $CH_3$ | $CH_3$ | NH | S | 4-$CF_3$ | B 1 |
| $CH_3$ | $CH_3$ | NH | S | 4-$C_2H_5$ | B 1 |
| $CH_3$ | $CH_3$ | NH | S | 4-$C_3H_7$ | B 1 |
| $CH_3$ | $CH_3$ | NH | S | 4-$C_4H_9$ | B 1 |
| $CH_3$ | $CH_3$ | NH | S | 4-i-$C_3H_7$ | B 1 |
| $CH_3$ | $CH_3$ | NH | S | 4-t-$C_4H_9$ | B 1 |
| $CH_3$ | $CH_3$ | NH | S | 2-$OCF_3$ | B 1 |
| $CH_3$ | $CH_3$ | NH | S | 3-$OCF_3$ | B 1 |
| $CH_3$ | $CH_3$ | NH | S | 4-$OCF_3$ | B 1 |
| $CH_3$ | $CH_3$ | NH | S | 3-$NO_2$ | B 1 |
| $CH_3$ | $CH_3$ | NH | S | 4-$NO_2$ | B 1 |
| $CH_3$ | $CH_3$ | NH | S | 3-$NH_2$ | B 1 |

| $R^1$ | $R^2$ | X | Y | $W_n$ | B |
|---|---|---|---|---|---|
| $CH_3$ | $CH_3$ | NH | S | 4-$NH_2$ | B 1 |
| $CH_3$ | $CH_3$ | NH | S | 4-$NHCOCH_3$ | B 1 |
| $CH_3$ | $CH_3$ | NH | S | 4-$NHSO_2CH_3$ | B 1 |
| $CH_3$ | $CH_3$ | NH | S | 4-$NHCOCF_3$ | B 1 |
| $CH_3$ | $CH_3$ | NH | S | 4-$NHSO_2CF_3$ | B 1 |
| $CH_3$ | $CH_3$ | NH | S | 4-Ph | B 1 |
| $CH_3$ | $CH_3$ | NH | S | 4-OPh | B 1 |
| $CH_3$ | $CH_3$ | NH | S | 4-$OCF_2CF_2H$ | B 1 |
| $CH_3$ | $CH_3$ | NH | S | 4-$COCH_3$ | B 1 |
| $CH_3$ | $CH_3$ | NH | S | 4-NHCOPh | B 1 |
| $CH_3$ | $CH_3$ | NH | S | 4-$NHCOOCH_3$ | B 1 |
| $CH_3$ | $CH_3$ | NH | S | 4-$NHCON(CH_3)_2$ | B 1 |
| $CH_3$ | $CH_3$ | NH | S | 2-Cl, 3-Cl | B 1 |
| $CH_3$ | $CH_3$ | NH | S | 2-Cl, 4-Cl | B 1 |
| $CH_3$ | $CH_3$ | NH | S | 2-Cl, 5-Cl | B 1 |
| $CH_3$ | $CH_3$ | NH | S | 2-Cl, 6-Cl | B 1 |
| $CH_3$ | $CH_3$ | NH | S | 3-Cl, 4-Cl | B 1 |
| $CH_3$ | $CH_3$ | NH | S | 3-Cl, 5-Cl | B 1 |
| $CH_3$ | $CH_3$ | NH | S | 2-$CH_3$, 3-$CH_3$ | B 1 |
| $CH_3$ | $CH_3$ | NH | S | 2-$CH_3$, 4-$CH_3$ | B 1 |

| $R^1$ | $R^2$ | X | Y | $W_n$ | B |
|---|---|---|---|---|---|
| $CH_3$ | $CH_3$ | NH | S | 2-$CH_3$, 5-$CH_3$ | B 1 |
| $CH_3$ | $CH_3$ | NH | S | 2-$CH_3$, 6-$CH_3$ | B 1 |
| $CH_3$ | $CH_3$ | NH | S | 3-$CH_3$, 4-$CH_3$ | B 1 |
| $CH_3$ | $CH_3$ | NH | S | 3-$CH_3$, 5-$CH_3$ | B 1 |
| $CH_3$ | $CH_3$ | NH | S | 2-Cl, 3-$CH_3$ | B 1 |
| $CH_3$ | $CH_3$ | NH | S | 2-Cl, 4-$CH_3$ | B 1 |
| $CH_3$ | $CH_3$ | NH | S | 2-Cl, 5-$CH_3$ | B 1 |
| $CH_3$ | $CH_3$ | NH | S | 3-Cl, 4-$CH_3$ | B 1 |
| $CH_3$ | $CH_3$ | NH | S | 3-Cl, 5-$CH_3$ | B 1 |
| $CH_3$ | $CH_3$ | NH | S | 2-$CH_3$, 3-Cl | B 1 |
| $CH_3$ | $CH_3$ | NH | S | 2-$CH_3$, 4-Cl | B 1 |
| $CH_3$ | $CH_3$ | NH | S | 2-$CH_3$, 5-Cl | B 1 |
| $CH_3$ | $CH_3$ | NH | S | 2-F, 3-F | B 1 |
| $CH_3$ | $CH_3$ | NH | S | 2-F, 4-F | B 1 |
| $CH_3$ | $CH_3$ | NH | S | 2-F, 5-F | B 1 |
| $CH_3$ | $CH_3$ | NH | S | 2-F, 6-F | B 1 |
| $CH_3$ | $CH_3$ | NH | S | 2-F, 3-Cl | B 1 |
| $CH_3$ | $CH_3$ | NH | S | 2-F, 4-Cl | B 1 |
| $CH_3$ | $CH_3$ | NH | S | 2-F, 5-Cl | B 1 |
| $CH_3$ | $CH_3$ | NH | S | 2-F, 6-Cl | B 1 |

| $R^1$ | $R^2$ | X | Y | $W_n$ | B |
|---|---|---|---|---|---|
| $CH_3$ | $CH_3$ | NH | S | 2-F, 3-$CH_3$ | B 1 |
| $CH_3$ | $CH_3$ | NH | S | 2-F, 4-$CH_3$ | B 1 |
| $CH_3$ | $CH_3$ | NH | S | 2-F, 3-Br | B 1 |
| $CH_3$ | $CH_3$ | NH | S | 2-F, 4-Br | B 1 |
| $CH_3$ | $CH_3$ | NH | S | 3-F, 4-Cl | B 1 |
| $CH_3$ | $CH_3$ | NH | S | 3-F, 5-Cl | B 1 |
| $CH_3$ | $CH_3$ | NH | S | 3-F, 4-$CH_3$ | B 1 |
| $CH_3$ | $CH_3$ | NH | S | 3-F, 5-$CH_3$ | B 1 |
| $CH_3$ | $CH_3$ | NH | S | 2-Br, 4-Cl | B 1 |
| $CH_3$ | $CH_3$ | NH | S | 2-Br, 4-Br | B 1 |
| $CH_3$ | $CH_3$ | NH | S | 2-Br, 4-$CH_3$ | B 1 |
| $CH_3$ | $CH_3$ | NH | S | 3-Br, 4-Cl | B 1 |
| $CH_3$ | $CH_3$ | NH | S | 3-Br, 4-Br | B 1 |
| $CH_3$ | $CH_3$ | NH | S | 3-Br, 4-$CH_3$ | B 1 |
| $CH_3$ | $CH_3$ | NH | S | 2-Cl, 4-Br | B 1 |
| $CH_3$ | $CH_3$ | NH | S | 3-Cl, 4-Br | B 1 |
| $CH_3$ | $CH_3$ | NH | S | 2-Cl, 4-I | B 1 |
| $CH_3$ | $CH_3$ | NH | S | 3-Cl, 4-I | B 1 |
| $CH_3$ | $CH_3$ | NH | S | 3-F, 4-Br | B 1 |
| $CH_3$ | $CH_3$ | NH | S | 3, 4-$OCH_2O$- | B 1 |

| $R^1$ | $R^2$ | X | Y | $W_n$ | B |
|---|---|---|---|---|---|
| $CH_3$ | $CH_3$ | NH | S | 2-$CF_3$, 3-Cl | B 1 |
| $CH_3$ | $CH_3$ | NH | S | 2-$CF_3$, 4-Cl | B 1 |
| $CH_3$ | $CH_3$ | NH | S | 2-$CF_3$, 3-Br | B 1 |
| $CH_3$ | $CH_3$ | NH | S | 2-$CF_3$, 4-Br | B 1 |
| $CH_3$ | $CH_3$ | NH | S | 2-F, 4-$NO_2$ | B 1 |
| $CH_3$ | $CH_3$ | NH | S | 3-F, 4-$NO_2$ | B 1 |
| $CH_3$ | $CH_3$ | NH | S | 2-Cl, 4-$NO_2$ | B 1 |
| $CH_3$ | $CH_3$ | NH | S | 3-Cl, 4-$NO_2$ | B 1 |
| $CH_3$ | $CH_3$ | NH | S | 2-F, 4-$OCH_3$ | B 1 |
| $CH_3$ | $CH_3$ | NH | S | 3-F, 4-$OCH_3$ | B 1 |
| $CH_3$ | $CH_3$ | NH | S | 2-Cl, 4-$OCH_3$ | B 1 |
| $CH_3$ | $CH_3$ | NH | S | 3-Cl, 4-$OCH_3$ | B 1 |
| $CH_3$ | $CH_3$ | NH | S | 2-$OCH_3$, 4-$OCH_3$ | B 1 |
| $CH_3$ | $CH_3$ | NH | S | 3-$OCH_3$, 4-$OCH_3$ | B 1 |
| $CH_3$ | $CH_3$ | NH | S | 2-Cl, 3-Cl, 4-Cl | B 1 |
| $CH_3$ | $CH_3$ | NH | S | 2-Cl, 4-Cl, 5-Cl | B 1 |
| $CH_3$ | $CH_3$ | NH | S | 2-Cl, 4-Cl, 6-Cl | B 1 |
| $CH_3$ | $CH_3$ | NH | S | 3-Cl, 4-Cl, 5-Cl | B 1 |
| $CH_3$ | $CH_3$ | NH | S | 2-Cl, 3-Cl, 4-$CH_3$ | B 1 |
| $CH_3$ | $CH_3$ | NH | S | 2-Cl, 3-Cl, 4-Br | B 1 |

| $R^1$ | $R^2$ | X | Y | $W_n$ | B |
|---|---|---|---|---|---|
| $CH_3$ | $CH_3$ | NH | S | 2-Cl, 3-Cl, 4-I | B 1 |
| $CH_3$ | $CH_3$ | NH | S | 2-Cl, 4-Br, 6-Cl | B 1 |
| $CH_3$ | $CH_3$ | NH | S | 2-Cl, 4-$CH_3$, 6-Cl | B 1 |
| $CH_3$ | $CH_3$ | NH | S | 2-Br, 4-Cl, 6-Cl | B 1 |
| $CH_3$ | $CH_3$ | NCHO | S | 4-Cl | B 1 |
| $CH_3$ | $CH_3$ | $NCOCH_3$ | S | 4-Cl | B 1 |
| $CH_3$ | $CH_3$ | $NCOOCH_3$ | S | 4-Cl | B 1 |
| $CH_3$ | $CH_3$ | $NCON(CH_3)_2$ | S | 4-Cl | B 1 |
| $CH_3$ | $CH_3$ | NCONHPh | S | 4-Cl | B 1 |
| $CH_3$ | $CH_3$ | $NCH_3$ | S | 4-Cl | B 1 |
| $CH_3$ | $CH_3$ | $NC_2H_5$ | S | 4-Cl | B 1 |
| $CH_3$ | $CH_3$ | $NC_3H_7$ | S | 4-Cl | B 1 |
| $CH_3$ | $CH_3$ | $NCH_2OCH_3$ | S | 4-Cl | B 1 |
| $CH_3$ | $CH_3$ | $NCH_2CH_2OCH_3$ | S | 4-Cl | B 1 |
| $CH_3$ | $CH_3$ | $NCH_2CH=CH_2$ | S | 4-Cl | B 1 |
| $CH_3$ | $CH_3$ | $NCH_2C\equiv CH$ | S | 4-Cl | B 1 |
| $CH_3$ | $CH_3$ | $NCH_2COCH_3$ | S | 4-Cl | B 1 |
| $CH_3$ | $CH_3$ | $NCH_2COOCH_3$ | S | 4-Cl | B 1 |
| $CH_3$ | $CH_3$ | $NCH_2COOC_2H_5$ | S | 4-Cl | B 1 |
| $CH_3$ | $CH_3$ | $NCH_2CN$ | S | 4-Cl | B 1 |

| $R^1$ | $R^2$ | X | Y | $W_n$ | B |
|---|---|---|---|---|---|
| $CH_3$ | $CH_3$ | $NCH_2Ph$ | S | 4-Cl | B 1 |
| $CH_3$ | $CH_3$ | $NCH_2C_6H_4$-4-Cl | S | 4-Cl | B 1 |
| $CH_3$ | $CH_3$ | $NCH_2C_6H_4$-4-$CH_3$ | S | 4-Cl | B 1 |
| $CH_3$ | $CH_3$ | $NCH_2CH_2Ph$ | S | 4-Cl | B 1 |
| $CH_3$ | $CH_3$ | $NSO_2CH_3$ | S | 4-Cl | B 1 |
| $CH_3$ | $CH_3$ | $NSO_2N(CH_3)_2$ | S | 4-Cl | B 1 |
| $CH_3$ | $CH_3$ | NCHO | S | 4-Cl | B 2 |
| $CH_3$ | $CH_3$ | $NCH_3$ | S | 4-Cl | B 2 |
| $CH_3$ | H | NH | S | 4-Cl | B 1 |
| $CH_3$ | H | NH | S | 2-Cl, 4-Cl | B 1 |
| $CH_3$ | H | NH | S | 3-Cl, 4-$CH_3$ | B 1 |
| $CH_3$ | $C_2H_5$ | NH | S | 4-Cl | B 1 |
| $CH_3$ | $C_2H_5$ | NH | S | 4-Br | B 1 |
| $CH_3$ | $C_2H_5$ | NH | S | 2-Cl, 4-Cl | B 1 |
| $CH_3$ | $C_2H_5$ | NH | S | 2-Cl, 4-$CH_3$ | B 1 |
| $CH_3$ | i-$C_3H_7$ | NH | S | 4-Cl | B 1 |
| $CH_3$ | i-$C_3H_7$ | NH | S | 4-Br | B 1 |
| $CH_3$ | i-$C_3H_7$ | NH | S | 2-Cl, 4-Cl | B 1 |
| $CH_3$ | i-$C_3H_7$ | NH | S | 2-Cl, 4-$CH_3$ | B 1 |
| $CH_3$ | t-$C_4H_9$ | NH | S | 4-Cl | B 1 |

| $R^1$ | $R^2$ | X | Y | $W_n$ | B |
|---|---|---|---|---|---|
| $CH_3$ | $t-C_4H_9$ | NH | S | 2-Cl, 4-Cl | B 1 |
| $CH_3$ | $t-C_4H_9$ | NH | S | 3-Cl, 4-$CH_3$ | B 1 |
| $CH_3$ | $CF_3$ | NH | S | 4-Cl | B 1 |
| $CH_3$ | $CF_3$ | NH | S | 4-Br | B 1 |
| $CH_3$ | $CF_3$ | NH | S | 2-Cl, 4-Cl | B 1 |
| $CH_3$ | $CF_3$ | NH | S | 3-Cl, 4-$CH_3$ | B 1 |
| $CH_3$ | H | NH | S | 4-Cl | B 2 |
| $CH_3$ | H | NH | S | 2-Cl, 4-Cl | B 2 |
| $CH_3$ | $C_2H_5$ | NH | S | 4-Cl | B 2 |
| $CH_3$ | $i-C_3H_7$ | NH | S | 4-Cl | B 2 |
| $CH_3$ | $t-C_4H_9$ | NH | S | 4-Cl | B 2 |
| $CH_3$ | $CF_3$ | NH | S | 2-Cl, 4-Cl | B 2 |
| $CH_3$ | $CH_2Ph$ | NH | S | 4-Cl | B 1 |
| $CH_3$ | $CH_2Ph$ | NH | S | 2-Cl, 4-Cl | B 1 |
| $CH_3$ | $CH_2Ph$ | NCHO | S | 2-Cl, 4-Cl | B 1 |
| $CH_3$ | CHO | NH | S | 4-Cl | B 1 |
| $CH_3$ | CHO | NCHO | S | 4-Cl | B 1 |
| $CH_3$ | CHO | NH | S | 2-Cl, 4-Cl | B 1 |
| $CH_3$ | CHO | NCHO | S | 2-Cl, 4-Cl | B 1 |
| $CH_3$ | $COCH_3$ | NH | S | 4-Cl | B 1 |

| $R^1$ | $R^2$ | X | Y | $W_n$ | B |
|---|---|---|---|---|---|
| $CH_3$ | $COCH_3$ | NH | S | 4-Br | B 1 |
| $CH_3$ | $COCH_3$ | NH | S | 2-Cl, 4-Cl | B 1 |
| $CH_3$ | $COCH_3$ | NCHO | S | 2-Cl, 4-Cl | B 1 |
| $CH_3$ | $SO_2CH_3$ | NH | S | 4-Cl | B 1 |
| $CH_3$ | $SO_2CH_3$ | NH | S | 2-Cl, 4-Cl | B 1 |
| $CH_3$ | $SO_2CH_3$ | NH | S | 3-Cl, 4-$CH_3$ | B 1 |
| $CH_3$ | $CONHCH_3$ | NH | S | 4-Cl | B 1 |
| $CH_3$ | $CONHCH_3$ | NH | S | 4-Br | B 1 |
| $CH_3$ | $CONHCH_3$ | NH | S | 2-Cl, 4-Cl | B 1 |
| $CH_3$ | $CONHCH_3$ | NH | S | 3-Cl, 4-$CH_3$ | B 1 |
| $CH_3$ | $CH_2Ph$ | NH | S | 4-Cl | B 2 |
| $CH_3$ | $CH_2Ph$ | NH | S | 2-Cl, 4-Cl | B 2 |
| $CH_3$ | $COCH_3$ | NH | S | 4-Cl | B 2 |
| $CH_3$ | $CONHCH_3$ | NH | S | 4-Cl | B 2 |
| $CH_3$ | $SO_2CH_3$ | NH | S | 4-Cl | B 2 |
| $CH_3$ | $SO_2CH_3$ | NH | S | 2-Cl, 4-Cl | B 2 |
| H | $CH_3$ | NH | S | 4-Cl | B 1 |
| H | $CH_3$ | NH | S | 2-Cl, 4-Cl | B 1 |
| H | $CH_3$ | NH | S | 3-Cl, 4-$CH_3$ | B 1 |
| $CF_3$ | $CH_3$ | NH | S | 4-Cl | B 1 |

| $R^1$ | $R^2$ | X | Y | $W_n$ | B |
|---|---|---|---|---|---|
| $CF_3$ | $CH_3$ | NH | S | 4-Br | B 1 |
| $CF_3$ | $CH_3$ | NH | S | 2-Cl, 4-Cl | B 1 |
| $CF_3$ | $CH_3$ | NH | S | 2-Cl, 4-$CH_3$ | B 1 |
| $C_2H_5$ | $CH_3$ | NH | S | 4-Cl | B 1 |
| $C_2H_5$ | $CH_3$ | NH | S | 4-Br | B 1 |
| $C_2H_5$ | $CH_3$ | NH | S | 2-Cl, 4-Cl | B 1 |
| $C_2H_5$ | $CH_3$ | NH | S | 2-Cl, 4-$CH_3$ | B 1 |
| i-$C_3H_7$ | $CH_3$ | NH | S | 4-Cl | B 1 |
| i-$C_3H_7$ | $CH_3$ | NH | S | 2-Cl, 4-Cl | B 1 |
| i-$C_3H_7$ | $CH_3$ | NH | S | 3-Cl, 4-$CH_3$ | B 1 |
| t-$C_4H_9$ | $CH_3$ | NH | S | 4-Cl | B 1 |
| t-$C_4H_9$ | $CH_3$ | NH | S | 4-Br | B 1 |
| t-$C_4H_9$ | $CH_3$ | NH | S | 2-Cl, 4-Cl | B 1 |
| t-$C_4H_9$ | $CH_3$ | NH | S | 3-Cl, 4-$CH_3$ | B 1 |
| H | $CH_3$ | NH | S | 4-Cl | B 2 |
| H | $CH_3$ | NH | S | 2-Cl, 4-Cl | B 2 |
| $C_2H_5$ | $CH_3$ | NH | S | 4-Cl | B 2 |
| i-$C_3H_7$ | $CH_3$ | NH | S | 4-Cl | B 2 |
| $CF_3$ | $CH_3$ | NH | S | 4-Cl | B 2 |
| $CF_3$ | $CH_3$ | NH | S | 2-Cl, 4-Cl | B 2 |

| $R^1$ | $R^2$ | X | Y | $W_n$ | B |
|---|---|---|---|---|---|
| Cl | $CH_3$ | NH | S | 4-Cl | B 1 |
| Cl | $CH_3$ | NH | S | 2-Cl, 4-Cl | B 1 |
| Cl | $CH_3$ | NH | S | 3-Cl, 4-$CH_3$ | B 1 |
| Cl | $CH_3$ | NCHO | S | 4-Cl | B 1 |
| Cl | $CH_3$ | NH | S | 4-Br | B 1 |
| Cl | $CH_3$ | NCHO | S | 2-Cl, 4-Cl | B 1 |
| Cl | $CH_3$ | NCHO | S | 2-Cl, 4-$CH_3$ | B 1 |
| $CH_3O$ | $CH_3$ | NH | S | 4-Cl | B 1 |
| $CH_3O$ | $CH_3$ | NH | S | 4-Br | B 1 |
| $CH_3O$ | $CH_3$ | NH | S | 2-Cl, 4-Cl | B 1 |
| $CH_3O$ | $CH_3$ | NH | S | 2-Cl, 4-$CH_3$ | B 1 |
| $CH_3O$ | $CH_3$ | NCHO | S | 4-Cl | B 1 |
| $CH_3O$ | $CH_3$ | NCHO | S | 2-Cl, 4-Cl | B 1 |
| $CH_3O$ | $CH_3$ | NCHO | S | 3-Cl, 4-$CH_3$ | B 1 |
| $CH_3S$ | $CH_3$ | NH | S | 4-Cl | B 1 |
| $CH_3S$ | $CH_3$ | NH | S | 4-Br | B 1 |
| $CH_3S$ | $CH_3$ | NH | S | 2-Cl, 4-Cl | B 1 |
| $CH_3S$ | $CH_3$ | NH | S | 3-Cl, 4-$CH_3$ | B 1 |
| $CH_3S$ | $CH_3$ | NCHO | S | 4-Cl | B 1 |
| $CH_3S$ | $CH_3$ | NCHO | S | 2-Cl, 4-Cl | B 1 |

| $R^1$ | $R^2$ | X | Y | $W_n$ | B |
|---|---|---|---|---|---|
| Cl | $CH_3$ | NH | S | 4-Cl | B2 |
| Cl | $CH_3$ | NH | S | 2-Cl, 4-Cl | B2 |
| $CH_3O$ | $CH_3$ | NH | S | 2-Cl, 4-Cl | B2 |
| $CH_3S$ | $CH_3$ | NH | S | 2-Cl, 4-Cl | B2 |
| $CH_3$ | $CH_3$ | SO | S | 4-Cl | B1 |
| $CH_3$ | $CH_3$ | $SO_2$ | S | 4-Cl | B1 |
| $CH_3$ | $CH_3$ | NH | SO | 4-Cl | B1 |
| $CH_3$ | $CH_3$ | NH | $SO_2$ | 4-Cl | B1 |
| $CH_3$ | $CH_3$ | NH | SO | 2-Cl, 4-Cl | B1 |
| $CH_3$ | $CH_3$ | NH | $SO_2$ | 2-Cl, 4-Cl | B1 |
| $CH_3$ | $CH_3$ | NH | SO | 3-Cl, 4-$CH_3$ | B1 |
| $CH_3$ | $CH_3$ | NH | $SO_2$ | 3-Cl, 4-$CH_3$ | B1 |
| $CH_3$ | $CH_3$ | S | O | 4-Cl | B1 |
| $CH_3$ | $CH_3$ | SO | O | 4-Cl | B1 |
| $CH_3$ | $CH_3$ | $SO_2$ | O | 4-Cl | B1 |
| $CH_3$ | $CH_3$ | S | O | 2-Cl, 4-Cl | B1 |
| $CH_3$ | $CH_3$ | SO | O | 2-Cl, 4-Cl | B1 |
| $CH_3$ | $CH_3$ | $SO_2$ | O | 2-Cl, 4-Cl | B1 |
| $CH_3$ | $CH_3$ | NH | O | 4-Cl | B1 |
| $CH_3$ | $CH_3$ | NH | O | 4-$CH_3$ | B1 |

| $R^1$ | $R^2$ | X | Y | $W_n$ | B |
|---|---|---|---|---|---|
| $CH_3$ | $CH_3$ | NH | O | 2-Cl, 4-Cl | B 1 |
| $CH_3$ | $CH_3$ | NH | O | 2-Cl, 4-$CH_3$ | B 1 |
| $CH_3$ | $CH_3$ | NH | O | 3-Cl, 4-$CH_3$ | B 1 |
| $CH_3$ | $CH_3$ | NH | O | 2-F, 4-Cl | B 1 |
| $CH_3$ | $CH_3$ | NCHO | O | 4-Cl | B 1 |
| $CH_3$ | $CH_3$ | $NCH_3$ | O | 4-Cl | B 1 |
| $CH_3$ | $CH_3$ | NH | O | 4-Cl | B 2 |
| $CH_3$ | $CH_3$ | NH | O | 2-Cl, 4-Cl | B 2 |
| $CH_3$ | $CH_3$ | NH | S | 4-F | B 2 |
| $CH_3$ | $CH_3$ | NH | S | 4-Cl | B 2 |
| $CH_3$ | $CH_3$ | NH | S | 4-$CH_3$ | B 2 |
| $CH_3$ | $CH_3$ | NH | S | 2-Cl, 4-Cl | B 2 |
| $CH_3$ | $CH_3$ | NH | S | 3-Cl, 4-Cl | B 2 |
| $CH_3$ | $CH_3$ | NH | S | 2-Cl, 4-$CH_3$ | B 2 |
| $CH_3$ | $CH_3$ | NH | S | 3-Cl, 4-$CH_3$ | B 2 |
| $CH_3$ | $CH_3$ | NH | S | 2-F, 4-F | B 2 |
| $CH_3$ | $CH_3$ | NH | S | 2-F, 4-Cl | B 2 |
| $CH_3$ | $CH_3$ | NH | S | 2-F, 4-Br | B 2 |
| $CH_3$ | $CH_3$ | NH | S | 2-F, 4-$CH_3$ | B 2 |
| $CH_3$ | $CH_3$ | NH | S | 3-F, 4-$CH_3$ | B 2 |

| $R^1$ | $R^2$ | X | Y | $W_n$ | B |
|---|---|---|---|---|---|
| $CH_3$ | $CH_3$ | NCHO | S | 4-Cl | B 2 |
| $CH_3$ | $CH_3$ | NCHO | S | 2-Cl, 4-Cl | B 2 |
| $CH_3$ | $CH_3$ | NCHO | S | 3-Cl, 4-$CH_3$ | B 2 |
| $CH_3$ | $CH_3$ | NH | S | 4-Cl | B 3 |
| $CH_3$ | $CH_3$ | NH | S | 4-Br | B 3 |
| $CH_3$ | $CH_3$ | NH | S | 2-Cl, 4-Cl | B 3 |
| $CH_3$ | $CH_3$ | NH | S | 2-Cl, 4-$CH_3$ | B 3 |
| $CH_3$ | $CH_3$ | NH | S | 3-Cl, 4-$CH_3$ | B 3 |
| $CH_3$ | $CH_3$ | NH | S | 4-Cl | B 4 |
| $CH_3$ | $CH_3$ | NH | S | 2-Cl, 4-Cl | B 4 |
| $CH_3$ | $CH_3$ | NH | S | 4-Cl | B 5 |
| $CH_3$ | $CH_3$ | NH | S | 2-Cl, 4-Cl | B 5 |
| $CH_3$ | $CH_3$ | NH | S | 4-Cl | B 6 |
| $CH_3$ | $CH_3$ | NH | S | 2-Cl, 4-Cl | B 6 |
| $CH_3$ | $CH_3$ | NH | S | 4-Cl | B 7 |
| $CH_3$ | $CH_3$ | NH | S | 2-Cl, 4-$CH_3$ | B 7 |
| $CH_3$ | $CH_3$ | NH | S | 4-Cl | B 8 |
| $CH_3$ | $CH_3$ | NH | S | 2-Cl, 4-Cl | B 8 |
| $CH_3$ | $CH_3$ | NH | S | 4-Cl | B 9 |
| $CH_3$ | $CH_3$ | NH | S | 2-Cl, 4-$CH_3$ | B 9 |

| $R^1$ | $R^2$ | X | Y | $W_n$ | B |
|---|---|---|---|---|---|
| $CH_3$ | $CH_3$ | NH | S | 4-Cl | B10 |
| $CH_3$ | $CH_3$ | NH | S | 2-Cl, 4-Cl | B10 |
| $CH_3$ | $CH_3$ | NH | S | 3-Cl, 4-Cl | B10 |
| $CH_3$ | $CH_3$ | NH | S | 2-F, 4-$CH_3$ | B10 |
| $CH_3$ | $CH_3$ | NH | S | 3-F, 4-$CH_3$ | B10 |
| $CH_3$ | $CH_3$ | NH | S | 2-F, 4-Cl | B10 |
| $CH_3$ | $CH_3$ | NH | S | 4-Cl | B11 |
| $CH_3$ | $CH_3$ | NH | S | 2-Cl, 4-$CH_3$ | B11 |
| $CH_3$ | $CH_3$ | NH | S | 4-Cl | B12 |
| $CH_3$ | $CH_3$ | NH | S | 2-Cl, 4-Cl | B12 |
| $CH_3$ | $CH_3$ | NH | S | 2-F, 4-$CH_3$ | B12 |
| $CH_3$ | $CH_3$ | NH | S | 3-F, 4-$CH_3$ | B12 |
| $CH_3$ | $CH_3$ | NH | S | 2-Cl, 4-$CH_3$ | B12 |
| $CH_3$ | $CH_3$ | NH | S | 3-Cl, 4-$CH_3$ | B12 |
| $CH_3$ | $CH_3$ | NCHO | S | 4-Cl | B12 |
| $CH_3$ | $CH_3$ | NCHO | S | 2-Cl, 4-Cl | B12 |
| $CH_3$ | $CH_3$ | NH | S | 4-Cl | B13 |
| $CH_3$ | $CH_3$ | NH | S | 2-Cl, 4-Cl | B13 |
| $CH_3$ | $CH_3$ | NH | S | 2-Cl, 4-$CH_3$ | B13 |

| $R^1$ | $R^2$ | X | Y | $W_n$ | B |
|---|---|---|---|---|---|
| $CH_3$ | $CH_3$ | NH | S | 2-F, 4-$CH_3$ | B 1 3 |
| $CH_3$ | $CH_3$ | NH | S | 4-Cl | B 1 4 |
| $CH_3$ | $CH_3$ | NH | S | 2-Cl, 4-Cl | B 1 4 |
| $CH_3$ | $CH_3$ | NH | S | 2-Cl, 4-$CH_3$ | B 1 4 |
| $CH_3$ | $CH_3$ | NH | S | 2-F, 4-$CH_3$ | B 1 4 |
| $CH_3$ | $CH_3$ | NH | S | 4-Cl | B 1 5 |
| $CH_3$ | $CH_3$ | NH | S | 2-Cl, 4-Cl | B 1 5 |
| $CH_3$ | $CH_3$ | NH | S | 2-Cl, 4-$CH_3$ | B 1 5 |
| $CH_3$ | $CH_3$ | NH | S | 2-F, 4-$CH_3$ | B 1 5 |
| $CH_3$ | $CH_3$ | NH | S | 4-Cl | B 1 6 |
| $CH_3$ | $CH_3$ | NH | S | 2-Cl, 4-Cl | B 1 6 |
| $CH_3$ | $CH_3$ | NH | S | 2-Cl, 4-$CH_3$ | B 1 6 |
| $CH_3$ | $CH_3$ | NH | S | 2-F, 4-$CH_3$ | B 1 6 |
| $CH_3$ | $CH_3$ | NH | S | 4-Cl | B 1 7 |
| $CH_3$ | $CH_3$ | NH | S | 2-Cl, 4-Cl | B 1 7 |
| $CH_3$ | $CH_3$ | NH | S | 2-Cl, 4-$CH_3$ | B 1 7 |
| $CH_3$ | $CH_3$ | NH | S | 2-F, 4-$CH_3$ | B 1 7 |
| $CH_3$ | $CH_3$ | NH | S | 4-Cl | B 1 8 |
| $CH_3$ | $CH_3$ | NH | S | 2-Cl, 4-Cl | B 1 8 |
| $CH_3$ | $CH_3$ | NH | S | 2-Cl, 4-$CH_3$ | B 1 8 |

| $R^1$ | $R^2$ | X | Y | $W_n$ | B |
|---|---|---|---|---|---|
| $CH_3$ | $CH_3$ | NH | S | 2-F, 4-$CH_3$ | B18 |
| $CH_3$ | $CH_3$ | NCHO | S | 2-Cl, 4-Cl | B1 |
| $CH_3$ | $CH_3$ | NCHO | S | 3-Cl, 4-Cl | B1 |
| $CH_3$ | $CH_3$ | NCHO | S | 2-F, 4-Cl | B1 |
| $CH_3$ | $CH_3$ | NCHO | S | 2-F, 4-$CH_3$ | B1 |
| $CH_3$ | $CH_3$ | NCHO | S | 3-F, 4-Cl | B1 |
| $CH_3$ | $CH_3$ | NCHO | S | 3-F, 4-$CH_3$ | B1 |
| $CH_3$ | $CH_3$ | NCHO | S | 2-Cl, 4-$CH_3$ | B1 |
| $CH_3$ | $CH_3$ | NCHO | S | 3-Cl, 4-$CH_3$ | B1 |
| $CH_3$ | $CH_3$ | NCHO | S | 2-Cl, 3-Cl, 4-Cl | B1 |
| $CH_3$ | $CH_3$ | NCHO | S | 2-Cl, 4-Cl, 5-Cl | B1 |
| $CH_3$ | $CH_3$ | $NCOCH_3$ | S | 2-Cl, 4-Cl | B1 |
| $CH_3$ | $CH_3$ | $NCOCH_3$ | S | 3-Cl, 4-Cl | B1 |
| $CH_3$ | $CH_3$ | $NCOCH_3$ | S | 2-F, 4-$CH_3$ | B1 |
| $CH_3$ | $CH_3$ | $NCOCH_3$ | S | 2-Cl, 3-Cl, 4-Cl | B1 |
| $CH_3$ | $CH_3$ | $NCH_3$ | S | 2-Cl, 4-Cl | B1 |
| $CH_3$ | $CH_3$ | $NCH_3$ | S | 3-Cl, 4-$CH_3$ | B1 |
| $CH_3$ | $CH_3$ | NNO | S | 2-Cl, 4-Cl | B1 |
| $CH_3$ | $CH_3$ | NNO | S | 3-Cl, 4-$CH_3$ | B1 |
| $CH_3$ | $CH_3$ | $NNH_2$ | S | 4-Cl | B1 |

| $R^1$ | $R^2$ | X | Y | $W_n$ | B |
|---|---|---|---|---|---|
| $CH_3$ | $CH_3$ | $NNH_2$ | S | 2-Cl, 4-Cl | B 1 |
| $CH_3$ | $CH_3$ | $NNH_2$ | S | 3-Cl, 4-$CH_3$ | B 1 |
| $CH_3$ | $CH_3$ | $NSO_2CH_3$ | S | 2-Cl, 4-Cl | B 1 |
| $CH_3$ | $CH_3$ | $NSO_2CH_3$ | S | 2-F, 4-$CH_3$ | B 1 |
| $CH_3$ | $CH_3$ | $NSO_2CH_3$ | S | 2-Cl, 3-Cl, 4-Cl | B 1 |
| $CH_3$ | $CH_3$ | $CH_2$ | S | 4-Cl | B 1 |
| $CH_3$ | $CH_3$ | $CH_2$ | S | 2-Cl, 4-Cl | B 1 |
| $CH_3$ | $CH_3$ | $CH_2$ | S | 2-Cl, 4-$CH_3$ | B 1 |
| $CH_3$ | $CH_3$ | $CH_2$ | S | 2-F, 4-$CH_3$ | B 1 |
| $CH_3$ | $CH_3$ | CH(OH) | S | 4-Cl | B 1 |
| $CH_3$ | $CH_3$ | CH(OH) | S | 2-Cl, 4-Cl | B 1 |
| $CH_3$ | $CH_3$ | CH(OH) | S | 2-Cl, 4-$CH_3$ | B 1 |
| $CH_3$ | $CH_3$ | CH(OH) | S | 3-Cl, 4-$CH_3$ | B 1 |
| $CH_3$ | $CH_3$ | CH(OH) | S | 2-F, 4-$CH_3$ | B 1 |
| $CH_3$ | $CH_3$ | CH(OH) | S | 3-F, 4-$CH_3$ | B 1 |
| $CH_3$ | $CH_3$ | $CH(OCH_3)$ | S | 4-Cl | B 1 |
| $CH_3$ | $CH_3$ | $CH(OCH_3)$ | S | 2-Cl, 4-Cl | B 1 |
| $CH_3$ | $CH_3$ | $CH(OCOCH_3)$ | S | 4-Cl | B 1 |
| $CH_3$ | $CH_3$ | $CH(OCOCH_3)$ | S | 2-Cl, 4-Cl | B 1 |
| $CH_3$ | $CH_3$ | $CH(OCOCH_3)$ | S | 2-Cl, 4-$CH_3$ | B 1 |

| $R^1$ | $R^2$ | X | Y | $W_n$ | B |
|---|---|---|---|---|---|
| $CH_3$ | $CH_3$ | $CH(OCOCH_3)$ | S | 2-F, 4-$CH_3$ | B 1 |
| $CH_3$ | $CH_3$ | CH(F) | S | 4-Cl | B 1 |
| $CH_3$ | $CH_3$ | CH(F) | S | 2-Cl, 4-Cl | B 1 |
| $CH_3$ | $CH_3$ | CH(Cl) | S | 4-Cl | B 1 |
| $CH_3$ | $CH_3$ | CH(Cl) | S | 2-Cl, 4-Cl | B 1 |
| $CH_3$ | $CH_3$ | C=O | S | 4-Cl | B 1 |
| $CH_3$ | $CH_3$ | C=O | S | 2-Cl, 4-Cl | B 1 |
| $CH_3$ | $CH_3$ | C=O | S | 2-Cl, 4-$CH_3$ | B 1 |
| $CH_3$ | $CH_3$ | C=O | S | 2-F, 4-$CH_3$ | B 1 |
| $CH_3$ | $CH_3$ | $CH_2$ | S | 4-Cl | B 2 |
| $CH_3$ | $CH_3$ | $CH_2$ | S | 2-Cl, 4-Cl | B 2 |
| $CH_3$ | $CH_3$ | $CH_2$ | S | 2-Cl, 4-$CH_3$ | B 2 |
| $CH_3$ | $CH_3$ | $CH_2$ | S | 2-F, 4-$CH_3$ | B 2 |
| $CH_3$ | $CH_3$ | CH(OH) | S | 4-Cl | B 2 |
| $CH_3$ | $CH_3$ | CH(OH) | S | 2-Cl, 4-Cl | B 2 |
| $CH_3$ | $CH_3$ | CH(OH) | S | 2-Cl, 4-$CH_3$ | B 2 |
| $CH_3$ | $CH_3$ | CH(OH) | S | 3-Cl, 4-$CH_3$ | B 2 |
| $CH_3$ | $CH_3$ | CH(OH) | S | 2-F, 4-$CH_3$ | B 2 |
| $CH_3$ | $CH_3$ | CH(OH) | S | 3-F, 4-$CH_3$ | B 2 |
| $CH_3$ | $CH_3$ | $CH(OCH_3)$ | S | 4-Cl | B 2 |

| $R^1$ | $R^2$ | X | Y | $W_n$ | B |
|---|---|---|---|---|---|
| $CH_3$ | $CH_3$ | $CH(OCH_3)$ | S | 2-Cl, 4-Cl | B 2 |
| $CH_3$ | $CH_3$ | $CH(OCOCH_3)$ | S | 4-Cl | B 2 |
| $CH_3$ | $CH_3$ | $CH(OCOCH_3)$ | S | 2-Cl, 4-Cl | B 2 |
| $CH_3$ | $CH_3$ | $CH(OCOCH_3)$ | S | 2-Cl, 4-$CH_3$ | B 2 |
| $CH_3$ | $CH_3$ | $CH(OCOCH_3)$ | S | 2-F, 4-$CH_3$ | B 2 |
| $CH_3$ | $CH_3$ | CH(F) | S | 4-Cl | B 2 |
| $CH_3$ | $CH_3$ | CH(F) | S | 2-Cl, 4-Cl | B 2 |
| $CH_3$ | $CH_3$ | CH(Cl) | S | 4-Cl | B 2 |
| $CH_3$ | $CH_3$ | CH(Cl) | S | 2-Cl, 4-Cl | B 2 |
| $CH_3$ | $CH_3$ | C=O | S | 4-Cl | B 2 |
| $CH_3$ | $CH_3$ | C=O | S | 2-Cl, 4-Cl | B 2 |
| $CH_3$ | $CH_3$ | C=O | S | 2-Cl, 4-$CH_3$ | B 2 |
| $CH_3$ | $CH_3$ | C=O | S | 2-F, 4-$CH_3$ | B 2 |
| $CH_3$ | $CH_3$ | $C(CH_3)(OH)$ | S | 4-Cl | B 2 |
| $CH_3$ | $CH_3$ | $C(CH_3)(OH)$ | S | 2-Cl, 4-Cl | B 2 |
| $CH_3$ | $CH_3$ | $C(CH_3)(OCOCH_3)$ | S | 4-Cl | B 2 |
| $CH_3$ | $CH_3$ | $C(CH_3)(OCOCH_3)$ | S | 2-Cl, 4-Cl | B 2 |
| $CH_3$ | $CH_3$ | $C(CH_3)(F)$ | S | 4-Cl | B 2 |
| $CH_3$ | $CH_3$ | $C(CH_3)(F)$ | S | 2-Cl, 4-Cl | B 2 |
| $CH_3$ | $CH_3$ | $C(C_2H_5)(OH)$ | S | 4-Cl | B 2 |

| $R^1$ | $R^2$ | X | Y | $W_n$ | B |
|---|---|---|---|---|---|
| $CH_3$ | $CH_3$ | $C(C_2H_5)(OH)$ | S | 2-Cl, 4-Cl | B 2 |
| $CH_3$ | $CH_3$ | $C(i-C_3H_7)(OH)$ | S | 4-Cl | B 2 |
| $CH_3$ | $CH_3$ | $C(i-C_3H_7)(OH)$ | S | 2-Cl, 4-Cl | B 2 |
| $CH_3$ | $CH_3$ | $C(i-C_3H_7)(OH)$ | S | 2-Cl, 4-$CH_3$ | B 2 |
| $CH_3$ | $CH_3$ | $C(i-C_3H_7)(OH)$ | S | 2-F, 4-$CH_3$ | B 2 |
| $CH_3$ | $CH_3$ | $CH(CH_3)$ | S | 4-Cl | B 2 |
| $CH_3$ | $CH_3$ | $CH(CH_3)$ | S | 2-Cl, 4-Cl | B 2 |
| $CH_3$ | $CH_3$ | $CH(CH_3)$ | S | 2-Cl, 4-$CH_3$ | B 2 |
| $CH_3$ | $CH_3$ | $CH(CH_3)$ | S | 2-F, 4-$CH_3$ | B 2 |
| Cl | $CH_3$ | $CH_2$ | S | 4-Cl | B 2 |
| Cl | $CH_3$ | $CH_2$ | S | 2-Cl, 4-Cl | B 2 |
| Cl | $CH_3$ | $CH_2$ | S | 2-Cl, 4-$CH_3$ | B 2 |
| Cl | $CH_3$ | $CH_2$ | S | 2-F, 4-$CH_3$ | B 2 |
| $CH_3O$ | $CH_3$ | $CH_2$ | S | 4-Cl | B 2 |
| $CH_3O$ | $CH_3$ | $CH_2$ | S | 2-Cl, 4-Cl | B 2 |
| $CH_3O$ | $CH_3$ | $CH_2$ | S | 2-Cl, 4-$CH_3$ | B 2 |
| $CH_3O$ | $CH_3$ | $CH_2$ | S | 2-F, 4-$CH_3$ | B 2 |

où B1 à B18 sont comme définis ci-après :

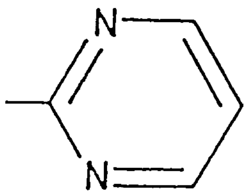
B1 ,

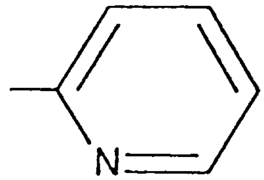
B2 ,

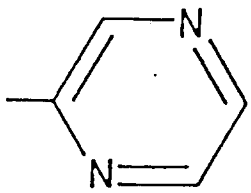
B3 ,

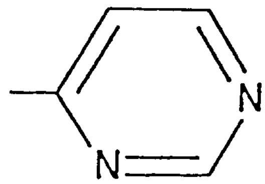
B4 ,

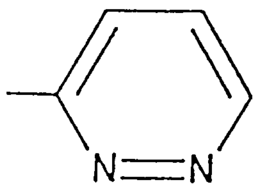
B5 ,

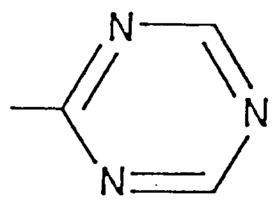
B6 ,

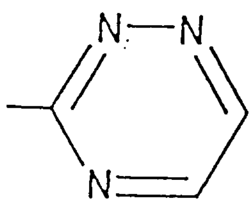
B7 ,

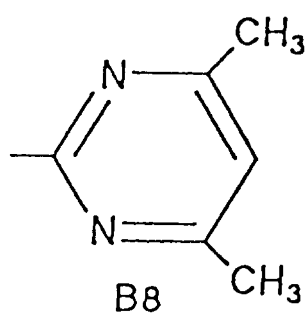
B8 ,

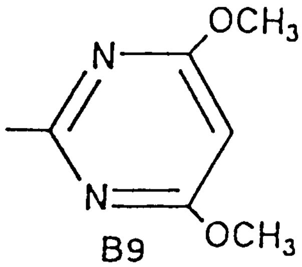
B9 ,

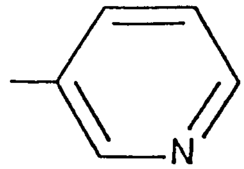
B10 ,

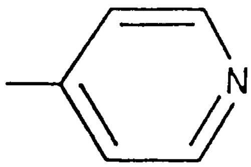
B11 ,

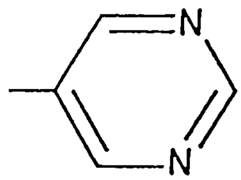
B12

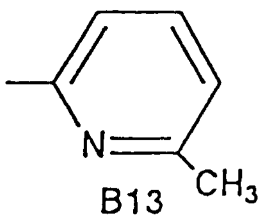
B13 ,

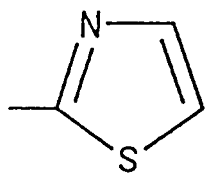
B14 ,

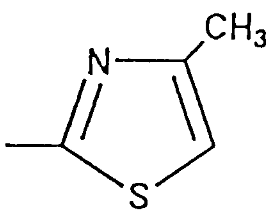
B15 ,

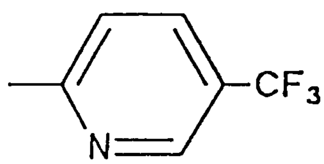
B16 ,

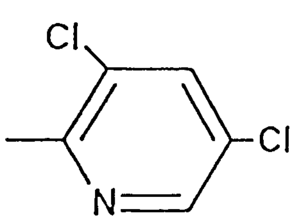
B17 ,

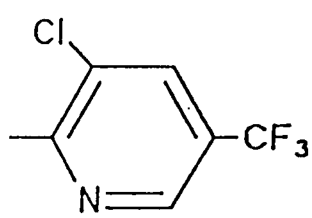
B18 ,

ou bien où les dérivés du pyrazole substitué sont définis par la formule [1b]

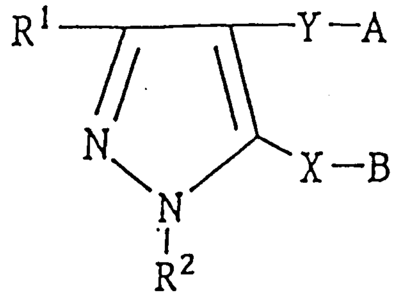

[1b]

ayant les définitions suivantes des substituants

| $R^1$ | $R^2$ | X | Y | A | B |
|---|---|---|---|---|---|
| $CH_3$ | $CH_3$ | NH | S | A 1 | B 1 |
| $CH_3$ | $CH_3$ | NH | S | A 1 | B 2 |
| $CH_3$ | $CH_3$ | NH | S | A 2 | B 1 |
| $CH_3$ | $CH_3$ | NH | S | A 2 | B 2 |
| $CH_3$ | $CH_3$ | NH | S | A 3 | B 1 |
| $CH_3$ | $CH_3$ | NH | S | A 3 | B 2 |
| $CH_3$ | $CH_3$ | NH | S | A 4 | B 1 |
| $CH_3$ | $CH_3$ | NH | S | A 4 | B 2 |
| $CH_3$ | $CH_3$ | NH | S | A 5 | B 1 |
| $CH_3$ | $CH_3$ | NH | S | A 5 | B 2 |
| $CH_3$ | $CH_3$ | NH | S | A 6 | B 1 |
| $CH_3$ | $CH_3$ | NH | S | A 6 | B 2 |

| $R^1$ | $R^2$ | X | Y | A | B |
|---|---|---|---|---|---|
| $CH_3$ | $CH_3$ | NH | S | A 7 | B 1 |
| $CH_3$ | $CH_3$ | NH | S | A 7 | B 2 |
| $CH_3$ | $CH_3$ | NH | S | A 8 | B 1 |
| $CH_3$ | $CH_3$ | NH | S | A 8 | B 2 |
| $CH_3$ | $CH_3$ | NH | S | A 9 | B 1 |
| $CH_3$ | $CH_3$ | NH | S | A 9 | B 2 |
| $CH_3$ | $CH_3$ | NH | S | A 10 | B 1 |
| $CH_3$ | $CH_3$ | NH | S | A 10 | B 2 |
| $CH_3$ | $CH_3$ | NH | S | A 11 | B 1 |
| $CH_3$ | $CH_3$ | NH | S | A 11 | B 2 |

où A1 à A11 sont comme définis ci-après

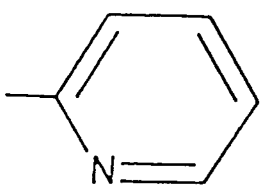
A1
,
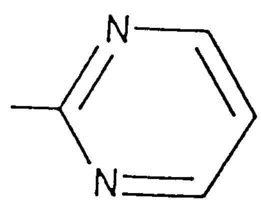
A2
,

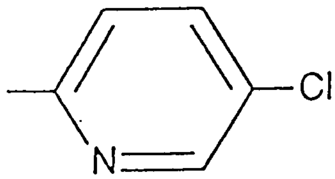

A3 ,

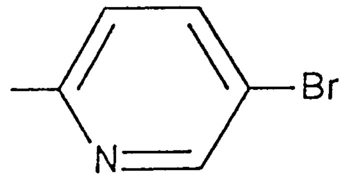

A4 ,

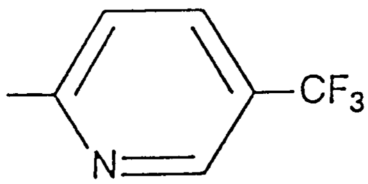

A5 ,

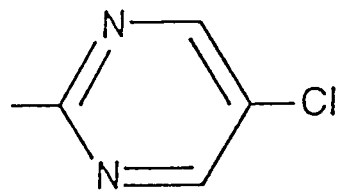

A6 ,

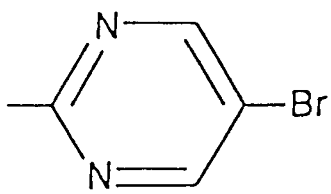

A7 ,

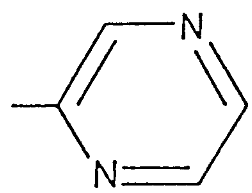

A8 ,

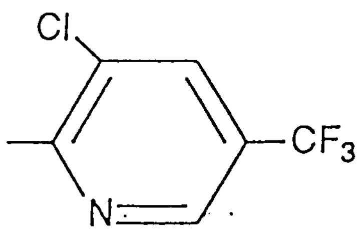

A9 ,

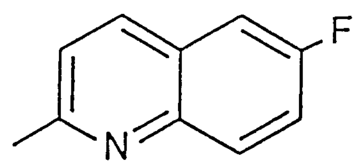

A10 ,

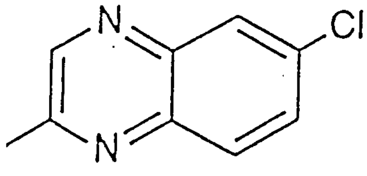

A11 ,

et B1 et B2 sont comme définis précédemment.

**2.** Dérivés du pyrazole substitués selon la revendication 1 qui sont des composés de formule [1a].

**3.** Dérivés du pyrazole substitués selon la revendication 1, selon laquelle X est -N($R^3$)-.

**4.** Dérivés du pyrazole substitués selon la revendication 2, selon laquelle Y est -S-.

**5.** Dérivés du pyrazole substitués selon la revendication 1 qui sont des composés de formule générale [1a], dans laquelle $R^1$ et $R^2$ désignent chacun un groupe alkyle, X est -N($R^3$)-, Y est -S- et a est B1, B2, B4, B10, B11 ou B12.

**6.** Un fongicide utilisé en agriculture et en horticulture contenant un ou plusieurs pyrazoles substitués selon la revendication 1, comme ingrédient actif.

**7.** Utilisation d'un dérivé du pyrazole substitué selon la revendication 1 comme fongicide.

**Revendications pour l'Etat contractant suivant : ES**

**1.** Une méthode de préparation des dérivés du pyrazole substitués de formule générale [1] :

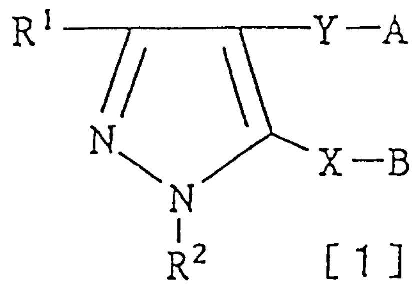

[1]

dans laquelle $R^1$ représente un atome d'hydrogène, un atome d'halogène, un groupe alkyle, un groupe alcoxy, un groupe alkylthio ou un groupe haloalkyle ;
$R^2$ représente un atome d'hydrogène, un groupe alkyle, un groupe haloalkyle, un groupe phénylalkyle, -$COR^6$ ou -$SO_2R^7$ ;
X représente -S-, -SO-, -$SO_2$-, -N($R^3$)-, -CO- ou -C($R^4$)($R^5$)-;
$R^3$ représente un atome d'hydrogène, un groupe alkyle, un groupe haloalkyle, un groupe alcényle, un groupe alcynyle, un groupe alcoxyalkyle, un groupe cyanoalkyle, un groupe alkylcarbonylalkyle, un groupe alcoxycarbonylalkyle, un groupe nitroso, un groupe amino, un groupe phénylalkyle, -$COR^6$ ou -$SO_2R^7$ ;
$R^4$ et $R^5$ représentent indépendamment un atome d'hydrogène, un atome d'halogène, un groupe alkyle, un groupe haloalkyle, un groupe alcényle, un groupe alcynyle ou -$OR^8$;
$R^8$ représente un atome d'hydrogène, un groupe alkyle, un groupe haloalkyle, un groupe alcényle, un groupe alcynyle, un groupe alcoxyalkyle, un groupe cyanoalkyle, un groupe alkylcarbonylalkyle, un groupe alcoxycarbonylalkyle, un groupe phénylalkyle, -$COR^6$ ou -$SO_2R^7$ ;
$R^6$ représente un atome d'hydrogène, un groupe alkyle, un groupe haloalkyle, un groupe phényle, un groupe phénylalkyle, un groupe alcoxy ou

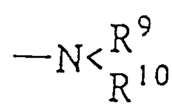 ;

$R^7$ représente un groupe alkyle, un groupe haloalkyle, un groupe phényle ou

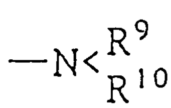 ;

$R^9$ et $R^{10}$ représentent indépendamment un atome d'hydrogène, un groupe alkyle ou un groupe phényle ;
Y représente un atome d'oxygène, -S-, -SO- ou $-SO_2-$ ;
A représente un groupe phényle ;
B représente

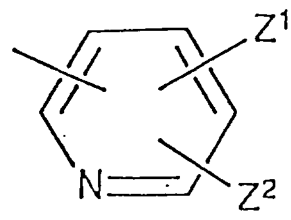
,
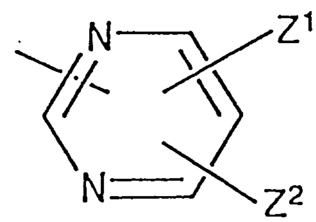
,
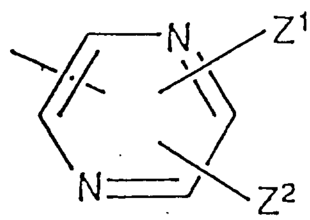
,
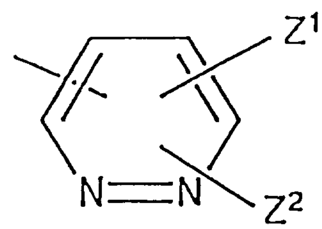
,
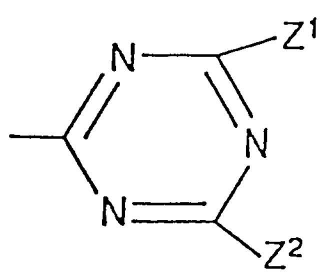
,
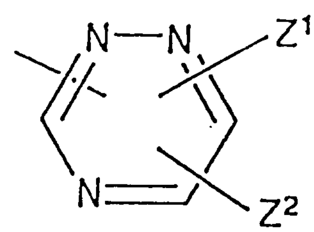

ou

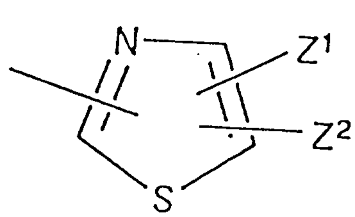
;

$Z^1$ et $Z^2$ représentent indépendamment un atome d'hydrogène, un atome d'halogène, un groupe alkyle, un groupe alcoxy ou un groupe haloalkyle,
et les dérivés du pyrazole substitués de formule générale [1] incluent les composés de formule [1a]

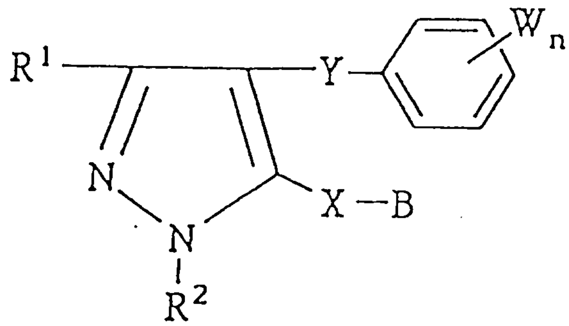

[1a]

ayant les significations des substituants suivantes

| $R^1$ | $R^2$ | X | Y | $W_n$ | B |
|---|---|---|---|---|---|
| H | $CH_3$ | S | S | 4-Cl | B 1 |
| $CH_3$ | $CH_3$ | S | S | 4-Cl | B 1 |
| $CF_3$ | $CH_3$ | S | S | 4-Cl | B 1 |
| $CH_3$ | $CH_3$ | S | S | 4-$CH_3$ | B 1 |
| $CF_3$ | $CH_3$ | S | S | 4-$CH_3$ | B 1 |
| $CH_3$ | H | S | S | 4-Cl | B 1 |
| $CH_3$ | H | S | S | 4-$CH_3$ | B 1 |
| $CH_3$ | $CF_3$ | S | S | 4-Cl | B 1 |
| $CH_3$ | $CF_3$ | S | S | 4-$CH_3$ | B 1 |
| $CH_3$ | $CH_3$ | S | S | 4-$CF_3$ | B 1 |
| $CH_3$ | $CH_3$ | S | S | 4-Br | B 1 |
| $CH_3$ | $CH_3$ | S | S | 4-$NO_2$ | B 1 |
| $CH_3$ | $CH_3$ | S | S | 4-$OCH_3$ | B 1 |
| $CH_3$ | $CH_3$ | S | S | 4-$C_2H_5$ | B 1 |
| $CH_3$ | $CH_3$ | S | S | 2-Cl | B 1 |
| $CH_3$ | $CH_3$ | S | S | 2-Cl, 4-Cl | B 1 |
| $CH_3$ | $CH_3$ | S | S | 2-Cl, 4-$CH_3$ | B 1 |
| $CH_3$ | $CH_3$ | S | S | 4-Cl | B 2 |
| $CF_3$ | $CH_3$ | S | S | 4-Cl | B 2 |

| $R^1$ | $R^2$ | X | Y | $W_n$ | B |
|---|---|---|---|---|---|
| $CH_3$ | $CH_3$ | S | S | 4-$CH_3$ | B 2 |
| $CF_3$ | $CH_3$ | S | S | 4-$CH_3$ | B 2 |
| $CH_3$ | $CH_3$ | S | S | 2-Cl, 4-Cl | B 2 |
| $CH_3$ | $CH_3$ | S | S | 2-Cl, 4-$CH_3$ | B 2 |
| $CH_3$ | $CH_3$ | S | O | 4-Cl | B 2 |
| $CH_3$ | $CH_3$ | S | O | 4-$CH_3$ | B 2 |
| $CF_3$ | $CH_3$ | S | O | 4-$CH_3$ | B 2 |
| $CH_3$ | $CH_3$ | S | O | 3-Cl, 4-$CH_3$ | B 2 |
| $C_2H_5$ | $CH_3$ | S | S | H | B 1 |
| $C_2H_5$ | $CH_3$ | S | S | 4-Cl | B 1 |
| $C_2H_5$ | $CH_3$ | S | S | 4-$CH_3$ | B 1 |
| $C_2H_5$ | $CH_3$ | S | S | 4-$OCH_3$ | B 1 |
| $C_2H_5$ | $CH_3$ | S | S | 3-$CF_3$ | B 1 |
| $C_2H_5$ | $CH_3$ | S | S | 2-Cl, 4-Cl | B 1 |
| $C_2H_5$ | $CH_3$ | S | S | 3-Cl, 4-Cl | B 1 |
| $C_2H_5$ | $CH_3$ | S | S | 2-Cl, 4-$CH_3$ | B 1 |
| i-$C_3H_7$ | $CH_3$ | S | S | 4-Cl | B 1 |
| i-$C_3H_7$ | $CH_3$ | S | S | 4-$CH_3$ | B 1 |
| i-$C_3H_7$ | $CH_3$ | S | S | 4-$OCH_3$ | B 1 |
| t-$C_4H_9$ | $CH_3$ | S | S | 4-Cl | B 1 |

| $R^1$ | $R^2$ | X | Y | $W_n$ | B |
|---|---|---|---|---|---|
| t-$C_4H_9$ | $CH_3$ | S | S | 4-$CH_3$ | B 1 |
| t-$C_4H_9$ | $CH_3$ | S | S | 4-$OCH_3$ | B 1 |
| $CH_3$ | $C_2H_5$ | S | S | 4-Cl | B 1 |
| $CH_3$ | $C_3H_7$ | S | S | 4-Cl | B 1 |
| $CH_3$ | $CH_3$ | NH | S | H | B 1 |
| $CF_3$ | $CH_3$ | NH | S | H | B 1 |
| $CH_3$ | $CH_3$ | NH | S | 4-Cl | B 1 |
| $CF_3$ | $CH_3$ | NH | S | 4-Cl | B 1 |
| H | $CH_3$ | NH | S | 4-Cl | B 1 |
| $CH_3$ | $CH_3$ | NH | S | 4-$CH_3$ | B 1 |
| $CF_3$ | $CH_3$ | NH | S | 4-$CH_3$ | B 1 |
| H | $CH_3$ | NH | S | 4-$CH_3$ | B 1 |
| $CH_3$ | $CH_3$ | NH | S | 4-F | B 1 |
| $CH_3$ | $CH_3$ | NH | S | 4-Br | B 1 |
| $CH_3$ | $CH_3$ | NH | S | 4-I | B 1 |
| $CH_3$ | $CH_3$ | NH | S | 2-F | B 1 |
| $CH_3$ | $CH_3$ | NH | S | 2-Cl | B 1 |
| $CH_3$ | $CH_3$ | NH | S | 2-Br | B 1 |
| $CH_3$ | $CH_3$ | NH | S | 2-I | B 1 |
| $CH_3$ | $CH_3$ | NH | S | 3-F | B 1 |

| $R^1$ | $R^2$ | X | Y | $W_n$ | B |
|---|---|---|---|---|---|
| $CH_3$ | $CH_3$ | NH | S | 3-Cl | B 1 |
| $CH_3$ | $CH_3$ | NH | S | 3-Br | B 1 |
| $CH_3$ | $CH_3$ | NH | S | 3-I | B 1 |
| $CH_3$ | $CH_3$ | NH | S | 2-$OCH_3$ | B 1 |
| $CH_3$ | $CH_3$ | NH | S | 3-$OCH_3$ | B 1 |
| $CH_3$ | $CH_3$ | NH | S | 4-$OCH_3$ | B 1 |
| $CH_3$ | $CH_3$ | NH | S | 2-$CF_3$ | B 1 |
| $CH_3$ | $CH_3$ | NH | S | 3-$CF_3$ | B 1 |
| $CH_3$ | $CH_3$ | NH | S | 4-$CF_3$ | B 1 |
| $CH_3$ | $CH_3$ | NH | S | 4-$C_2H_5$ | B 1 |
| $CH_3$ | $CH_3$ | NH | S | 4-$C_3H_7$ | B 1 |
| $CH_3$ | $CH_3$ | NH | S | 4-$C_4H_9$ | B 1 |
| $CH_3$ | $CH_3$ | NH | S | 4-i-$C_3H_7$ | B 1 |
| $CH_3$ | $CH_3$ | NH | S | 4-t-$C_4H_9$ | B 1 |
| $CH_3$ | $CH_3$ | NH | S | 2-$OCF_3$ | B 1 |
| $CH_3$ | $CH_3$ | NH | S | 3-$OCF_3$ | B 1 |
| $CH_3$ | $CH_3$ | NH | S | 4-$OCF_3$ | B 1 |
| $CH_3$ | $CH_3$ | NH | S | 3-$NO_2$ | B 1 |
| $CH_3$ | $CH_3$ | NH | S | 4-$NO_2$ | B 1 |
| $CH_3$ | $CH_3$ | NH | S | 3-$NH_2$ | B 1 |

| $R^1$ | $R^2$ | X | Y | $W_n$ | B |
|---|---|---|---|---|---|
| $CH_3$ | $CH_3$ | NH | S | 4-$NH_2$ | B 1 |
| $CH_3$ | $CH_3$ | NH | S | 4-$NHCOCH_3$ | B 1 |
| $CH_3$ | $CH_3$ | NH | S | 4-$NHSO_2CH_3$ | B 1 |
| $CH_3$ | $CH_3$ | NH | S | 4-$NHCOCF_3$ | B 1 |
| $CH_3$ | $CH_3$ | NH | S | 4-$NHSO_2CF_3$ | B 1 |
| $CH_3$ | $CH_3$ | NH | S | 4-Ph | B 1 |
| $CH_3$ | $CH_3$ | NH | S | 4-OPh | B 1 |
| $CH_3$ | $CH_3$ | NH | S | 4-$OCF_2CF_2H$ | B 1 |
| $CH_3$ | $CH_3$ | NH | S | 4-$COCH_3$ | B 1 |
| $CH_3$ | $CH_3$ | NH | S | 4-NHCOPh | B 1 |
| $CH_3$ | $CH_3$ | NH | S | 4-$NHCOOCH_3$ | B 1 |
| $CH_3$ | $CH_3$ | NH | S | 4-$NHCON(CH_3)_2$ | B 1 |
| $CH_3$ | $CH_3$ | NH | S | 2-Cl, 3-Cl | B 1 |
| $CH_3$ | $CH_3$ | NH | S | 2-Cl, 4-Cl | B 1 |
| $CH_3$ | $CH_3$ | NH | S | 2-Cl, 5-Cl | B 1 |
| $CH_3$ | $CH_3$ | NH | S | 2-Cl, 6-Cl | B 1 |
| $CH_3$ | $CH_3$ | NH | S | 3-Cl, 4-Cl | B 1 |
| $CH_3$ | $CH_3$ | NH | S | 3-Cl, 5-Cl | B 1 |
| $CH_3$ | $CH_3$ | NH | S | 2-$CH_3$, 3-$CH_3$ | B 1 |
| $CH_3$ | $CH_3$ | NH | S | 2-$CH_3$, 4-$CH_3$ | B 1 |

| $R^1$ | $R^2$ | X | Y | $W_n$ | B |
|---|---|---|---|---|---|
| $CH_3$ | $CH_3$ | NH | S | 2-$CH_3$, 5-$CH_3$ | B 1 |
| $CH_3$ | $CH_3$ | NH | S | 2-$CH_3$, 6-$CH_3$ | B 1 |
| $CH_3$ | $CH_3$ | NH | S | 3-$CH_3$, 4-$CH_3$ | B 1 |
| $CH_3$ | $CH_3$ | NH | S | 3-$CH_3$, 5-$CH_3$ | B 1 |
| $CH_3$ | $CH_3$ | NH | S | 2-Cl, 3-$CH_3$ | B 1 |
| $CH_3$ | $CH_3$ | NH | S | 2-Cl, 4-$CH_3$ | B 1 |
| $CH_3$ | $CH_3$ | NH | S | 2-Cl, 5-$CH_3$ | B 1 |
| $CH_3$ | $CH_3$ | NH | S | 3-Cl, 4-$CH_3$ | B 1 |
| $CH_3$ | $CH_3$ | NH | S | 3-Cl, 5-$CH_3$ | B 1 |
| $CH_3$ | $CH_3$ | NH | S | 2-$CH_3$, 3-Cl | B 1 |
| $CH_3$ | $CH_3$ | NH | S | 2-$CH_3$, 4-Cl | B 1 |
| $CH_3$ | $CH_3$ | NH | S | 2-$CH_3$, 5-Cl | B 1 |
| $CH_3$ | $CH_3$ | NH | S | 2-F, 3-F | B 1 |
| $CH_3$ | $CH_3$ | NH | S | 2-F, 4-F | B 1 |
| $CH_3$ | $CH_3$ | NH | S | 2-F, 5-F | B 1 |
| $CH_3$ | $CH_3$ | NH | S | 2-F, 6-F | B 1 |
| $CH_3$ | $CH_3$ | NH | S | 2-F, 3-Cl | B 1 |
| $CH_3$ | $CH_3$ | NH | S | 2-F, 4-Cl | B 1 |
| $CH_3$ | $CH_3$ | NH | S | 2-F, 5-Cl | B 1 |
| $CH_3$ | $CH_3$ | NH | S | 2-F, 6-Cl | B 1 |

| $R^1$ | $R^2$ | X | Y | $W_n$ | B |
|---|---|---|---|---|---|
| $CH_3$ | $CH_3$ | NH | S | 2-F, 3-$CH_3$ | B 1 |
| $CH_3$ | $CH_3$ | NH | S | 2-F, 4-$CH_3$ | B 1 |
| $CH_3$ | $CH_3$ | NH | S | 2-F, 3-Br | B 1 |
| $CH_3$ | $CH_3$ | NH | S | 2-F, 4-Br | B 1 |
| $CH_3$ | $CH_3$ | NH | S | 3-F, 4-Cl | B 1 |
| $CH_3$ | $CH_3$ | NH | S | 3-F, 5-Cl | B 1 |
| $CH_3$ | $CH_3$ | NH | S | 3-F, 4-$CH_3$ | B 1 |
| $CH_3$ | $CH_3$ | NH | S | 3-F, 5-$CH_3$ | B 1 |
| $CH_3$ | $CH_3$ | NH | S | 2-Br, 4-Cl | B 1 |
| $CH_3$ | $CH_3$ | NH | S | 2-Br, 4-Br | B 1 |
| $CH_3$ | $CH_3$ | NH | S | 2-Br, 4-$CH_3$ | B 1 |
| $CH_3$ | $CH_3$ | NH | S | 3-Br, 4-Cl | B 1 |
| $CH_3$ | $CH_3$ | NH | S | 3-Br, 4-Br | B 1 |
| $CH_3$ | $CH_3$ | NH | S | 3-Br, 4-$CH_3$ | B 1 |
| $CH_3$ | $CH_3$ | NH | S | 2-Cl, 4-Br | B 1 |
| $CH_3$ | $CH_3$ | NH | S | 3-Cl, 4-Br | B 1 |
| $CH_3$ | $CH_3$ | NH | S | 2-Cl, 4-I | B 1 |
| $CH_3$ | $CH_3$ | NH | S | 3-Cl, 4-I | B 1 |
| $CH_3$ | $CH_3$ | NH | S | 3-F, 4-Br | B 1 |
| $CH_3$ | $CH_3$ | NH | S | 3, 4-$OCH_2O$- | B 1 |

| $R^1$ | $R^2$ | X | Y | $W_n$ | B |
|---|---|---|---|---|---|
| $CH_3$ | $CH_3$ | NH | S | 2-$CF_3$, 3-Cl | B 1 |
| $CH_3$ | $CH_3$ | NH | S | 2-$CF_3$, 4-Cl | B 1 |
| $CH_3$ | $CH_3$ | NH | S | 2-$CF_3$, 3-Br | B 1 |
| $CH_3$ | $CH_3$ | NH | S | 2-$CF_3$, 4-Br | B 1 |
| $CH_3$ | $CH_3$ | NH | S | 2-F, 4-$NO_2$ | B 1 |
| $CH_3$ | $CH_3$ | NH | S | 3-F, 4-$NO_2$ | B 1 |
| $CH_3$ | $CH_3$ | NH | S | 2-Cl, 4-$NO_2$ | B 1 |
| $CH_3$ | $CH_3$ | NH | S | 3-Cl, 4-$NO_2$ | B 1 |
| $CH_3$ | $CH_3$ | NH | S | 2-F, 4-$OCH_3$ | B 1 |
| $CH_3$ | $CH_3$ | NH | S | 3-F, 4-$OCH_3$ | B 1 |
| $CH_3$ | $CH_3$ | NH | S | 2-Cl, 4-$OCH_3$ | B 1 |
| $CH_3$ | $CH_3$ | NH | S | 3-Cl, 4-$OCH_3$ | B 1 |
| $CH_3$ | $CH_3$ | NH | S | 2-$OCH_3$, 4-$OCH_3$ | B 1 |
| $CH_3$ | $CH_3$ | NH | S | 3-$OCH_3$, 4-$OCH_3$ | B 1 |
| $CH_3$ | $CH_3$ | NH | S | 2-Cl, 3-Cl, 4-Cl | B 1 |
| $CH_3$ | $CH_3$ | NH | S | 2-Cl, 4-Cl, 5-Cl | B 1 |
| $CH_3$ | $CH_3$ | NH | S | 2-Cl, 4-Cl, 6-Cl | B 1 |
| $CH_3$ | $CH_3$ | NH | S | 3-Cl, 4-Cl, 5-Cl | B 1 |
| $CH_3$ | $CH_3$ | NH | S | 2-Cl, 3-Cl, 4-$CH_3$ | B 1 |
| $CH_3$ | $CH_3$ | NH | S | 2-Cl, 3-Cl, 4-Br | B 1 |

| $R^1$ | $R^2$ | X | Y | $W_n$ | B |
|---|---|---|---|---|---|
| $CH_3$ | $CH_3$ | NH | S | 2-Cl, 3-Cl, 4-I | B 1 |
| $CH_3$ | $CH_3$ | NH | S | 2-Cl, 4-Br, 6-Cl | B 1 |
| $CH_3$ | $CH_3$ | NH | S | 2-Cl, 4-$CH_3$, 6-Cl | B 1 |
| $CH_3$ | $CH_3$ | NH | S | 2-Br, 4-Cl, 6-Cl | B 1 |
| $CH_3$ | $CH_3$ | NCHO | S | 4-Cl | B 1 |
| $CH_3$ | $CH_3$ | $NCOCH_3$ | S | 4-Cl | B 1 |
| $CH_3$ | $CH_3$ | $NCOOCH_3$ | S | 4-Cl | B 1 |
| $CH_3$ | $CH_3$ | $NCON(CH_3)_2$ | S | 4-Cl | B 1 |
| $CH_3$ | $CH_3$ | NCONHPh | S | 4-Cl | B 1 |
| $CH_3$ | $CH_3$ | $NCH_3$ | S | 4-Cl | B 1 |
| $CH_3$ | $CH_3$ | $NC_2H_5$ | S | 4-Cl | B 1 |
| $CH_3$ | $CH_3$ | $NC_3H_7$ | S | 4-Cl | B 1 |
| $CH_3$ | $CH_3$ | $NCH_2OCH_3$ | S | 4-Cl | B 1 |
| $CH_3$ | $CH_3$ | $NCH_2CH_2OCH_3$ | S | 4-Cl | B 1 |
| $CH_3$ | $CH_3$ | $NCH_2CH=CH_2$ | S | 4-Cl | B 1 |
| $CH_3$ | $CH_3$ | $NCH_2C\equiv CH$ | S | 4-Cl | B 1 |
| $CH_3$ | $CH_3$ | $NCH_2COCH_3$ | S | 4-Cl | B 1 |
| $CH_3$ | $CH_3$ | $NCH_2COOCH_3$ | S | 4-Cl | B 1 |
| $CH_3$ | $CH_3$ | $NCH_2COOC_2H_5$ | S | 4-Cl | B 1 |
| $CH_3$ | $CH_3$ | $NCH_2CN$ | S | 4-Cl | B 1 |

| $R^1$ | $R^2$ | X | Y | $W_n$ | B |
|---|---|---|---|---|---|
| $CH_3$ | $CH_3$ | $NCH_2Ph$ | S | 4-Cl | B 1 |
| $CH_3$ | $CH_3$ | $NCH_2C_6H_4$-4-Cl | S | 4-Cl | B 1 |
| $CH_3$ | $CH_3$ | $NCH_2C_6H_4$-4-$CH_3$ | S | 4-Cl | B 1 |
| $CH_3$ | $CH_3$ | $NCH_2CH_2Ph$ | S | 4-Cl | B 1 |
| $CH_3$ | $CH_3$ | $NSO_2CH_3$ | S | 4-Cl | B 1 |
| $CH_3$ | $CH_3$ | $NSO_2N(CH_3)_2$ | S | 4-Cl | B 1 |
| $CH_3$ | $CH_3$ | NCHO | S | 4-Cl | B 2 |
| $CH_3$ | $CH_3$ | $NCH_3$ | S | 4-Cl | B 2 |
| $CH_3$ | H | NH | S | 4-Cl | B 1 |
| $CH_3$ | H | NH | S | 2-Cl, 4-Cl | B 1 |
| $CH_3$ | H | NH | S | 3-Cl, 4-$CH_3$ | B 1 |
| $CH_3$ | $C_2H_5$ | NH | S | 4-Cl | B 1 |
| $CH_3$ | $C_2H_5$ | NH | S | 4-Br | B 1 |
| $CH_3$ | $C_2H_5$ | NH | S | 2-Cl, 4-Cl | B 1 |
| $CH_3$ | $C_2H_5$ | NH | S | 2-Cl, 4-$CH_3$ | B 1 |
| $CH_3$ | i-$C_3H_7$ | NH | S | 4-Cl | B 1 |
| $CH_3$ | i-$C_3H_7$ | NH | S | 4-Br | B 1 |
| $CH_3$ | i-$C_3H_7$ | NH | S | 2-Cl, 4-Cl | B 1 |
| $CH_3$ | i-$C_3H_7$ | NH | S | 2-Cl, 4-$CH_3$ | B 1 |
| $CH_3$ | t-$C_4H_9$ | NH | S | 4-Cl | B 1 |

| $R^1$ | $R^2$ | X | Y | $W_n$ | B |
|---|---|---|---|---|---|
| $CH_3$ | t-$C_4H_9$ | NH | S | 2-Cl, 4-Cl | B 1 |
| $CH_3$ | t-$C_4H_9$ | NH | S | 3-Cl, 4-$CH_3$ | B 1 |
| $CH_3$ | $CF_3$ | NH | S | 4-Cl | B 1 |
| $CH_3$ | $CF_3$ | NH | S | 4-Br | B 1 |
| $CH_3$ | $CF_3$ | NH | S | 2-Cl, 4-Cl | B 1 |
| $CH_3$ | $CF_3$ | NH | S | 3-Cl, 4-$CH_3$ | B 1 |
| $CH_3$ | H | NH | S | 4-Cl | B 2 |
| $CH_3$ | H | NH | S | 2-Cl, 4-Cl | B 2 |
| $CH_3$ | $C_2H_5$ | NH | S | 4-Cl | B 2 |
| $CH_3$ | i-$C_3H_7$ | NH | S | 4-Cl | B 2 |
| $CH_3$ | t-$C_4H_9$ | NH | S | 4-Cl | B 2 |
| $CH_3$ | $CF_3$ | NH | S | 2-Cl, 4-Cl | B 2 |
| $CH_3$ | $CH_2Ph$ | NH | S | 4-Cl | B 1 |
| $CH_3$ | $CH_2Ph$ | NH | S | 2-Cl, 4-Cl | B 1 |
| $CH_3$ | $CH_2Ph$ | NCHO | S | 2-Cl, 4-Cl | B 1 |
| $CH_3$ | CHO | NH | S | 4-Cl | B 1 |
| $CH_3$ | CHO | NCHO | S | 4-Cl | B 1 |
| $CH_3$ | CHO | NH | S | 2-Cl, 4-Cl | B 1 |
| $CH_3$ | CHO | NCHO | S | 2-Cl, 4-Cl | B 1 |
| $CH_3$ | $COCH_3$ | NH | S | 4-Cl | B 1 |

| $R^1$ | $R^2$ | X | Y | $W_n$ | B |
|---|---|---|---|---|---|
| $CH_3$ | $COCH_3$ | NH | S | 4-Br | B 1 |
| $CH_3$ | $COCH_3$ | NH | S | 2-Cl, 4-Cl | B 1 |
| $CH_3$ | $COCH_3$ | NCHO | S | 2-Cl, 4-Cl | B 1 |
| $CH_3$ | $SO_2CH_3$ | NH | S | 4-Cl | B 1 |
| $CH_3$ | $SO_2CH_3$ | NH | S | 2-Cl, 4-Cl | B 1 |
| $CH_3$ | $SO_2CH_3$ | NH | S | 3-Cl, 4-$CH_3$ | B 1 |
| $CH_3$ | $CONHCH_3$ | NH | S | 4-Cl | B 1 |
| $CH_3$ | $CONHCH_3$ | NH | S | 4-Br | B 1 |
| $CH_3$ | $CONHCH_3$ | NH | S | 2-Cl, 4-Cl | B 1 |
| $CH_3$ | $CONHCH_3$ | NH | S | 3-Cl, 4-$CH_3$ | B 1 |
| $CH_3$ | $CH_2Ph$ | NH | S | 4-Cl | B 2 |
| $CH_3$ | $CH_2Ph$ | NH | S | 2-Cl, 4-Cl | B 2 |
| $CH_3$ | $COCH_3$ | NH | S | 4-Cl | B 2 |
| $CH_3$ | $CONHCH_3$ | NH | S | 4-Cl | B 2 |
| $CH_3$ | $SO_2CH_3$ | NH | S | 4-Cl | B 2 |
| $CH_3$ | $SO_2CH_3$ | NH | S | 2-Cl, 4-Cl | B 2 |
| H | $CH_3$ | NH | S | 4-Cl | B 1 |
| H | $CH_3$ | NH | S | 2-Cl, 4-Cl | B 1 |
| H | $CH_3$ | NH | S | 3-Cl, 4-$CH_3$ | B 1 |
| $CF_3$ | $CH_3$ | NH | S | 4-Cl | B 1 |

| $R^1$ | $R^2$ | X | Y | $W_n$ | B |
|---|---|---|---|---|---|
| $CF_3$ | $CH_3$ | NH | S | 4-Br | B 1 |
| $CF_3$ | $CH_3$ | NH | S | 2-Cl, 4-Cl | B 1 |
| $CF_3$ | $CH_3$ | NH | S | 2-Cl, 4-$CH_3$ | B 1 |
| $C_2H_5$ | $CH_3$ | NH | S | 4-Cl | B 1 |
| $C_2H_5$ | $CH_3$ | NH | S | 4-Br | B 1 |
| $C_2H_5$ | $CH_3$ | NH | S | 2-Cl, 4-Cl | B 1 |
| $C_2H_5$ | $CH_3$ | NH | S | 2-Cl, 4-$CH_3$ | B 1 |
| i-$C_3H_7$ | $CH_3$ | NH | S | 4-Cl | B 1 |
| i-$C_3H_7$ | $CH_3$ | NH | S | 2-Cl, 4-Cl | B 1 |
| i-$C_3H_7$ | $CH_3$ | NH | S | 3-Cl, 4-$CH_3$ | B 1 |
| t-$C_4H_9$ | $CH_3$ | NH | S | 4-Cl | B 1 |
| t-$C_4H_9$ | $CH_3$ | NH | S | 4-Br | B 1 |
| t-$C_4H_9$ | $CH_3$ | NH | S | 2-Cl, 4-Cl | B 1 |
| t-$C_4H_9$ | $CH_3$ | NH | S | 3-Cl, 4-$CH_3$ | B 1 |
| H | $CH_3$ | NH | S | 4-Cl | B 2 |
| H | $CH_3$ | NH | S | 2-Cl, 4-Cl | B 2 |
| $C_2H_5$ | $CH_3$ | NH | S | 4-Cl | B 2 |
| i-$C_3H_7$ | $CH_3$ | NH | S | 4-Cl | B 2 |
| $CF_3$ | $CH_3$ | NH | S | 4-Cl | B 2 |
| $CF_3$ | $CH_3$ | NH | S | 2-Cl, 4-Cl | B 2 |

| $R^1$ | $R^2$ | X | Y | $W_n$ | B |
|---|---|---|---|---|---|
| Cl | $CH_3$ | NH | S | 4-Cl | B 1 |
| Cl | $CH_3$ | NH | S | 2-Cl, 4-Cl | B 1 |
| Cl | $CH_3$ | NH | S | 3-Cl, 4-$CH_3$ | B 1 |
| Cl | $CH_3$ | NCHO | S | 4-Cl | B 1 |
| Cl | $CH_3$ | NH | S | 4-Br | B 1 |
| Cl | $CH_3$ | NCHO | S | 2-Cl, 4-Cl | B 1 |
| Cl | $CH_3$ | NCHO | S | 2-Cl, 4-$CH_3$ | B 1 |
| $CH_3O$ | $CH_3$ | NH | S | 4-Cl | B 1 |
| $CH_3O$ | $CH_3$ | NH | S | 4-Br | B 1 |
| $CH_3O$ | $CH_3$ | NH | S | 2-Cl, 4-Cl | B 1 |
| $CH_3O$ | $CH_3$ | NH | S | 2-Cl, 4-$CH_3$ | B 1 |
| $CH_3O$ | $CH_3$ | NCHO | S | 4-Cl | B 1 |
| $CH_3O$ | $CH_3$ | NCHO | S | 2-Cl, 4-Cl | B 1 |
| $CH_3O$ | $CH_3$ | NCHO | S | 3-Cl, 4-$CH_3$ | B 1 |
| $CH_3S$ | $CH_3$ | NH | S | 4-Cl | B 1 |
| $CH_3S$ | $CH_3$ | NH | S | 4-Br | B 1 |
| $CH_3S$ | $CH_3$ | NH | S | 2-Cl, 4-Cl | B 1 |
| $CH_3S$ | $CH_3$ | NH | S | 3-Cl, 4-$CH_3$ | B 1 |
| $CH_3S$ | $CH_3$ | NCHO | S | 4-Cl | B 1 |
| $CH_3S$ | $CH_3$ | NCHO | S | 2-Cl, 4-Cl | B 1 |

| $R^1$ | $R^2$ | X | Y | $W_n$ | B |
|---|---|---|---|---|---|
| Cl | $CH_3$ | NH | S | 4-Cl | B 2 |
| Cl | $CH_3$ | NH | S | 2-Cl, 4-Cl | B 2 |
| $CH_3O$ | $CH_3$ | NH | S | 2-Cl, 4-Cl | B 2 |
| $CH_3S$ | $CH_3$ | NH | S | 2-Cl, 4-Cl | B 2 |
| $CH_3$ | $CH_3$ | SO | S | 4-Cl | B 1 |
| $CH_3$ | $CH_3$ | $SO_2$ | S | 4-Cl | B 1 |
| $CH_3$ | $CH_3$ | NH | SO | 4-Cl | B 1 |
| $CH_3$ | $CH_3$ | NH | $SO_2$ | 4-Cl | B 1 |
| $CH_3$ | $CH_3$ | NH | SO | 2-Cl, 4-Cl | B 1 |
| $CH_3$ | $CH_3$ | NH | $SO_2$ | 2-Cl, 4-Cl | B 1 |
| $CH_3$ | $CH_3$ | NH | SO | 3-Cl, 4-$CH_3$ | B 1 |
| $CH_3$ | $CH_3$ | NH | $SO_2$ | 3-Cl, 4-$CH_3$ | B 1 |
| $CH_3$ | $CH_3$ | S | O | 4-Cl | B 1 |
| $CH_3$ | $CH_3$ | SO | O | 4-Cl | B 1 |
| $CH_3$ | $CH_3$ | $SO_2$ | O | 4-Cl | B 1 |
| $CH_3$ | $CH_3$ | S | O | 2-Cl, 4-Cl | B 1 |
| $CH_3$ | $CH_3$ | SO | O | 2-Cl, 4-Cl | B 1 |
| $CH_3$ | $CH_3$ | $SO_2$ | O | 2-Cl, 4-Cl | B 1 |
| $CH_3$ | $CH_3$ | NH | O | 4-Cl | B 1 |
| $CH_3$ | $CH_3$ | NH | O | 4-$CH_3$ | B 1 |

| $R^1$ | $R^2$ | X | Y | $W_n$ | B |
|---|---|---|---|---|---|
| $CH_3$ | $CH_3$ | NH | O | 2-Cl, 4-Cl | B 1 |
| $CH_3$ | $CH_3$ | NH | O | 2-Cl, 4-$CH_3$ | B 1 |
| $CH_3$ | $CH_3$ | NH | O | 3-Cl, 4-$CH_3$ | B 1 |
| $CH_3$ | $CH_3$ | NH | O | 2-F, 4-Cl | B 1 |
| $CH_3$ | $CH_3$ | NCHO | O | 4-Cl | B 1 |
| $CH_3$ | $CH_3$ | $NCH_3$ | O | 4-Cl | B 1 |
| $CH_3$ | $CH_3$ | NH | O | 4-Cl | B 2 |
| $CH_3$ | $CH_3$ | NH | O | 2-Cl, 4-Cl | B 2 |
| $CH_3$ | $CH_3$ | NH | S | 4-F | B 2 |
| $CH_3$ | $CH_3$ | NH | S | 4-Cl | B 2 |
| $CH_3$ | $CH_3$ | NH | S | 4-$CH_3$ | B 2 |
| $CH_3$ | $CH_3$ | NH | S | 2-Cl, 4-Cl | B 2 |
| $CH_3$ | $CH_3$ | NH | S | 3-Cl, 4-Cl | B 2 |
| $CH_3$ | $CH_3$ | NH | S | 2-Cl, 4-$CH_3$ | B 2 |
| $CH_3$ | $CH_3$ | NH | S | 3-Cl, 4-$CH_3$ | B 2 |
| $CH_3$ | $CH_3$ | NH | S | 2-F, 4-F | B 2 |
| $CH_3$ | $CH_3$ | NH | S | 2-F, 4-Cl | B 2 |
| $CH_3$ | $CH_3$ | NH | S | 2-F, 4-Br | B 2 |
| $CH_3$ | $CH_3$ | NH | S | 2-F, 4-$CH_3$ | B 2 |
| $CH_3$ | $CH_3$ | NH | S | 3-F, 4-$CH_3$ | B 2 |

| $R^1$ | $R^2$ | X | Y | $W_n$ | B |
|---|---|---|---|---|---|
| $CH_3$ | $CH_3$ | NCHO | S | 4-Cl | B 2 |
| $CH_3$ | $CH_3$ | NCHO | S | 2-Cl, 4-Cl | B 2 |
| $CH_3$ | $CH_3$ | NCHO | S | 3-Cl, 4-$CH_3$ | B 2 |
| $CH_3$ | $CH_3$ | NH | S | 4-Cl | B 3 |
| $CH_3$ | $CH_3$ | NH | S | 4-Br | B 3 |
| $CH_3$ | $CH_3$ | NH | S | 2-Cl, 4-Cl | B 3 |
| $CH_3$ | $CH_3$ | NH | S | 2-Cl, 4-$CH_3$ | B 3 |
| $CH_3$ | $CH_3$ | NH | S | 3-Cl, 4-$CH_3$ | B 3 |
| $CH_3$ | $CH_3$ | NH | S | 4-Cl | B 4 |
| $CH_3$ | $CH_3$ | NH | S | 2-Cl, 4-Cl | B 4 |
| $CH_3$ | $CH_3$ | NH | S | 4-Cl | B 5 |
| $CH_3$ | $CH_3$ | NH | S | 2-Cl, 4-Cl | B 5 |
| $CH_3$ | $CH_3$ | NH | S | 4-Cl | B 6 |
| $CH_3$ | $CH_3$ | NH | S | 2-Cl, 4-Cl | B 6 |
| $CH_3$ | $CH_3$ | NH | S | 4-Cl | B 7 |
| $CH_3$ | $CH_3$ | NH | S | 2-Cl, 4-$CH_3$ | B 7 |
| $CH_3$ | $CH_3$ | NH | S | 4-Cl | B 8 |
| $CH_3$ | $CH_3$ | NH | S | 2-Cl, 4-Cl | B 8 |
| $CH_3$ | $CH_3$ | NH | S | 4-Cl | B 9 |
| $CH_3$ | $CH_3$ | NH | S | 2-Cl, 4-$CH_3$ | B 9 |

| $R^1$ | $R^2$ | X | Y | $W_n$ | B |
|---|---|---|---|---|---|
| $CH_3$ | $CH_3$ | NH | S | 4-Cl | B10 |
| $CH_3$ | $CH_3$ | NH | S | 2-Cl, 4-Cl | B10 |
| $CH_3$ | $CH_3$ | NH | S | 3-Cl, 4-Cl | B10 |
| $CH_3$ | $CH_3$ | NH | S | 2-F, 4-$CH_3$ | B10 |
| $CH_3$ | $CH_3$ | NH | S | 3-F, 4-$CH_3$ | B10 |
| $CH_3$ | $CH_3$ | NH | S | 2-F, 4-Cl | B10 |
| $CH_3$ | $CH_3$ | NH | S | 4-Cl | B11 |
| $CH_3$ | $CH_3$ | NH | S | 2-Cl, 4-$CH_3$ | B11 |
| $CH_3$ | $CH_3$ | NH | S | 4-Cl | B12 |
| $CH_3$ | $CH_3$ | NH | S | 2-Cl, 4-Cl | B12 |
| $CH_3$ | $CH_3$ | NH | S | 2-F, 4-$CH_3$ | B12 |
| $CH_3$ | $CH_3$ | NH | S | 3-F, 4-$CH_3$ | B12 |
| $CH_3$ | $CH_3$ | NH | S | 2-Cl, 4-$CH_3$ | B12 |
| $CH_3$ | $CH_3$ | NH | S | 3-Cl, 4-$CH_3$ | B12 |
| $CH_3$ | $CH_3$ | NCHO | S | 4-Cl | B12 |
| $CH_3$ | $CH_3$ | NCHO | S | 2-Cl, 4-Cl | B12 |
| $CH_3$ | $CH_3$ | NH | S | 4-Cl | B13 |
| $CH_3$ | $CH_3$ | NH | S | 2-Cl, 4-Cl | B13 |
| $CH_3$ | $CH_3$ | NH | S | 2-Cl, 4-$CH_3$ | B13 |

| $R^1$ | $R^2$ | X | Y | $W_n$ | B |
|---|---|---|---|---|---|
| $CH_3$ | $CH_3$ | NH | S | 2-F, 4-$CH_3$ | B 1 3 |
| $CH_3$ | $CH_3$ | NH | S | 4-Cl | B 1 4 |
| $CH_3$ | $CH_3$ | NH | S | 2-Cl, 4-Cl | B 1 4 |
| $CH_3$ | $CH_3$ | NH | S | 2-Cl, 4-$CH_3$ | B 1 4 |
| $CH_3$ | $CH_3$ | NH | S | 2-F, 4-$CH_3$ | B 1 4 |
| $CH_3$ | $CH_3$ | NH | S | 4-Cl | B 1 5 |
| $CH_3$ | $CH_3$ | NH | S | 2-Cl, 4-Cl | B 1 5 |
| $CH_3$ | $CH_3$ | NH | S | 2-Cl, 4-$CH_3$ | B 1 5 |
| $CH_3$ | $CH_3$ | NH | S | 2-F, 4-$CH_3$ | B 1 5 |
| $CH_3$ | $CH_3$ | NH | S | 4-Cl | B 1 6 |
| $CH_3$ | $CH_3$ | NH | S | 2-Cl, 4-Cl | B 1 6 |
| $CH_3$ | $CH_3$ | NH | S | 2-Cl, 4-$CH_3$ | B 1 6 |
| $CH_3$ | $CH_3$ | NH | S | 2-F, 4-$CH_3$ | B 1 6 |
| $CH_3$ | $CH_3$ | NH | S | 4-Cl | B 1 7 |
| $CH_3$ | $CH_3$ | NH | S | 2-Cl, 4-Cl | B 1 7 |
| $CH_3$ | $CH_3$ | NH | S | 2-Cl, 4-$CH_3$ | B 1 7 |
| $CH_3$ | $CH_3$ | NH | S | 2-F, 4-$CH_3$ | B 1 7 |
| $CH_3$ | $CH_3$ | NH | S | 4-Cl | B 1 8 |
| $CH_3$ | $CH_3$ | NH | S | 2-Cl, 4-Cl | B 1 8 |
| $CH_3$ | $CH_3$ | NH | S | 2-Cl, 4-$CH_3$ | B 1 8 |

| $R^1$ | $R^2$ | X | Y | $W_n$ | B |
|---|---|---|---|---|---|
| $CH_3$ | $CH_3$ | NH | S | 2-F, 4-$CH_3$ | B18 |
| $CH_3$ | $CH_3$ | NCHO | S | 2-Cl, 4-Cl | B1 |
| $CH_3$ | $CH_3$ | NCHO | S | 3-Cl, 4-Cl | B1 |
| $CH_3$ | $CH_3$ | NCHO | S | 2-F, 4-Cl | B1 |
| $CH_3$ | $CH_3$ | NCHO | S | 2-F, 4-$CH_3$ | B1 |
| $CH_3$ | $CH_3$ | NCHO | S | 3-F, 4-Cl | B1 |
| $CH_3$ | $CH_3$ | NCHO | S | 3-F, 4-$CH_3$ | B1 |
| $CH_3$ | $CH_3$ | NCHO | S | 2-Cl, 4-$CH_3$ | B1 |
| $CH_3$ | $CH_3$ | NCHO | S | 3-Cl, 4-$CH_3$ | B1 |
| $CH_3$ | $CH_3$ | NCHO | S | 2-Cl, 3-Cl, 4-Cl | B1 |
| $CH_3$ | $CH_3$ | NCHO | S | 2-Cl, 4-Cl, 5-Cl | B1 |
| $CH_3$ | $CH_3$ | $NCOCH_3$ | S | 2-Cl, 4-Cl | B1 |
| $CH_3$ | $CH_3$ | $NCOCH_3$ | S | 3-Cl, 4-Cl | B1 |
| $CH_3$ | $CH_3$ | $NCOCH_3$ | S | 2-F, 4-$CH_3$ | B1 |
| $CH_3$ | $CH_3$ | $NCOCH_3$ | S | 2-Cl, 3-Cl, 4-Cl | B1 |
| $CH_3$ | $CH_3$ | $NCH_3$ | S | 2-Cl, 4-Cl | B1 |
| $CH_3$ | $CH_3$ | $NCH_3$ | S | 3-Cl, 4-$CH_3$ | B1 |
| $CH_3$ | $CH_3$ | NNO | S | 2-Cl, 4-Cl | B1 |
| $CH_3$ | $CH_3$ | NNO | S | 3-Cl, 4-$CH_3$ | B1 |
| $CH_3$ | $CH_3$ | $NNH_2$ | S | 4-Cl | B1 |

| $R^1$ | $R^2$ | X | Y | $W_n$ | B |
|---|---|---|---|---|---|
| $CH_3$ | $CH_3$ | $NNH_2$ | S | 2-Cl, 4-Cl | B 1 |
| $CH_3$ | $CH_3$ | $NNH_2$ | S | 3-Cl, 4-$CH_3$ | B 1 |
| $CH_3$ | $CH_3$ | $NSO_2CH_3$ | S | 2-Cl, 4-Cl | B 1 |
| $CH_3$ | $CH_3$ | $NSO_2CH_3$ | S | 2-F, 4-$CH_3$ | B 1 |
| $CH_3$ | $CH_3$ | $NSO_2CH_3$ | S | 2-Cl, 3-Cl, 4-Cl | B 1 |
| $CH_3$ | $CH_3$ | $CH_2$ | S | 4-Cl | B 1 |
| $CH_3$ | $CH_3$ | $CH_2$ | S | 2-Cl, 4-Cl | B 1 |
| $CH_3$ | $CH_3$ | $CH_2$ | S | 2-Cl, 4-$CH_3$ | B 1 |
| $CH_3$ | $CH_3$ | $CH_2$ | S | 2-F, 4-$CH_3$ | B 1 |
| $CH_3$ | $CH_3$ | CH(OH) | S | 4-Cl | B 1 |
| $CH_3$ | $CH_3$ | CH(OH) | S | 2-Cl, 4-Cl | B 1 |
| $CH_3$ | $CH_3$ | CH(OH) | S | 2-Cl, 4-$CH_3$ | B 1 |
| $CH_3$ | $CH_3$ | CH(OH) | S | 3-Cl, 4-$CH_3$ | B 1 |
| $CH_3$ | $CH_3$ | CH(OH) | S | 2-F, 4-$CH_3$ | B 1 |
| $CH_3$ | $CH_3$ | CH(OH) | S | 3-F, 4-$CH_3$ | B 1 |
| $CH_3$ | $CH_3$ | $CH(OCH_3)$ | S | 4-Cl | B 1 |
| $CH_3$ | $CH_3$ | $CH(OCH_3)$ | S | 2-Cl, 4-Cl | B 1 |
| $CH_3$ | $CH_3$ | $CH(OCOCH_3)$ | S | 4-Cl | B 1 |
| $CH_3$ | $CH_3$ | $CH(OCOCH_3)$ | S | 2-Cl, 4-Cl | B 1 |
| $CH_3$ | $CH_3$ | $CH(OCOCH_3)$ | S | 2-Cl, 4-$CH_3$ | B 1 |

| $R^1$ | $R^2$ | X | Y | $W_n$ | B |
|---|---|---|---|---|---|
| $CH_3$ | $CH_3$ | $CH(OCOCH_3)$ | S | 2-F, 4-$CH_3$ | B 1 |
| $CH_3$ | $CH_3$ | CH(F) | S | 4-Cl | B 1 |
| $CH_3$ | $CH_3$ | CH(F) | S | 2-Cl, 4-Cl | B 1 |
| $CH_3$ | $CH_3$ | CH(Cl) | S | 4-Cl | B 1 |
| $CH_3$ | $CH_3$ | CH(Cl) | S | 2-Cl, 4-Cl | B 1 |
| $CH_3$ | $CH_3$ | C=O | S | 4-Cl | B 1 |
| $CH_3$ | $CH_3$ | C=O | S | 2-Cl, 4-Cl | B 1 |
| $CH_3$ | $CH_3$ | C=O | S | 2-Cl, 4-$CH_3$ | B 1 |
| $CH_3$ | $CH_3$ | C=O | S | 2-F, 4-$CH_3$ | B 1 |
| $CH_3$ | $CH_3$ | $CH_2$ | S | 4-Cl | B 2 |
| $CH_3$ | $CH_3$ | $CH_2$ | S | 2-Cl, 4-Cl | B 2 |
| $CH_3$ | $CH_3$ | $CH_2$ | S | 2-Cl, 4-$CH_3$ | B 2 |
| $CH_3$ | $CH_3$ | $CH_2$ | S | 2-F, 4-$CH_3$ | B 2 |
| $CH_3$ | $CH_3$ | CH(OH) | S | 4-Cl | B 2 |
| $CH_3$ | $CH_3$ | CH(OH) | S | 2-Cl, 4-Cl | B 2 |
| $CH_3$ | $CH_3$ | CH(OH) | S | 2-Cl, 4-$CH_3$ | B 2 |
| $CH_3$ | $CH_3$ | CH(OH) | S | 3-Cl, 4-$CH_3$ | B 2 |
| $CH_3$ | $CH_3$ | CH(OH) | S | 2-F, 4-$CH_3$ | B 2 |
| $CH_3$ | $CH_3$ | CH(OH) | S | 3-F, 4-$CH_3$ | B 2 |
| $CH_3$ | $CH_3$ | $CH(OCH_3)$ | S | 4-Cl | B 2 |

| $R^1$ | $R^2$ | X | Y | $W_n$ | B |
|---|---|---|---|---|---|
| $CH_3$ | $CH_3$ | $CH(OCH_3)$ | S | 2-Cl, 4-Cl | B 2 |
| $CH_3$ | $CH_3$ | $CH(OCOCH_3)$ | S | 4-Cl | B 2 |
| $CH_3$ | $CH_3$ | $CH(OCOCH_3)$ | S | 2-Cl, 4-Cl | B 2 |
| $CH_3$ | $CH_3$ | $CH(OCOCH_3)$ | S | 2-Cl, 4-$CH_3$ | B 2 |
| $CH_3$ | $CH_3$ | $CH(OCOCH_3)$ | S | 2-F, 4-$CH_3$ | B 2 |
| $CH_3$ | $CH_3$ | CH(F) | S | 4-Cl | B 2 |
| $CH_3$ | $CH_3$ | CH(F) | S | 2-Cl, 4-Cl | B 2 |
| $CH_3$ | $CH_3$ | CH(Cl) | S | 4-Cl | B 2 |
| $CH_3$ | $CH_3$ | CH(Cl) | S | 2-Cl, 4-Cl | B 2 |
| $CH_3$ | $CH_3$ | C=O | S | 4-Cl | B 2 |
| $CH_3$ | $CH_3$ | C=O | S | 2-Cl, 4-Cl | B 2 |
| $CH_3$ | $CH_3$ | C=O | S | 2-Cl, 4-$CH_3$ | B 2 |
| $CH_3$ | $CH_3$ | C=O | S | 2-F, 4-$CH_3$ | B 2 |
| $CH_3$ | $CH_3$ | $C(CH_3)(OH)$ | S | 4-Cl | B 2 |
| $CH_3$ | $CH_3$ | $C(CH_3)(OH)$ | S | 2-Cl, 4-Cl | B 2 |
| $CH_3$ | $CH_3$ | $C(CH_3)(OCOCH_3)$ | S | 4-Cl | B 2 |
| $CH_3$ | $CH_3$ | $C(CH_3)(OCOCH_3)$ | S | 2-Cl, 4-Cl | B 2 |
| $CH_3$ | $CH_3$ | $C(CH_3)(F)$ | S | 4-Cl | B 2 |
| $CH_3$ | $CH_3$ | $C(CH_3)(F)$ | S | 2-Cl, 4-Cl | B 2 |
| $CH_3$ | $CH_3$ | $C(C_2H_5)(OH)$ | S | 4-Cl | B 2 |

| $R^1$ | $R^2$ | X | Y | $W_n$ | B |
|---|---|---|---|---|---|
| $CH_3$ | $CH_3$ | $C(C_2H_5)(OH)$ | S | 2-Cl, 4-Cl | B 2 |
| $CH_3$ | $CH_3$ | $C(i-C_3H_7)(OH)$ | S | 4-Cl | B 2 |
| $CH_3$ | $CH_3$ | $C(i-C_3H_7)(OH)$ | S | 2-Cl, 4-Cl | B 2 |
| $CH_3$ | $CH_3$ | $C(i-C_3H_7)(OH)$ | S | 2-Cl, 4-$CH_3$ | B 2 |
| $CH_3$ | $CH_3$ | $C(i-C_3H_7)(OH)$ | S | 2-F, 4-$CH_3$ | B 2 |
| $CH_3$ | $CH_3$ | $CH(CH_3)$ | S | 4-Cl | B 2 |
| $CH_3$ | $CH_3$ | $CH(CH_3)$ | S | 2-Cl, 4-Cl | B 2 |
| $CH_3$ | $CH_3$ | $CH(CH_3)$ | S | 2-Cl, 4-$CH_3$ | B 2 |
| $CH_3$ | $CH_3$ | $CH(CH_3)$ | S | 2-F, 4-$CH_3$ | B 2 |
| Cl | $CH_3$ | $CH_2$ | S | 4-Cl | B 2 |
| Cl | $CH_3$ | $CH_2$ | S | 2-Cl, 4-Cl | B 2 |
| Cl | $CH_3$ | $CH_2$ | S | 2-Cl, 4-$CH_3$ | B 2 |
| Cl | $CH_3$ | $CH_2$ | S | 2-F, 4-$CH_3$ | B 2 |
| $CH_3O$ | $CH_3$ | $CH_2$ | S | 4-Cl | B 2 |
| $CH_3O$ | $CH_3$ | $CH_2$ | S | 2-Cl, 4-Cl | B 2 |
| $CH_3O$ | $CH_3$ | $CH_2$ | S | 2-Cl, 4-$CH_3$ | B 2 |
| $CH_3O$ | $CH_3$ | $CH_2$ | S | 2-F, 4-$CH_3$ | B 2 |

où B1 à B18 sont comme définis ci-après :

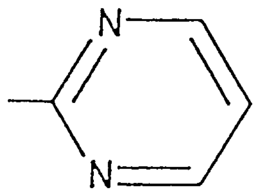

B1

,

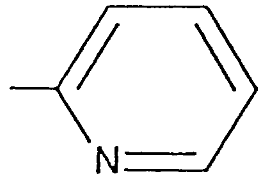

B2

,

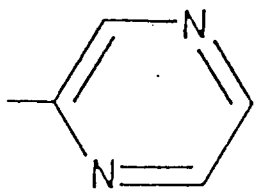

B3

,

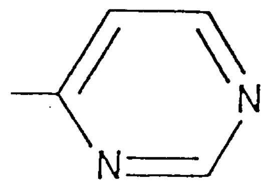

B4

,

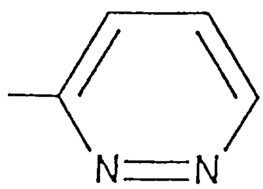

B5

,

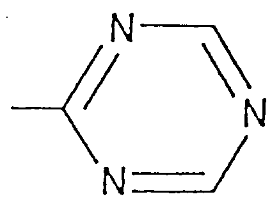

B6

,

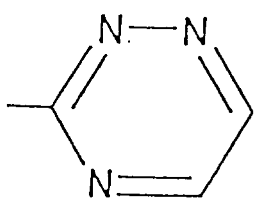

B7

,

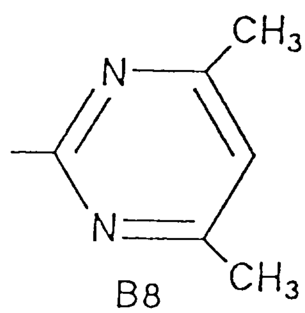

B8

,

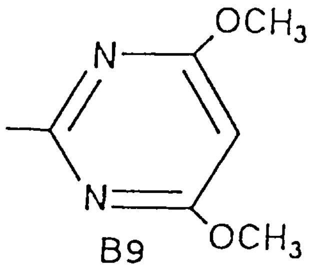
B9 ,

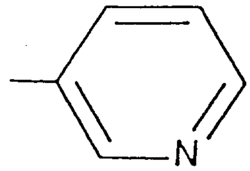
B10 ,

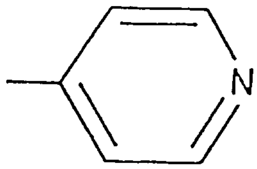
B11 ,

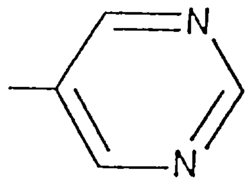
B12

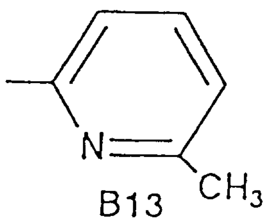
B13 ,

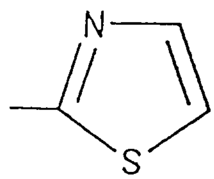
B14 ,

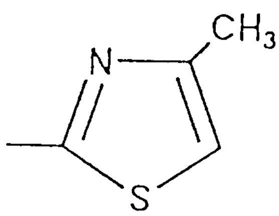
B15 ,

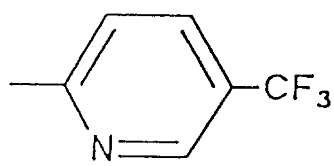
B16 ,

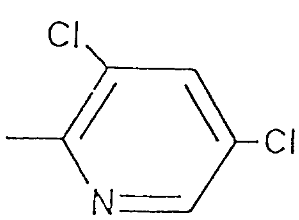
B17 ,

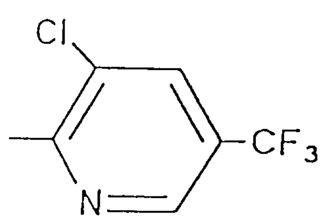
B18 ,

ou bien où les dérivés du pyrazole substitué sont définis par la formule [1b]

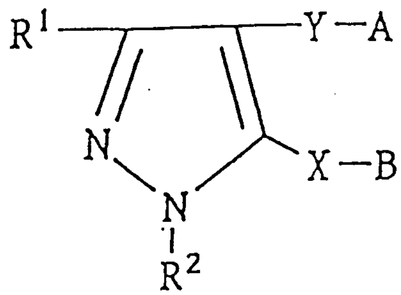

[1b]

ayant les définitions suivantes des substituants

| $R^1$ | $R^2$ | X | Y | A | B |
|---|---|---|---|---|---|
| $CH_3$ | $CH_3$ | NH | S | A 1 | B 1 |
| $CH_3$ | $CH_3$ | NH | S | A 1 | B 2 |
| $CH_3$ | $CH_3$ | NH | S | A 2 | B 1 |
| $CH_3$ | $CH_3$ | NH | S | A 2 | B 2 |
| $CH_3$ | $CH_3$ | NH | S | A 3 | B 1 |
| $CH_3$ | $CH_3$ | NH | S | A 3 | B 2 |
| $CH_3$ | $CH_3$ | NH | S | A 4 | B 1 |
| $CH_3$ | $CH_3$ | NH | S | A 4 | B 2 |
| $CH_3$ | $CH_3$ | NH | S | A 5 | B 1 |
| $CH_3$ | $CH_3$ | NH | S | A 5 | B 2 |

| $R^1$ | $R^2$ | X | Y | A | B |
|---|---|---|---|---|---|
| $CH_3$ | $CH_3$ | NH | S | A 6 | B 1 |
| $CH_3$ | $CH_3$ | NH | S | A 6 | B 2 |
| $CH_3$ | $CH_3$ | NH | S | A 7 | B 1 |
| $CH_3$ | $CH_3$ | NH | S | A 7 | B 2 |
| $CH_3$ | $CH_3$ | NH | S | A 8 | B 1 |
| $CH_3$ | $CH_3$ | NH | S | A 8 | B 2 |
| $CH_3$ | $CH_3$ | NH | S | A 9 | B 1 |
| $CH_3$ | $CH_3$ | NH | S | A 9 | B 2 |
| $CH_3$ | $CH_3$ | NH | S | A 10 | B 1 |
| $CH_3$ | $CH_3$ | NH | S | A 10 | B 2 |
| $CH_3$ | $CH_3$ | NH | S | A 11 | B 1 |
| $CH_3$ | $CH_3$ | NH | S | A 11 | B 2 |

où A1 à A11 sont comme définis ci-après

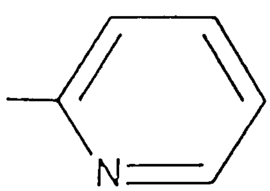
A1 ,

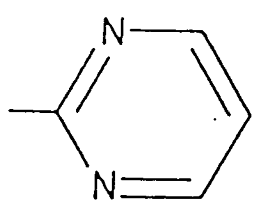
A2 ,

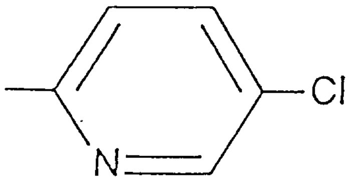

A3 ,

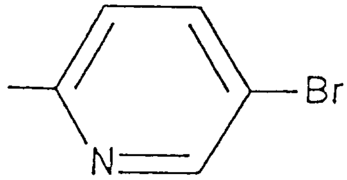

A4 ,

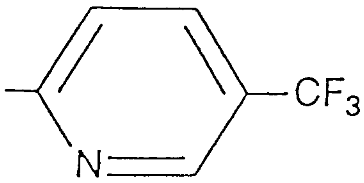

A5 ,

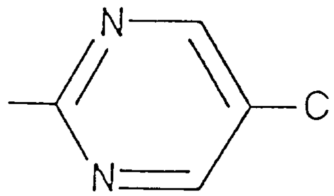

A6 ,

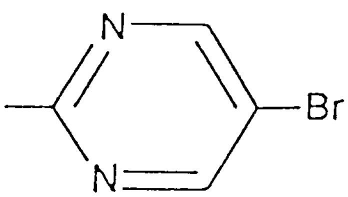

A7 ,

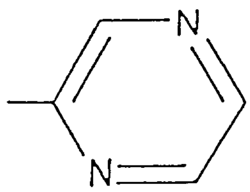

A8 ,

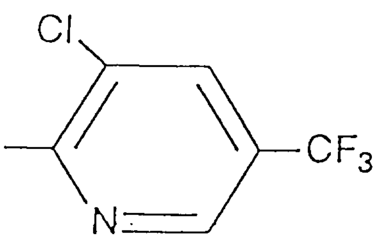

A9 ,

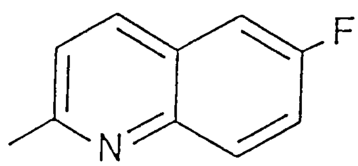

A10 ,

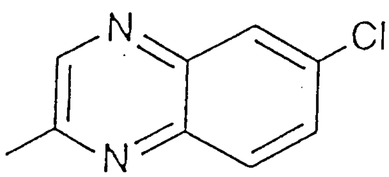

A11 ,

et B1 et B2 sont comme définis précédemment, conformément aux schémas réactionnels suivants :

(Méthode 1)

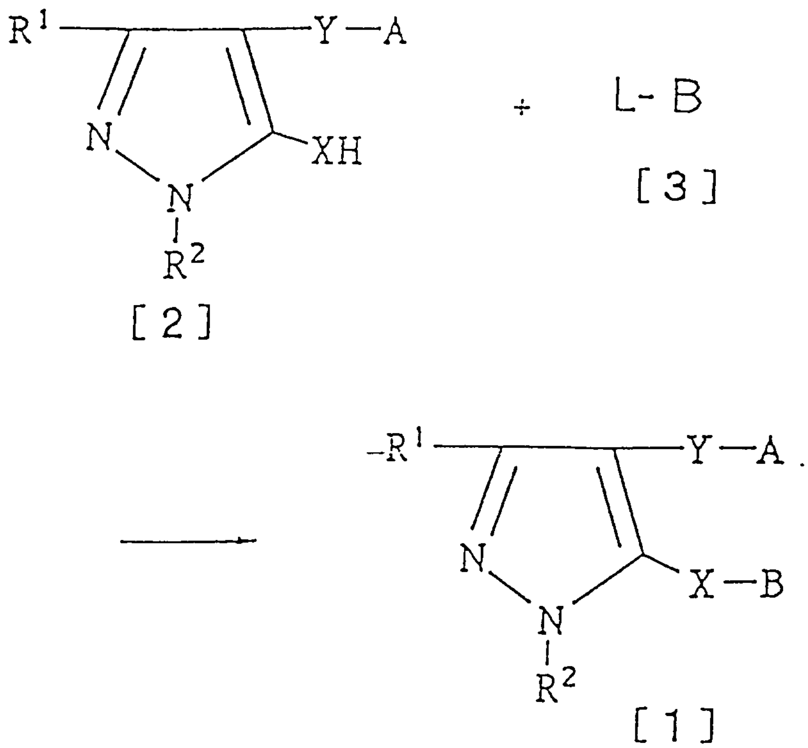

[2] [3] [1]

dans lesquels $R^1$, $R^2$, X, Y et A ont les significations mentionnées ci-dessus, L représente un groupe séparable et B a la même signification que celle mentionnée précédemment,

(Méthode 2)

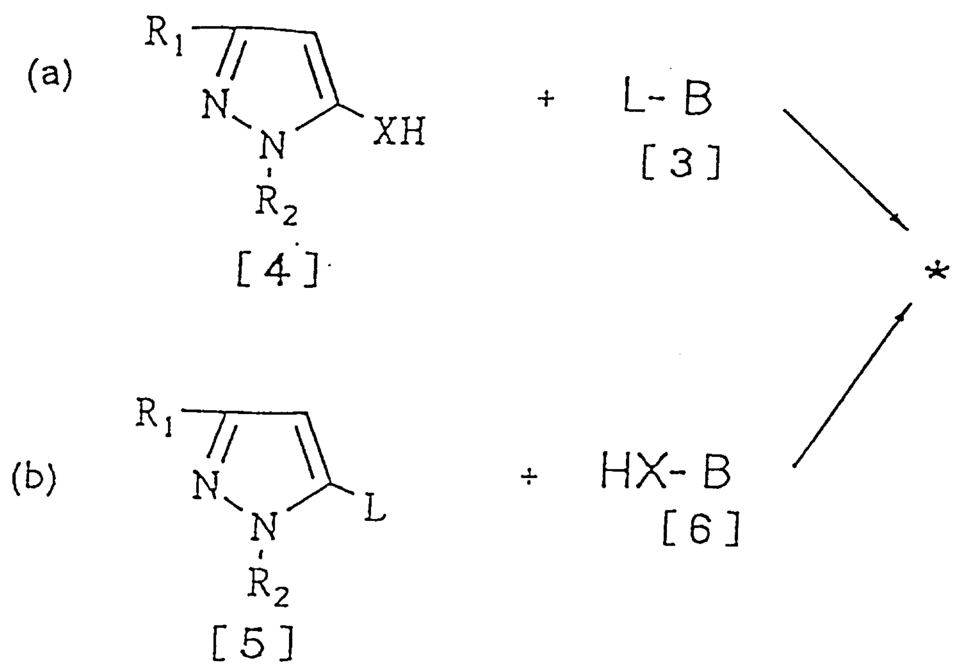

(a) [4] [3]

(b) [5] [6]

lorsque Y ≠ 0 :

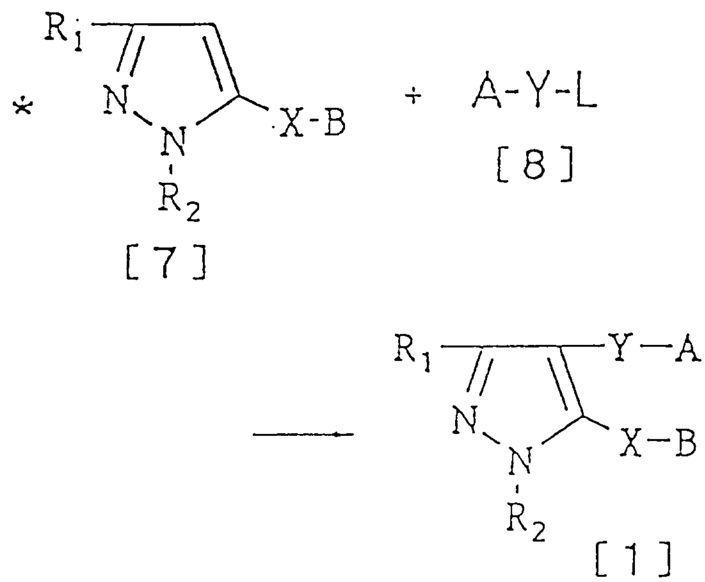

[7] [8]

[1]

dans lesquels $R^1$, $R^2$, B, X et A ont les mêmes significations que mentionné précédemment et L représente un groupe séparable, et Y a la même signification que celle mentionnée précédemment, à l'exception de l'atome d'oxygène,

(Méthode 3)

lorsque X = N–$R^3$, $R^3 \neq$ H :

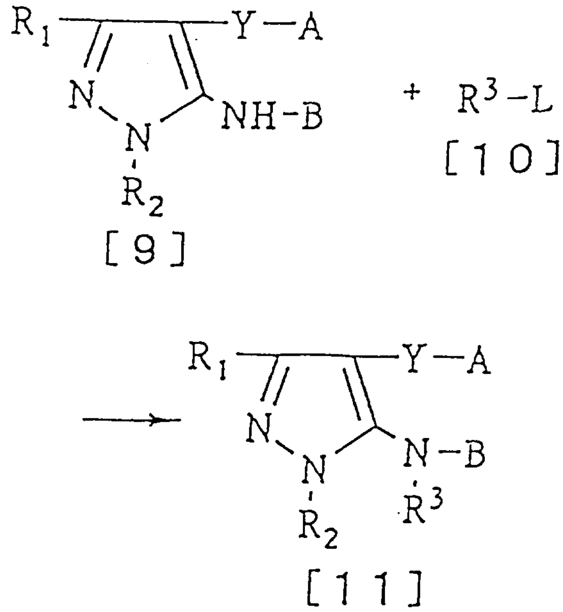


[9] [10]

[11]

dans lesquelles $R^1$, $R^2$, A, B et Y ont les mêmes significations que mentionné précédemment, L représente un groupe séparable et $R^3$ a la même signification que celle mentionnée précédemment, à l'exception de l'atome d'hydrogène.

**2.** Une méthode de préparation des dérivés du pyrazole substitués selon la revendication 1, selon laquelle lesdits dérivés du pyrazole substitués sont des composés de formule [1a].

**3.** Une méthode de préparation des dérivés du pyrazole substitués selon la revendication 1, selon laquelle X est -N($R^3$)-.

**4.** Une méthode de préparation des dérivés du pyrazole substitués selon la revendication 2, selon laquelle Y est -S-.

**5.** Une méthode de préparation des dérivés du pyrazole substitués selon la revendication 1, qui sont des composés de formule générale [1a], dans laquelle $R^1$ et $R^2$ représentent chacun un groupe alkyle, X est -N($R^3$)-, Y est -S- et B est B1, B2, B4, B10, B11 ou B12.

**6.** Un fongicide à utiliser en agriculture et en horticulture, contenant un ou plusieurs pyrazoles substitués selon la revendication 1, comme ingrédient actif.

**7.** Une méthode de préparation d'un fongicide à utiliser en agriculture et en horticulture par préparation d'un fongicide contenant un ou plusieurs pyrazoles substitués tels que définis dans la revendication 1 comme ingrédient actif.